(19) Europäisches Patentamt
European Patent Office
Office européen des brevets

(11) EP 4 524 136 A1

(12) EUROPEAN PATENT APPLICATION
published in accordance with Art. 153(4) EPC

(43) Date of publication:
**19.03.2025 Bulletin 2025/12**

(21) Application number: **23803001.9**

(22) Date of filing: **11.05.2023**

(51) International Patent Classification (IPC):
***C07D 401/14*** (2006.01) ***C07D 405/14*** (2006.01)
***C07D 413/14*** (2006.01) ***A61K 31/497*** (2006.01)
***A61K 31/506*** (2006.01) ***A61P 35/00*** (2006.01)

(52) Cooperative Patent Classification (CPC):
**A61K 31/38; A61K 31/41; A61K 31/438; A61K 31/455; A61K 31/495; A61K 31/497; A61K 31/506; A61K 31/63; A61P 35/00; C07D 401/04; C07D 401/12; C07D 401/14; C07D 405/04; C07D 405/14; C07D 413/14;** (Cont.)

(86) International application number:
**PCT/CN2023/093538**

(87) International publication number:
**WO 2023/217232 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**13.05.2022 CN 202210520744**
**25.07.2022 CN 202210878192**
**27.09.2022 CN 202211183092**
**02.12.2022 CN 202211537992**
**16.01.2023 CN 202310071265**
**22.03.2023 CN 202310288871**
**27.04.2023 CN 202310475180**

(71) Applicant: **Shanghai Apeiron Therapeutics Company Limited**
**Shanghai 201210 (CN)**

(72) Inventors:
- **XIAO, Yisong**
**Shanghai 201210 (CN)**
- **GU, Xiaohui**
**Shanghai 201210 (CN)**
- **LAI, Kunmin**
**Shanghai 201210 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) KINESIN KIF18A INHIBITOR AND USE THEREOF

(57) The present invention discloses a kinesin KIF18A inhibitor that regulates the KIF18A protein, influencing cell cycle and proliferation processes for the treatment of cancers and cancer-related diseases, along with its applications. The present invention also encompasses pharmaceutical compositions containing the compounds and methods for treating conditions associated with KIF18A activity.

EP 4 524 136 A1

(52) Cooperative Patent Classification (CPC): (Cont.)
**C07D 471/04; C07D 487/04; C07D 491/04;
C07D 491/048; C07D 498/04; C07D 513/04;
C07D 519/00**

## Description

This application claims priority from the following applications:

1) A Chinese patent application submitted to China National Intellectual Property Administration on May 13, 2022, with the application number 202210520744.0 and the invention name "Kinesin KIF18A Inhibitor and Use Thereof";
2) A Chinese patent application submitted to China National Intellectual Property Administration on July 25, 2022, with the application number 202210878192.0 and the invention name "Kinesin KIF18A Inhibitor and Use Thereof";
3) A Chinese patent application submitted to China National Intellectual Property Administration on September 27, 2022, with the application number 202211183092.2 and the invention name "Kinesin KIF18A Inhibitor and Use Thereof";
4) A Chinese patent application submitted to China National Intellectual Property Administration on December 02, 2022, with the application number 202211537992.2 and the invention name "Kinesin KIF18A Inhibitor and Use Thereof";
5) A Chinese patent application submitted to China National Intellectual Property Administration on January 16, 2023, with the application number 202310071265.X and the invention name "Kinesin KIF18A Inhibitor and Use Thereof";
6) A Chinese patent application submitted to China National Intellectual Property Administration on March 22, 2023, with the application number 202310288871.7 and the invention name "Kinesin KIF18A Inhibitor and Use Thereof";
7) A Chinese patent application submitted to China National Intellectual Property Administration on April 27, 2023, with the application number 202310475180.8 and the invention name "Kinesin KIF18A Inhibitor and Use Thereof";

The entire contents of which are incorporated herein by reference.

## TECHNICAL FIELD

The present invention belongs to the field of medicine and relates to a class of kinesin KIF18A inhibitors and their use for inhibiting cancer cell proliferation and treating cancers.

## BACKGROUND ART

KIF18A is a member of the kinesin-8 family. It moves towards the plus ends of microtubules within cells using microtubules as tracks, relying on the energy released from ATP hydrolysis. Upon reaching the plus ends of microtubules, KIF18A regulates the dynamic instability of microtubules and exerts an activity similar to that of microtubule depolymerase. During mitosis, KIF18A regulates spindle microtubule dynamics and chromosome amplitude, playing a critical role in the timely completion of chromosome alignment at mitosis, maintenance of genomic stability and successful completion of mitosis.

The KIF18A gene belongs to the kinesin-8 subfamily and is a plus-end directed motor.

KIF18A is believed to influence the dynamics of the plus ends of centromeric microtubules, thereby regulating correct chromosome orientation and spindle tension. Depletion of human KIF18A results in longer spindles, increased chromosome oscillation at metaphase and activated mitotic spindle assembly checkpoint in HeLa cervical cancer cells (MI Mayr et al., Current Biology 17, 488-98, 2007). KIF18A appears to be a viable target for cancer therapy. KIF18A is overexpressed in multiple types of cancers, including but not limited to colon, breast, lung, pancreatic, prostatic, bladder, head, neck, cervical and ovarian cancers. Furthermore, in cancer cell lines, gene deletion or knockout or KIF18A inhibition affects the mitotic spindle apparatus. In particular, inhibition of KIF18A has been found to induce mitotic cell arrest, a vulnerability known to promote mitotic cell death via apoptosis, mitotic catastrophe or death following polyphasic-driven lethality or interphase mitotic slippage. As a result, there is a strong interest in finding inhibitors of KIF 18A protein. Therefore, inhibition of KIF18A ATPase activity is a promising approach to developing new anticancer agents.

## CONTENT OF THE INVENTION

The present invention belongs to the field of medicine and relates to a class of kinesin KIF18A inhibitors, specifically to the said compounds or their stereoisomers, tautomers, mesomers, racemates, enantiomers, diastereoisomers or their mixture forms or pharmaceutically acceptable salts, co-crystals, metabolites, solvates, prodrugs or isotopic labels, their preparation methods and pharmaceutical compositions containing such compounds and their use as therapeutic agents, especially the use to inhibit cancer cell proliferation and treat cancers.

The present invention provides a novel class of compounds that regulate KIF18A protein, alone or in microtubule-bound complexes, for the treatment of KIF18A-mediated disorders and/or diseases, including cancer, inflammation or ciliopathy.

The compounds of the present invention exhibit MT-based regulatory activity on KIF18A, specifically inhibitory activity on KIF18A. To this end, the present invention also provides the use of these compounds and their pharmaceutically acceptable salts in the preparation and manufacture of pharmaceutical compositions or medicines for therapeutic, prophylactic, acute or chronic treatment of KIF18A-mediated diseases and disorders (including but not limited to cancers).

The present invention provides an Example: a compound having the structure of formula (I), or its pharmaceutically acceptable salts, stereoisomers, isotope isomers, prodrugs, hydrates, or solvates:

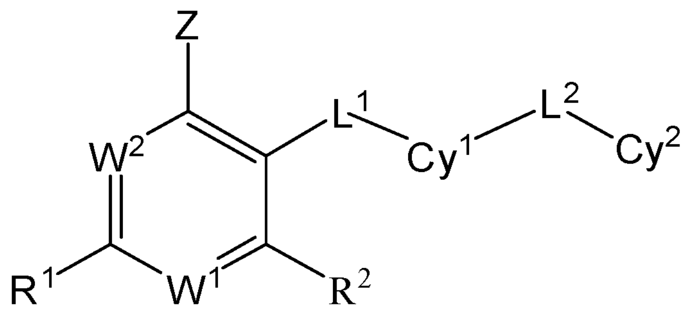

Formula (I)

wherein, $W^1$ represents $CR^{W1}$ or N;

wherein, $W^2$ represents $CR^{W2}$ or N;

wherein, Z represents $-CR^TR^{T'}$ or $-NR^SR^{S'}$;

wherein, $R^T$ and $R^{T'}$, together with the C atom they are attached to, form a ring having a structure selected from the following:

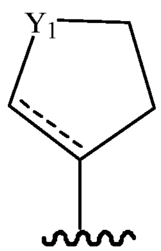

,

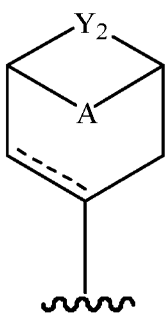

and/or

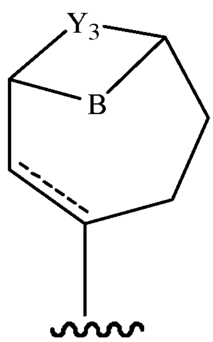

;

wherein, $R^S$ and $R^{S'}$, together with the N atom they are attached to, form a ring having a structure selected from the following:

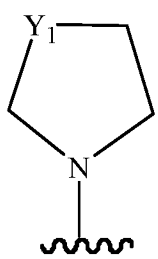

,

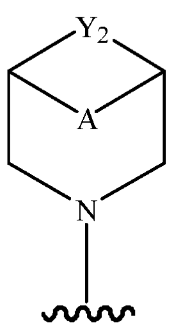

and/or

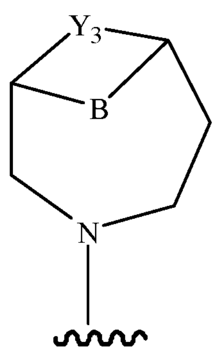

wherein, $Y^1$, $Y^2$ and $Y^3$ each independently represent $-(CR^aR^b)_o-(NR^a)_p-(CR^{a'}R^{b'})_q-$;

wherein, A and B each independently represent $-(CR^aR^b)m-$;

wherein, $L^1$ represents 5-6-membered heteroaryl,

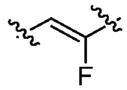, 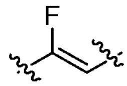, 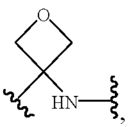,

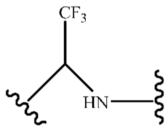, 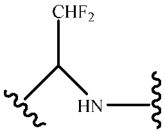 and 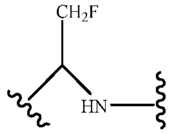;

wherein, $L^2$ represents absence, $-C_1-C_6$ alkylene-, $-NR^a-$, $-NR^a(C_1-C_6$ alkylene)-, $-C(O)NR^a(C_1-C_6$ alkylene)-, -O-, $-O-(C_1-C_6$ alkylene), -S-, -S(O)-, $-S(O)_2-$, $-S(O)_2NR^a-$ or $-S(O)(NR^a)-$:

wherein, $R^1$ represents $L^3-R^3$ or

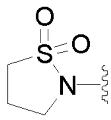;

wherein, $R^2$ represents hydrogen, halogen, cyano, nitro, hydroxy($C_1-C_6$ alkyl), $C_1-C_6$ alkyl, $C_3-C_8$ cycloalkyl, $C_1-C_6$ haloalkyl, $-OR^a$, $-SO_3R^a$, $-SR^a$, $-SF_5$, $-S(O)R^a$, -O-, $C_1-C_6$ haloalkyl or $-NR^aR^b$;

wherein, $L^3$ represents absence, $C_1-C_6$ alkylene, $-NR^a-$, $-NR^aSO_2-$, $-SO_2NR^a-$, $-S(=O)(NR^a)-$, $-P(O)(OR^a)_2$, $-NR^a-P(O)(OR^a)_2$ or $-NR^aP(O)(R^a)_2$;

wherein, $R^3$ represents absence, hydrogen, or $C_1-C_6$ alkyl or $C_3-C_6$ cycloalkyl,

wherein the $C_1-C_6$ alkyl or $C_3-C_6$ cycloalkyl can be optionally independently substituted with 0-3 substituents selected from halogen, $-OR^a$, $-NR^aR^b$, cyano and $-O-C_1-C_6$ haloalkyl;

wherein, $R^{W1}$ and $R^{W2}$ each independently represent hydrogen, halogen, cyano, nitro, hydroxy($C_1-C_6$ alkyl), $C_1-C_6$ alkyl, $C_3-C_8$ cycloalkyl, $C_1-C_6$ haloalkyl, $-OR^a$, $-SO_3R^a$, $-S(O)R^a$, -O-, $C_1-C_6$ haloalkyl, $-SR^a$, $-SF_5$ or $-NR^aR^b$;

wherein, $Cy^1$ represents 6-12-membered aryl or 5-12-membered heteroaryl; the $Cy^1$ may be optionally substituted with 0-3 substituents selected from the following: halogen, $C_1-C_6$ alkyl, $C_1-C_6$ haloalkyl, hydroxy($C_1-C_6$ alkyl), $OR^a$, $-O-(C_1-C_6$ haloalkyl), 5-6-membered heteroaryl, phenyl, $-SR^a$, $-SF_5$, cyano, nitro, $-NR^aR^b$, $-NH-(C_0-C_6$ alkyl)$-R^M$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$ and $-S(O)_2NR^aR^b$;

wherein, $Cy^2$ represents a 3-12-membered saturated or unsaturated monocyclic or bicyclic ring which may optionally contain 0-3 heteroatoms selected from O, N and S; the $Cy^2$ may be optionally substituted with 0-3 substituents selected from the following: halogen, $C_1-C_6$ haloalkyl, $C_1-C_6$ haloalkyl, hydroxy($C_1-C_6$ alkyl), $-OR^a$, $-O-(C_1-C_6$ haloalkyl), $-SR^a$, $-SF_5$, cyano, nitro, $-NR^aR^b$, $-NR^aC(O)R^b$, $-C(O)NR^aR^b$, $-OC(O)R^a$, $-C(O)OR^a$, $-S(O)R^a$, $-S(O)_2R^a$ and $-S(O)_2NR^aR^b$;

wherein, $R^M$ represents $-OR^a$, $-NR^aR^b$, 5-6-membered heterocyclyl, 5-10-membered heteroaryl and 6-10-membered aryl;

wherein, $R^a$, $R^b$, $R^{a'}$ and $R^{b'}$ each independently represent hydrogen, halogen, $C_1-C_6$ alkyl, $C_3-C_6$ cycloalkyl, $C_1-C_6$ haloalkyl or hydroxy($C_1-C_6$ alkyl); Or $R^a$ and $R^b$, together with the atom they are attached to, form a 3-6-membered saturated or unsaturated ring which may optionally contain 0-2 heteroatoms selected from O, S and N; Or $R^{a'}$ and $R^{b'}$, together with the atom they are attached to, form a 3-6-membered saturated or unsaturated ring which may optionally contain 0-2 heteroatoms selected from O, S and N;

wherein, the dashed line represents a single or double bond;

wherein, m, o, p and q independently represent integers from 0 to 3.

In a preferred Example of the present invention, $W^1$ represents CH or N.

In a preferred Example of the present invention, $W^2$ represents CH or N.

In a preferred Example of the present invention, $L^3$ represents $-NR^aSO_2-$, $-SO_2NR^a-$ or $-S(=O)(NR^a)-$.

In a preferred Example of the present invention, $L^3$ represents $-NR^aSO_2-$.

In a preferred Example of the present invention, $R^3$ represents hydrogen, or $C_1-C_6$ alkyl or $C_3-C_6$ cycloalkyl, wherein the $C_1-C_6$ alkyl or $C_3-C_6$ cycloalkyl can be optionally independently substituted with 0-3 substituents selected from halogen, $-OR^a$, $-NR^aR^b$, cyano and $-O-C_1-C_6$ haloalkyl.

In a preferred Example of the present invention, $R^3$ represents $C_1-C_6$ alkyl substituted with 0-3 substituents selected from halogen, $-OR^a$, $-NR^aR^b$, cyano and $-O-C_1-C_6$ haloalkyl.

In a preferred Example of the present invention, Z represents $NR^SR^{S'}$.

In a preferred Example of the present invention, Z represents:

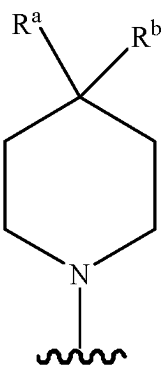 , 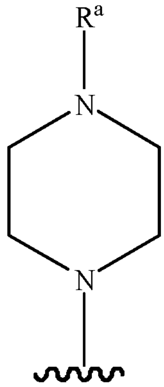 , 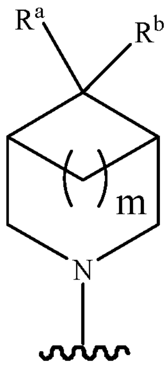 and/or 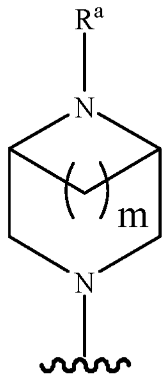 ;

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy($C_1$-$C_6$ alkyl); Or $R^a$ and $R^b$, together with the atom they are attached to, form a 3-6-membered saturated or unsaturated ring which may optionally contain 0-2 heteroatoms selected from O, S and N.

In a preferred Example of the present invention, Z represents:

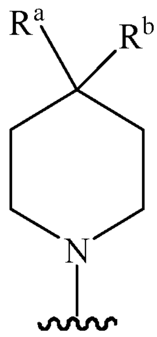 ,

wherein $R^a$ and $R^b$ each independently represent hydrogen, $C_1$-$C_6$ alkyl, $C_3$-$C_6$ cycloalkyl, $C_1$-$C_6$ haloalkyl or hydroxy($C_1$-$C_6$ alkyl); Or $R^a$ and $R^b$, together with the atom they are attached to, form a 3-6-membered saturated or unsaturated ring which may optionally contain 0-2 heteroatoms selected from O, S and N.

In a preferred Example of the present invention, Z represents:

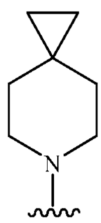 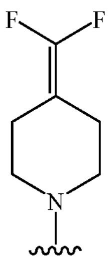

In a preferred Example of the present invention, $L^1$ represents 5-6 membered heteroaryl,

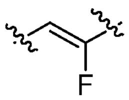 , 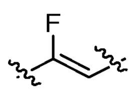 , 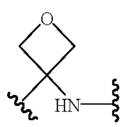 , 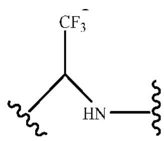 , 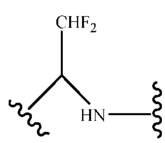 ,

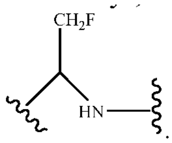

In a preferred Example of the present invention, $Cy^1$ represents any one of the following groups substituted with 0-3 substituents selected from halogen, $C_1$-$C_6$ alkyl, $C_1$-$C_6$ haloalkyl, hydroxy($C_1$-$C_6$ alkyl), $OR^a$, -O-($C_1$-$C_6$ haloalkyl), 5-6 membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$ and $-S(O)_2NR^aR^b$:

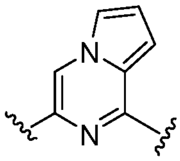 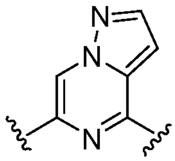 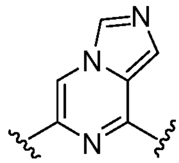 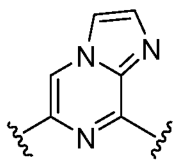

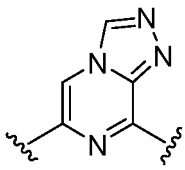 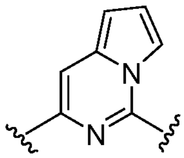 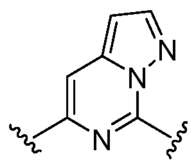 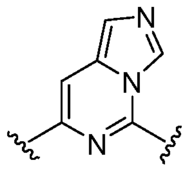

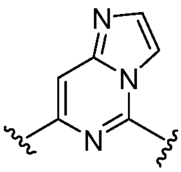 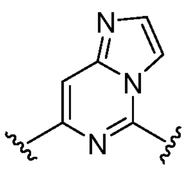 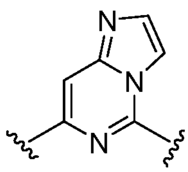 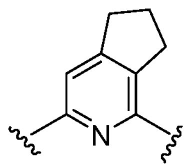

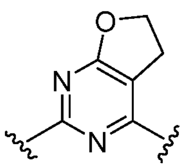 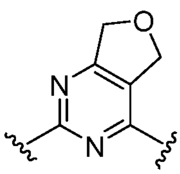 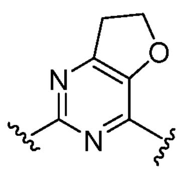 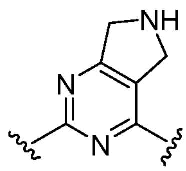

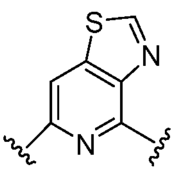 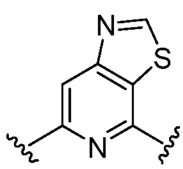 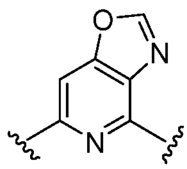 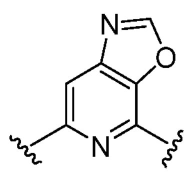

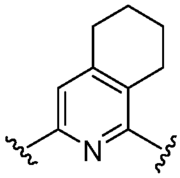 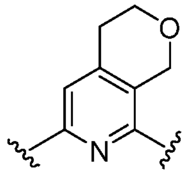 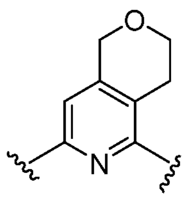 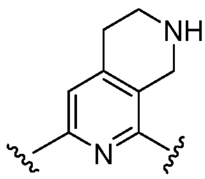

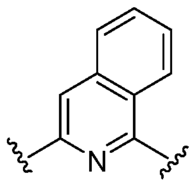 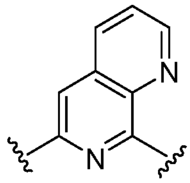 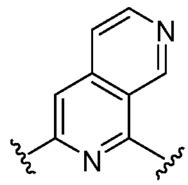 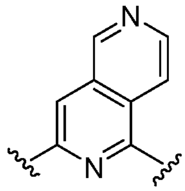

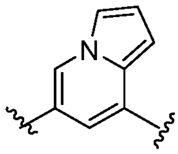 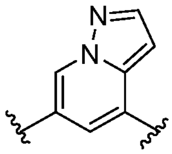 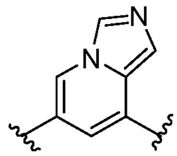 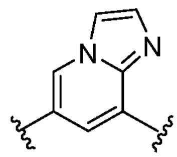

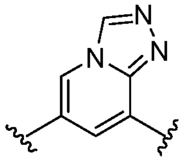 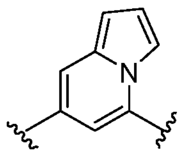 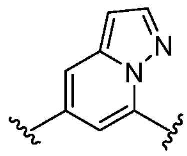 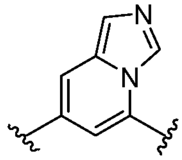

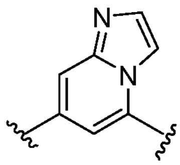 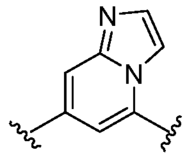 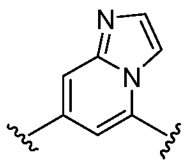 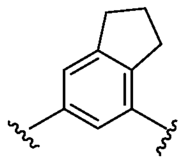

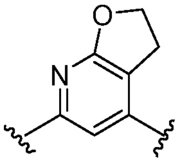 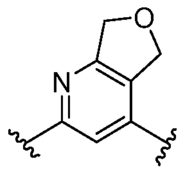 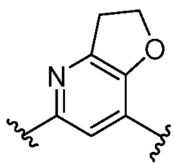 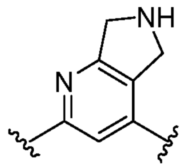

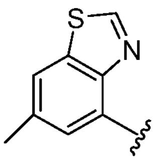 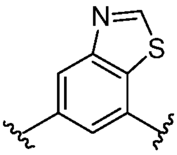 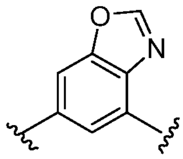 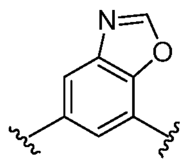

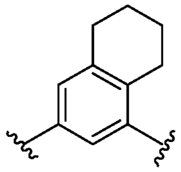 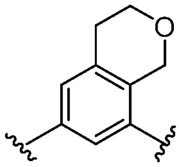 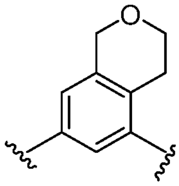 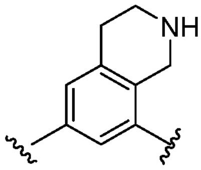

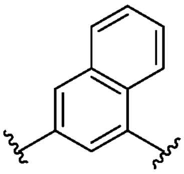 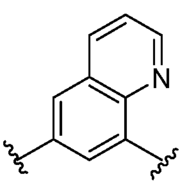 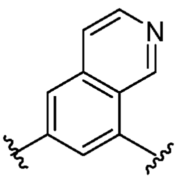 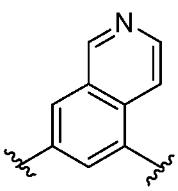

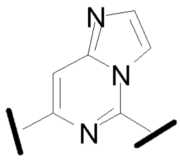 、 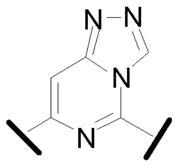 、 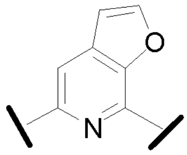 、 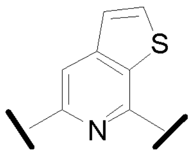

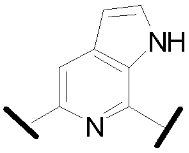 、 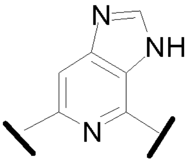 、 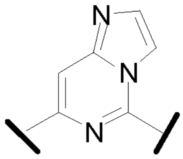 、 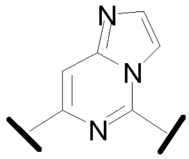

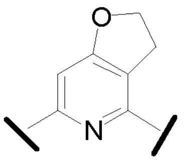 、 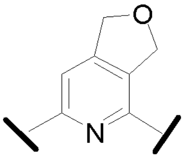 、 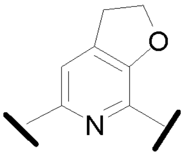 、 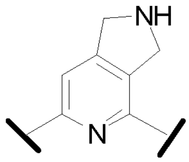

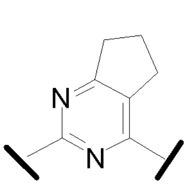 、 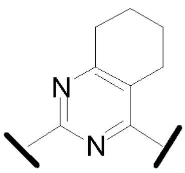 、 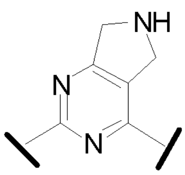 、 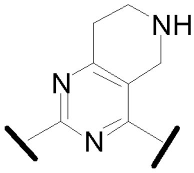

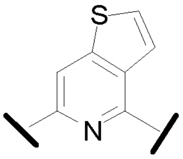 、 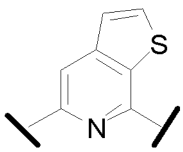 、 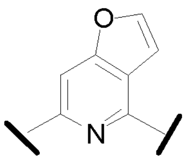 、 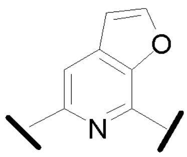

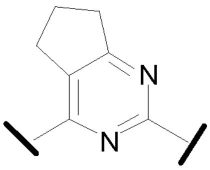 、 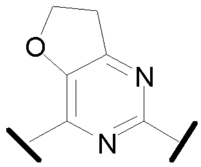 、 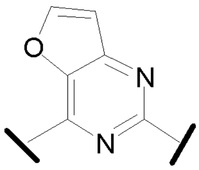 、 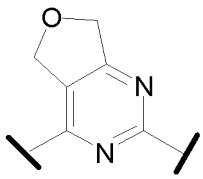

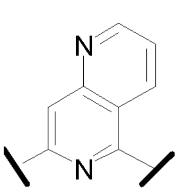 、 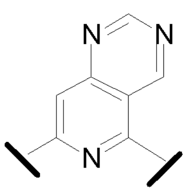 、 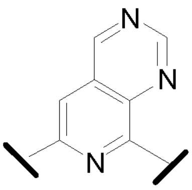 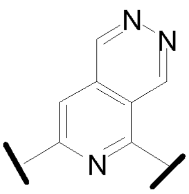

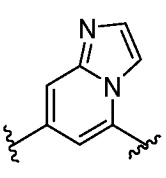 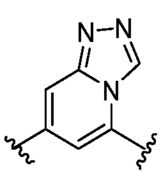 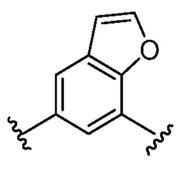 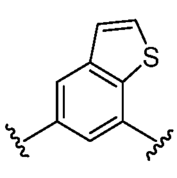

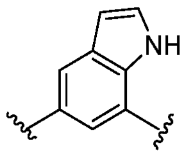 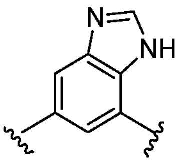 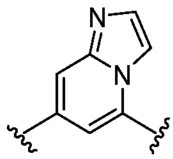 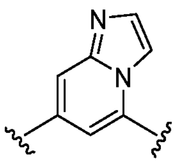

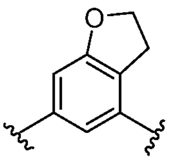 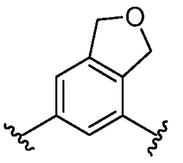 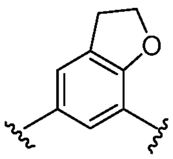 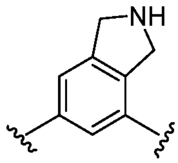

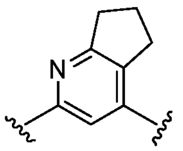 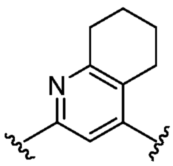 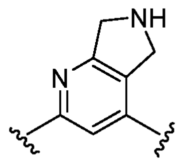 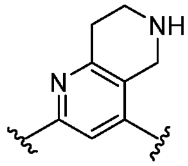

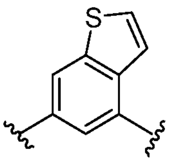 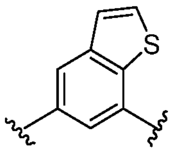 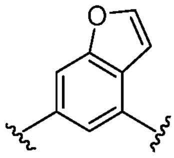 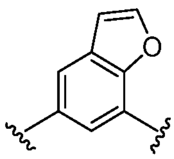

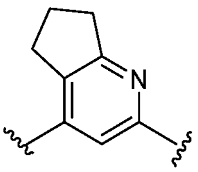 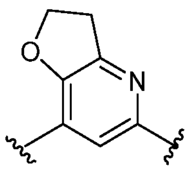 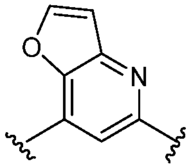 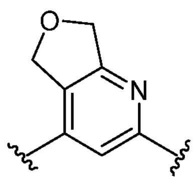

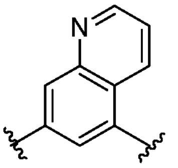 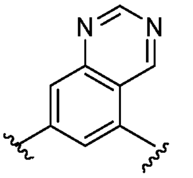 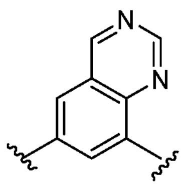 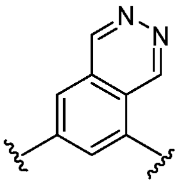

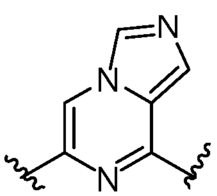 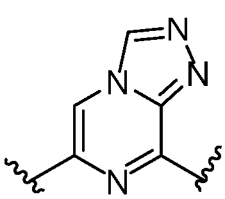 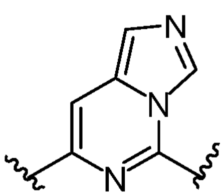 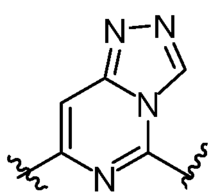

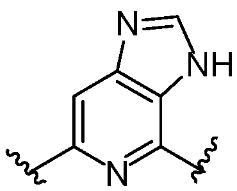 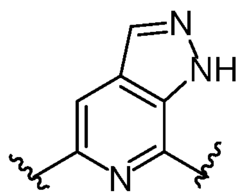 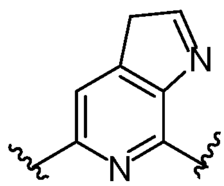 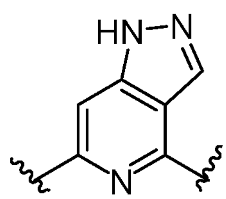

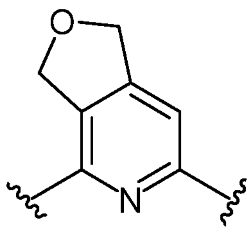
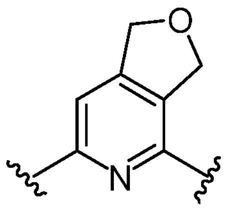
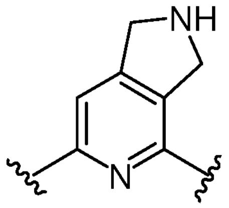
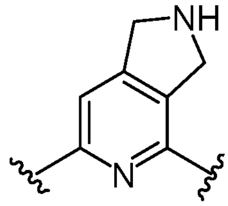
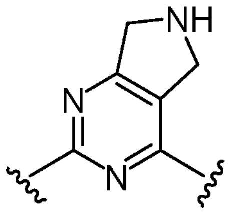
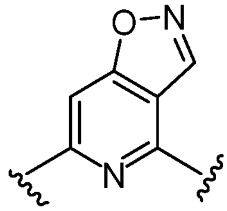
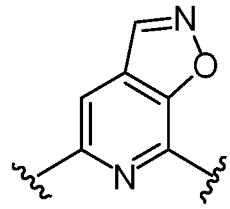
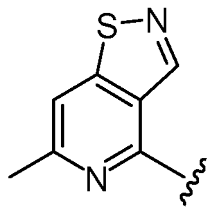
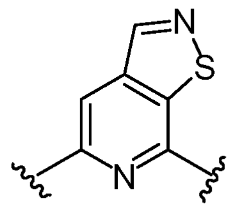
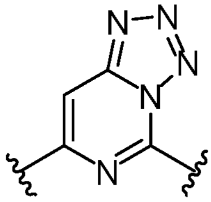
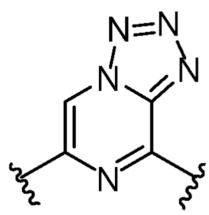
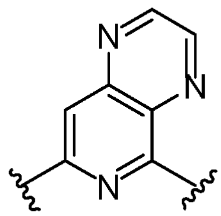
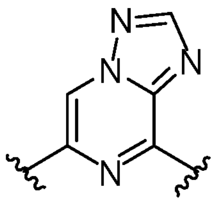
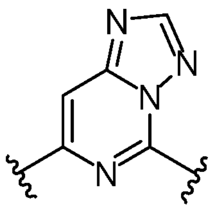
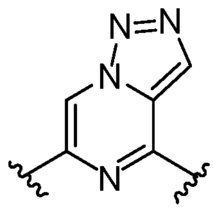

and/or

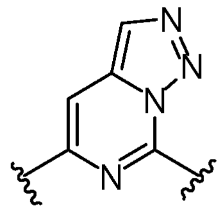
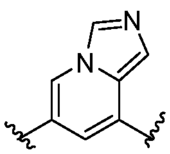
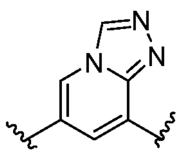
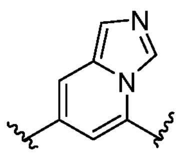
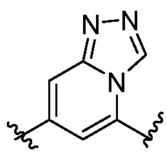
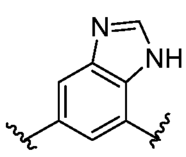
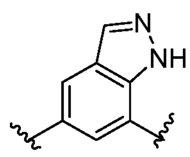
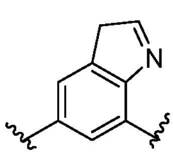
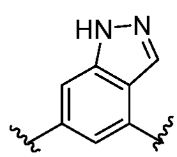
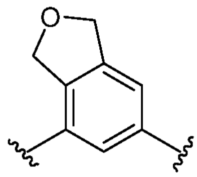
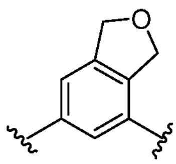
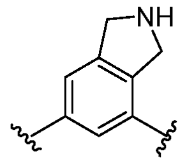
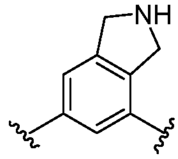

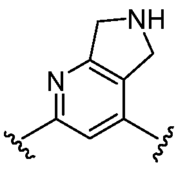
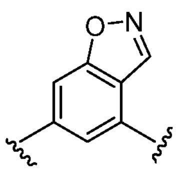
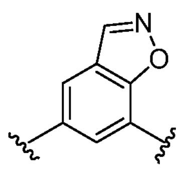
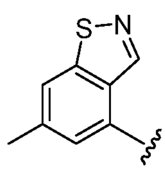
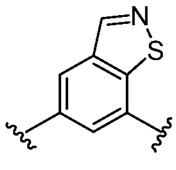
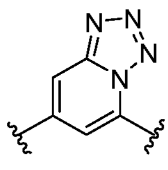
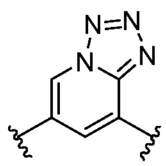
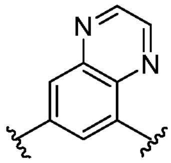
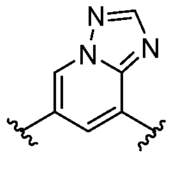
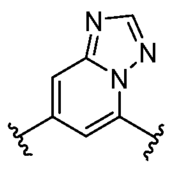
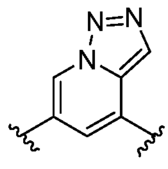
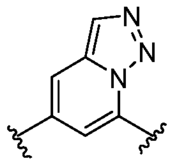

For example:

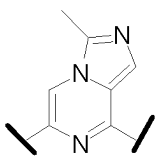
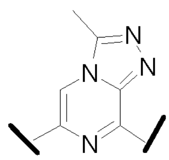
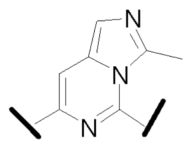
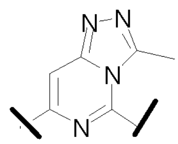
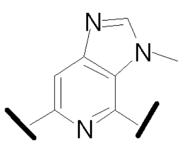
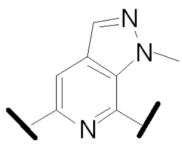
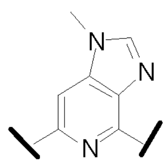
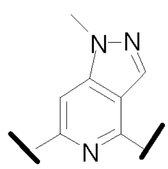
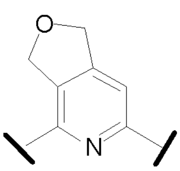
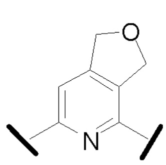
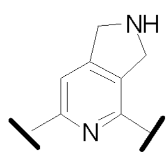
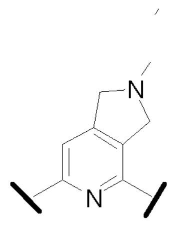
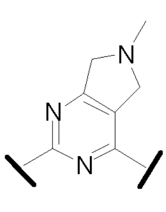
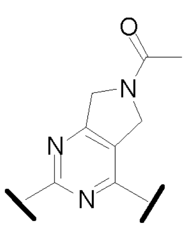
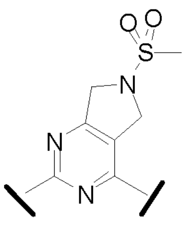
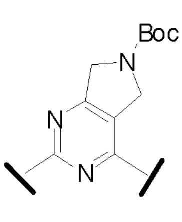

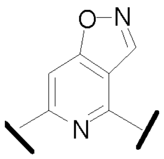 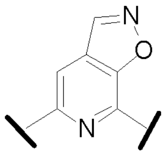 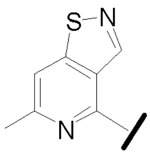 and/or 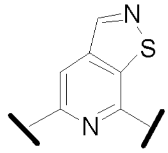

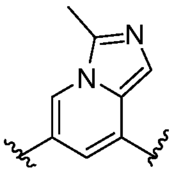 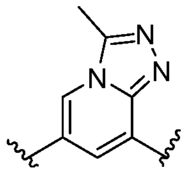 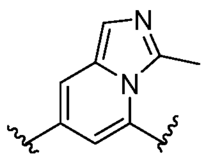 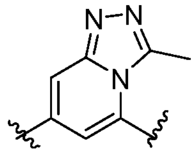

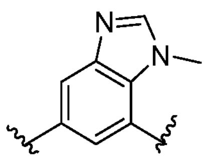 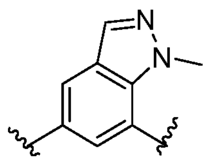 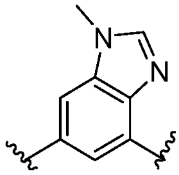 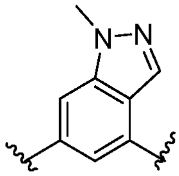

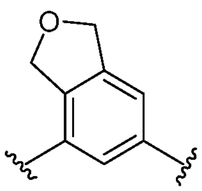 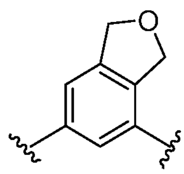 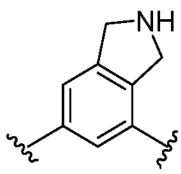 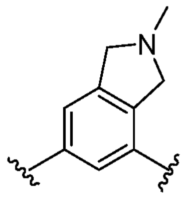

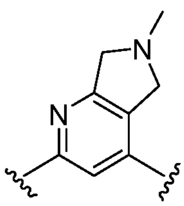 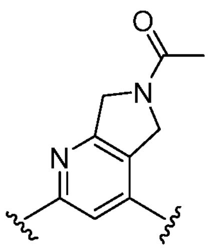 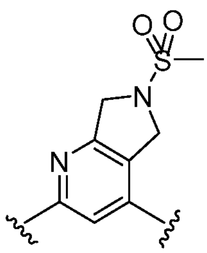 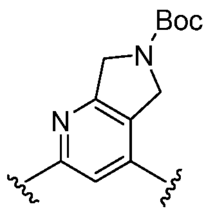

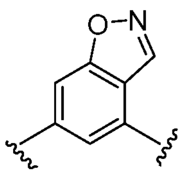 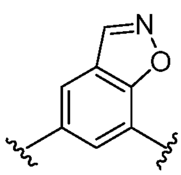 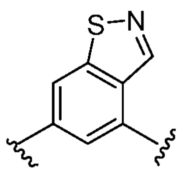 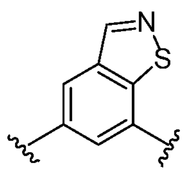 ;

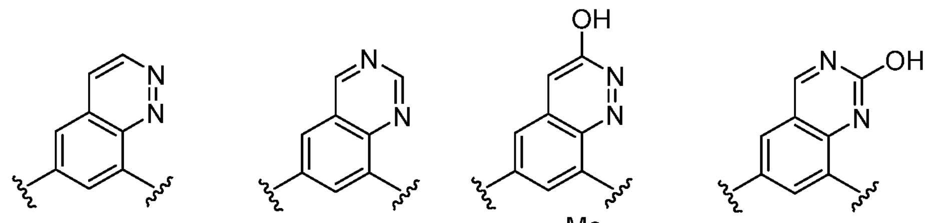

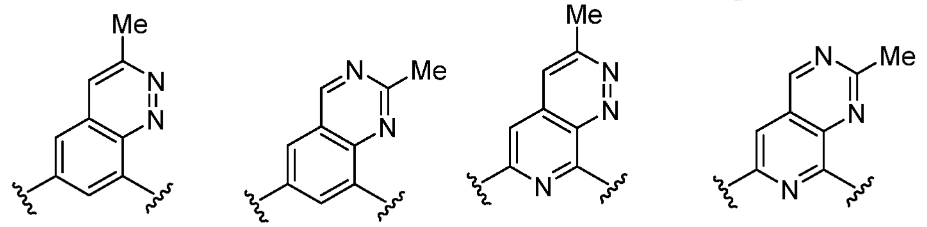

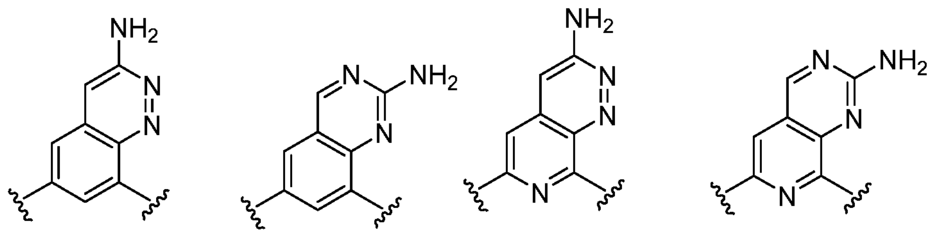

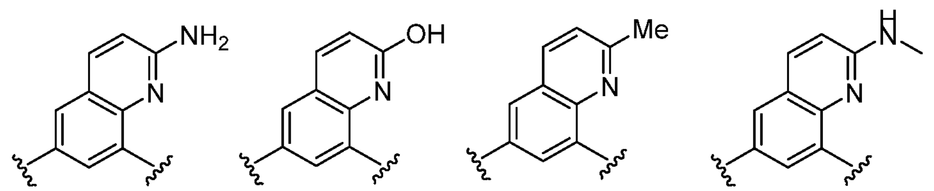

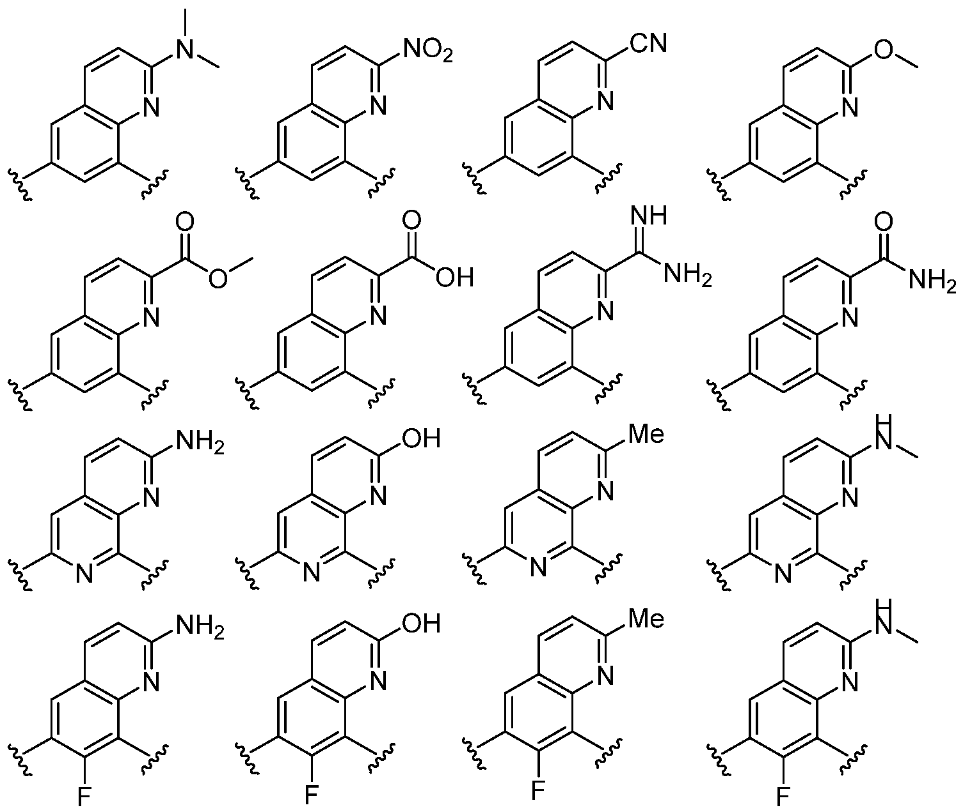

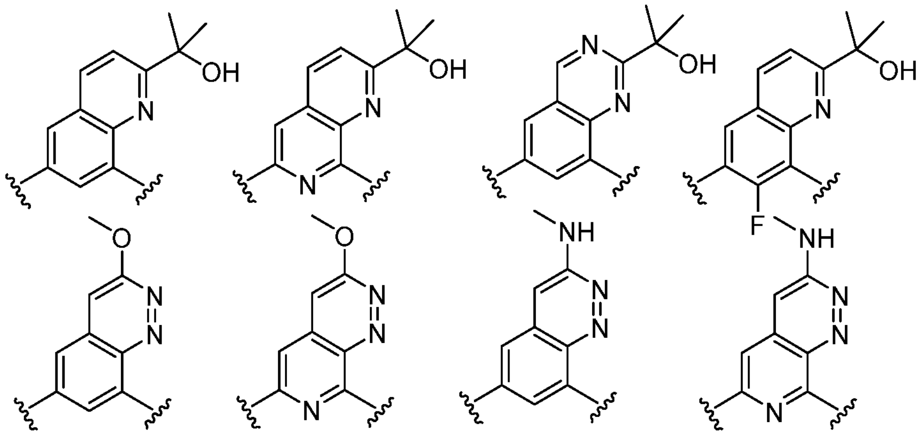

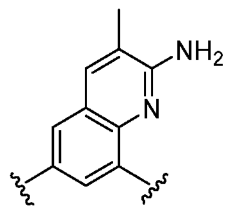
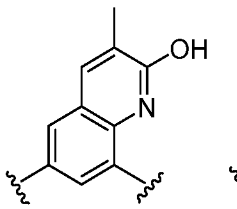
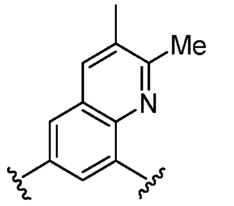
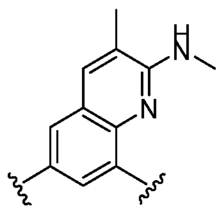

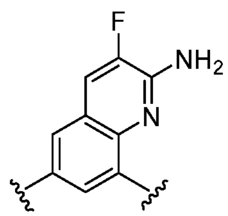
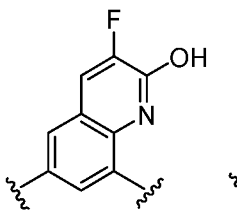
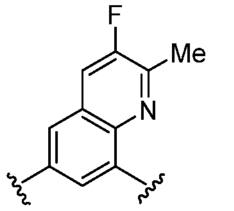
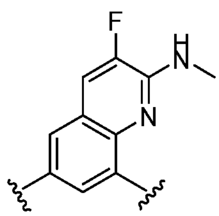

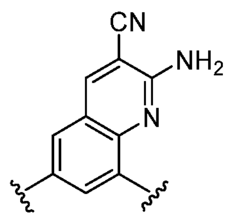
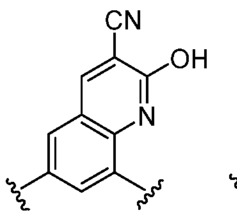
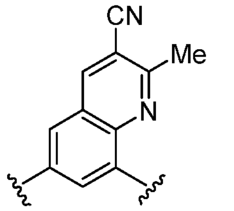
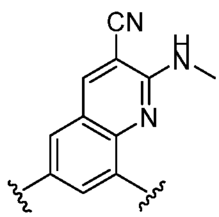

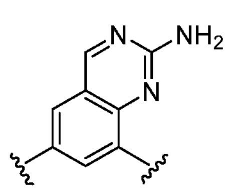
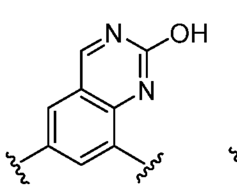
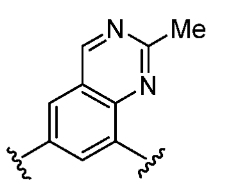
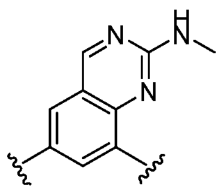

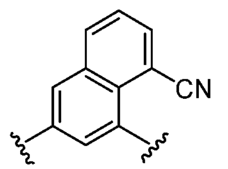
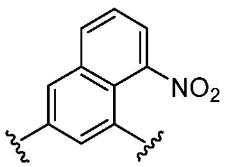
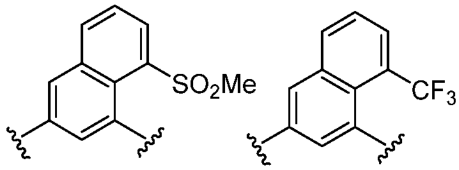

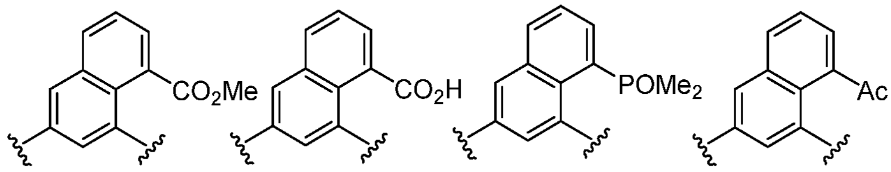

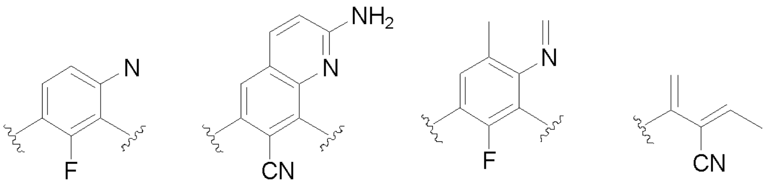

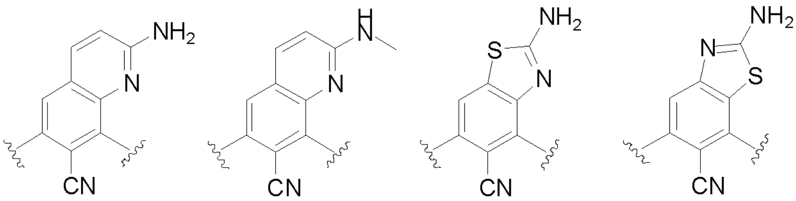

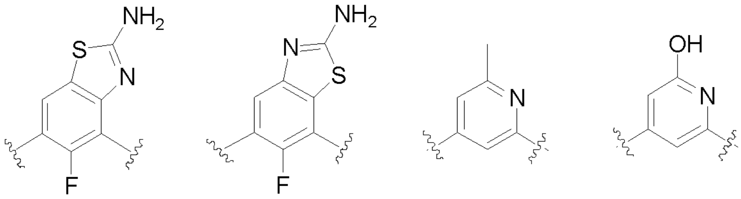

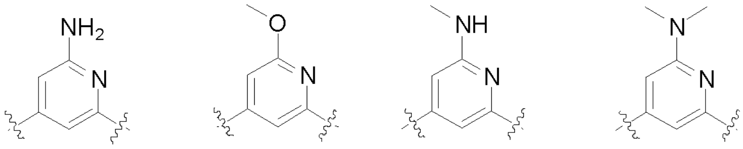

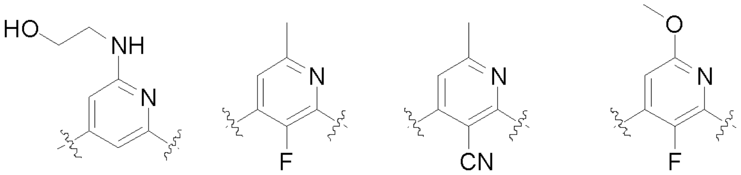

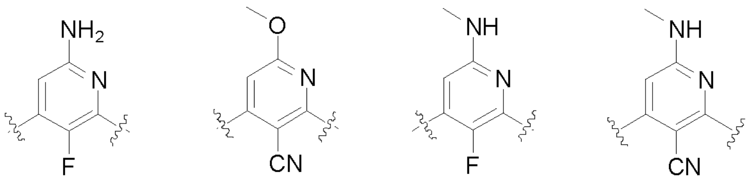

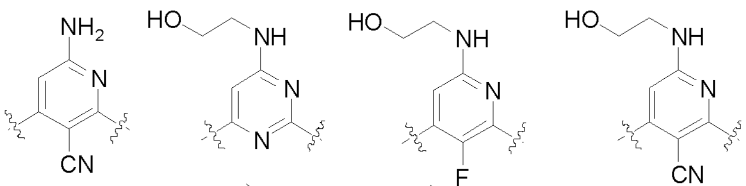

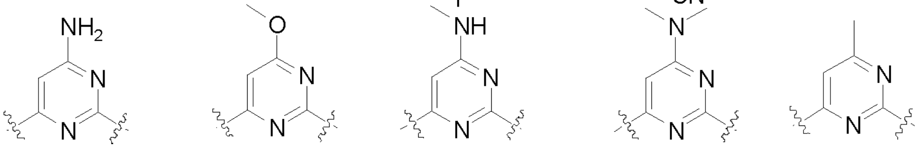
.

In a preferred Example of the present invention, $L^1$ represents 5-6 membered heteroaryl,

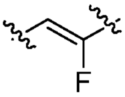 , 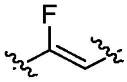 , 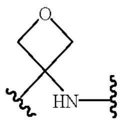 , 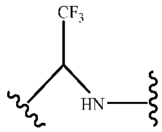 , 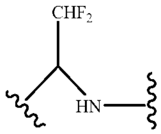 ,

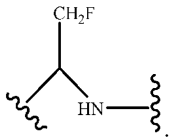.

In a preferred Example of the present invention, $L^1$ represents any one of the following groups:

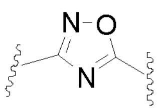 、 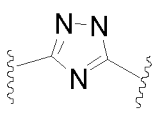 、 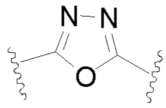 、 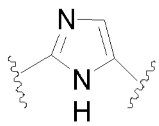 、 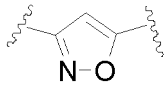 、

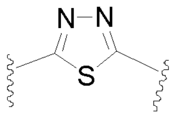 、 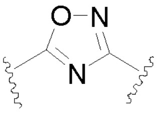 、 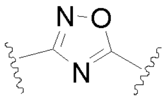 、 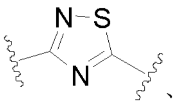 、 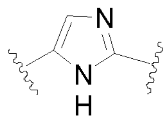

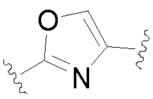 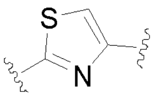 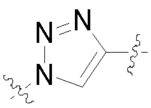 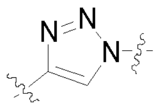 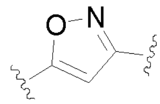

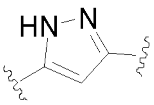 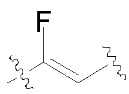 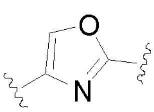 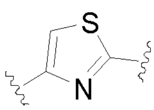 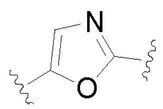

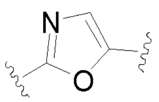 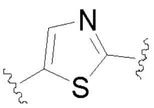 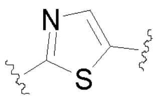 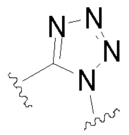 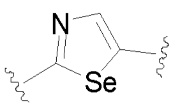

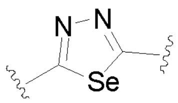 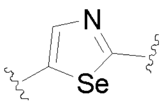 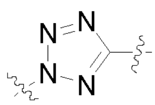 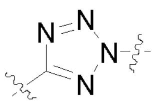 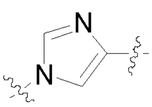

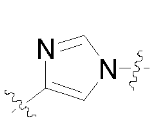 and/or 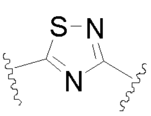

In a preferred Example of the present invention, $Cy^1$ represents any one of the following groups substituted with 0-3 substituents selected from halogen, $C_1$-$C_6$ alkyl, $C_1$-$C_6$ haloalkyl, hydroxy($C_1$-$C_6$ alkyl), $OR^a$, -O-($C_1$-$C_6$ haloalkyl), 5-6 membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$ and $-S(O)_2NR^aR^b$:

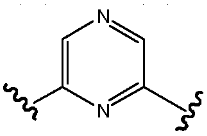 , 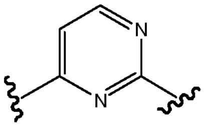 , 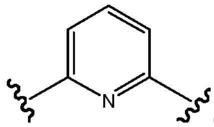 and/or 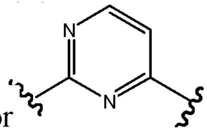

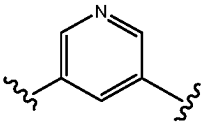 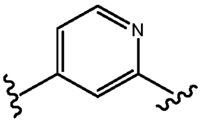 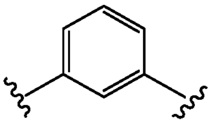 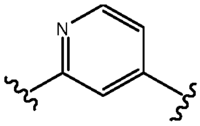 .

In a preferred Example of the present invention, $Cy^1$ represents the following groups substituted with 0-3 substituents selected from halogen, $C_1$-$C_6$ alkyl, $C_1$-$C_6$ haloalkyl, hydroxy($C_1$-$C_6$ alkyl), $OR^a$, -O- ($C_1$-$C_6$ haloalkyl), 5-6 membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$ and $-S(O)_2NR^aR^b$:

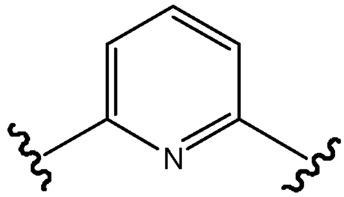 .

In a preferred Example of the present invention, $Cy^1$ represents

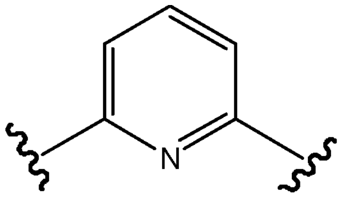

optionally substituted with phenyl, pyridinyl, thiazolyl, oxazolyl, pyrazolyl, imidazolyl and N-methylpyrazolyl.

In a preferred Example of the present invention $Cy^1$ represents the following groups:

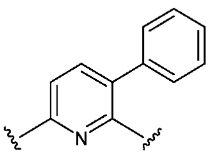 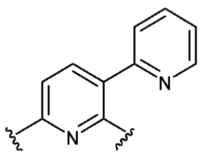 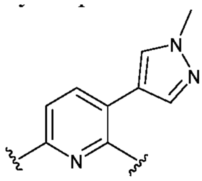

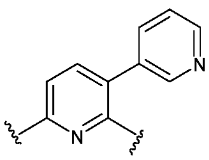 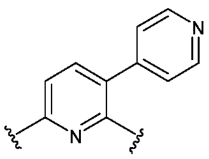 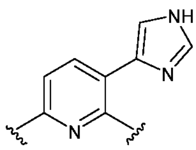

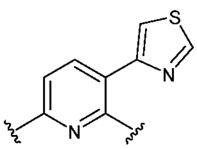 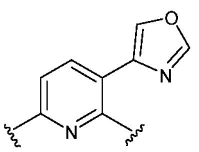 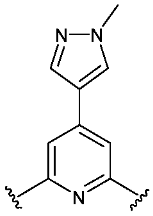 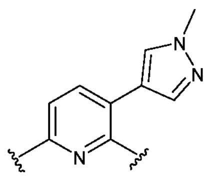

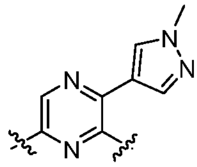
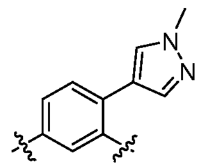
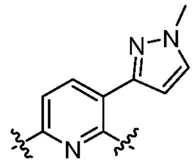
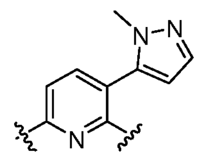
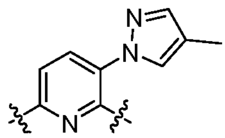
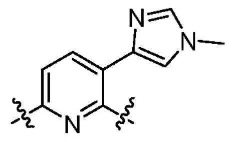
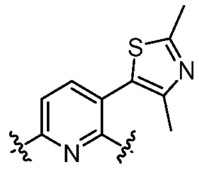
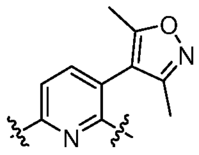
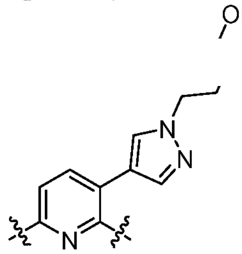
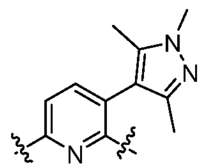
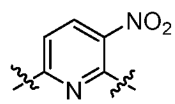
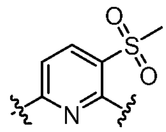
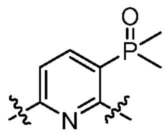
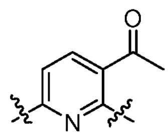
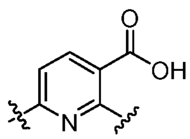
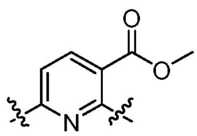
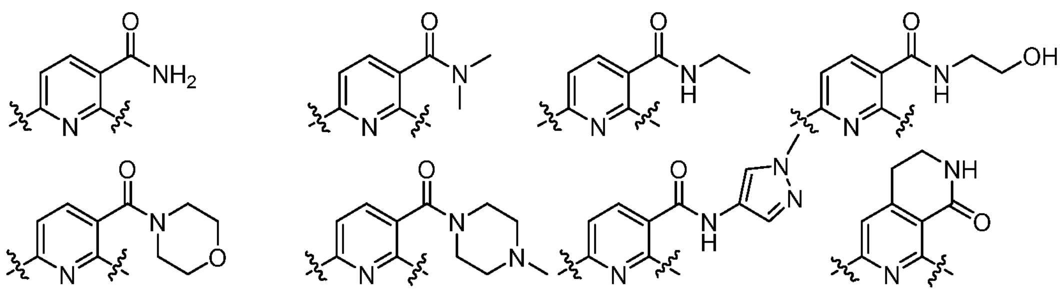

In a preferred Example of the present invention, $L^2$ represents absence, -$C_1$-$C_6$ alkylene- or -NH-.

In a preferred Example of the present invention, $L^2$ represents absence.

In a preferred Example of the present invention, $Cy^2$ represents a saturated, partially saturated or unsaturated 3-, 4-, 5-, 6-, 7-membered monocyclic ring, or a 3-, 4-, 5-, 6-, 7-membered fused ring, which contains 0-3 N heteroatoms and 0-2 O or S heteroatoms and can be optionally furthermore substituted with 0-3 substituents selected from the following: halogen, cyano, nitro, hydroxy($C_1$-$C_6$ alkyl), $C_1$-$C_6$ alkyl, $C_3$-$C_8$ cycloalkyl, $C_1$-$C_6$ haloalkyl, -$OR^a$, -$SO_3R^a$, -$S(O)R^a$, -O-($C_1$-$C_6$ haloalkyl), -$SR^a$, -$SF_5$ or $NR^aR^b$-substituted morpholinyl, piperidinyl, azetidine, pyrrolidinyl, cyclopropyl, cyclobutyl, cyclopentyl, cyclohexyl, piperazinyl or tetrahydrofuryl; wherein, $R^a$ and $R^b$ each independently represent hydrogen, halogen, $C_1$-$C_6$ alkyl, $C_3$-$C_8$ cycloalkyl, $C_1$-$C_6$ haloalkyl or hydroxy($C_1$-$C_6$ alkyl); Or $R^a$ and $R^b$, together with the atom they are attached to, form a 3-6 membered ring which contains 0-2 heteroatoms selected from O, N and S.

In a preferred Example of the present invention, $Cy^2$ represents

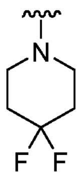
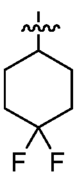
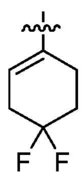
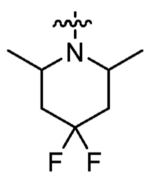
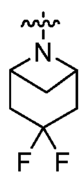
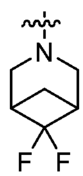
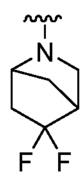

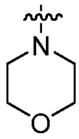 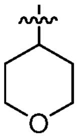 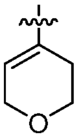 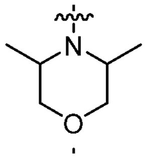 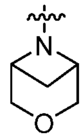 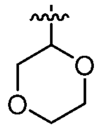 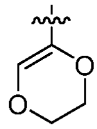

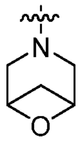 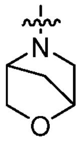 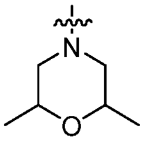 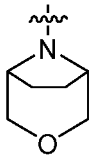 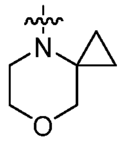 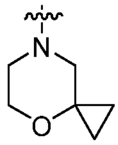 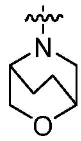

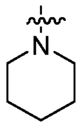 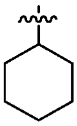 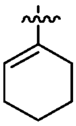 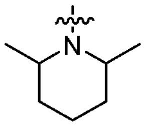 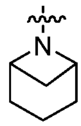 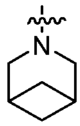 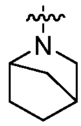

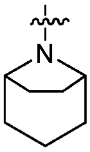 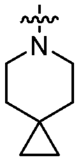 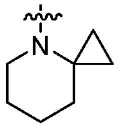 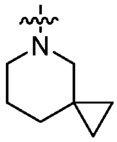 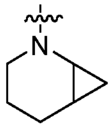 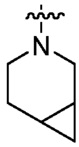 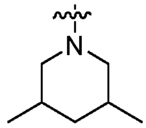

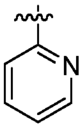 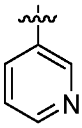 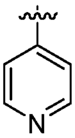 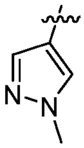 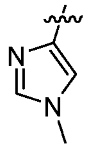 .

In particular, the present invention provides compounds having the following structures:

| | | | |
|---|---|---|---|
| 1 | 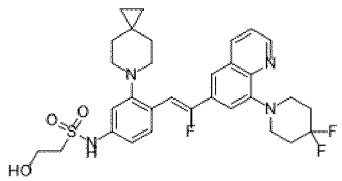 | 2 | 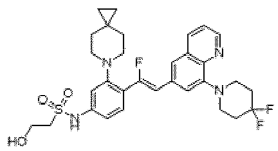 |
| 3 | 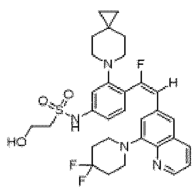 | 4 | 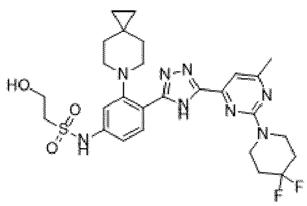 |
| 5 | 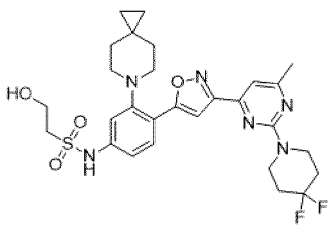 | 6 | 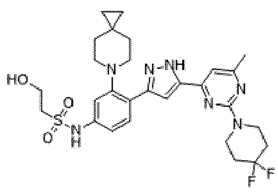 |

(continued)

| | | | |
|---|---|---|---|
| 7 | 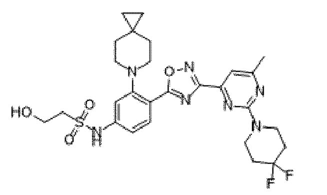 | 8 | 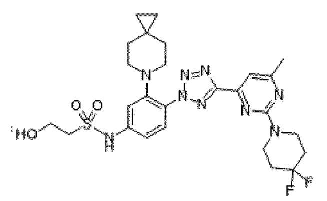 |
| 9 | 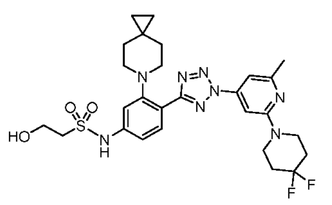 | 10 | 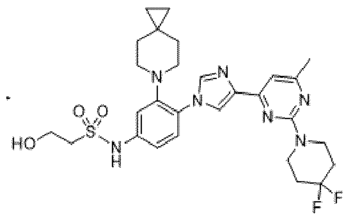 |
| 11 | 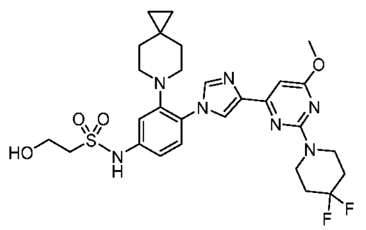 | 12 | 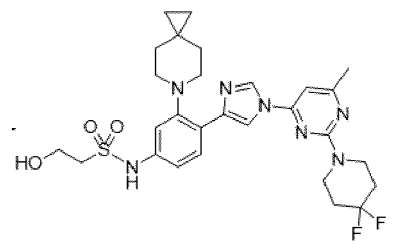 |
| 13 | 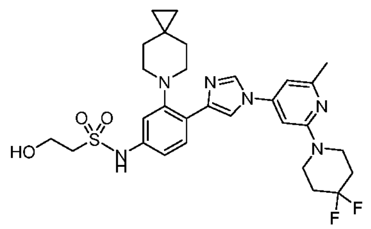 | 14 | 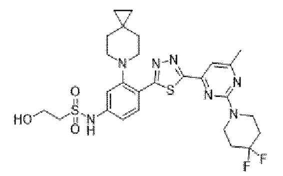 |
| 15 | 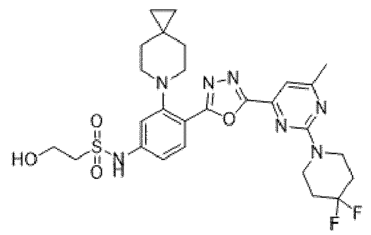 | 16 | 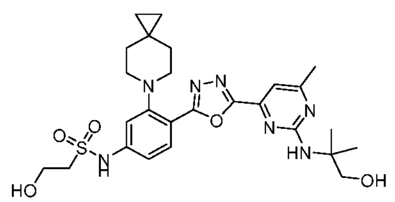 |
| 17 | 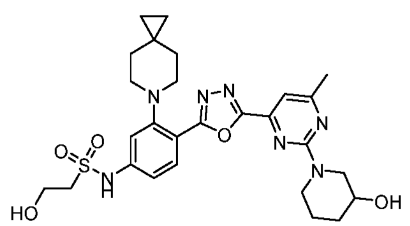 | 18 | 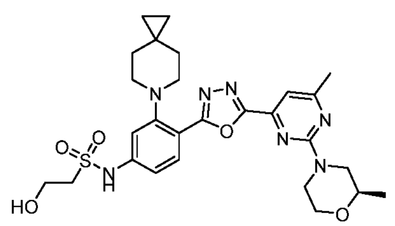 |
| 19 | 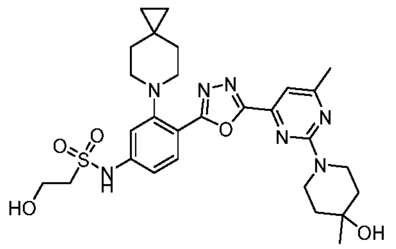 | 20 | 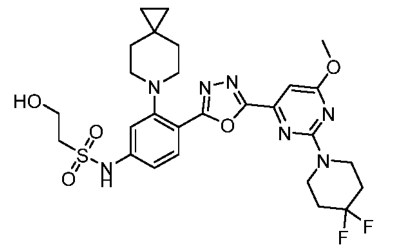 |

(continued)

| | | | |
|---|---|---|---|
| 21 | 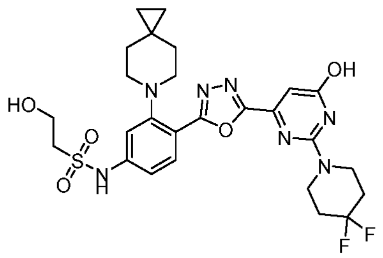 | 22 | 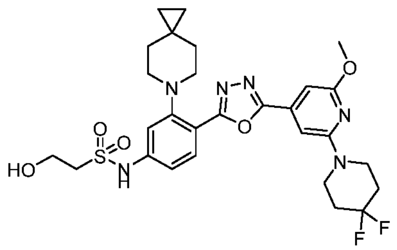 |
| 23 | 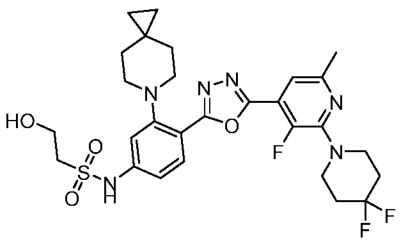 | 24 | 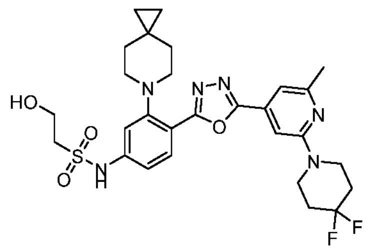 |
| 25 | 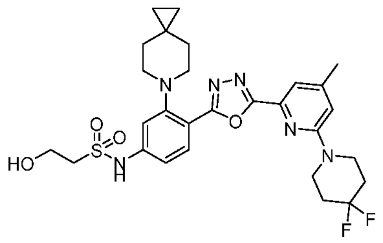 | 26 | 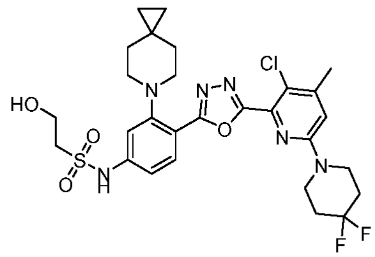 |
| 27 | 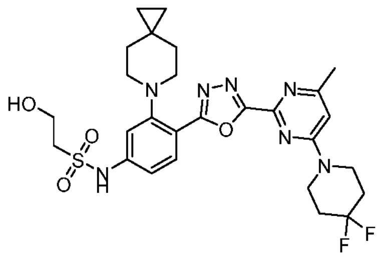 | 28 | 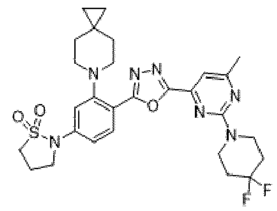 |
| 29 | 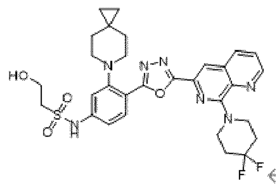 | 30 | 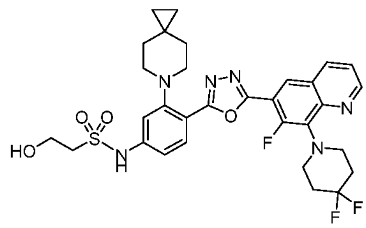 |
| 31 | 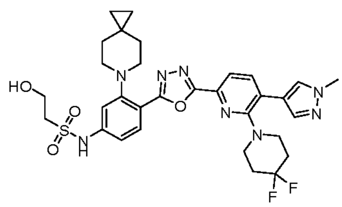 | 32 | 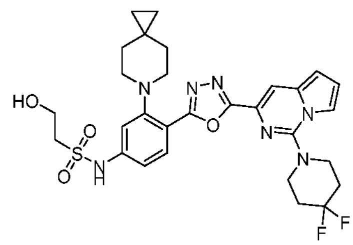 |

(continued)

| | | | |
|---|---|---|---|
| 33 | 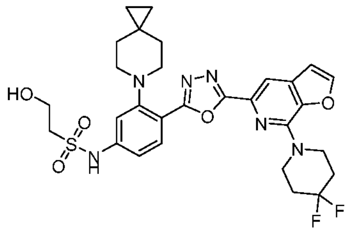 | 34 | 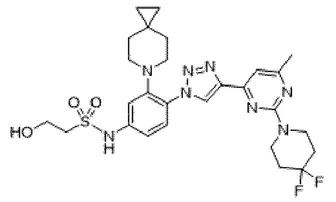 |
| 35 | 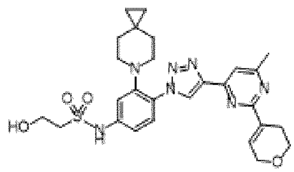 | 36 | 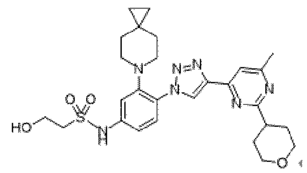 |
| 37 | 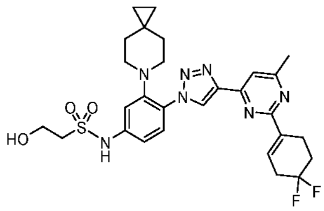 | 38 | 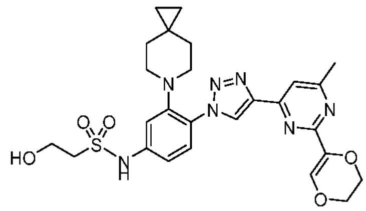 |
| 39 | 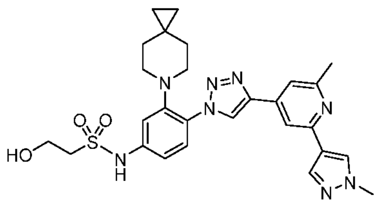 | 40 | 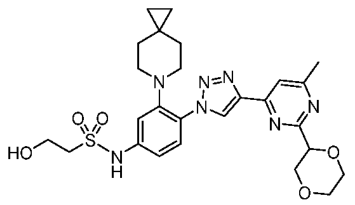 |
| 41 | 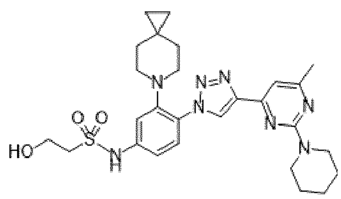 | 42 | 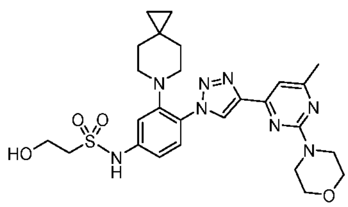 |
| 43 | 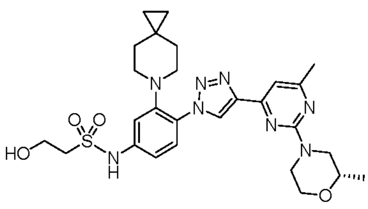 | 44 | 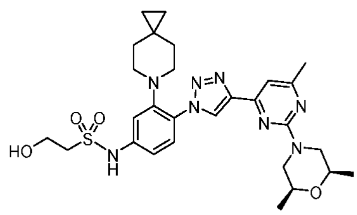 |
| 45 | 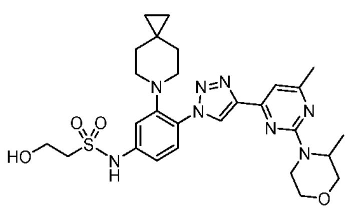 | 46 | 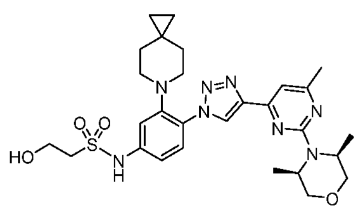 |

(continued)

| | | | |
|---|---|---|---|
| 47 | 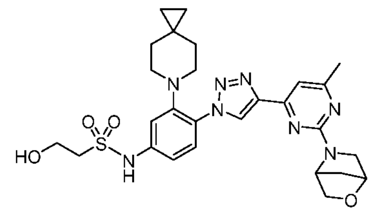 | 48 | 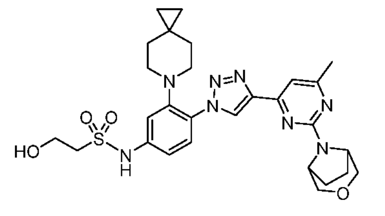 |
| 49 | 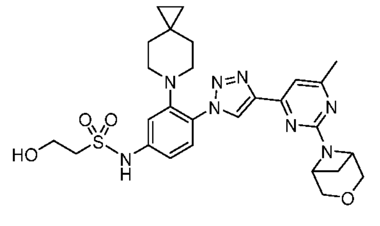 | 50 | 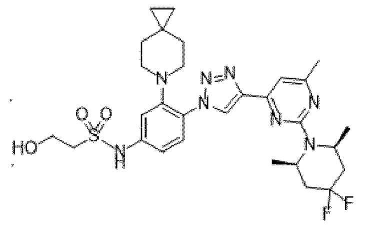 |
| 51 | 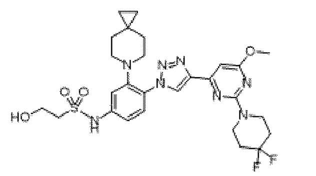 | 52 | 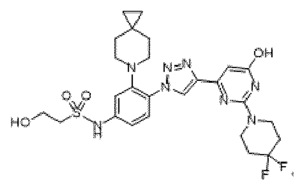 |
| 53 | 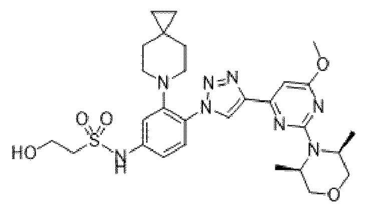 | 54 | 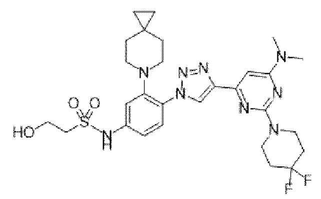 |
| 55 | 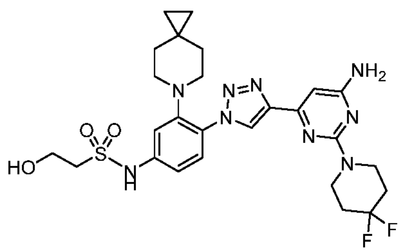 | 56 | 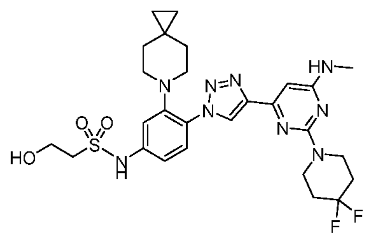 |
| 57 | 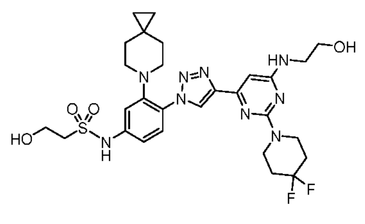 | 58 | 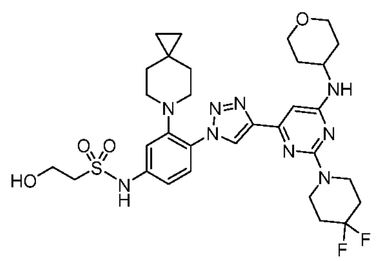 |
| 59 | 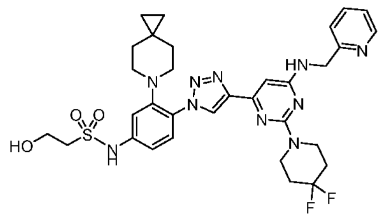 | 60 | 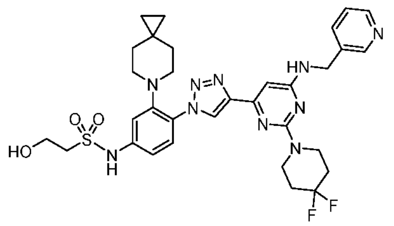 |

(continued)

| | | | |
|---|---|---|---|
| 61 | 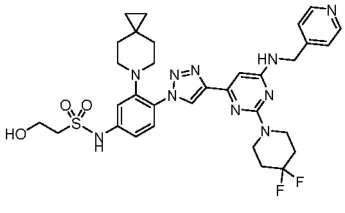 | 62 | 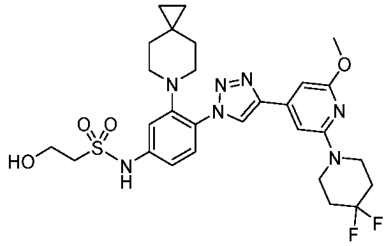 |
| 63 | 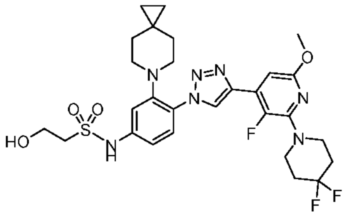 | 64 | 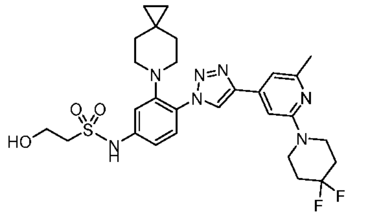 |
| 65 | 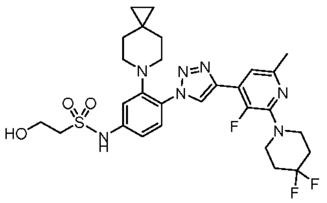 | 66 | 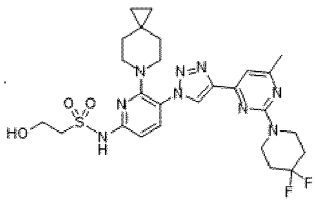 |
| 67 | 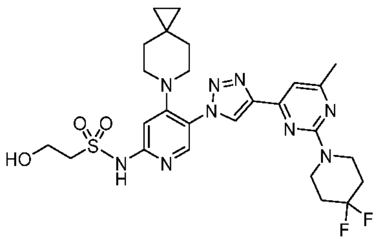 | 68 | 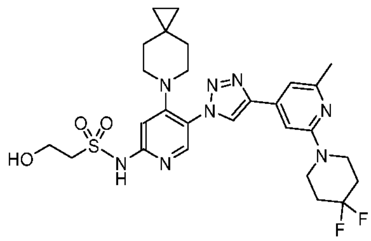 |
| 69 | 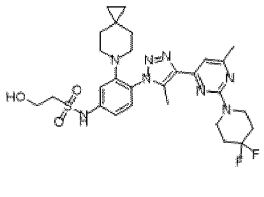 | 70 | 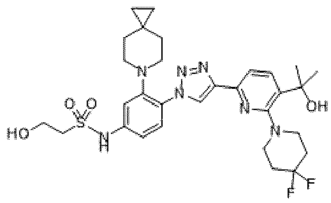 |
| 71 | 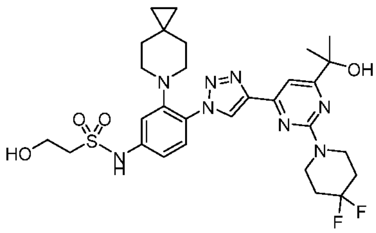 | 72 | 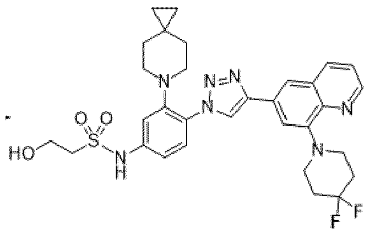 |
| 73 | 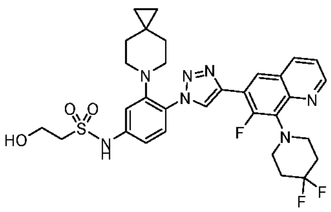 | 74 | 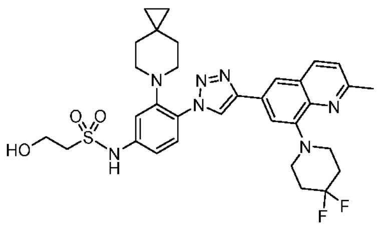 |

(continued)

| | | | |
|---|---|---|---|
| 75 | 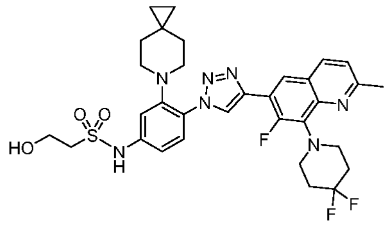 | 76 | 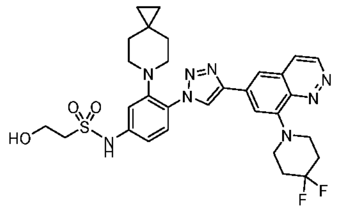 |
| 77 | 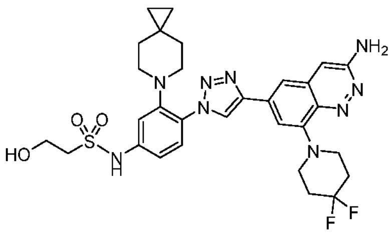 | 78 | 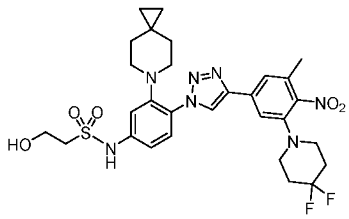 |
| 79 | 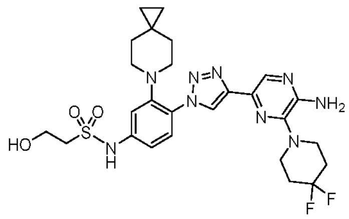 | 80 | 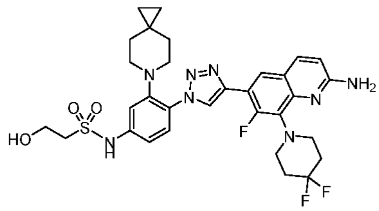 |
| 81 | 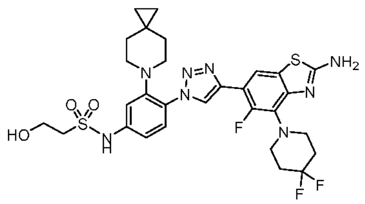 | 82 | 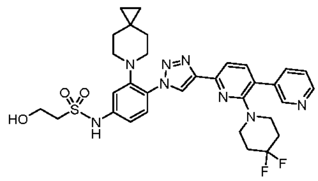 |
| 83 | 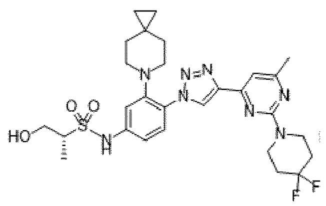 | 84 | 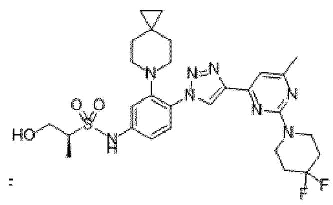 |
| 85 | 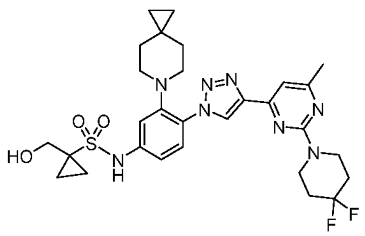 | 86 | 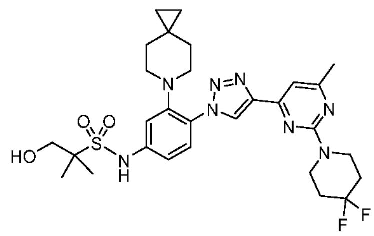 |
| 87A | 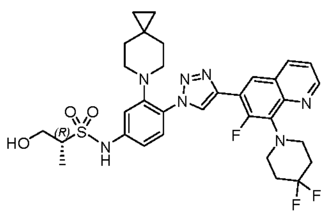 | 87B | 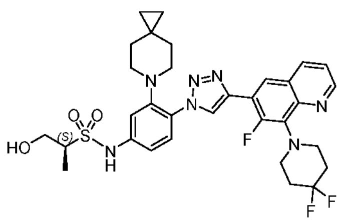 |

(continued)

| | | | |
|---|---|---|---|
| 88 | 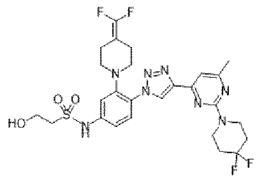 | 89 | 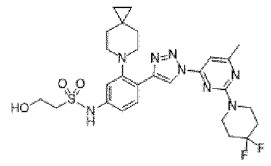 |
| 90 | 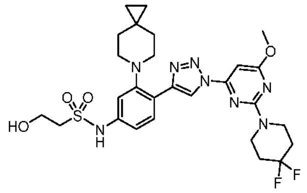 | 91 | 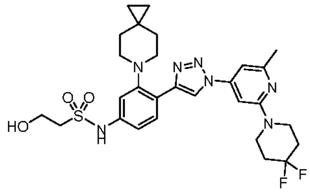 |
| 92 | 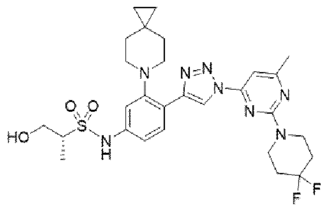 | 93 | 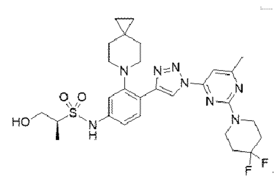 |
| 94 | 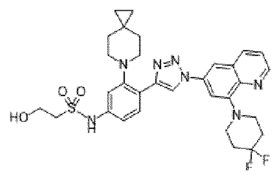 | 95 | 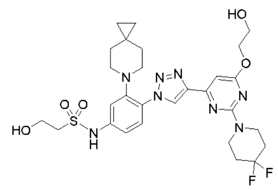 |
| 96 | 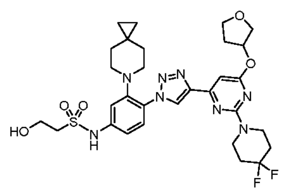 | 97 | 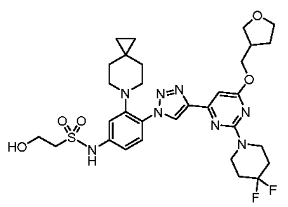 |
| 98 | 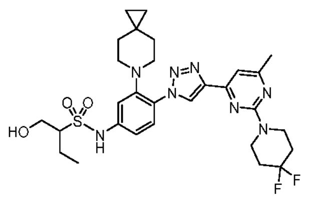 | 99 | 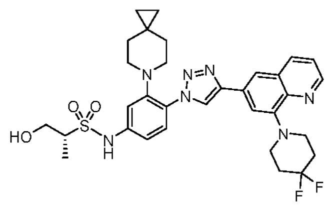 |
| 100 | 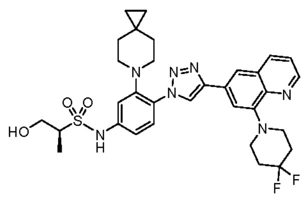 | 101 | 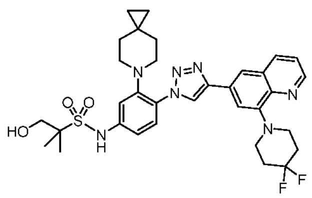 |
| 102 | 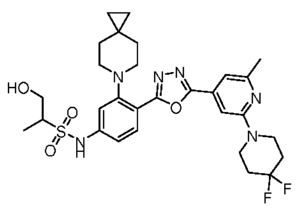 | 103 | 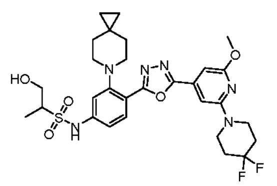 |

(continued)

| | | | |
|---|---|---|---|
| 104 | 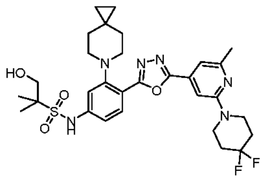 | 105 | 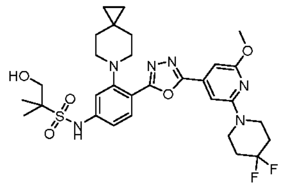 |
| 106 | 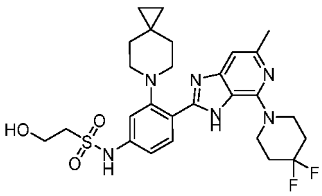 | | |

In addition, the present invention provides a pharmaceutical composition that contains any one of the compounds in the Examples of the present invention or its pharmaceutically acceptable salts, stereoisomers, isotope isomers, prodrugs, hydrates or solvates and pharmaceutically acceptable carriers.

In addition, the present invention provides a method for treating tumors by inhibiting KIF 18A, comprising administering any one of the compounds of the present invention or its pharmaceutically acceptable salts, stereoisomers, isotope isomers, prodrugs, hydrates or solvates to an individual in need.

**Definitions**

Unless otherwise specified, the compounds of the present invention may encompass, in addition to their specific structures, their pharmaceutically acceptable salts, stereoisomers, isotope isomers (e.g., deuterated compounds), solvates, hydrates, prodrugs and metabolites. Thus, the pharmaceutically acceptable salts, stereoisomers, isotope isomers, solvates, hydrates, prodrugs, and metabolites of these compounds are also considered within the scope of protection.

Unless otherwise specified, the terms used in this patent specification and claims are defined as follows. Furthermore, many of the groups defined herein may be optionally substituted. A list of typical substituents is presented in Definitions by way of example and is not intended to limit the substituents defined elsewhere in this patent specification and claims.

The term "alkyl" refers to a linear or branched saturated aliphatic hydrocarbyl or linker, comprising 1-20 carbon atoms, preferably 1-12 carbon atoms, more preferably 1-8 carbon atoms, 1-6 carbon atoms or 1-4 carbon atoms. "Lower alkyl" specifically refers to an alkyl comprising 1-4 carbon atoms. Examples of alkyl include $-(CH_2)_3-$, methyl, trifluoromethyl, ethyl, propyl, isopropyl, n-butyl, isobutyl, tert-butyl and pentyl. Alkyl may be either substituted or unsubstituted. Typical substituents include cycloalkyl, aryl, heteroaryl, heterocycloalkyl, hydroxyl, alkoxy, aryloxy, mercapto, alkylthio, arylthio, cyano, halo, carbonyl, thiocarbonyl, O-carbamoyl, N-carbamoyl, O-thiocarbamoyl, N-thiocarbamoyl, C-acylamino, N-amido, C-carboxyl, O-carboxyl, nitro, silyl, amino and $-NR^xR^y$; Wherein, $R^x$ and $R^y$ are independently selected from hydrogen, alkyl, cycloalkyl, aryl, carbonyl, acetyl, sulfonyl, trifluoromethanesulfonyl and a fused 5- or 6-membered heterocyclyl ring.

The term "alkenyl" refers to a linear or branched hydrocarbyl containing one or more double bonds and typically comprising 2-20 carbon atoms. For example, "$C_2$-$C_6$ alkenyl" comprises 2-6 carbon atoms. Examples of alkenyl include, but are not limited to, vinyl, propenyl, butenyl and 1-methyl-2-buten-1-yl.

The term "alkynyl" refers to a linear or branched hydrocarbyl containing one or more triple bonds and typically comprising 2-20 carbon atoms. For example, "C2-C6 alkynyl" comprises 2-6 carbon atoms. Examples of alkynyl include, but are not limited to, ethynyl, 1-propynyl and 1-butynyl.

The term "alkoxy" or "alkyloxy" refers to -O-alkyl. "$C_1$-$C_6$ alkoxy" (or alkyloxy) is intended to include $C_1$, $C_2$, $C_3$, $C_4$, $C_5$ and $C_6$ alkoxy. Examples of alkoxy include, but are not limited to, methoxy, ethoxy, propoxy (e.g., n-propoxy and isopropoxy) and tert-butoxy. Similarly, "alkylthio" or "thioalkoxy" refers to sulfur-bridged alkyl comprising a specified number of carbon atoms as defined above; for example, methyl-S- and ethyl-S-.

The term "cycloalkyl" refers to a 3- to 8-membered all-carbon monocyclic or bicyclic ring, a 5-/6- or 6-/6-membered fused all-carbon bicyclic ring, or a fused polycyclic ring (in a "fused" ring system, each ring shares at least one adjacent carbon atom with other rings) group, in which one or more of the rings may contain one or more double bonds but none of such rings has an intact conjugated π-electron system, or two rings forms a spiro by sharing one carbon. Examples of cycloalkyl include, but are not limited to, cyclopropane, cyclobutane, cyclopentane, cyclopentene, cyclohexane,

cyclohexadiene, adamantane, cycloheptane and cycloheptatriene. Bicyclic alkyl includes bridged, spiro or fused-ring cycloalkyl. Illustrative examples of cycloalkyl are derived from but not limited to the following:

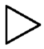 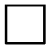 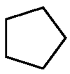 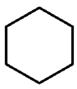 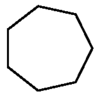 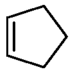 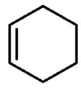 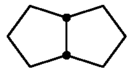

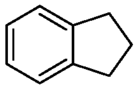 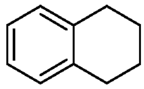 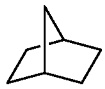 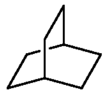 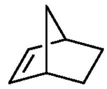 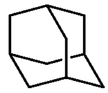

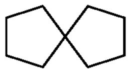 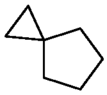 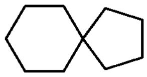 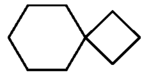

The term "aryl" refers to an all-carbon monocyclic or fused-ring polycyclic group comprising 6-12 carbon atoms, with an intact conjugated $\pi$-electron system. Examples of aryl include, but are not limited to, phenyl, naphthyl and anthracenyl. Aryl may be either substituted or unsubstituted. Typical substituents include halo, trihalomethyl, alkyl, hydroxyl, alkoxy, aryloxy, mercapto, alkylthio, arylthio, cyano, nitro, carbonyl, thiocarbonyl, C-carboxyl, O-carbamoyl, N-carbamoyl, O-thiocarbamoyl, N-thiocarbamoyl, C-acylamino, N-amido, sulfinyl, sulfonyl, amino and $-NR^aR^b$, wherein $R^a$ and $R^b$ are as defined above. The aryl-fused saturated or unsaturated cycloalkyl/saturated or unsaturated heterocycloalkyl may be regarded as a special substituent of aryl, and typical examples include but are not limited to:

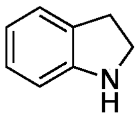 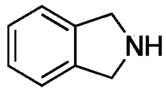 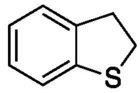 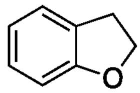 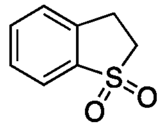

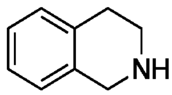 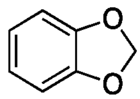

The term "heteroaryl" refers to a monocyclic or fused ring comprising 5-12 ring atoms, with one, two, three or four ring heteroatoms selected from N, O and S and the remaining ring atoms being C, having an intact conjugated $\pi$-electron system. Typical examples of heteroaryl include, but are not limited to, acridinyl, azetidinyl, azocinyl, benzimidazolyl, benzofuryl, benzothiofuranyl, benzothienyl, benzoxazolyl, benzoxazolinyl, benzothiazolyl, benzotriazolyl, benzotetrazolyl, benzoisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofura[2,3-b]tetrahydrofuryl, furanyl, furazanyl, imidazolidinyl, imidazolinyl, imidazolyl, 1H-indazolyl, imidazopyridinyl, indolenyl, dihydroindolyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridoxazolyl, pyridinoimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolyl, 4H-quinolizinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienoxazolyl, thienoimidazolyl, thienyl, triazinyl, 1,2,3-triazolyl, 1,2,4-triazolyl, 1,2,5-triazolyl, 1,3,4-triazolyl and xanthyl, quinolyl, isoquinolinyl, phthalazinyl, quinazolinyl, indolyl, isoindolyl, dihydroindolyl, 1H-indazolyl, benzimidazolyl, 1,2,3,4-tetrahydroquinolyl, 1,2,3,4-tetrahydroisoquinolyl, 5,6,7,8-tetrahydro-quinolyl, 2,3-dihydro-benzofuranyl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and monocyclic "heteroaryl", including but not limited to "-phenylbipyridyl-", "-phenylbipyrimidinyl", "-pyridylbiphenyl", "-pyridylbipyrimidinyl-" and "-pyrimidinylbiphenyl-"; wherein the present invention also includes fused ring and spiro ring compounds containing, for example, heterocyclic rings mentioned above.

A pharmaceutically acceptable heteroaryl is sufficiently stable to be linked to a compound of the present invention for formulation into a pharmaceutical composition and subsequent administration to patients in need.

Unless otherwise defined, the definitions of substituents in the present invention are independent and not interrelated. For example, the definition of $R^a$ (or $R^b$) is independent across different substituents. Specifically, when one definition is selected for $R^a$ (or $R^b$) in a substituent, it does not imply that the same definition applies to $R^a$ (or $R^b$) in other substituents. More specifically, when $R^a$ (or $R^b$) is defined as hydrogen in $NR^aR^b$, for example (non-exhaustive), it does not imply that $R^a$ (or $R^b$) in -C(O)-$NR^aR^b$ must be hydrogen.

"Halo" or "halogen" includes fluorine, chlorine, bromine and iodine. "Haloalkyl" is intended to include branched and linear saturated aliphatic hydrocarbyl having a specified number of carbon atoms substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl also include "fluoroalkyl" which is intended to include branched and linear saturated aliphatic hydrocarbyl having a specified number of carbon atoms and substituted with 1 or more fluorine atoms.

"Haloalkoxy" or "haloalkyloxy" refers to oxygen-bridged haloalkyl having a specified number of carbon atoms as defined above. For example, "C1-C6 haloalkoxy" is intended to include $C_1$, $C_2$, $C_3$, $C_4$, $C_5$ and $C_6$ haloalkoxy. Examples of haloalkoxy include, but are not limited to, trifluoromethoxy, 2,2,2-trifluoroethoxy and pentafluoroethoxy. Similarly, "haloalkylthio" or "thiohaloalkoxy" refers to sulfur-bridged haloalkyl comprising a specified number of carbon atoms as defined above; for example, trifluoromethyl-S- and pentafluoroethyl-S-.

In the present disclosure, the expression Cx1-Cx2 is used when referring to some substituents, which means that the number of carbon atoms in the substituent may be x1 to x2. For example, $C_0$-$C_8$ means that the group contains 0, 1, 2, 3, 4, 5, 6, 7 or 8 carbon atoms, $C_1$-$C_8$ means that the group contains 1, 2, 3, 4, 5, 6, 7 or 8 carbon atoms, $C_2$-$C_8$ means that the group contains 2, 3, 4, 5, 6, 7 or 8 carbon atoms, $C_3$-$C_8$ means that the group contains 3, 4, 5, 6, 7 or 8 carbon atoms, $C_4$-$C_8$ means that the group contains 4, 5, 6, 7 or 8 carbon atoms, $C_0$-$C_6$ means that the group contains 0, 1, 2, 3, 4, 5 or 6 carbon atoms, $C_1$-$C_6$ means that the group contains 1, 2, 3, 4, 5 or 6 carbon atoms, $C_2$-$C_6$ means that the group contains 2, 3, 4, 5 or 6 carbon atoms, and $C_3$-$C_6$ means that the group contains 3, 4, 5 or 6 carbon atoms.

In the present disclosure, the expression " x1-x2-membered ring" is used when referring to cyclic groups (e.g., aryl, heteroaryl, cycloalkyl and heterocycloalkyl), which means that the number of ring atoms in the group may be x1 to x2. For example, 3-12-membered cyclic group may be a 3, 4, 5, 6, 7, 8, 9, 10, 11 or 12-membered ring comprising 3, 4, 5, 6, 7, 8, 9, 10, 11 or 12 ring atoms; 3-6-membered cyclic group may be a 3, 4, 5 or 6-membered ring comprising 3, 4, 5 or 6 ring atoms; 3-8-membered cyclic group may be a 3, 4, 5, 6, 7 or 8-membered ring comprising 3, 4, 5, 6, 7 or 8 ring atoms; 3-9-membered cyclic group may be a 3, 4, 5, 6, 7, 8 or 9-membered ring comprising 3, 4, 5, 6, 7, 8 or 9 ring atoms; 4-7-membered cyclic group may be a 4, 5, 6 or 7-membered ring comprising 4, 5, 6 or 7 ring atoms; 5-8-membered cyclic group may be a 5, 6, 7 or 8-membered ring comprising 5, 6, 7 or 8 ring atoms; 5-12-membered cyclic group may be a 5, 6, 7, 8, 9, 10, 11 or 12-membered ring comprising 5, 6, 7, 8, 9, 10, 11 or 12 ring atoms; 6-12-membered cyclic group may be a 6, 7, 8, 9, 10, 11 or 12-membered ring comprising 6, 7, 8, 9, 10, 11 or 12 ring atoms. The ring atoms may be carbon atoms or heteroatoms, such as heteroatoms selected from N, O and S. The heterocyclic rings may contain 1, 2, 3, 4, 5, 6, 7, 8, 9, 10 or more ring heteroatoms, such as heteroatoms selected from N, O and S.

In the present invention, one or more halogens may be each independently selected from fluorine, chlorine, bromine and iodine.

The term "substituted" as used herein means that at least one hydrogen atom is substituted by a non-hydrogen group, provided that normal valence is maintained and the substitution results in a stable compound. The term "cyclic double bond" as used herein refers to a double bond formed between two adjacent ring atoms (e.g. C=C, C=N or N=N).

Where nitrogen atoms (e.g. amines) are present on the compounds of the invention, these nitrogen atoms may be converted to N-oxides by treatment with an oxidizing agent (e.g. mCPBA and/or hydrogen peroxide) to obtain other compounds of the invention. Therefore, the nitrogen atoms shown and claimed are deemed to encompass the nitrogen atoms shown and their N-oxides to obtain derivatives of the present invention.

When any variable occurs more than once in any composition or formula of a compound, its definition in each occurrence is independent of its definition in every other occurrence Therefore, for example, if a group is shown substituted with 0-3 Rs, the group may be optionally substituted with up to three R groups, with R defined independently in each occurrence. Furthermore, combinations of substituents and/or variables are permitted only if such combinations result in stable compounds.

The term "patient" as used herein refers to an organism to be treated by a method of the present invention. Such organisms preferably include, but are not limited to, mammals (e.g. rodents, apes/monkeys, horses, cattle, pigs, dogs and cats) and most preferably humans.

The term "effective amount" as used herein refers to an amount of a drug or agent (i.e., a compound of the invention) that will elicit, for example, a biological or medical response in a tissue, a system, an animal or a human sought by a researcher or clinician. Furthermore, the term "therapeutically effective amount" refers to an amount that results in improved treatment, cure, prevention or alleviation of a disease, condition or side effect, or reduction in the progression

rate of a disease or condition, as compared to a corresponding subject who does not receive such amount. An effective amount may be administered in one or more doses and is not intended to be limited to a particular formulation or route of administration. The term also includes an amount effective to enhance normal physiological functions within its range.

The term "treatment" as used herein includes its broad sense and encompasses both therapeutic and/or prophylactic treatment of an object. Specifically, "treatment" includes any treatment that results in the alleviation, inhibition, elimination and relief and/or prevention of a condition, disease and disorder, such as alleviation, reduction, regulation, relief, elimination, prophylaxis, prevention or remission of symptoms thereof. The therapeutic treatment includes alleviating, inhibiting or relieving symptoms or conditions of a disease; inhibiting the occurrence of complications; alleviating the underlying metabolic syndrome; inhibiting the occurrence of a disease or condition such as controlling the progression of the disease or condition; alleviating a disease or condition; reducing a disease or condition; alleviating complications caused by a disease or condition, or treating signs caused by a disease or condition. The prophylactic treatment includes a prior treatment to prevent, block or delay, slow the onset or progression of a disease or condition or reduce the severity of the disease or condition.

Similarly, the term "therapeutic agent" also includes agents or reagents used in the therapeutic and/or prophylactic treatment of an object.

The terms "pharmaceutical" or "pharmaceutically acceptable" as used herein refer to compounds, substances, compositions and/or dosage forms that, within the scope of sound medical judgment, are suitable for contact with human and animal tissues without excessive toxicity, irritation, allergic reactions and/or other issues or complications, and commensurate with a reasonable benefit/risk ratio.

Specific Pharmaceutical and Medical Terms

The term "cancer" as used herein refers to an abnormal growth of cells that is uncontrollable and, under certain conditions, capable of metastasis (spread). This type of cancer includes, but is not limited to, solid tumors [such as bladder, intestine, brain, chest, uterus, heart, kidney, lung, lymphoid tissue (lymphoma), ovary, pancreas or other endocrine organs (e.g. thyroid gland), prostate and skin (melanoma)] or blood tumors (e.g. non-leukemic leukemia).

The term "combined administration" or similar terms thereof, as used herein, refers to the administration of several selected therapeutic agents to a patient in the same or different modes of administration and at the same or different times.

The term "enhancement" or "capable of enhancing" as used herein refers to an expected outcome of an increase or extension in either potency or duration. Thus, in the context of enhancing the therapeutic effect of a drug, the term "capable of enhancing" refers to the ability of the drug to increase or extend its potency or duration within the system. The term "enhancement value" as used herein refers to the ability to maximize the efficacy of another therapeutic agent in an ideal system.

The term "immune disease" refers to a disease or condition resulting from an adverse or harmful response to endogenous or exogenous antigens, typically leading to cellular dysfunction, or functional damage and impairment, or damage to organs or tissues that may produce immune symptoms.

The term "kit" is synonymous with "product packaging."

The terms "object", "subject" or "patient" include both mammals and non-mammals. Mammals include, but are not limited to, mammals including humans and non-human primates (such as orangutans, apes and monkeys), agricultural animals (such as cattle, horses, goats, sheep and pigs), domestic animals (such as rabbits and dogs), and laboratory animals including rodents (such as rats, mice and guinea pigs). Non-mammals include, but are not limited to, birds and fish. In a preferred Example, the selected mammal is human.

As used herein, a compound or pharmaceutical composition, after administered, can relieve a disease, symptom or condition, particularly to reduce severity, delay onset, slow down progression or shorten duration. This applies whether the administration is fixed or temporary, continuous or intermittent, and can be attributed to or associated with the administration.

Routes of Administration

Suitable routes of administration include, but are not limited to, oral, intravenous, rectal, aerosol, parenteral, ocular, pulmonary, transdermal, vaginal, ear canal, nasal and topical. In addition, by way of example only, routes of parenteral administration include intramuscular, subcutaneous, intravenous, intramedullary, ventricular, intraperitoneal, intralymphatic and intranasal.

The mode of administration of the compounds of the present invention may be topical. In certain Examples, long-acting formulations are administered by (subcutaneous or intramuscular) implantation or intramuscular injection. In

another Example, administration is achieved by a targeted drug delivery system. For example, liposomes coated with organ-specific antibodies. In this Example, the liposomes are selectively directed to and absorbed by specific organs.

Pharmaceutical Composition and Dosage

The term "pharmaceutical carrier" as used herein refers to a pharmaceutical substance, composition or vehicle, such as a liquid or solid filler, a diluent, an excipient, a manufacturing aid (e.g., lubricant, talc, magnesium stearate, calcium stearate or zinc stearate or stearic acid) or a solvent-encapsulated substance, used to carry or deliver the target compound from one organ or part of the body to another. Each carrier must be "acceptable" in the sense of being compatible with other ingredients of the formulation and harmless to patients.

The term "pharmaceutical composition" refers to a composition comprising a compound of the present invention and optionally a pharmaceutical carrier. The "pharmaceutical carrier" refers to a medium generally accepted in the art for delivering a biologically active agent to animals (particularly mammals), including (i.e.) adjuvants, excipients or vehicles such as diluents, preservatives, fillers, flow regulators, disintegrants, wetting agents, emulsifiers, suspending agents, sweeteners, corrigents, perfuming agents, antibacterial agents, antifungal agents, lubricants and dispersants, depending on the mode of administration and nature of the dosage form.

The pharmaceutical composition of the present invention may comprise a therapeutically effective amount of one or more compounds described in the present invention formulated with optionally one or more pharmaceutical carriers (additives) and/or diluents, and optionally one or more other therapeutic agents. The compounds of the present invention may be administered by any suitable means for any of the above uses, for example orally, such as in the form of tablets, pills, powders, granules, elixirs, tinctures, suspensions (including nanosuspensions, microsuspensions and spray-dried dispersions), syrups and emulsions; sublingually; buccally; parenterally, such as by subcutaneous, intravenous, intramuscular or intrasternal injection or infusion techniques (e.g. in the form of sterile injectable aqueous or non-aqueous solutions or suspensions); nasally, including administering to the nasal membrane, such as by inhalation spray; topically, such as in the form of creams or ointments; rectally, such as in the form of suppositories; or intratumorally. They may be administered alone; however, they are typically administered via a drug carrier selected based on the chosen route of administration and standard pharmaceutical practice.

Pharmaceutical carriers are formulated based on various factors known to those skilled in the art. These factors include but are not limited to: the type and nature of the active agent formulated; the subject to whom the composition containing the active agent is to be administered; the intended route of administration of the composition; and the targeted therapeutic indication. Pharmaceutical carriers include aqueous and non-aqueous liquid media and various solid and semisolid dosage forms.

The above-mentioned carriers may comprise many different ingredients and additives in addition to the active agent. These ingredients are included in the formulation for various reasons known to those skilled in the art, such as stabilizing active agents and binding agents. Descriptions of suitable pharmaceutical carriers and factors involved in carrier selection can be obtained from several readily available sources, such as Allen L. V. Jr. et al. Remington: The Science and Practice of Pharmacy( 2 Volumes), 22nd Edition (2012), Pharmaceutical Press.

Dosage regimens of the compounds of the present invention will, of course, vary depending on known factors such as the pharmacodynamic properties of the particular agent and its mode and route of administration; the species, age, sex, health status, medical condition and weight of the recipient; the nature and severity of symptoms; the type of concurrent treatment; the frequency of treatment; the route of administration, renal and hepatic function of the patient and expected effects. According to general guidance, the daily oral dose of each active ingredient when used for a given effect should range from about 0.001 mg/day to 10-5000 mg/day, preferably from about 0.01 mg/day to 1000 mg/day, and most preferably from about 0.1 mg/day to 250 mg/day. During constant-rate infusion, the most preferred intravenous dose should range from about 0.01 mg/kg/min to 10 mg/kg/min. The compounds of the present invention may be administered in a single daily dose, or divided into two, three or four doses daily.

The compounds are typically administered in the form of mixtures with suitable pharmaceutical diluents, excipients or carriers (collectively referred to herein as pharmaceutical carriers) appropriately selected according to the intended form of administration (e.g., oral tablets, capsules, elixirs and syrups) and conventional pharmaceutical practice.

A dosage form (pharmaceutical composition) suitable for administration may contain about 1 mg-2000 mg of active ingredient per dosage unit. In any of these pharmaceutical compositions, the active ingredient will generally be present in an amount of about 0.1-95% by weight based on the total weight of the composition.

The scope of the present invention includes pharmaceutical compositions comprising a therapeutically effective amount of at least one compound of the invention as an active ingredient, alone or in combination with a pharmaceutical carrier. Optionally, the compounds of the invention may be used alone, in combination with other compounds of the invention or in combination with one or more other therapeutic agents (e.g. anticancer agents or other pharmaceutically active substances).

The compounds of the invention (which may be used in a suitable hydrated form) and/or the pharmaceutical compositions of the invention are formulated into pharmaceutical dosage forms by conventional methods known to those skilled in the art, regardless of the selected route of administration.

The actual dose level of the active ingredient in a pharmaceutical composition of the present invention may be adjusted to reach an amount effective for achieving the desired therapeutic response, composition and mode of administration and ensuring nontoxicity for a particular patient.

The dosage level will be selected depending on a variety of factors, including the activity of the particular compound of the invention or its ester, salt or amide; route of administration; duration of administration; the excretion rate of the particular compound; the rate and extent of absorption; duration of treatment; other medications, compounds and/or substances used in combination with the particular compound; factors well known in the medical art such as age, sex, weight, condition, general health and previous medical history of the patient to be treated.

A physician or veterinarian of ordinary skill in the art can readily determine and prescribe an effective amount of a desired pharmaceutical composition. For example, to achieve a desired therapeutic effect, a physician or veterinarian may start with a dose level lower than the desired level for any compound of the present invention incorporated in a pharmaceutical composition, and then gradually increase the dose level until the desired effect is achieved. Typically, a suitable daily dose for a compound of the invention will be the lowest dose of the compound effective to produce a therapeutic effect. This effective dose usually depends on the factors mentioned above. Typically, oral, intravenous, intracerebroventricular and subcutaneous doses for compounds of the invention range from about 0.01 to 50 mg/kg body weight per day for use in patients. If desired, an effective daily dose of the active compound may be administered in two, three, four, five, six or more sub-doses at a suitable interval throughout the day, optionally in unit dosage form. In certain Examples of the present invention, administration is once daily.

Although the compounds of the present invention may be administered alone, they are preferably administered in the form of pharmaceutical formulations (compositions).

Kit/Product Packaging

For use in the treatment of the above indications, kit/product packaging is also described herein. A kit may consist of a delivery device, a drug pack, or a container box which may be divided into several compartments to accommodate one or more containers such as vials, tubes and the like, each containing a separate ingredient involved in a method described herein. Suitable containers include bottles, vials, syringes and test tubes. These containers are made of acceptable materials such as glass or plastic.

For example, a container may contain one or more compounds described herein, either as a pharmaceutical ingredient or in a mixture with other ingredients described herein. The container may have a sterile outlet (e.g. the container may be an intravenous infusion bag or bottle, and its stopper can be pierced by a hypodermic needle). A kit may contain a compound and a description, label or instructions for use for a method described herein.

A typical kit may comprise one or more containers, each containing one or more materials (e.g. reagents, concentrated stock solutions and/or devices) to accommodate commercial promotion and user needs for the use of the compound. These materials include, but are not limited to, buffers, diluents, filters, needles, syringes, delivery devices, bags, containers, bottles and/or test tubes, accompanied by a list of contents and/or instructions for use, and a description of built-in packaging (if any). The entire set of instructions should be included in the kit.

A label may appear on or closely associated with a container. The presence of a label on a container means that letters, numbers or other features are pasted, molded and engraved onto the container; the label may also appear inside a container box or transport box containing multiple containers, such as in a product insert. A label may be used to indicate a specific therapeutic use of the contents. The label may also carry instructions for the use of the contents, such as described in the methods above.

All features described in the specification (including any stated claim, abstract and figure) and/or all steps involved in any method or process may exist in any combination unless some features or steps are mutually exclusive in the same combination.

The above-mentioned features in the present invention or the features mentioned in Examples may be combined arbitrarily. All features disclosed in the specification may be used in conjunction with any composition form. Each feature disclosed in the specification may be replaced by any alternative feature that provides the same, equivalent or similar purpose. Therefore, unless otherwise specified, the features disclosed are only general examples of equivalent or similar features.

The present invention will be further described below in conjunction with specific Examples. It should be noted that these Examples are not intended to define the scope of the present invention but merely to describe the present invention. The experimental methods with no specific conditions indicated in the following Examples usually follow conventional conditions or the conditions recommended by manufacturers. Unless otherwise stated, all percentages, ratios, proportions or parts are measured by weight.

The unit of weight-to-volume percentage in the present invention is well known to those skilled in the art, for example, it refers to the weight of solute in 100 mL of solution. Unless otherwise defined, all technical and scientific terms used herein have the same meanings as those familiar to those skilled in the art. In addition, any methods and materials similar or equivalent to those described herein may be used in the methods of the present invention. The preferred methods and materials described herein are for exemplary purposes only.

SPECIFIC IMPLEMENTATIONS

The present invention is further described through the following specific Examples, which are not intended to limit the scope of the present invention to these Examples. Experimental methods with no specific conditions indicated in the following Examples should be selected according to conventional methods and conditions, or commercial specifications.
NMR was measured using Bruker AVANCE-400 NMR spectrometer. Solvents used for measurement were indicated in the spectrum analysis.
MS measurement was conducted using Agilent 1200-G1956A/1200-6110A/1200-6140A/1260-6125B/Prime-6125B/1260-6120 liquid chromatograph-mass spectrometer (LC/MS), SHIMADZU 20A-2010/20A-2020 LC/MS and Waters ACQ-QDA LC/MS.
HPLC analysis was performed using a SHIMADZU 20A high performance liquid chromatograph.
SFC analysis and determination were conducted using Waters UPCC with PDA Detector and QDa Detector ultra-high performance convergence chromatograph, Waters UPC$^2$ with PDA detector ultra-high performance convergence chromatograph, Agilent 1260 with DAD detector high-performance liquid chromatograph, Shimadzu LC-20AB with PDA detector high-performance liquid chromatograph and Shimadzu LC-20AD with PDA detector high-performance liquid chromatograph.
Preparative HPLC separation was conducted using Shimadzu LC-20AP pump, Shimadzu LH-40 Liquid Handler, Shimadzu SPD-20A Detector, Gilson GX-281 Liquid Handler, Gilson 322 pump and Gilson 156 UV Detector preparative chromatographs.
SFC separation was conducted using The Berger MG II, MG III, Sepiatec's Prep SFC 100 system, Waters Prep 80Q SFC SYSTEM, Prep 150 AP SFC SYSTEM, Prep 200 SFC SYSTEM and Prep 350 SFC SYSTEM.
Flash column chromatography separation was conducted using Biotage IsoleraOne flash-preparative chromatograph.
The GF254 acrylic adhesive silica gel plate manufactured by Anhui Liangchen Silicon Material Co., Ltd. was used for thin-layer chromatography (TLC). The specification of the TLC silica gel plate was 0.25 mm and that for products separated and purified by TLC was 0.5 mm.
Pressurized hydrogenation reaction was conducted using hydrogenation bottles and hydrogen gas cylinders.
Microwave reaction was conducted using Biotage Initiator + microwave synthesizer. The glove box used was customized by DELLIX.
The present invention is further described through the following Examples, which are not intended to impose any limitations on the present invention. The compounds of the present invention can be prepared by a variety of synthetic methods known to those skilled in the art, including the specific Examples listed below, Examples formed by their combination with other chemical synthesis methods and equivalent alternatives well known to those skilled in the art. Preferred Examples include, but are not limited to, the Examples of the present invention. It will be apparent to those skilled in the art that various changes and improvements can be made to these Examples without departing from the spirit and scope of the present invention.

**Example 1:**

**N-(4-(2-(8-(4,4-difluoropiperidin-1-yl)quinolin-6-yl)-2-fluorovinyl)-3-(6-azaspiro[ 2.5]octan-6-yl)phenyl)-2-hydroxyethane-1-sulfonamide**

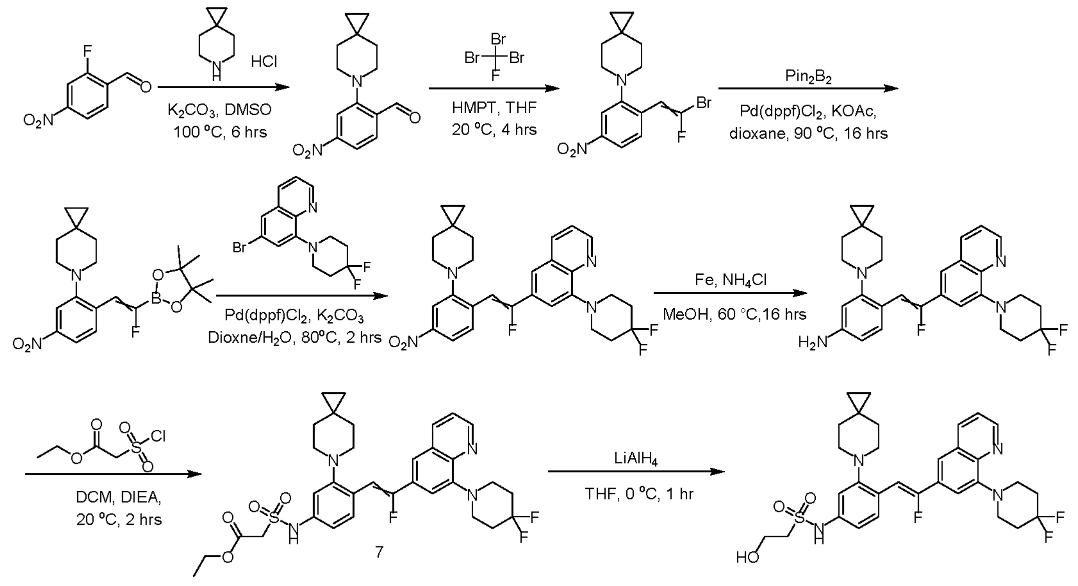


7

Step 1: To a solution of 2-fluoro-4-nitrobenzaldehyde (4.50 g, 26.6 mmol) in dimethyl sulfoxide (50 mL) were added 6-azaspiro[2.5]octane hydrochloride (4.32 g, 29.3 mmol) and potassium carbonate (11.3 g, 79.8 mmol). The mixture was stirred at 100°C for 6 h. The reaction solution was cooled to ambient temperature and extracted with water (150 mL) and ethyl acetate (100 mL×3). The combined organic layer was washed with saturated saline (150 mL×3), dried over anhydrous magnesium sulfate and filtered. The filtrate was concentrated to give a residue. The residue was purified by flash column chromatography (silica gel, 0-9% gradient of ethyl acetate/petroleum ether) to give 2-{6-azaspiro[2.5]octan-6-yl}-4-nitrobenzaldehyde as an orange solid (4.60 g, 17.7 mmol, yield: 66%). LCMS (ESI): [M+H]+ = 261.2; $^1$H NMR (400 MHz, DMSO-d6) δ ppm 10.19 (s, 1H), 7.90 (d, *J* = 2.0 Hz, 1H), 7.88 - 7.82 (m, 2H), 3.23 - 3.15 (m, 4H), 1.61 - 1.51 (m, 4H), 0.37 (s, 4H)

Step 2: To tetrahydrofuran (THF) (50 mL) were added 2-{6-azaspiro[2.5]octan-6-yl}-4-nitrobenzaldehyde (4.60 g, 17.7 mmol) and tribromofluoromethane (12.8 g, 47.3 mmol). Hexamethyltriaminophosphine (8.74 g, 53.0 mmol) was slowly added dropwise. The mixture was stirred at 20°C for 4 h. The reaction solution was poured into water (150 mL) and extracted with ethyl acetate (100 mL×2). The combined organic layer was washed with saturated saline (100 mL), dried over anhydrous magnesium sulfate and filtered. The filtrate was concentrated under reduced pressure to give a residue. The residue was purified by flash column chromatography (silica gel, 0-6% gradient of ethyl acetate/petroleum ether) to give 6-[2-(2-bromo-2-fluoroethenyl)-5-nitrophenyl]-6-azaspiro[2.5]octane as an orange solid (4.50 g, 12.7 mmol, yield: 72%). LCMS (ESI): [M+H]+ = 355.0; $^1$H NMR (400 MHz, DMSO-d6) δ ppm 7.97 - 7.87 (m, 1H), 7.81 (dd, *J* = 2.4, 8.4 Hz, 1H), 7.76 (dd, *J* = 4.0, 8.4 Hz, 1H), 6.51 - 6.32 (m, 1H), 2.98 (br d, *J* = 4.4 Hz, 4H), 1.52 (br d, *J*= 3.6 Hz, 4H), 0.36 (s, 4H)

Step 3: To a solution of 6-[2-(2-bromo-2-fluoroethenyl)-5-nitrophenyl]-6-azaspiro [2.5] octane (4.50 g, 12.7 mmol) in dioxane (50 mL) were added bis(pinacolato)diboron (4.83 g, 19.0 mmol), potassium acetate (3.77 g, 38.0 mmol) and [1,1-bis(diphenylphosphine)ferrocene] palladium dichloride (0.96 g, 1.27 mmol). The mixture was replaced with nitrogen three times, and heated to 90°C and stirred for 16 h under nitrogen atmosphere. The reaction was cooled to ambient temperature and extracted with water (50 mL) and ethyl acetate (50 mL×3). The combined organic layer was washed with saturated saline (100 mL), dried over anhydrous magnesium sulfate and filtered. The filtrate was concentrated under reduced pressure to give a residue. The residue was purified by flash column chromatography (silica gel, 0-20% gradient of ethyl acetate/petroleum ether) to give 6-{2-[2-fluoro-2-(4,4,5,5-tetramethyl-1,3,2-dioxaborolan-2-yl)vinyl]-5-nitrophenyl }-6 -azaspiro[2.5]octane as an orange solid (2.10 g, 5.22 mmol, yield: 39%). LCMS (ESI): [M+H]+ = 320.9; $^1$H NMR (400 MHz, DMSO-d6) δ ppm 7.94 - 7.87 (m, 2H), 7.82 (d, *J* = 2.0 Hz, 1H), 6.75 - 6.57 (m, 1H), 2.99 (br t, *J* = 5.2 Hz, 4H), 1.51 (br s, 4H), 1.28 (s, 12H), 0.37 (s, 4H)

Step 4: To dioxane (40 mL)/water (8 mL) were added 6-bromo-8-(4,4-difluoropiperidin-1-yl)quinoline (1.14 g, 3.48 mmol) and 6-{2-[2-fluoro-2-(4,4,5,5-tetramethyl-1,3,2-dioxaborolan-2-yl)ethenyl]-5-nitrophenyl} -6-azaspiro[2.5]octane (2.10 g, 5.22 mmol), and then potassium carbonate (0.98 g, 6.96 mmol) and [1,1-bis(diphenylphosphine) ferrocene] palladium dichloride (0.26 g, 0.35 mmol). The mixture was replaced with nitrogen three times, and heated to 80°C and stirred for 2 h under nitrogenatmosphere. The reaction solution was cooled to ambient temperature and filtered. The filter cake was washed with dioxane (50 mL)/water (10 mL) to give 6-[2-(2-{6-azaspiro[2.5]octan-6-yl}-4-nitrophenyl)-1-fluoroethenyl]-8-(4,4-difluoropi peridin-1-yl)quinoline as a yellow solid (1.10 g, 2.10 mmol, yield: 60%). LCMS (ESI): [M+H]+ = 523.1; $^1$H NMR (400 MHz, $CDCl_3$) δ ppm 8.94 (br d, *J* = 3.2 Hz, 1H), 8.21 (br d, *J* = 8.0 Hz, 1H),

8.08 (br d, $J$ = 9.2 Hz, 1H), 8.01 - 7.91 (m, 2H), 7.81 (s, 1H), 7.50 - 7.40 (m, 2H), 7.01 - 6.85 (m, 1H), 3.63 - 3.55 (m, 4H), 3.17 - 3.06 (m, 4H), 2.42 - 2.31 (m, 4H), 1.71 - 1.61 (m, 4H), 0.43 (s, 4H)

Step 5: To a solution of 6-[2-(2-{6-azaspiro[2.5]octan-6-yl}-4-nitrophenyl)-1-fluoroethenyl]-8-(4,4-difluoropi peridin-1-yl)quinoline (1.10 g, 2.10 mmol) in methanol (18 mL)/saturated aqueous ammonium chloride solution (6 mL) was added iron powder (1.19 g, 21.0 mmol). The mixture was stirred at 60°C for 16 h. The hot reaction solution was filtered under reduced pressureand extracted with water (50 mL) and ethyl acetate (50 mL×3). The combined organic layer was washed with saturated saline (50 mL), dried over anhydrous magnesium sulfate and filtered. The filtrate was concentrated under reduced pressure to give 3-{6-azaspiro[2.5]octan-6-yl}-4-{2-[8-(4,4-difluoropiperidin-1-yl)quinolin-6-yl]-2-flu oroethenyl }aniline as a green solid (0.25 g, crude). LCMS (ESI): [M+H]+ = 493.3

Step 6: To a solution of 3-{6-azaspiro[2.5]octan-6-yl}-4-{2-[8-(4,4-difluoropiperidin-l-yl)quinolin-6-yl]-2-flu oroethenyl } aniline (250 mg, 0.51 mmol) and N,N-diisopropylethylamine (134 mg, 1.02 mmol) in dichloromethane (5 mL) was slowly added ethyl 2-(chlorosulfonyl)acetate (114 mg, 0.61 mmol) dropwise. The mixture was stirred at 20°C for 2 h. The reaction solution was extracted with water (20 mL) and ethyl acetate (20 mL×3). The organic phases were combined, washed with saturated saline (20 mL), dried over anhydrous magnesium sulfate and filtered. The filtrate was concentrated under reduced pressure to give a residue. The residue was purified by flash column chromatography (silica gel, 0-33% gradient of ethyl acetate/petroleum ether) to give ethyl 2-[(3-{6-azaspiro[2.5]octan-6-yl}-4-{2-[8-(4,4-difluoropiperidin-1-yl)quinolin-6-yl]-2 -fluoroethenyl}phenyl)sulfamoyl]acetate as a yellow solid (150 mg, 0.24 mmol, yield: 46%). LCMS (ESI): [M+H]+ = 643.2

Step 7: To a solution of THF (3 mL) was added ethyl 2-[(3-{6-azaspiro[2.5]octan-6-yl}-4-{2-[8-(4,4-difluoropiperidin-1-yl)quinolin-6-yl]-2 -fluoroethenyl}phenyl)sulfamoyl]acetate (150 mg, 0.24 mmol). Lithium tetrahydridoaluminate (12.8 mg, 0.36 mmol) was added portionwise at 0°C. The mixture was stirred at 0°C for 1 h. The reaction solution was quenched with water (0.02 mL) and 10% aqueous sodium hydroxide solution (0.04 mL), and filtered. The filtrate was concentrated under reduced pressure to give a residue. The residue was purified by preparative HPLC (C18, 48-88% gradient of water (formic acid)/acetonitrile) to give N-(4-(2-(8-(4,4-difluoropiperidin-1-yl)quinolin-6-yl)-2-fluorovinyl)-3-(6-azaspiro[2.5] octan-6-yl)phenyl)-2-hydroxyethane-1-sulfonamide as a yellow solid compound (11.4 mg, 0.02 mmol, yield: 8%). LCMS (ESI): [M+H]+ = 601.6; $^1$H NMR (400 MHz, DMSO-d6) δ ppm 9.79 (s, 1H), 8.88 (br s, 1H), 8.42 (br d, $J$ = 8.0 Hz, 1H), 7.92 - 7.69 (m, 2H), 7.57 (br dd, $J$ = 4.0, 8.0 Hz, 1H), 7.35 (br s, 1H), 7.01 (br s, 1H), 6.98 - 6.78 (m, 2H), 4.98 (s, 1H), 3.75 (br t, $J$ = 6.4 Hz, 2H), 3.60 - 3.50 (m, 4H), 3.28 (br s, 2H), 3.02 - 2.84 (m, 4H), 2.31 - 2.15 (m, 4H), 1.70 - 1.44 (m, 4H), 0.36 (s, 4H)

**Example 2:**

**(Z)-N-(4-(2-(8-(4,4-difluoropiperidin-1-yl)quinolin-6-yl)-1-fluorovinyl)-3-(6-azasp iro[2.5]octan-6-yl)phenyl)-2-hydroxyethane-1-sulfonamide**

**Example 3:**

**(E)-N-(4-(2-(8-(4,4-difluoropiperidin-1-yl)quinolin-6-yl)-1-fluorovinyl)-3-(6-azasp iro[2.5]octan-6-yl)phenyl)-2-hydroxyethane-1-sulfonamide**

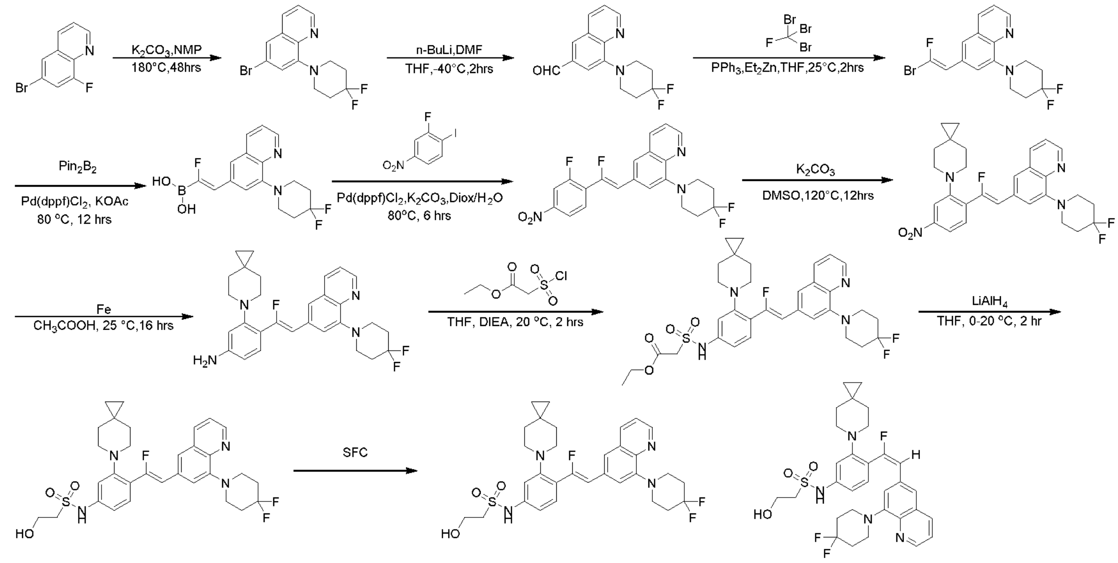


Step 1: To a solution of 6-bromo-8-fluoroquinoline (5.00 g, 22.1 mmol) in N-methylpyrrolidone (50 mL) were added 4,4-difluoropiperidine hydrochloride (5.23 g, 33.2 mmol) and potassium carbonate (9.17 g, 66.4 mmol). The mixture was stirred at 180°C for 48 h. The reaction solution was cooled to ambient temperature and extracted with water (100 mL) and ethyl acetate (300 mL×3). The organic phases were combined, washed with saturated saline (200 mL), dried over anhydrous magnesium sulfate and filtered. The filtrate was concentrated under reduced pressure to give a residue. The residue was purified by flash column chromatography (silica gel, 0-20% gradient of ethyl acetate/petroleum ether) to give 6-bromo-8-(4,4-difluoropiperidin-1-yl)quinoline as a colorless oil (6.80 g, 20.8 mmol, 94%: yield). LCMS (ESI): [M+H]+ = 326.9

Step 2: To a solution of 6-bromo-8-(4,4-difluoropiperidin-1-yl)quinoline (5.00 g, 15.3 mmol) in THF (100 mL) was added n-butyllithium (9.17 mL, 22.9 mmol) dropwise at -40°C. The temperature was kept below -40°C during the addition. The mixture was stirred at -40°C for 0.5 h. N,N-dimethylformamide (10.00 mL) was added dropwise to the reaction solution at this temperature. The solution was stirred at this temperature for 2 h. To the reaction solution, water (200 mL) and ethyl acetate (300 mL×3) were added for extraction. The organic phases were combined, washed with saturated saline (200 mL), dried over anhydrous magnesium sulfate and filtered. The filtrate was concentrated under reduced pressure to give a residue. The residue was purified by flash column chromatography (silica gel, 0-8% gradient of THF/petroleum ether) to give 8-(4,4-difluoropiperidin-1-yl)quinoline-6-carbaldehyde as a yellow solid (1.60 g, 5.81 mmol, yield: 38%). LCMS (ESI): [M+H]+ = 277.0; $^1$H NMR (400 MHz, DMSO-d6) $\delta$ ppm 10.12 (s, 1H), 9.04 (dd, *J* = 2.0, 4.4 Hz, 1H), 8.57 (dd, *J* = 2.0, 8.4 Hz, 1H), 8.24 (d, *J* = 1.2 Hz, 1H), 7.67 (dd, *J* = 4.0, 8.0 Hz, 1H), 7.52 (d, *J* = 1.2 Hz, 1H), 3.53 (br t, *J* = 5.2 Hz, 4H), 2.28 - 2.13 (m, 4H)

Step 3: To a solution of THF (100 mL) were added 8-(4,4-difluoropiperidin-1-yl)quinoline-6-carbaldehyde (5.00 g, 18.1 mmol) and tribromofluoromethane (14.7 g, 54.2 mmol) at 0°C. Then, triphenylphosphine (14.2 g, 54.2 mmol) was slowly added, and diethylzinc (54.2 mL, 54.2 mmol) solution was added dropwise. The mixture was stirred at 0-25°C for 2 h. The reaction solution was poured into water (150 mL) and extracted with ethyl acetate (100 mL×2). The combined organic layer was washed with saturated saline (100 mL), dried over anhydrous magnesium sulfate and filtered. The filtrate was concentrated under reduced pressure to give a residue. The residue was purified by flash column chromatography (silica gel, 0-15% gradient of ethyl acetate/petroleum ether) to give 6-[(1E)-2-bromo-2-fluoroethenyl]-8-(4,4-difluoropiperidin-1-yl)quinoline as an orange solid (3.60 g, 9.77 mmol, yield: 54%). LCMS (ESI): [M+H]+ = 372.8

Step 4: To a solution of 6-[(1E)-2-bromo-2-fluoroethenyl]-8-(4,4-difluoropiperidin-1-yl)quinoline (3.60 g, 9.70 mmol) in dioxane (50 mL) were added bis(pinacolato)diboron (3.69 g, 14.5 mmol), potassium acetate (2.88 g, 29.1 mmol) and [1,1-bis(diphenylphosphine)ferrocene]palladium dichloride (0.73 g, 0.97 mmol). The mixture was replaced with nitrogen three times, and heated to 80°C and stirred for 12 h under nitrogen atmosphere. The reaction solution was cooled to ambient temperature and extracted with water (150 mL) and ethyl acetate (200 mL × 3). The combined organic layer was washed with saturated saline (100 mL), dried over anhydrous magnesium sulfate and filtered. The filtrate was concentrated under reduced pressure to give a residue. The residue was purified by flash column chromatography (silica gel, 0-15% gradient of ethyl acetate/petroleum ether) to give [(1Z)-2-[8-(4,4-difluoropiperidin-1-yl)quinolin-6-yl]-1-fluoroethenyl]boronic acid as an orange solid (1.90 g, 5.63 mmol, yield: 58%). LCMS (ESI): [M+H]+ = 337.1

Step 5: To dioxane (40 mL)/water (8 mL) were added 2-fluoro-1-iodo-4-nitrobenzene (2.14 g, 8.03 mmol) and

[(1Z)-2-[8-(4,4-difluoropiperidin-1-yl)quinolin-6-yl]-1-fluoroethenyl]boric acid (1.80 g, 5.36 mmol). Then, potassium carbonate (2.22 g, 16.1 mmol) and [1,1-bis(diphenylphosphine)ferrocene]palladium dichloride (0.41 g, 0.54 mmol) were added. The mixture was replaced with nitrogen three times, and heated to 80°C and stirred for 6 h under nitrogen atmosphere. The reaction solution was cooled to ambient temperature and extracted with water (150 mL) and ethyl acetate (200 mL × 3). The combined organic layer was washed with saturated saline (100 mL), dried over anhydrous magnesium sulfate and filtered. The filtrate was concentrated under reduced pressure to give a residue. The residue was purified by flash column chromatography (silica gel, 0-15% gradient of ethyl acetate/petroleum ether) to give 6-[2-(2-{6-azaspiro[2.5]octan-6-yl}-4-nitrophenyl)-1-fluoroethenyl]-8-(4,4-difluoropi peridin-1-yl)quinoline as a yellow solid (0.83 g, 1.93 mmol, yield: 36%). LCMS (ESI): [M+H]+ = 432.1

Step 6: To a solution of 6-[2-(2-{6-azaspiro[2.5]octan-6-yl}-4-nitrophenyl)-1-fluoroethenyl]-8-(4,4-difluoropi peridin-1-yl)quinoline (400 mg, 0.93 mmol) in dimethyl sulfoxide (8 mL) were added 6-azaspiro[2.5]octane hydrochloride (207 mg, 1.39 mmol) and potassium carbonate (384 mg, 2.78 mmol). The mixture was stirred at 120°C for 12 h. The reaction solution was cooled to ambient temperature and extracted with water (20 mL) and ethyl acetate (30 mL×3). The organic phases were combined, washed with saturated saline (20 mL), dried over anhydrous magnesium sulfate and filtered. The filtrate was concentrated under reduced pressure to give a residue. The residue was purified by flash column chromatography (silica gel, 0-8% gradient of THF/petroleum ether) to give 6-[(1Z)-2-(2-{6-azaspiro[2.5]octan-6-yl}-4-nitrophenyl)-2-fluoroethenyl]-8-(4,4-diflu oropiperidin-1-yl)quinoline as a yellow solid (0.34 g, 0.65 mmol, yield: 70%). LCMS (ESI): [M+H]+ = 523.1

Step 7: To a solution of 6-[(1Z)-2-(2-{6-azaspiro[2.5]octan-6-yl}-4-nitrophenyl)-2-fluoroethenyl]-8-(4,4-diflu oropiperidin-1-yl)quinoline (280 mg, 0.54 mmol) in acetic acid (6 mL) was added iron powder (302 mg, 5.36 mmol). The mixture was stirred at 25°C for 16 h. The hot reaction solution was filtered under reduced pressure and extracted with water (20 mL) and ethyl acetate (20 mL×3). The combined organic layer was washed with saturated saline (20 mL), dried over anhydrous magnesium sulfate and filtered. The filtrate was concentrated under reduced pressure to give a residue. The residue was purified by flash column chromatography (silica gel, 0-30% gradient of THF/petroleum ether) to give 3-{6-azaspiro[2.5]octan-6-yl}-4-[(1Z)-2-[8-(4,4-difluoropiperidin-1-yl)quinolin-6-yl]-1-fluoroethenyl]aniline as a yellow solid (67.0 mg, 0.14 mmol, yield: 25%). LCMS (ESI): [M+H]+ = 493.1

Step 8: To a solution of 3-{6-azaspiro[2.5]octan-6-yl}-4-[(1Z)-2-[8-(4,4-difluoropiperidin-1-yl)quinolin-6-yl]-1-fluoroethenyl]aniline (150 mg, 0.30 mmol) and N,N-diisopropylethylamine (80.3 mg, 0.61 mmol) in THF (4 mL) was slowly added ethyl 2-(chlorosulfonyl)acetate (68.2 mg, 0.37 mmol) dropwise. The mixture was stirred at 20°C for 2 h. The reaction solution was extracted with water (20 mL) and ethyl acetate (20 mL×3). The combined organic layer was washed with saturated saline (20 mL), dried over anhydrous magnesium sulfate and filtered. The filtrate was concentrated under reduced pressure to give a residue. The residue was purified by flash column chromatography (silica gel, 0-30% gradient of THF/petroleum ether) to give ethyl 2-{[(4-{(1Z)-2-[8-(4,4-difluoropiperidinyl)(6-quinolinyl)]-1-fluoroethenyl}-3-(6-azas piro[2.5]oct-6-yl)phenyl)amino]sulfonyl}acetate as a yellow solid (120 mg, 0.18 mmol, yield: 61%). LCMS (ESI): [M+H]+ = 643.1

Step 9: To a solution of THF (3 mL) was added ethyl 2-{[(4-{(1Z)-2-[8-(4,4-difluoropiperidinyl)(6-quinolinyl)]-1-fluoroethenyl}-3-(6-azas piro[2.5]oct-6-yl)phenyl)amino]sulfonyl}acetate (100 mg, 0.16 mmol). Then, lithium tetrahydridoaluminate (8.33 mg, 0.23 mmol) was added portionwise at 0°C. The mixture was stirred at 0°C for 2 h. The reaction solution was quenched with water (0.02 mL) and 10% aqueous sodium hydroxide solution (0.04 mL), and filtered. The filtrate was concentrated under reduced pressure to give a residue. The residue was purified by preparative HPLC (C18, 46-86% gradient of water (ammonium bicarbonate)/acetonitrile), lyophilized to give a white mixture, and separated by chiral SFC (IG, system: carbon dioxide/ethanol (0.1% ammonia water) to give (Z)-N-(4-(2-(8-(4,4-difluoropiperidin-1-yl)quinolin-6-yl)-1-fluorovinyl)-3-(6-azaspiro [2.5]octan-6-yl)phenyl)-2-hydroxyethane-1-sulfonamide (12.0 mg, 0.02 mmol, yield: 13%) (**Example 2**) and (E)-N-(4-(2-(8-(4,4-difluoropiperidin-1-yl)quinolin-6-yl)-1-fluorovinyl)-3-(6-azaspiro [2.5]octan-6-yl)phenyl)-2-hydroxyethane-1-sulfonamide (5.00 mg, 0.008 mmol, yield: 5%) (**Example 3**).

**Example 2:** LCMS (ESI): [M+H]+ = 601.2; $^1$H NMR (400 MHz, DMSO-d6) δ ppm 9.98 (s, 1H), 8.77 (dd, *J* = 1.6, 4.0 Hz, 1H), 8.13 (dd, *J* = 1.6, 8.4 Hz, 1H), 7.44 (dd, *J* = 4.0, 8.4 Hz, 1H), 7.33 (br dd, *J* = 0.8, 8.8 Hz, 1H), 7.30 (br s, 1H), 6.93 - 6.86 (m, 2H), 6.71 (d, *J* = 1.6 Hz, 1H), 6.67 - 6.59 (m, 1H), 4.99 (br s, 1H), 3.81 - 3.75 (m, 2H), 3.32 - 3.30 (m, 2H), 3.13 (br s, 4H), 2.87 (br s, 4H), 2.13 - 2.00 (m, 4H), 1.03 (br s, 4H), 0.16 (s, 4H)

**Example 3:** LCMS (ESI): [M+H]+ = 601.2; $^1$H NMR (400 MHz, DMSO-d6) δ ppm 9.98 (s, 1H), 8.85 (br d, *J* = 3.2 Hz, 1H), 8.35 (br d, *J* = 8.0 Hz, 1H), 7.75 (s, 1H), 7.51 (br d, *J* = 8.0 Hz, 2H), 7.44 (s, 1H), 7.09 - 6.86 (m, 3H), 4.96 (br t, *J* = 5.6 Hz, 1H), 3.76 (q, *J* = 6.0 Hz, 2H), 3.50 (br s, 4H), 3.31 - 3.27 (m, 2H), 3.04 (br s, 4H), 2.31 - 2.15 (m, 4H), 1.52 (br s, 4H), 0.34 (s, 4H)

**Example 4:**

**N-(4-(5-(2-(4,4-difluoropiperidin-1-yl)-6-methylpyrimidin-4-yl)-4H-1,2,4-triazol-3-yl)-3-(6-azaspiro[2.5]octan-6-yl) phenyl)-2-hydroxyethane-1-sulfonamide**

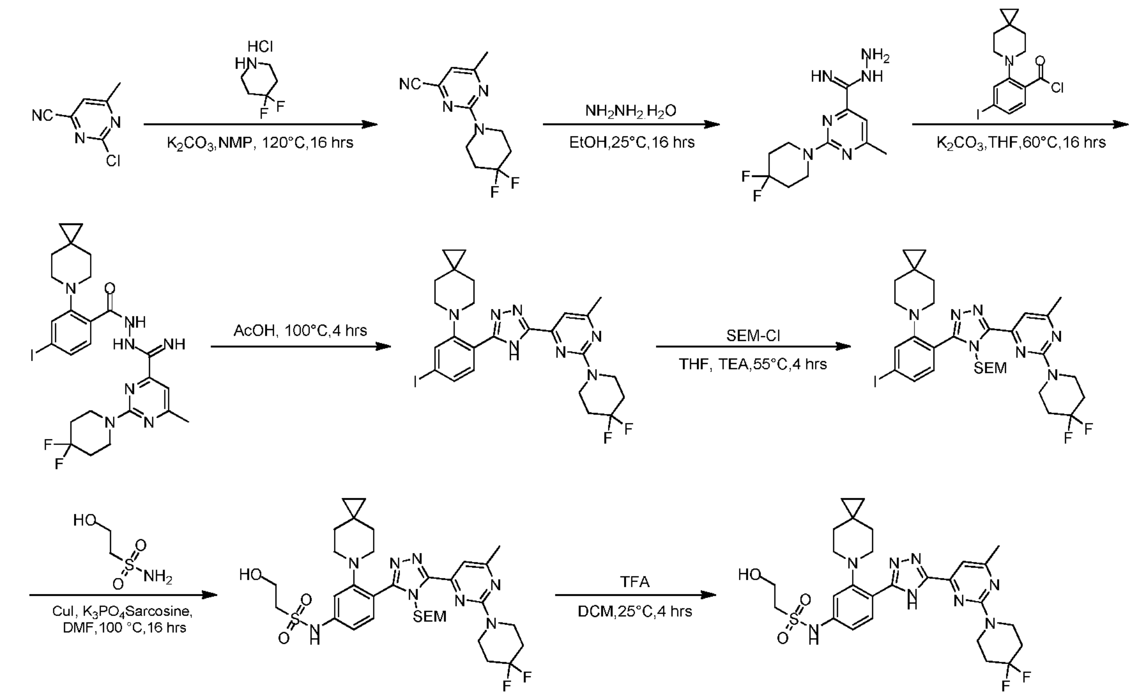


Step 1: To a solution of 2-chloro-6-methylpyrimidin-4-carbonitrile (1.80 g, 11.7 mmol) in 1-methyl-2-pyrrolidone (30 mL) were added 4,4-difluoropiperidine hydrochloride (2.77 g, 17.5 mmol) and potassium carbonate (4.96 g, 35.1 mmol). The mixture was heated to 120°C and stirred for 16 h. The reaction solution was diluted with water (50 mL) and extracted with dichloromethane (50 mL×3). The combined organic layer was dried over anhydrous sodium sulfate, filtered and concentrated under reduced pressure to give a residue. The residue was purified by flash silica gel chromatography (silica gel, 0-6% gradient of ethyl acetate/petroleum ether) to give 2-(4,4-difluoropiperidin-1-yl)-6-methylpyrimidin-4-carbonitrile as a white solid (1.60 g, 11.7 mmol, yield: 57.3%). LCMS (ESI): [M+H]+ = 239.0

Step 2: To a solution of 2-(4,4-difluoropiperidin-1-yl)-6-methylpyrimidin-4-carbonitrile (1.50 g, 6.30 mmol) in ethanol (20.0 mL) was added hydrazine hydrate (1.97 g, 31.48 mmol). The mixture was stirred at 25°C for 16 h. The reaction solution was concentrated under reduced pressure to give 2-(4,4-difluoropiperidin-1-yl)-6-methylpyrimidin-4-carboximidohydrazide as a white solid (1.60 g, 6.29 mmol, yield: 94.0%). LCMS (ESI): $[M+H]^+$ = 271.1

Step 3: To a solution of 2-(4,4-difluoropiperidin-1-yl)-6-methylpyrimidin-4-carboximidohydrazide (0.50 g, 1.85 mmol) in THF (5.00 mL) were added 4-iodo-2-(6-azaspiro[2.5]octane-6-yl)benzoyl chloride (1.04 g, 2.77 mmol) and N,N-diisopropylethylamine (0.73 g, 5.55 mmol). The mixture was heated to 60°C and stirred for 16 h. The reaction solution was concentrated under reduced pressure to give N'-((2-(4,4-difluoropiperidin-1-yl)-6-methylpyrimidin-4-yl)(imino) methyl)-4-iodo-2-( 6-azaspiro[2.5]octan-6-yl)benzohydrazide as a white solid (1.2 g, 1.84 mmol, crude). LCMS (ESI): $[M+H]^+$ = 610.1

Step 4: To a solution of acetic acid (20.0 mL) was added N'-((2-(4,4-difluoropiperidin-1-yl)-6-methylpyrimidin-4-yl)(amino)methyl)-4-iodo-2-(6-azaspiro[2.5]octan-6-yl)benzohydrazide (1.10 g, 1.80 mmol). The mixture was heated to 100°C. and stirred for 4 hours. Water (10 mL) was added to the reaction solution. Th solution was extracted with dichloromethane (10 mLx3). The combined organic layer was dried over anhydrous sodium sulfate, filtered and concentrated under reduced pressure to give a residue. The residue was purified by flash silica gel chromatography (silica gel, 0-7% ethyl acetate/petroleum ether) to give 6-(2-(5-(2-(4,4-difluoropiperidin-1-yl)-6-methylpyrimidin-4-yl)-4H-1,2,4-triazol-3-yl) -5-iodophenyl)-6-azaspiro[2.5]octane as a white solid (0.56 g, 1.80 mmol, yield: 54.3%). LCMS (ESI): $[M+H]^+$ = 592.2

Step 5: To a solution of 6-(2-(5-(2-(4,4-difluoropiperidin-1-yl)-6-methylpyrimidin-4-yl)-4H-1,2,4-triazol-3-yl) -5-iodophenyl)-6-azaspiro[2.5]octane (1.35 g, 2.28 mmol) in THF (20.0 mL) were added triethylamine (0.71 g, 6.85 mmol) and (2-(chloromethoxy)ethyl)trimethylsilane (0.46 g, 2.74 mmol). The mixture was heated to 55°C and stirred for 4 h, and water (50 mL) was added to the reaction solution, which was extracted with dichloromethane (50 mL × 3). The combined organic layer was dried over anhydrous sodium sulfate, filtered and concentrated under reduced pressure to give a residue. The residue was purified by flash column chromatography (silica gel, 0-6% ethyl acetate/petroleum ether) to give 6-(2-(5-(2-(4,4-difluoropiperidin-1-yl)-6-methylpyrimidin-4-yl)-4-((2-(trimethylsilyl) ethoxy)methyl)-4H-1,2,4-triazol-3-yl)-5-iodophenyl)-6-azaspiro[2.5]octane (0.43 g, 2.28 mmol, yield: 26.1%) as a colorless and transparent oil. LCMS (ESI): $[M+H]^+$ = 722.3

Step 6: To a solution of N,N-dimethylformamide (4.00 mL) were added 2-hydroxyethyl sulfonamide (103 mg, 0.83 mmol) and sarcosine (56.8 mg, 0.63 mmol), then, cuprous iodide (61.9 mg, 0.32 mmol) and potassium phosphate (552 mg, 2.54 mmol) were added at 25°C, and the mixture was stirred at 50°C for 5 min. Then, 6-(2-(5-(2-(4,4-difluoropiperidin-1-yl)-6-methylpyrimidin-4-yl)-4-((2-(trimethylsilyl) ethoxy)methyl)-4H-1,2,4-triazol-3-yl)-5-iodophenyl)-6-azaspiro[2.5]octane (460 mg, 0.63 mmol) was added portionwise and the mixture was stirred at 100°C for 16 h. Water (20 mL) was added to the reaction mixture, which was extracted with ethyl acetate (30 mL × 2), the organic phases were combined and washed twice with saline, dried over anhydrous magnesium sulfate, filtered, and the filtrate was concentrated under reduced pressure to give N-(4-(5-(2-(4,4-difluoropiperidin-1-yl)-6-methylpyrimidin-4-yl)-4-((2-(trimethylsilyl) ethoxy)methyl)-4H-1,2,4-triazol-3-yl)-3-(6-azaspiro[2.5]octan-6-yl)phenyl)-1-yl-2-hy droxyethanesulfonamide (0.35 g, 0.63 mmol, crude) as a yellow oil. LCMS (ESI): $[M+H]^+$ = 719.7

Step 7: N-(4-(5-(2-(4,4-difluoropiperidin-1-yl)-6-methylpyrimidin-4-yl)-4-((2-(trimethylsilyl) ethoxy)methyl)-4H-1,2,4-triazol-3-yl)-3-(6-azaspiro[2.5]octan-6-yl)phenyl)-1-yl-2-hy droxyethanesulfonamide (0.35 g, 0.63 mmol) was added to a mixture of trifluoroacetic acid (2.00 mL) and dichloromethane (2.00 mL), and the mixture was stirred at 25°C for 4 h. The reaction mixture was extracted with water (20 mL) and ethyl acetate (60 mL × 2). The organic phases were combined and washed twice with saline, dried over anhydrous magnesium sulfate, and filtered. The filtrate was concentrated under reduced pressure to give the residue. The residue was purified by preparative HPLC (C18, 18-58% gradient of water (formic acid)/acetonitrile) to give N-(4-(5-(2-(4,4-difluoropiperidin-1-yl)-6-methylpyrimidin-4-yl)-4H-1,2,4-triazol-3-yl) -3-(6-azaspiro[2.5]octan-6-yl)phenyl)-2-hydroxyethane-1-sulfonamide (92 mg, 0.59 mmol, yield: 26%) as a white solid. LCMS (ESI): $[M+H]^+$ = 589.5. $^1$H NMR (400 MHz, DMSO-d6) δ ppm 14.19 (s, 1H), 9.99 (s, 1H), 7.72 (br d, J = 7.5 Hz, 1H), 7.23 (s, 1H), 7.18 - 6.78 (m, 2H), 5.54 - 4.38 (m, 1H), 4.01 (br s, 4H), 3.76 (t, J = 6.6 Hz, 2H), 3.30 (br s, 2H), 2.99 - 2.76 (m, 4H), 2.39 (s, 3H), 2.10 - 1.92 (m, 4H), 1.62-1.32 (m, 4H), 0.40 - 0.18 (m, 4H)

**Example 5:**

**N-(4-(3-(2-(4,4-difluoropiperidin-1-yl)-6-methylpyrimidin-4-yl)isoxazol-5-yl)-3-(6 -azaspiro[2.5]octan-6-yl)phenyl)-2-hydroxyethane-1-sulfonamide**

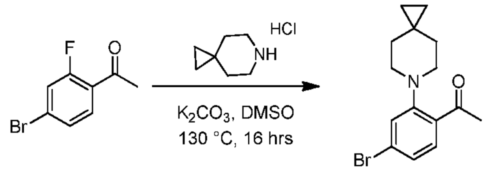


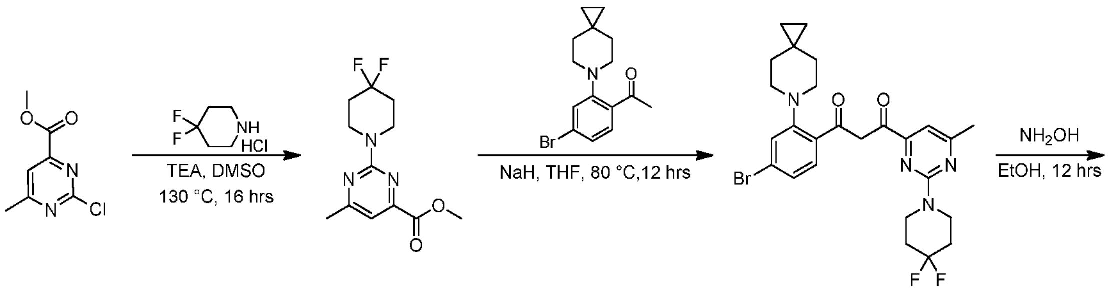


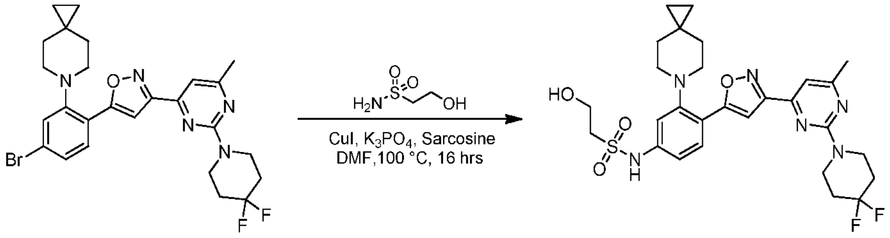


Step 1: To a solution of 1-(4-bromo-2-fluorophenyl)ethanone (5.00 g, 23.0 mmol) and potassium carbonate (9.55 g, 69.1 mmol) in DMSO (50 mL) was added 6-azaspiro[2.5]octane hydrochloride (3.40 g, 23.0 mmol), and the mixture was stirred at 130°C for 16 h. The reaction mixture was filtered and poured into water (300 mL). The mixture was extracted with ethyl acetate (400 mL × 2), and the combined organic layer was washed with saturated saline (250 mL), dried over anhydrous magnesium sulfate, filtered, and concentrated under reduced pressure to give the residue. The residue was purified by flash column chromatography (silica gel, 0-15% gradient of ethyl acetate/petroleum ether) to give 1-(4-bromo-2-(6-azaspiro[2.5]octan-6-yl)phenyl)ethanone (6.60 g, 21.4 mmol, yield: 93%) as a yellow oil. LCMS (ESI): [M+H]+ = 309.9.

Step 2: To a solution of methyl 2-chloro-6-methylpyrimidin-4-carboxylate (5.00 g, 26.8 mmol) and triethylamine (8.13 g, 80.4 mmol) in DMSO (50 mL) was added 4,4-difluoropiperidine hydrochloride (4.22 g, 26.8 mmol), and the mixture was stirred at 130°C for 16 h. The reaction mixture was poured into water (300 mL). The mixture was extracted with ethyl acetate (400 mL × 2), and the combined organic layer was washed with saturated saline (250 mL), dried over anhydrous magnesium sulfate, filtered, and concentrated under reduced pressure to give the residue. The residue was purified by flash column chromatography (silica gel, 0-15% gradient of ethyl acetate/petroleum ether) to give methyl 2-(4,4-difluoropiperidin-1-yl)-6-methylpyrimidin-4-carboxylate (2.70 g, 9.92 mmol, yield: 37%) as a white solid. LCMS (ESI): $[M+H]^+$ = 272.0

Step 3: Sodium hydrogen (0.39 g, 9.73 mmol, 60% purity) was added to a solution of 1-acetyl-2-(6-azaspiro[2.5]oct-6-yl)-4-bromobenzene (2.00 g, 6.49 mmol) in THF (40 mL) under nitrogen protection, and the mixture was stirred at 25°C for 15 min. Then, another starting material methyl 2-(4,4-difluoropiperidin-1-yl)-6-methylpyrimidin-4-carboxylate (1.76 g, 6.49 mmol) was added, and the reaction solution was stirred at 80°C for 12 h. The reaction mixture was filtered, poured into water (80 mL), and extracted with methyl tert-butyl ether (100 mL × 3), and the organic phases were separated and combined, washed with saturated saline (40 mL), dried over anhydrous magnesium sulfate, filtered, and concentrated under reduced pressure to give the residue. The residue was purified by flash column chromatography (silica gel, 0-15% gradient of ethyl acetate/petroleum ether) to give 1-(4-bromo-2-(6-azaspiro[2.5]octan-6-yl)phenyl)-3-(2-(4,4-difluoropiperidin-1-yl)-6-methylpyrimidin-4-yl)propane-1,3-dione (2.40 g, 4.41 mmol, yield: 68%) as a yellow solid. LCMS (ESI): $[M+H]^+$ = 548.8.

Step 4: Hydroxylamine hydrochloride (0.25 g, 3.65 mmol) was added to a solution of 1-(4-bromo-2-(6-azaspiro[2.5]octan-6-yl)phenyl)-3-(2-(4,4-difluoropiperidin-1-yl)-6-methylpyrimidin-4-yl)propane-1,3-dione (1.00 g, 1.83 mmol) in ethanol (20 mL) at 25°C, and the reaction mixture was stirred at 100°C for 12 h. The reaction solution was slowly added into water (50 mL) dropwise, and the precipitate was separated. The cake was collected by filtration with a sand core funnel, and dried with an oil pump to give the crude. The crude was purified by chiral separation to give 5-(4-bromo-2-(6-azaspiro[2.5]octan-6-yl)phenyl)-3-(2-(4,4-difluoropiperidin-1-yl)-6-methylpyrimidin-4-yl)isoxazole (0.46 g, 0.84 mmol, yield: 46%)

as a white solid compound. LCMS (ESI): [M+H]+ =545.8.

Step 5: Cuprous iodide (35.0 mg, 0.18 mmol) and potassium phosphate (312 mg, 1.47 mmol) were added to a solution of 2-hydroxyethanesulfonamide (55.2 mg, 0.44 mmol) and 2-(methylamino)acetic acid (32.7 mg, 0.37 mmol) in N,N-dimethylformamide (4 mL) at 25 °C. The reaction mixture was heated to 50°C under nitrogen protection and stirred for 5 min, then 5-(4-bromo-2-(6-azaspiro[2.5]octan-6-yl)phenyl)-3-(2-(4,4-difluoropiperidin-1-yl)-6-methylpyrimidin-4-yl)isoxazole (200 mg, 0.37 mmol) was added at 50°C, the nitrogen was replaced again, and finally the reaction solution was stirred at 100°C for 16 h. The reaction mixture was concentrated under reduced pressure, and the residue was diluted with N,N-dimethylformamide (10 mL). The diluent was purified by preparative HPLC (C18, 48-88% gradient of water (formic acid)/acetonitrile) to give N-(4-(3-(2-(4,4-difluoropiperidin-1-yl)-6-methylpyrimidin-4-yl)isoxazol-5-yl)-3-(6-az aspiro[2.5]octan-6-yl)phenyl)-2-hydroxyethane-1-sulfonamide (120 mg, 0.20 mmol. yield: 55%) as a white solid compound. LCMS (ESI): $[M+H]^+$ =589.5. $^1H$ NMR (400 MHz, DMSO-d6) δ ppm 10.11 (s, 1H), 7.82 (d, J = 8.5 Hz, 1H), 7.69 (s, 1H), 7.16 - 7.15 (m, 2H), 7.04 (dd, J = 1.9, 8.5 Hz, 1H), 4.94(br s, 1H), 4.08 - 3.91 (m, 4H), 3.76 (br t, J = 6.4 Hz, 2H), 3.16- 3.05(m, 2H), 2.95 - 2.81 (m, 4H), 2.42 (s, 3H), 2.14 - 1.93 (m, 4H), 1.69 - 1.44 (m, 4H), 0.36 (s, 4H)

**Example 6:**

**N-(4-(5-(2-(4,4-difluoropiperidin-1-yl)-6-methylpyrimidin-4-yl)-1H-pyrazol-3-yl)-3-(6-azaspiro[2.5]octan-6-yl)phenyl)-2-hydroxyethane-1-sulfonamide**

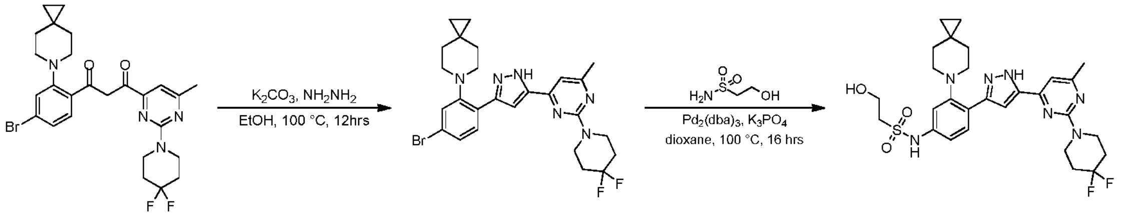


Step 1: Hydrazine monohydrochloride (93.9 mg, 1.37 mmol) and potassium carbonate (190 mg, 2.74 mmol) were added to a solution of 1-(4-bromo-2-(6-azaspiro[2.5]octan-6-yl)phenyl)-3-(2-(4,4-difluoropiperidin-1-yl)-6-methylpyrimidin-4-yl)propane-1,3-dione (500 mg, 0.91 mmol) in ethanol (10 mL) at 25°C, and the reaction mixture was stirred at 100°C for 12 h. The reaction solution was slowly added dropwise into water (50 mL), and the precipitate was separated. The cake was collected by filtration with a sand core funnel, washed with water (50 mL), and dried by an oil pump to give 6-(5-bromo-2-(5-(2-(4,4-difluoropiperidin-1-yl)-6-methylpyrimidin-4-yl)-1H-pyrazol-3-yl)phenyl)-6-azaspiro[2.5]octane (410 mg, 0.76 mmol, yield: 83%) as a bright yellow solid compound. LCMS (ESI): $[M+H]^+$ =542.9

Step 2: To a solution of 6-(5-bromo-2-(5-(2-(4,4-difluoropiperidin-1-yl)-6-methylpyrimidin-4-yl)-1H-pyrazol-3-yl)phenyl)-6-azaspiro[2.5]octane (250 mg, 0.46 mmol) in dioxane (5 mL) were added 2-hydroxyethanesulfonamide (57.6 mg, 0.46 mmol), potassium phosphate (391 mg, 1.84 mmol), di-tert-butyl-[2-(2,4,6-triisopropylphenyl) phenyl] phosphine (39.1 mg, 0.09 mmol) and tris(dibenzylideneacetone) dipalladium (42.1 mg, 0.05 mmol) at 25 °C, and the reaction mixture was heated to 100°C under nitrogen protection and stirred for 16 h. The reaction mixture was concentrated under reduced pressure, and the residue was diluted with N,N-dimethylformamide (10 mL). The diluent was purified by preparative HPLC (C18, 24-64% gradient of water (formic acid)/acetonitrile) to give N-(4-(5-(2-(4,4-difluoropiperidin-1-yl)-6-methylpyrimidin-4-yl)-1H-pyrazol-3-yl)-3-( 6-azaspiro[2.5]octan-6-yl)phenyl)-2-hydroxyethane-1-sulfonamide (133 mg, 0.23 mmol. yield: 49%) as a white solid compound. LCMS (ESI): $[M+H]^+$ =588.3. $^1H$-NMR (400 MHz, DMSO-d6) δ ppm 14.52 - 12.29 (m, 1H), 9.84 (br s, 1H), 7.59 (br d, J = 8.0 Hz, 1H), 7.50 (br s, 1H), 7.12 - 7.03 (m, 2H), 6.95 (t, J = 1.0 Hz, 1H), 4.08 - 3.92 (m, 4H), 3.76 (t, J = 6.7 Hz, 2H), 3.35- 3.30 (m, 1H), 3.29 (t, J = 6.6 Hz, 2H), 2.98 - 2.74 (m, 4H), 2.37 (s, 3H), 2.09 - 1.94 (m, 4H), 1.66 - 1.34 (m, 4H), 0.32 (s, 4H)

**Example 7:**

**N-(4-(3-(2-(4,4-difluoropiperidin-1-yl)-6-methylpyrimidin-4-yl)-1,2,4-oxadiazol-5 -yl)-3-(6-azaspiro[2.5]octan-6-yl)phenyl)-2-hydroxyethanesulfonamide**

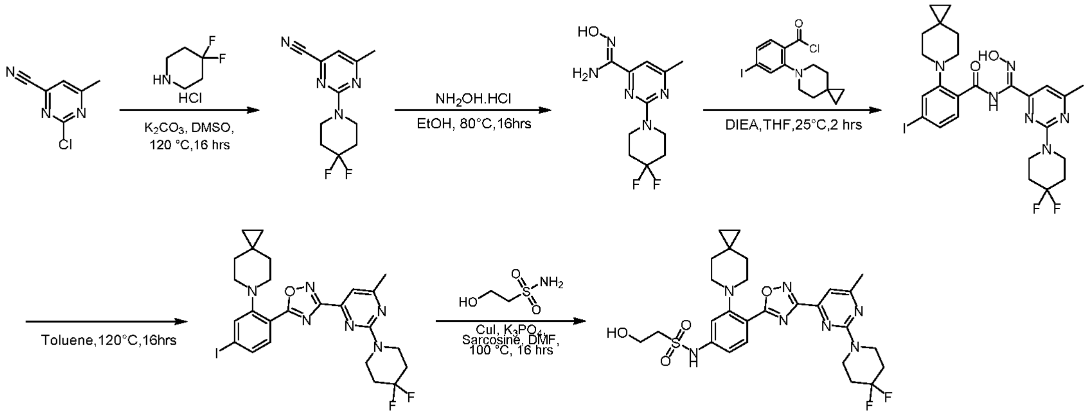


Step 1: To a solution of 2-chloro-6-methylpyrimidin-4-carbonitrile (5.00 g, 32.5 mmol) in DMSO (50.0 mL) were added 4,4-difluoropiperidine hydrochloride (7.70 g, 48.8 mmol) and potassium carbonate (13.8 g, 97.6 mmol). The mixture was heated to 120°C and stirred for 16 h. The reaction solution was diluted with water (50 mL) and extracted with ethyl acetate (50 mL×3). The organic phases were combined, dried over anhydrous sodium sulfate, and filtered, and The filtrate was concentrated under reduced pressure to give the residue. The residue was purified by flash column chromatography (silica gel, 0-6% gradient of ethyl acetate/petroleum ether) to give 2-(4,4-difluoropiperidin-1-yl)-6-methylpyrimidin-4-carbonitrile (4.50 g, 25.5 mmol, yield: 58%) as a white solid. LCMS (ESI): $[M+H]^+$ =239.0

Step 2: Triethylamine (1.30 g, 12.6 mmol) was added to a solution of 2-(4,4-difluoropiperidin-1-yl)-6-methylpyrimidin-4-carbonitrile (1.00 g, 4.20 mmol) and hydroxylamine hydrochloride (0.58 g, 8.39 mmol) in ethanol (20.0 mL). The mixture was heated to 80°C and stirred for 16 h. Water (20 mL) was added to the reaction solution to precipitate the white solid, and the residue was collected by filtration to give (Z)-2-(4,4-difluoropiperidin-1-yl)-N'-hydroxy-6-methylpyrimidin-4-carboxamidine (1.10 g, 4.19 mmol, yield: 97%). LCMS (ESI): $[M+H]^+$ =272.0

Step 3: N,N-diisopropylethylamine (729 mg, 5.53 mmol) was added to a solution of (Z)-2-(4,4-difluoropiperidin-1-yl)-N'-hydroxy-6-methylpyrimidin-4-carboxamidine (500 mg, 1.84 mmol) and 4-iodo-2-(6-azaspiro[2.5]octane-6-yl) benzoyl chloride (900 mg, 2.40 mmol) in THF (4.00 mL). The mixture was stirred at 25°C for 2 h. The reaction solution was diluted with water (5 mL) and extracted with dichloromethane (5 mL × 3), and the organic layer was washed with saturated saline (20 mL), dried over magnesium sulfate, filtered, and concentrated under reduced pressure to give the residue. The residue was purified by flash column chromatography (silica gel, 0-10% gradient of ethyl acetate/petroleum ether) to give (*Z*)-*N*-((2-(4,4-difluoropiperidin-1-yl)-6-methylpyrimidin-4-yl)(oxime)methyl)-4-iodo -2-(6-azaspiro[2.5]octan-6-yl)benzamide (1.30 g, 1.80 mmol, yield: 79%) as a brown solid. LCMS (ESI): $[M+H]^+$ =611.1

Step 4: The stirred solution of (Z)-N-((2-(4,4-difluoropiperidin-1-yl)-6-methylpyrimidin-4-yl)(oxime)methyl)-4-iodo -2-(6-azaspiro[2.5]octan-6-yl)benzamide (1.10 g, 1.80 mmol) in toluene (10.00 mL) was heated to 120°C and stirred for 16 h. The reaction solution was diluted with water (10 mL) and extracted with dichloromethane (10 mL×3). The organic phases were combined, dried over anhydrous sodium sulfate, and filtered, and The filtrate was concentrated under reduced pressure to give the residue. The residue was purified by flash column chromatography (silica gel, 0-3% gradient of ethyl acetate/petroleum ether) to give 3-(2-(4,4-difluoropiperidin-1-yl)-6-methylpyrimidin-4-yl)-5-(4-iodo-2-(6-azaspiro[2.5] octan-6-yl)phenyl)-1,2,4-oxadiazole (420 mg, 1.80 mmol, yield: 39%) as a yellow solid. LCMS (ESI): $[M+H]^+$ =593.1

Step 5: Cuprous iodide (115 mg, 0.59 mmol) was added to a solution of potassium phosphate (512 mg, 2.36 mmol), 2-hydroxyethanesulfonamide (148 mg, 1.18 mmol), and sarcosine (53.7 mg, 0.59 mmol) in dimethylformamide (5.00 mL). The mixture was heated to 50°C and stirred for 5 min. The mixture was added with 3-(2-(4,4-difluoropiperidin-1-yl)-6-methylpyrimidin-4-yl)-5-(4-iodo-2-(6-azaspiro[2.5] octan-6-yl)phenyl)-1,2,4-oxadiazole (900 mg, 1.30 mmol) and then heated to 100°C under nitrogen protection with stirring for 16 h. The reaction solution was diluted with water (10 mL) and extracted with dichloromethane (10 mL × 3). The organic phases were combined, dried over anhydrous sodium sulfate, and filtered, and The filtrate was concentrated under reduced pressure to give the residue. The residue was purified by preparative HPLC (C18, 50-90% gradient of water (formic acid)/acetonitrile) to give N-(4-(3-(2-(4,4-difluoropiperidin-1-yl)-6-methylpyrimidin-4-yl)-1,2,4-oxadiazol-5-yl) -3-(6-azaspiro[2.5]octan-6-yl) phenyl)-2-hydroxyethanesulfonamide (227 mg, 0.59 mmol, yield: 65%) as the white solid compound. LCMS (ESI): $[M+H]^+$ =590.5. $^1H$ NMR (400 MHz, DMSO-d6) $\delta$ ppm 10.27 (s, 1H), 8.00 (d, J = 8.4 Hz, 1H), 7.26 (s, 1H), 7.10 (d, J = 2.0 Hz, 1H), 6.99 (dd, J = 2.0, 8.8 Hz, 1H), 4.95 (br s, 1H), 4.00 (br t, J = 5.6 Hz, 4H), 3.78 (br t, J = 6.0 Hz, 2H), 3.39 (br t, J = 6.4 Hz, 2H), 2.98 (br t, J = 4.8 Hz, 4H), 2.45 (s, 3H), 2.13 - 1.98 (m, 4H), 1.55 (m, 4H), 0.35 (s, 4H)

**Example 8:**

**N-(4-(5-(2-(4,4-difluoropiperidin-1-yl)-6-methylpyrimidin-4-yl)-2H-tetrazol-2-yl) -3-(6-azaspiro[2.5]octan-6-yl) phenyl)-2-hydroxyethane-1-sulfonamide**

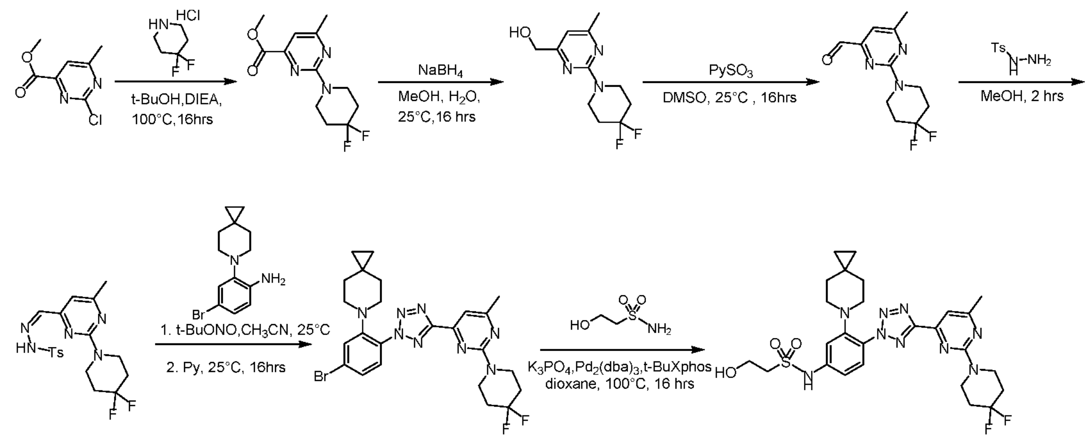


Step 1: To a solution of methyl 2-chloro-6-methylpyrimidin-4-carboxylate (5.00 g, 26.8 mmol) and N,N-diisopropylethylamine (10.6 g, 80.4 mmol) in tert-butanol (100 mL) was added 4,4-difluoropiperidine hydrochloride (4.22 g, 26.8 mmol), and the mixture was stirred at 100°C for 16 h. The reaction solution was diluted with water (100 mL) and extracted with ethyl acetate (100 mL × 2), and the organic layer was washed with saturated saline (100 mL × 2), dried over magnesium sulfate, filtered, and concentrated under reduced pressure to give the residue. The residue was separated by column chromatography (silica gel, 0-15% gradient of ethyl acetate/petroleum ether) to give methyl 2-(4,4-difluoropiperidin-1-yl)-6-methylpyrimidin-4-carboxylate (5.60 g, 20.6 mmol, yield: 77.0%) as a yellow solid. LCMS (ESI): $[M+H]^+$ =271.9.

Step 2: Sodium borohydride (2.51 g, 66.3 mmol) was added to a solution of methyl 2-(4,4-difluoropiperidin-1-yl)-6-methylpyrimidin-4-carboxylate (6.00 g, 22.1 mmol) in methanol (100 mL), and the mixture was stirred at 25°C for 16 h. The reaction solution was diluted with water (100 mL) and extracted with ethyl acetate (100 mL × 2), and the organic layer was washed with saturated saline (100 mL × 2), dried over magnesium sulfate, filtered, and concentrated under reduced pressure to give [2-(4,4-difluoropiperidin-1-yl)-6-methylpyrimidin-4-yl]methanol (5.00 g, 20.5 mmol, yield: 92.9%) as the crude. LCMS (ESI): $[M+H]^+$ =244.1.

Step 3: Pyridine sulfur trioxide (9.81 g, 61.6 mmol) was added to a solution of [2-(4,4-difluoropiperidin-1-yl)-6-methylpyrimidin-4-yl]methanol (5.00 g, 20.5 mmol) and triethylamine (10.4 g, 102 mmol) in DMSO (100 mL), and the mixture was stirred at 25°C for 16 h. The reaction solution was diluted with water (100 mL) and extracted with ethyl acetate (100 mL × 2), and the organic layer was washed with saturated saline (100 mL × 2), dried over magnesium sulfate, filtered, and concentrated under reduced pressure to give 2-(4,4-difluoropiperidin-1-yl)-6-methylpyrimidin-4-carbaldehyde (3.00 g, 12.4 mmol, yield: 60.5%) as the crude. LCMS (ESI): $[M+H]^+$ =242.0.

Step 4: To a solution of 2-(4,4-difluoropiperidin-1-yl)-6-methylpyrimidin-4-carbaldehyde (1.00 g, 4.14 mmol) in methanol (20 mL) was added 4-methylbenzene-1-sulfonylhydrazine (0.77 g, 4.14 mmol), and the mixture was stirred at 25°C for 2 h. The reaction solution was filtered and the filtrate was concentrated under reduced pressure to give N'-[(Z)-[2-(4,4-difluoropiperidin-1-yl)-6-methylpyrimidin-4-yl]methylidene]-4-methy lbenzene-1-sulfonylhydrazine (0.68 g, 1.66 mmol, yield: 40.0%). LCMS (ESI): $[M+H]^+$ =410.2.

Step 5: Tert-butyl nitrite (308 mg, 2.93 mmol) was added to a solution of 2-{6-azaspiro[2.5]octan-6-yl}-4-bromoaniline (453 mg, 1.61 mmol) in acetonitrile (6 mL). The mixture was stirred at 25°C for 1 h. Then, the solution of N'-[(Z)-[2-(4,4-difluoropiperidin-1-yl)-6-methylpyrimidin-4-yl]methylidene]-4-methy lbenzene-1-sulfonylhydrazine (0.22 g, 0.44 mmol) in pyridine (6 mL) was added to the reaction solution. The mixture was stirred at 25°C for 16 h. The reaction solution was diluted with water (100 mL) and extracted with ethyl acetate (100 mL × 2), and the organic layer was washed with saturated saline (100 mL × 2), dried over magnesium sulfate, filtered, and concentrated under reduced pressure to give the residue. The residue was separated by column chromatography (silica gel, 0-5% gradient of ethyl acetate/petroleum ether) to give 6-(5-bromo-2-{5-[2-(4,4-difluoropiperidin-1-yl)-6-methylpyrimidin-4-yl]-2H-1,2,3,4-tetrazol-2-yl}phenyl)-6-azaspiro[2.5]octane (390 mg, 0.72 mmol, yield: 49.0%) as a yellow solid. LCMS (ESI): $[M+H]^+$ =545.1
Step 6: In dioxane (8.00 mL) was dissolved 6-(5-bromo-2-{5-[2-(4,4-difluoropiperidin-1-yl)-6-methylpyrimidin-4-yl]-2H-1,2,3,4-tetrazol-2-yl}phenyl)-6-azaspiro[2.5]octane (365 mg, 0.67 mmol) and 2-hydroxyethanesulfonamide

(108 mg, 0.87 mmol), and potassium phosphate (426 mg, 2.00 mmol), 2-di-tert-butylphosphino-2',4',6'-triisopropylbiphenyl (56.8 mg, 0.13 mmol), and tris(dibenzylideneacetone) dipalladium (61.3 mg, 0.06 mmol) were added. The mixture was heated to 100°C under nitrogen protection and stirred for 16 h. The reaction solution was cooled to ambient temperature and extracted with water (20 mL) and ethyl acetate (20 mL × 2). The organic phases were combined and washed with ammonia (20 mL × 2), dried over anhydrous magnesium sulfate and filtered. The filtrate was concentrated under reduced pressure to give a residue. The residue was purified by preparative HPLC (C18, 36-76% gradient of water (formic acid)/acetonitrile) to give N-(4-(5-(2-(4,4-difluoropiperidin-1-yl)-6-methylpyrimidin-4-yl)-2H-tetrazol-2-yl)-3-( 6-azaspiro[2.5]octan-6-yl)phenyl)-2-hydroxyethane-1-sulfonamide (220 mg, 0.37 mmol, yield: 55.7%) as a yellow solid. LCMS (ESI): $[M+H]^+$ =590.3, $^1H$ NMR (400 MHz, DMSO-d6) δ 9.96 (s, 1H), 7.35 (d, *J* = 8.4 Hz, 1H), 7.12 (s, 1H), 6.93 (d, *J* = 2.4 Hz, 1H), 6.79 (dd, *J* = 2.0, 8.4 Hz, 1H), 5.32 - 4.63 (m, 1H), 3.76 (br t, *J* = 5.6 Hz, 4H), 3.55 (t, *J* = 6.4 Hz, 2H), 3.18 - 3.15 (m, 2H), 2.55 - 2.49 (m, 4H), 2.22 (s, 3H), 1.88 - 1.74 (m, 4H), 1.00 - 0.93 (m, 4H), 0.00 (s, 4H)

**Example 9:**

**N-(4-(2-(2-(4,4-difluoropiperidin-1-yl)-6-methylpyridin-4-yl)-2H-tetrazol-5-yl)-3-(6-azaspiro[2.5]octan-6-yl)phenyl)-2-hydroxyethane-1-sulfonamide**

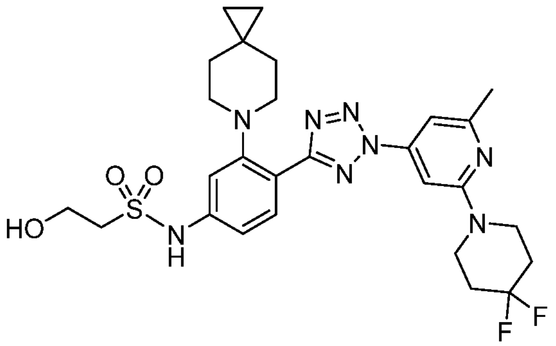

**Example 9** was prepared with reference to the synthetic route of **Example 8:** LCMS (ESI): $[M+H]^+$ = 589.3. $^1H$ NMR (400 MHz, DMSO-$d_6$) δ 10.07 (s, 1H), 7.95 (d, *J* = 8.4 Hz, 1H), 7.38 (s, 1H), 7.33 (s, 1H), 7.14 (d, *J* = 2.0 Hz, 1H), 7.02 (dd, *J* = 2.0, 8.4 Hz, 1H), 5.51 - 4.53 (m, 1H), 3.85 - 3.72 (m, 6H), 3.36 - 3.33 (m, 2H), 2.93 (br t, *J* = 4.8 Hz, 4H), 2.48 (br s, 3H), 2.11 - 2.00 (m, 4H), 1.55 - 1.38 (m, 4H), 0.32 (s, 4H)

**Example 10:**

**N-(4-(4-(2-(4,4-difluoropiperidin-1-yl)-6-methylpyrimidin-4-yl)-1H-imidazol-1-yl) -3-(6-azaspiro[2.5]octan-6-yl) phenyl)-2-hydroxyethane-1-sulfonamide**

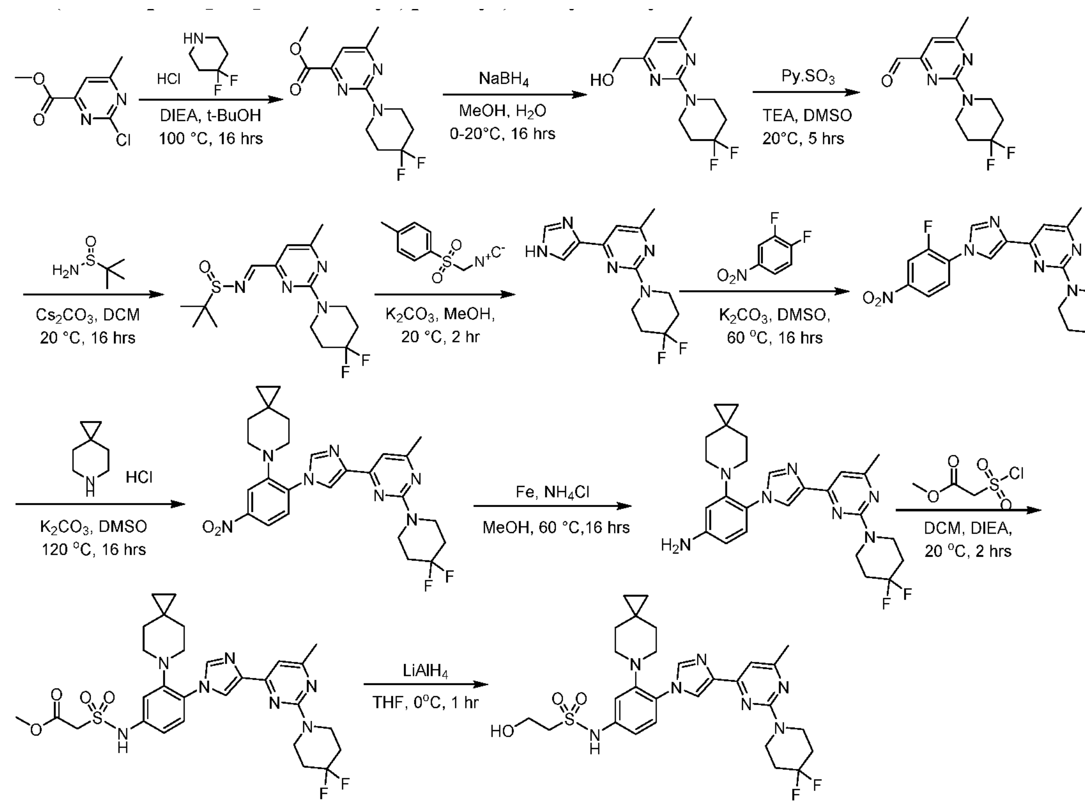


Step 1: To a solution of methyl 2-chloro-6-methylpyrimidin-4-carboxylate (5.00 g, 26.8 mmol) in tert-butanol (50 mL) was added 4,4-difluoropiperidine hydrochloride (4.64 g, 29.5 mmol) and N,N-diisopropylethylamine (10.6 g, 80.4 mmol). The mixture was stirred at 100°C for 16 h. The reaction solution was cooled to ambient temperature and extracted with water (100 mL) and ethyl acetate (100 mL × 3). The combined organic layer was washed with saturated saline (100 mL), dried over anhydrous magnesium sulfate, and filtered, and The filtrate was concentrated under reduced pressure to give methyl 2-(4,4-difluoropiperidin-1-yl)-6-methylpyrimidin-4-carboxylate as a yellow solid (6.20 g, 22.9 mmol, yield: 85%). LCMS (ESI): [M+H]+ = 272.0

Step 2: Methyl 2-(4,4-difluoropiperidin-1-yl)-6-methylpyrimidin-4-carboxylate (6.20 g, 22.9 mmol) was added to methanol (90 mL)/water (15 mL), and sodium borohydride (2.56 g, 66.21 mmol) was added portionwise at 0°C. The mixture was stirred at 20°C for 16 h. The reaction solution was quenched slowly with water (150 mL) and extracted with ethyl acetate (100 mL × 2). The combined organic layer was washed with saturated saline (100 mL), dried over anhydrous magnesium sulfate, and filtered, and The filtrate was concentrated under reduced pressure to give [2-(4,4-difluoropiperidin-1-yl)-6-methylpyrimidin-4-yl]methanol as a yellow oil (5.10 g, 21.0 mmol, yield: 92%). LCMS (ESI): [M+H]+ = 244.0

Step 3: Triethylamine (10.4 g, 103 mmol) was added in a solution of [2-(4,4-difluoropiperidin-1-yl)-6-methylpyrimidin-4-yl]methanol (5.10 g, 21.0 mmol) in DMSO (100 mL) dropwise, and then pyridine sulfur trioxide complex (10.0 g, 62.9 mmol) was added portionwise. The mixture was stirred at 20°C for 5 h. The reaction solution was extracted with water (100 mL) and ethyl acetate (200 mL×3). The combined organic layer was washed with saturated saline (100 mL × 2), dried over anhydrous magnesium sulfate, and filtered, and The filtrate was concentrated under reduced pressure to give 2-(4,4-difluoropiperidin-1-yl)-6-methylpyrimidin-4-carbaldehyde (5.00 g, crude) as a brown oil. LCMS (ESI): [M+H]+ = 242.1

Step 4: To a solution of N-[(E)-[2-(4,4-difluoropiperidin-1-yl)-6-methylpyrimidin-4-yl]methylidene]-2-methyl propane-2-sulfenamide (5.00 g, 20.7 mmol) in dichloromethane (60 mL) were added 2-methyl-2-propanesulfinamide (2.64 g, 21.8 mmol) and cesium carbonate (8.27 g, 24.9 mmol). The mixture was stirred at 20°C for 16 h. The reaction solution was diluted with water (100 mL) and extracted with ethyl acetate (100 mL×3). The combined organic layer was washed with saturated saline (100 mL), dried over anhydrous magnesium sulfate, and filtered. The filtrate was concentrated under reduced pressure to give N-[(E)-[2-(4,4-difluoropiperidin-1-yl)-6-methylpyrimidin-4-yl]methylidene]-2-methyl propane-2-sulfenamide (6.20 g, 18.0 mmol, yield: 87%) as an orange solid. LCMS (ESI): [M+H]+ = 345.0

Step 5: Potassium carbonate (6.35 g, 45.0 mmol) and p-toluenesulfonylmethyl isocyanide (3.87 g, 19.8 mmol) were added to a solution of N-[(E)-[2-(4,4-difluoropiperidin-1-yl)-6-methylpyrimidin-4-yl]methylidene]-2-methyl propane-2-sulfenamide (6.20 g, 18.0 mmol) in methanol (70 mL). The mixture was stirred at 20°C for 2 h. The reaction solution was cooled to ambient temperature and extracted with water (50 mL) and ethyl acetate (50 mL×3). The combined organic layer was washed with saturated saline (50 mL), dried over anhydrous magnesium sulfate and filtered. The filtrate was concentrated under reduced pressure to give a residue. The residue was purified by flash column chromatography (silica gel, 0-6% gradient of methanol/dichloromethane) to give 2-(4,4-difluoropiperidin-1-yl)-4-(1H-imidazol-4-yl)-6-methylpyrimidine (4.20 g, 15.12 mmol, yield: 84%) as an off-white solid. LCMS (ESI): [M+H]+ = 280.0

Step 6: To a solution of 2-(4,4-difluoropiperidin-1-yl)-4-(1H-imidazol-4-yl)-6-methylpyrimidine (1.00 g, 3.58 mmol) in DMSO (20 mL) were added 1,2-difluoro-4-nitrobenzene (0.57 g, 3.58 mmol) and potassium carbonate (1.51 g, 10.7 mmol). The mixture was stirred at 60°C for 16 h. The reaction was cooled to ambient temperature and extracted with water (50 mL) and ethyl acetate (50 mL×3). The organic phases were combined and washed with saturated saline (50 mL×2), dried over anhydrous magnesium sulfate, and filtered. The filtrate was concentrated under reduced pressure to give a residue. The residue was purified by flash column chromatography (silica gel, 0-33% gradient of ethyl acetate/petroleum ether) to give 2-(4,4-difluoropiperidin-1-yl)-4-[1-(2-fluoro-4-nitrophenyl)-1H-imidazol-4-yl]-6-met hylpyrimidine (1.20 g, 2.87 mmol, yield: 87%) as a pale yellow solid. LCMS (ESI): [M+H]+ = 419.1

Step 7: To a solution of 2-(4,4-difluoropiperidin-1-yl)-4-[1-(2-fluoro-4-nitrophenyl)-1H-imidazol-4-yl]-6-met hylpyrimidine (1.20 g, 2.87 mmol) in DMSO (20 mL) was added 6-azaspiro [2.5] octane hydrochloride (0.47 g, 3.16 mmol) and potassium carbonate (1.21 g, 8.60 mmol). The mixture was stirred at 120°C for 16 h. The reaction solution was diluted with water (50 mL) and extracted with ethyl acetate (50 mL×3). The combined organic layer was washed with saturated saline (20 mL × 2), dried over anhydrous magnesium sulfate and filtered. The filtrate was concentrated under reduced pressure to give a residue. The residue was purified by flash column chromatography (silica gel, 0-50% gradient of ethyl acetate/petroleum ether) to give 6-(2-{4-[2-(4,4-difluoropiperidin-1-yl)-6-methylpyrimidin-4-yl]-1H-imidazol-1-yl}-5 -nitrophenyl)-6-azaspiro[2.5]octane (0.60 g, 1.18 mmol, yield: 41%) as a white solid. LCMS (ESI): [M+H]+ = 510.3

Step 8: Iron powder (332 mg, 5.89 mmol) was added portionwise in 6-(2-{4-[2-(4,4-difluoropiperidin-1-yl)-6-methylpyrimidin-4-yl]-1H-imidazol-1-yl}-5 -nitrophenyl)-6-azaspiro[2.5]octane (500 mg, 0.98 mmol) in methanol (9 mL)/saturated aqueous ammonium chloride solution (3 mL). The mixture was stirred at 60°C for 16 h. The hot reaction solution was filtered under reduced pressure and extracted with water (20 mL) and ethyl acetate (20 mL×3). The combined organic layer was washed with saturated saline (20 mL), dried over anhydrous magnesium sulfate and filtered. The filtrate was concentrated under reduced pressure to give a residue. The residue was purified by flash column chromatography (silica gel, 0-50% gradient of ethyl acetate/petroleum ether) to give 3-{6-azaspiro[2.5]octan-6-yl}-4-{4-[2-(4,4-difluoropiperidin-1-yl)-6-methylpyrimidin -4-yl]-1H-imidazol-1-yl}aniline (380 mg, 0.75 mmol, yield: 81%). LCMS (ESI): [M+H]+ = 480.2

Step 9: Methyl chlorosulfonylacetate (294 mg, 1.58 mmol) was slowly added dropwise in a solution of 3-{6-azaspiro[2.5]octan-6-yl}-4-{4-[2-(4,4-difluoropiperidin-1-yl)-6-methylpyrimidin -4-yl]-1H-imidazol-1-yl}aniline (350 mg, 0.73 mmol) and N,N-diisopropylethylamine (193 mg, 1.46 mmol) in dichloromethane (7 mL). The mixture was stirred at 20°C for 2 h. The reaction solution was extracted with water (20 mL) and ethyl acetate (20 mL×3). The organic phases were combined, washed with saturated saline (20 mL), dried over anhydrous magnesium sulfate and filtered. The filtrate was concentrated under reduced pressure to give a residue. The residue was purified by flash column chromatography (silica gel, 0-50% gradient of ethyl acetate/petroleum ether) to give methyl 2-[(3-{6-azaspiro[2.5]octan-6-yl}-4-{4-[2-(4,4-difluoropiperidin-1-yl)-6-methylpyrim idin-4-yl]-1H-imidazol-1-yl}phenyl)sulfamoyl]acetate (210 mg, 0.34 mmol, yield: 47%) as a white solid. LCMS (ESI): [M+H]+ = 616.2

Step 10: Methyl 2-[(3-{6-azaspiro[2.5]octan-6-yl}-4-{4-[2-(4,4-difluoropiperidin-1-yl)-6-methylpyrim idin-4-yl]-1H-imidazol-1-yl}phenyl)sulfamoyl]acetate (200 mg, 0.32 mmol) was added to a THF solution (4 mL), and lithium tetrahydridoaluminate (34.8 mg, 0.97 mmol) was added portionwise at 0 °C. The mixture was stirred at 0°C for 1 h. The reaction solution was quenched with water (0.03 mL) and 10% aqueous sodium hydroxide solution (0.06 mL) and filtered. The filtrate was concentrated under reduced pressure to give a residue. The residue was purified by preparative HPLC (C18, 32-72% gradient of water (formic acid)/acetonitrile) to give N-(4-(4-(2-(4,4-difluoropiperidin-1-yl)-6-methylpyrimidin-4-yl)-1H-imidazol-1-yl)-3-(6-azaspiro[2.5]octan-6-yl)phenyl)-2-hydroxyethane-1-sulfonamide (35.0 mg, 0.06 mmol, yield: 18%) as the white solid compound. LCMS (ESI): $[M+H]^+$ = 588.6, $^1$H NMR (400 MHz, DMSO-d6) δ ppm 9.88 (s, 1H), 8.30 (d, *J* = 1.2 Hz, 1H), 8.09 (d, *J* = 1.2 Hz, 1H), 7.39 (d, *J* = 8.4 Hz, 1H), 7.10 - 7.03 (m, 2H), 6.96 (dd, *J* = 2.4, 8.4 Hz, 1H), 4.97 (s, 1H), 3.95 (br t, *J* = 5.2 Hz, 4H), 3.76 (t, *J* = 6.8 Hz, 2H), 3.27 (br s, 2H), 2.71 - 2.64 (m, 4H), 2.34 (s, 3H), 2.06 - 1.91 (m, 4H), 1.32 (br s, 4H), 0.25 (s, 4H)

**Example 11:**

**N-(4-(4-(2-(4,4-difluoropiperidin-1-yl)-6-methoxypyrimidin-4-yl)-1H-imidazol-1-yl)-3-(6-azaspiro[2.5]octan-6-yl)phenyl)-2-hydroxyethane-1-sulfonamide**

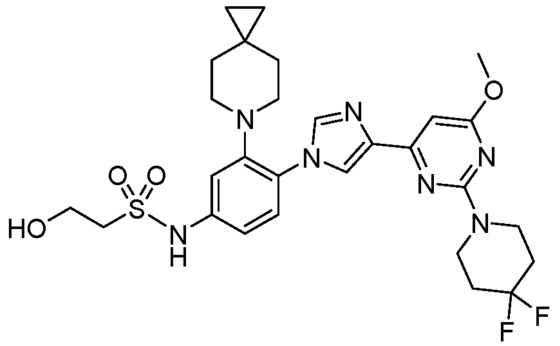

LCMS (ESI): [M+H]+ = 604.5; $^1$H NMR (400 MHz, DMSO-d6) δ ppm 9.89 (s, 1H), 8.25 (d, *J* = 1.2 Hz, 1H), 8.07 (d, *J* = 1.2 Hz, 1H), 7.38 (d, *J* = 8.4 Hz, 1H), 7.07 (d, *J* = 2.4 Hz, 1H), 6.95 (dd, *J* = 2.4, 8.4 Hz, 1H), 6.53 (s, 1H), 4.97 (m, 1H), 3.95 (br t, *J* = 5.2 Hz, 4H), 3.88 (s, 3H), 3.76 (t, *J* = 6.4 Hz, 2H), 3.30 - 3.26 (m, 2H), 2.69 (br t, *J* = 5.2 Hz, 4H), 2.09 - 1.91 (m, 4H), 1.32 (br s, 4H), 0.25 (s, 4H)

**Example 12:**

**N-(4-(1-(2-(4,4-difluoropiperidin-1-yl)-6-methylpyrimidin-4-yl)-1H-imidazol-4-yl) -3-(6-azaspiro[2.5]octan-6-yl)phenyl)-2-hydroxyethane-1-sulfonamide**

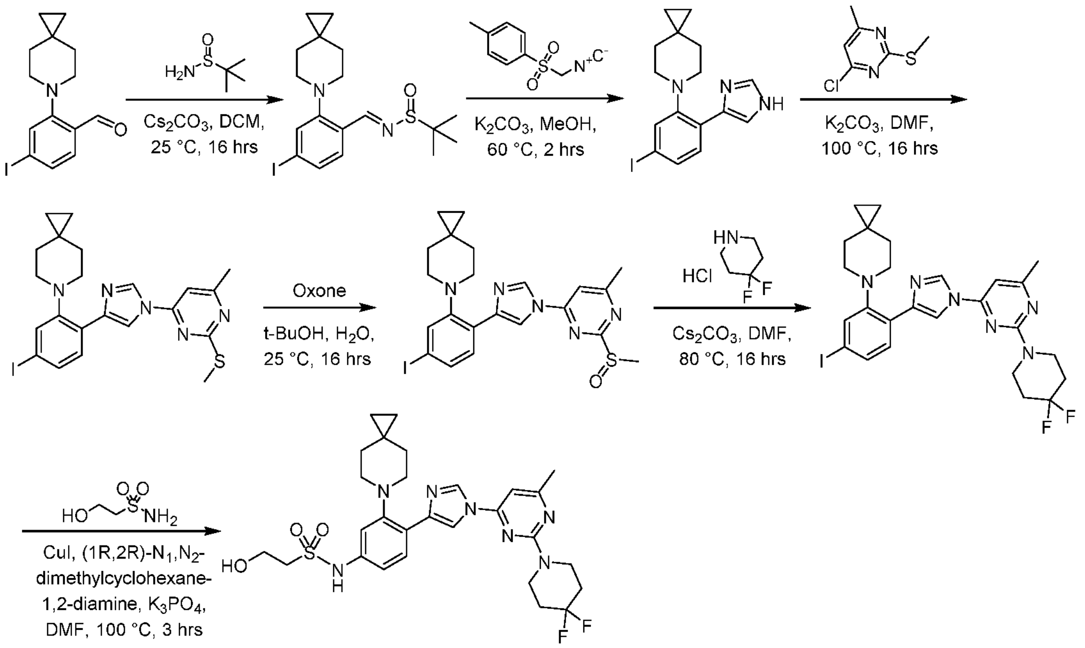


Step 1: To a solution of 2-(6-azaspiro[2.5]oct-6-yl)-4-iodobenzaldehyde (1.50 g, 4.40 mmol) in dichloromethane (20 mL) was added 2-methyl-2-propanesulfinamide (0.53 g, 4.40 mmol) and cesium carbonate (1.61 g, 4.84 mmol). The mixture was stirred at 25°C for 16 h. The reaction solution was extracted with water (50 mL) and ethyl acetate (50 mL×3). The combined organic layer was washed with saturated saline (50 mL), dried over anhydrous magnesium sulfate and filtered. The filtrate was concentrated under reduced pressure to give a residue. The residue was purified

by flash column chromatography (silica gel, 0-11% gradient of ethyl acetate/petroleum ether) to give N-[(E)-(2-{6-azaspiro[2.5]octan-6-yl}-4-iodophenyl)methylidene]-2-methyl-2-propan esulfinamide (1.80 g, 4.05 mmol, yield: 92%) as a brown solid. LCMS (ESI): [M+H]+ = 445.0

Step 2: Potassium carbonate (1.43 g, 10.1 mmol) and p-toluenesulfonylmethyl isocyanide (5.10 g, 90.3 mmol) were added to a solution of N-[(E)-(2-{6-azaspiro[2.5]octan-6-yl}-4-iodophenyl)methylidene]-2-methyl-2-propan esulfinamide (1.80 g, 4.05 mmol) in methanol (30 mL). The mixture was stirred at 60°C for 2 h. The reaction solution was cooled to ambient temperature and extracted with water (50 mL) and ethyl acetate (50 mL×3). The combined organic layer was washed with saturated saline (50 mL), dried over anhydrous magnesium sulfate and filtered. The filtrate was concentrated under reduced pressure to give a residue. The residue was purified by flash column chromatography (silica gel, 0-6% gradient of methanol/dichloromethane) to give 6-[2-(1H-imidazol-4-yl)-5-iodophenyl]-6-azaspiro [2.5]octane (0.90 g, 2.38 mmol, yield: 59%) as an off-white solid. LCMS (ESI): [M+H]+ = 380.0

Step 3: To a solution of 6-[2-(1H-imidazol-4-yl)-5-iodophenyl]-6-azaspiro[2.5]octane (560 mg, 1.48 mmol) in N,N-dimethylformamide (10 mL) was added 2-methylthio-4-chloro-6-methylpyrimidine (284 mg, 1.62 mmol) and potassium carbonate (312 mg, 2.21 mmol). The mixture was stirred at 100°C for 16 h. The reaction solution was cooled to ambient temperature and extracted with water (50 mL) and ethyl acetate (80 mL × 3). The organic phases were combined, dried over anhydrous magnesium sulfate, and filtered. The filtrate was concentrated under reduced pressure to give a residue. The residue was purified by flash column chromatography (silica gel, 0-25% gradient of ethyl acetate/petroleum ether) to give 6-(5-iodo-2-{1-[6-methyl-2-(methylmercapto)pyrimidin-4-yl]-1H-imidazol-4-yl}phen yl)-6-azaspiro[2.5]octane (570 mg, 1.15 mmol, yield: 78%) as an off-white solid. LCMS (ESI): [M+H]+ = 518.0

Step 4: Potassium monopersulfate (455 mg, 2.71 mmol) was added to a solution of 6-(5-iodo-2-{1-[6-methyl-2-(methylmercapto)pyrimidin-4-yl]-1H-imidazol-4-yl}phen yl)-6-azaspiro[2.5]octane (560 mg, 1.08 mmol) in tert-butanol (3 mL)/water (3 mL), and the mixture was stirred at 25°C for 16 h. The reaction solution was diluted with water (20 mL) and extracted with ethyl acetate (20 mL×3). The combined organic layer was washed with saturated saline (20 mL), dried over anhydrous magnesium sulfate, and filtered, and The filtrate was concentrated under reduced pressure to give 6-{5-iodo-2-[1-(2-methylsulfinyl-6-methylpyrimidin-4-yl)-1H-imidazol-4-yl]phenyl} -6-azaspiro [2.5]octane (420 mg, crude) as a brown solid. LCMS (ESI): [M+H]+ = 534.0

Step 5: To a solution of 6-{5-iodo-2-[1-(2-methylsulfinyl-6-methylpyrimidin-4-yl)-1H-imidazol-4-yl]phenyl} -6-azaspiro [2.5]octane (400 mg, 0.75 mmol) in N,N-dimethylformamide (5 mL) was added 4,4-difluoropiperidine hydrochloride (126 mg, 0.80 mmol) and cesium carbonate (363 mg, 1.09 mmol). The reaction solution was stirred at 80°C for 16 h. The reaction solution was cooled to ambient temperature and extracted with water (30 mL) and ethyl acetate (30 mL × 3). The combined organic layer was washed with saturated saline (30 mL), dried over anhydrous magnesium sulfate, and filtered. The filtrate was concentrated under reduced pressure to give a residue. The residue was purified by flash column chromatography (silica gel, 0-25% gradient of ethyl acetate/petroleum ether) to give 6-(2-{1-[2-(4,4-difluoropiperidin-1-yl)-6-methylpyrimidin-4-yl]-1H-imidazol-4-yl}-5 -iodophenyl)-6-azaspiro[2.5]octane (130 mg, 0.22 mmol, yield: 29%) as a white solid. LCMS (ESI): [M+H]+ = 591.1

Step 6: To N,N-dimethylformamide (2.5 mL) was added 2-hydroxyethyl sulfonamide (33.1 mg, 0.26 mmol) and (1R,2R)-(-)-N,N-dimethylcyclohexane-1,2-diamine (29.2 mg, 0.20 mmol), cuprous iodide (19.8 mg, 0.10 mmol) and potassium phosphate (176 mg, 0.81 mmol) were added at 25°C, and the mixture was stirred at 50°C for 5 min. Then 6-(2-{ 1-[2-(4,4-difluoropiperidin-1-yl)-6-methylpyrimidin-4-yl]-1H-imidazol-4-yl}-5 -iodophenyl)-6-azaspiro[2.5]octane (120 mg, 0.20 mmol) was added portionwise, and the mixture was replaced with nitrogen three times, heated to 100°C under nitrogen protection, and stirred for 3 h. The reaction solution was cooled to ambient temperature and extracted with water (10 mL) and ethyl acetate (10 mL × 3). The combined organic layer was washed with saturated saline (10 mL × 3), dried over anhydrous magnesium sulfate and filtered. The filtrate was under reduced pressure to give a residue. The residue was purified by preparative HPLC (C18, 16-56% gradient of water (formic acid)/acetonitrile) to give N-(4-(1-(2-(4,4-difluoropiperidin-1-yl)-6-methylpyrimidin-4-yl)-1H-imidazol-4-yl)-3-(6-azaspiro[2.5]octan-6-yl)phenyl)-2-hydroxyethane-1-sulfonamide (40.5 mg, 0.07 mmol, yield: 34%) as a white solid. LCMS (ESI): [M+H]+ = 590.3, $^1$H NMR (400 MHz, DMSO-d6) δ ppm 10.72 (s, 1H), 9.11 (s, 1H), 8.20 (s, 1H), 7.22 (s, 1H), 6.66 (s, 1H), 4.97 (s, 1H), 3.97 (br t, *J* = 5.2 Hz, 4H), 3.84 - 3.75 (m, 2H), 3.63 (br d, *J* = 2.8 Hz, 2H), 2.83 - 2.71 (m, 4H), 2.39 (s, 3H), 2.06 - 1.91 (m, 4H), 1.29 - 1.22 (m, 4H), 0.24 (s, 4H)

**Example 13:**

**N-(4-(1-(2-(4,4-difluoropiperidin-1-yl)-6-methylpyridin-4-yl)-1H-imidazol-4-yl)-3 -(6-azaspiro[2.5]octan-6-yl)phenyl)-2-hydroxyethane-1-sulfonamide**

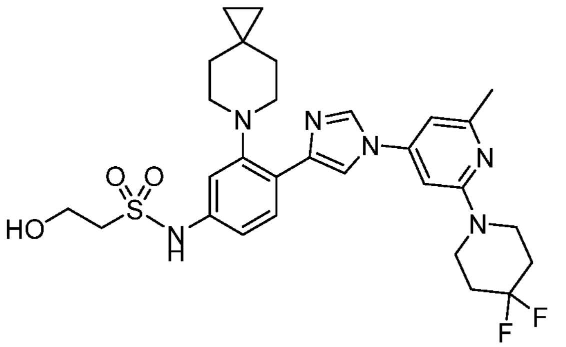

LCMS (ESI): $[M+H]^+$ = 587.3; $^1H$ NMR (400 MHz, DMSO-$d_6$) δ ppm 9.73 (s, 1H), 8.50 (s, 1H), 8.41 (s, 1H), 7.90 (d, *J* = 8.4 Hz, 1H), 7.10 (s, 1H), 7.02 - 6.93 (m, 2H), 6.90 (s, 1H), 4.95 (br s, 1H), 3.86 - 3.69 (m, 6H), 3.26 (br t, *J* = 6.4 Hz, 2H), 2.95 - 2.80 (m, 4H), 2.39 (s, 3H), 2.07 - 1.93 (m, 4H), 1.6 - 1.4 (m, 4H), 0.34 (s, 4H)

**Example 14:**

**N-(4-(5-(2-(4,4-difluoropiperidin-1-yl)-6-methylpyrimidin-4-yl)-1,3,4-thiadiazol-2 -yl)-3-(6-azaspiro[2.5]oct-6-yl) phenyl)-2-hydroxyethane-1-sulfonamide**

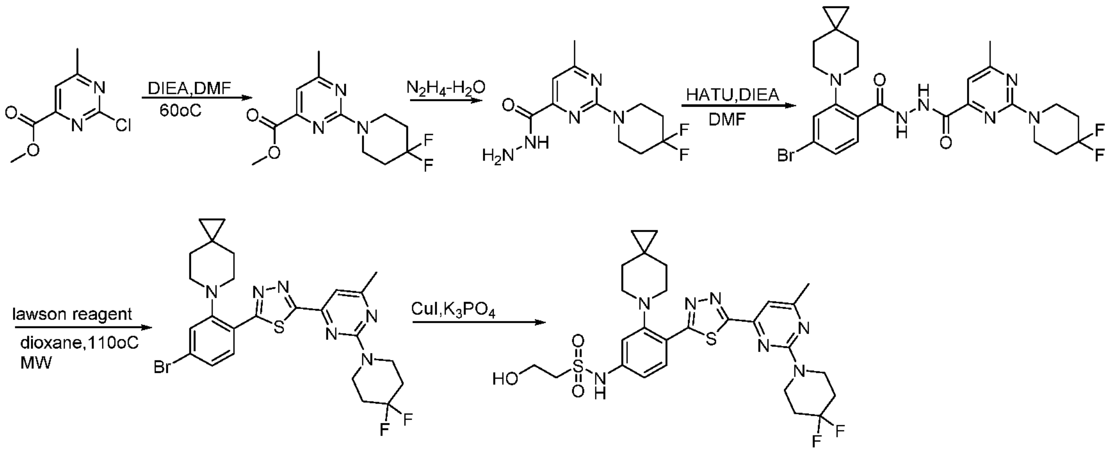


Step 1: Methyl 2-chloro-6-methylpyrimidin-4-carboxylate (3.00 g, 16.08 mmol), 4,4-difluoropiperidine hydrochloride (3.04 g, 19.29 mmol) and N,N-diisopropylethylamine (7.46 g, 56.54 mmol) were added to N,N-dimethylformamide (10 mL), and the reaction solution was stirred at 60°C for 3 h. The reaction solution was poured into the saturated aqueous ammonium chloride solution (50 mL) and extracted with ethyl acetate (50 mL × 3), and the organic phases were combined, washed with saturated aqueous sodium chloride solution (50 mL × 3), dried over anhydrous sodium sulfate, and concentrated to give crude methyl 2-(4,4-difluoropiperidin-1-yl)-6-methylpyrimidin-4-carboxylate (4.40 g, 86%). LCMS (ESI): $[M+H]^+$ =272.1.

Step 2: To 85% hydrazine hydrate (10 mL) was added 2-(4,4-difluoropiperidin-1-yl)-6-methylpyrimidin-4-carboxylate (4.40 g, 16.22 mmol), and the reaction solution was stirred at 40°C for 2 h. The reaction solution was poured into water (50 mL), suction filtration was performed, and the solid obtained by filtration was washed with water (50 mL × 3) and dried to give crude 2-(4,4-difluoropiperidin-1-yl)-6-methylpyrimidin-4-carbohydrazide (4.40 g, 100%). LCMS (ESI): $[M+H]^+$ =272.1.

Step 3: To N,N-dimethylformamide (10 mL) was added 2-(4,4-difluoropiperidin-1-yl)-6-methylpyrimidin-4-carbohydrazide (2.00 g, 7.37 mmol), 4-bromo-2-(6-azaspiro[2.5]oct-6-yl) benzoic acid (2.74 g, 8.85 mmol), 2-(7-azobenzotriazole)-N,N,N',N'-tetramethylurea hexafluorophosphate (5.72 g, 14.74 mmol) and N,N-diisopropylethylamine (1.94 g, 14.74 mmol). The reaction solution was stirred at 25°C for 2 h. The reaction solution was poured into saturated aqueous ammonium chloride solution (50 mL) and extracted with ethyl acetate (50 mL × 3), and the organic phases were combined, dried over anhydrous sodium sulfate, and concentrated to give the crude. The crude was washed with methanol (50 mL × 3) to give N'-(4-bromo-2-(6-azaspiro[2.5]octyl-6-yl)benzoyl)-2-(4,4-difluoropiperidin-1-yl)-6-m ethylpyrimidin-4-carbohydrazide (3.50 g, 84%). $^1$HNMR (400 MHz, Chloroform-d) δ 13.82 (s, 1H), 10.69 (s, 1H), 8.13 (d, J = 8.4 Hz, 1H), 7.56 (d, J = 1.9 Hz, 1H), 7.51 - 7.42 (m, 1H), 7.26 (s, 1H), 4.05 (dd, J = 6.8, 5.0 Hz, 4H), 3.05 (t, J = 5.4 Hz, 4H), 2.46 (s, 3H), 2.12 - 1.98 (m, 4H), 1.78 (s, 4H), 0.44 (s, 4H).

Step 4: N'-(4-bromo-2-(6-azaspiro[2.5]octyl-6-yl)benzoyl)-2-(4,4-difluoropiperidin-1-yl)-6-m ethylpyrimidin-4-carbohydrazide (800 mg, 1.42 mmol) and Lawesson reagent (580 mg, 1.42 mmol) were added to 1,4-dioxane (5 mL), and the reaction solution was heated by microwave at 110°C for 15 min. The reaction solution was poured into water (30 mL) and extracted with ethyl acetate (30 mL × 3). The organic phases were combined, dried over anhydrous sodium sulfate, and concentrated to obtain the crude. The crude was purified by flash column chromatography (silica gel, 0-10% gradient of ethyl acetate/petroleum ether) to give 2-(4-bromo-2-(6-azaspiro[2.5]oct-6-yl)phenyl)-5-(2-(4,4-difluoropiperidin-1-yl)-6-me thylpyrimidin-4-yl)-1,3,4-thiadiazole (500 mg, 63%). $^1$H NMR (400 MHz, Chloroform-d) δ 8.33 (d, *J* = 8.5 Hz, 1H), 7.47 (s, 1H), 7.43 (d, *J* = 10.5 Hz, 2H), 4.07 (dd, *J* = 7.0, 4.7 Hz, 4H), 2.97 (t, *J* = 5.4 Hz, 4H), 2.12 - 1.98 (m, 4H), 1.67 (s, 4H), 0.41 (s, 4H).

Step 5: To N,N-dimethylformamide (4 mL) was added 2-(4-bromo-2-(6-azaspiro[2.5]oct-6-yl)phenyl)-5-(2-(4,4-difluoropiperidin-1-yl)-6-me thylpyrimidin-4-yl)-1,3,4-thiadiazole (400 mg, 0.71 mmol), 2-hydroxy-1-sulfonamide (136 mg, 1.07 mmol), cuprous iodide (692 mg, 3.56 mmol), potassium phosphate (463 mg, 2.14 mmol), and (1S,2S)-(+)-N,N'-dimethyl-1,2-cyclohexanediamine (1.02 g, 7.12 mmol), nitrogen replacement was performed, and the reaction solution was reacted at 115°C for 4 h. The reaction solution was poured into the saturated aqueous ammonium chloride solution (30 mL) and extracted with ethyl acetate (30 mL×3), and the organic phases were combined, washed with the saturated aqueous sodium chloride solution (30 mL × 3), dried with anhydrous sodium sulfate, and concentrated to give the crude. The crude was purified by flash column chromatography (silica gel, 0-10% gradient of ethyl acetate/petroleum ether) to give N-(4-(5-(2-(4,4-difluoropiperidin-1-yl)-6-methylpyrimidin-4-yl)-1,3,4-thiadiazol-2-yl) -3-(6-azaspiro[2.5]oct-6-yl)phenyl)-2-hydroxyethane-1-sulfonamide (46 mg, 11%). LCMS (ESI): $[M+H]^+$ =606.4. $^1$H NMR (400 MHz, DMSO-$d_6$) δ 10.20 (s, 1H), 8.29 (d, *J* = 8.7 Hz, 1H), 7.38 - 7.31 (m, 2H), 7.17 (dd, *J* = 8.7, 2.2 Hz, 1H), 4.95 (s, 1H), 3.98 (t, *J* = 5.8 Hz, 4H), 3.77 (t, *J* = 6.5 Hz, 2H), 3.36 (t, *J* = 6.5 Hz, 2H), 2.89 (t, *J* = 5.3 Hz, 4H), 2.45 (s, 3H), 2.10 - 1.99 (m, 4H), 1.65 (d, *J*= 7.0 Hz, 4H), 0.39 (s, 4H).

**Example 15:**

**N-(4-(5-(2-(4,4-difluoropiperidin-1-yl)-6-methylpyrimidin-4-yl)-1,3,4-oxadiazol-2 -yl)-3-(6-azaspiro[2.5]octan-6-yl)phenyl)-2-hydroxyethane-1-sulfonamide**

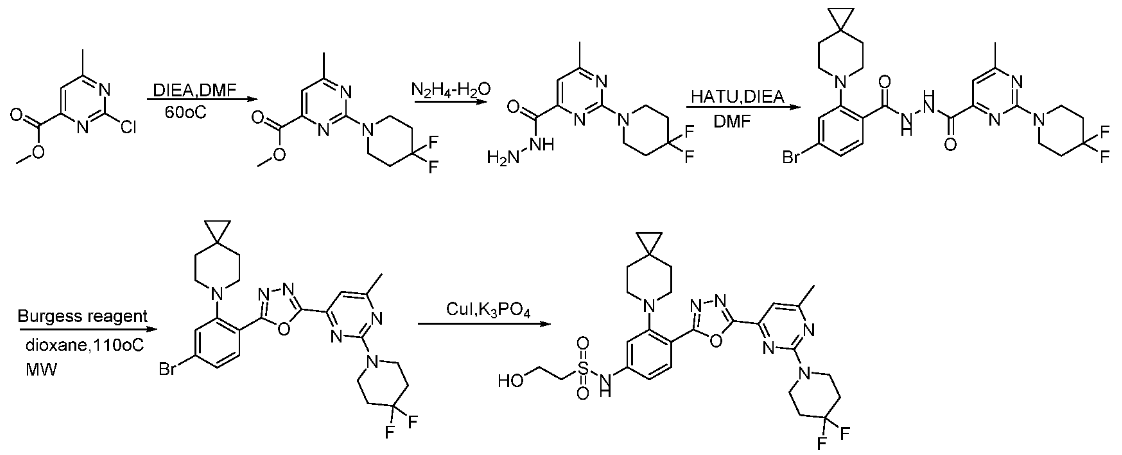


Step 1: Methyl 2-chloro-6-methylpyrimidin-4-carboxylate (3.00 g, 16.08 mmol), 4,4-difluoropiperidine hydrochloride (3.04 g, 19.29 mmol) and N,N-diisopropylethylamine (7.46 g, 56.54 mmol) were added to N,N-dimethylformamide (10 mL), and the reaction solution was stirred at 60°C for 3 h. The reaction solution was poured into the saturated aqueous ammonium chloride solution (50 mL) and extracted with ethyl acetate (50 mL × 3), and the organic phases were combined, washed with saturated aqueous sodium chloride solution (50 mL × 3), dried over anhydrous sodium sulfate, and concentrated to give crude methyl 2-(4,4-difluoropiperidin-1-yl)-6-methylpyrimidin-4-carboxylate (4.40 g, 86%). LCMS (ESI): $[M+H]^+$ =272.1.

Step 2: To 85% hydrazine hydrate (10 mL) was added 2-(4,4-difluoropiperidin-1-yl)-6-methylpyrimidin-4-carboxylate (4.40 g, 16.22 mmol), and the reaction solution was stirred at 40°C for 2 h. The reaction solution was poured into

water (50 mL), suction filtration was performed, and the solid obtained by filtration was washed with water (50 mL × 3) and dried to give crude 2-(4,4-difluoropiperidin-1-yl)-6-methylpyrimidin-4-carbohydrazide (4.40 g, 100%). LCMS (ESI): $[M+H]^+$ =272.1.

Step 3: To N,N-dimethylformamide (10 mL) was added 2-(4,4-difluoropiperidin-1-yl)-6-methylpyrimidin-4-carbohydrazide (2.00 g, 7.37 mmol), 4-bromo-2-(6-azaspiro[2.5]oct-6-yl) benzoic acid (2.74 g, 8.85 mmol), 2-(7-azobenzotriazole)-N,N,N',N'-tetramethylurea hexafluorophosphate (5.72 g, 14.74 mmol) and N,N-diisopropylethylamine (1.94 g, 14.74 mmol). The reaction solution was stirred at 25°C for 2 h. The reaction solution was poured into saturated aqueous ammonium chloride solution (50 mL) and extracted with ethyl acetate (50 mL × 3), and the organic phases were combined, dried over anhydrous sodium sulfate, and concentrated to give the crude. The crude was washed with methanol (50 mL × 3) to give N'-(4-bromo-2-(6-azaspiro[2.5]octyl-6-yl)benzoyl)-2-(4,4-difluoropiperidin-1-yl)-6-m ethylpyrimidin-4-carbohydrazide (3.50 g, 84%). $^1$HNMR (400 MHz, Chloroform-d) δ 13.82 (s, 1H), 10.69 (s, 1H), 8.13 (d, J = 8.4 Hz, 1H), 7.56 (d, J = 1.9 Hz, 1H), 7.51 - 7.42 (m, 1H), 7.26 (s, 1H), 4.05 (dd, J = 6.8, 5.0 Hz, 4H), 3.05 (t, J = 5.4 Hz, 4H), 2.46 (s, 3H), 2.12 - 1.98 (m, 4H), 1.78 (s, 4H), 0.44 (s, 4H).

Step 4: N'-(4-bromo-2-(6-azaspiro[2.5]octyl-6-yl)benzoyl)-2-(4,4-difluoropiperidin-1-yl)-6-m ethylpyrimidin-4-carbohydrazide (800 mg, 1.42 mmol) and Burgess reagent (345 mg, 1.42 mmol) were added to 1,4-dioxane (5 mL), and the reaction solution was heated by microwave at 115°C for 2 h. The reaction solution was poured into water (30 mL) and extracted with ethyl acetate (30 mL × 3). The organic phases were combined, dried over anhydrous sodium sulfate, and concentrated to obtain the crude. The crude was purified by flash column chromatography (silica gel, 0-10% gradient of ethyl acetate/petroleum ether) to give 2-(4-bromo-2-(6-azaspiro[2.5]oct-6-yl)phenyl)-5-(2-(4,4-difluoropiperidin-1-yl)-6-me thylpyrimidin-4-yl)-1,3,4-oxadiazole (450 mg, 58%). $^1$H NMR (400 MHz, Chloroform-d) δ 7.90 (d, *J* = 8.4 Hz, 1H), 7.38-7.30 (m, 2H), 7.26 - 7.23 (m, 1H), 4.12 (t, *J* = 5.8 Hz, 4H), 3.05 (t, *J* = 5.3 Hz, 4H), 2.49 (s, 3H), 2.06 (tt, *J* = 13.7, 5.9 Hz, 4H), 1.67 (d, *J* = 70.8 Hz, 4H), 0.35 (s, 4H).

Step 5: To N,N-dimethylformamide (4 mL) was added 2-(4-bromo-2-(6-azaspiro[2.5]oct-6-yl)phenyl)-5-(2-(4,4-difluoropiperidin-1-yl)-6-me thylpyrimidin-4-yl)-1,3,4-oxadiazol (400 mg, 0.73 mmol), 2-hydroxy-1-sulfonamide (140 mg, 1.10 mmol), potassium phosphate (477 mg, 2.20 mmol), cuprous iodide (427 mg, 2.20 mmol) and (1S,2S)-(+)-N,N'-dimethyl-1,2-cyclohexanediamine (632 g, 4.40 mmol), nitrogen replacement was performed, and the reaction solution was reacted at 115°C for 4 h. The reaction solution was poured into the saturated aqueous ammonium chloride solution (30 mL) and extracted with ethyl acetate (30 mL×3), and the organic phases were combined, washed with the saturated aqueous sodium chloride solution (30 mL × 3), dried with anhydrous sodium sulfate, and concentrated to give the crude. The crude was purified by flash column chromatography (silica gel, 0-10% gradient of ethyl acetate/petroleum ether) to give N-(4-(5-(2-(4,4-difluoropiperidin-1-yl)-6-methylpyrimidin-4-yl)-1,3,4-oxadiazol-2-yl) -3-(6-azaspiro[2.5]oct-6-yl)phenyl)-2-hydroxyethane-1-sulfonamide (78 mg, 18%). LCMS (ESI): $[M+H]^+$ =590.2. $^1$H NMR (400 MHz, DMSO-$d_6$) δ 10.18 (s, 1H), 7.92 (d, *J* = 8.6 Hz, 1H), 7.36 (s, 1H), 7.07 (d, *J* = 2.1 Hz, 1H), 6.97 (dd, *J* = 8.5, 2.1 Hz, 1H), 4.94 (s, 1H), 4.02 (td, *J* = 5.3, 2.4 Hz, 4H), 3.77 (t, *J* = 6.5 Hz, 2H), 3.37 (t, *J* = 6.5 Hz, 2H), 2.92 (t, *J* = 5.2 Hz, 4H), 2.44 (s, 3H), 2.05 (qd, *J* = 13.8, 13.1, 6.6 Hz,4H), 1.52 (s, 4H), 0.31 (s, 4H).

**Examples 16 to 27 were prepared with reference to the synthetic route of Example 15:**

**Example 16:**

**2-hydroxy-N-(4-(5-(2-((1-hydroxy-2-methylpropan-2-yl)amino)-6-methylpyrimid in-4-yl)-1,3,4-oxadiazol-2-yl)-3-(6-azaspiro[2.5]octan-6-yl)phenyl)ethane-1-sulfon amide**

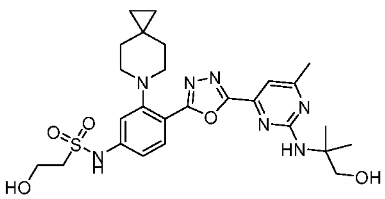

LCMS (ESI)m/z: $[M+H]^+$ = 558. $^1$H NMR (400 MHz, DMSO) δ 10.23 (s, 1H), 7.82 (d, *J* = 8.6 Hz, 1H), 7.36 (s, 1H), 7.11 (d, *J* = 2.0 Hz, 1H), 6.99 (dd, *J* = 8.4, 2.0 Hz, 1H), 5.02 (s, 2H), 3.77 (t, *J* = 6.4 Hz, 2H), 3.55 (s, 2H), 3.37 (t, *J* = 6.4 Hz, 2H), 2.97 (t, *J*= 5.2 Hz, 4H), 2.44 (s, 3H), 1.53 - 1.44 (m, 4H), 1.40 (s, 6H), 0.30 (s, 4H).

**Example 17:**

**2-hydroxy-N-(4-(5-(2-(3-hydroxypiperidin-1-yl)-6-methylpyrimidin-4-yl)-1,3,4-ox adiazol-2-yl)-3-(6-azaspiro[2.5] octan-6-yl)phenyl)ethane-1-sulfonamide**

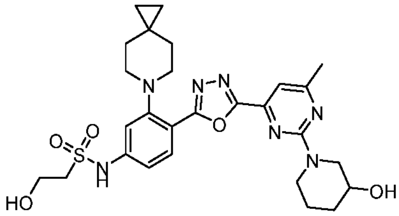

LCMS (ESI)m/z: $[M+H]^+$ = 570. $^1$H NMR (400 MHz, DMSO) δ 10.21 (s, 1H), 7.90 (d, *J* = 8.6 Hz, 1H), 7.24 (s, 1H), 7.10 (d, *J* = 2.0 Hz, 1H), 6.98 (dd, *J* = 8.6, 2.0 Hz, 1H), 5.35 - 5.31 (m, 2H), 4.58 - 4.37 (m, 2H), 3.77 (t, *J* = 6.4 Hz, 2H), 3.51 (dt, *J* = 8.8, 4.4 Hz, 1H), 3.37 (t, *J* = 6.4 Hz, 2H), 3.17 (ddd, *J* = 13.2, 9.6, 2.8 Hz, 1H), 3.05 - 2.89 (m, 5H), 2.41 (s, 3H), 1.98 - 1.88 (m, 1H), 1.82 - 1.71 (m, 1H), 1.61 - 1.38 (m, 6H), 0.31 (s, 4H).

**Example 18:**

**(R)-2-hydroxy-N-(4-(5-(6-methyl-2-(2-methylmorpholino)pyrimidin-4-yl)-1,3,4-o xadiazol-2-yl)-3-(6-azaspiro[2.5] octan-6-yl)phenyl)ethane-1-sulfonamide**

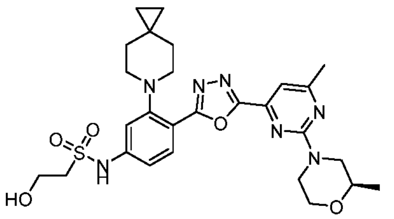

LCMS (ESI)m/z: $[M+H]^+$ = 570. $^1$H NMR (400 MHz, DMSO) δ 10.21 (s, 1H), 7.92 (d, *J* = 8.5 Hz, 1H), 7.34 (s, 1H), 7.08 (d, *J* = 2.1 Hz, 1H), 6.97 (dd, *J* = 8.4, 2.0 Hz, 1H), 4.59 (t, *J* = 14.0 Hz, 2H), 4.24 (s, 1H), 3.94 (dd, *J* = 11.2, 3.2 Hz, 1H), 3.77 (t, *J* = 6.4 Hz, 2H), 3.59 - 3.48 (m, 2H), 3.37 (t, *J* = 6.4 Hz, 2H), 3.02 (ddd, *J* = 15.2, 12.2, 3.6 Hz, 1H), 2.92 (t, *J* = 5.2 Hz, 4H), 2.71 (dd, *J* = 13.4, 10.4 Hz, 1H), 2.43 (s, 3H), 1.53 (s, 4H), 1.18 (d, *J* = 6.0 Hz, 3H), 0.31‘ (s, 4H).

**Example 19:**

**2-hydroxy-N-(4-(5-(2-(4-hydroxy-4-methylpiperidin-1-yl)-6-methylpyrimidin-4-y 1)-1,3,4-oxadiazol-2-yl)-3-(6-azaspiro [2.5] octan-6-yl)phenyl)ethane-1-sulfonamide**

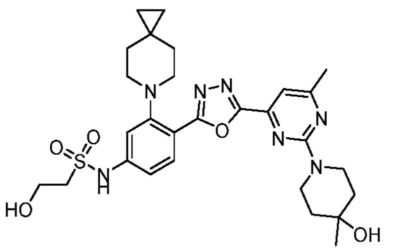

LCMS (ESI)m/z: $[M+H]^+$ = 584. $^1$H NMR (400 MHz, DMSO) δ 10.20 (s, 1H), 7.90 (d, *J* = 8.6 Hz, 1H), 7.23 (s, 1H), 7.08 (d, *J* = 2.0 Hz, 1H), 6.97 (dd, *J* = 8.4, 2.0 Hz, 1H), 5.00 (s, 2H), 4.36 (d, *J* = 12.4 Hz, 2H), 3.77 (t, *J* = 6.4 Hz, 2H), 3.60 -

3.42 (m, 2H), 3.37 (t, *J* = 6.4 Hz, 2H), 2.92 (t, *J* = 5.2 Hz, 4H), 2.40 (s, 3H), 1.61 - 1.22 (m, 8H), 1.17 (s, 3H), 0.31 (s, 4H).

Example 20:

N-(4-(5-(2-(4,4-difluoropiperidin-1-yl)-6-methoxypyrimidin-4-yl)-1,3,4-oxadiazol-2-yl)-3-(6-azaspiro[2.5]octan-6-yl)phenyl)-2-hydroxyethane-1-sulfonamide

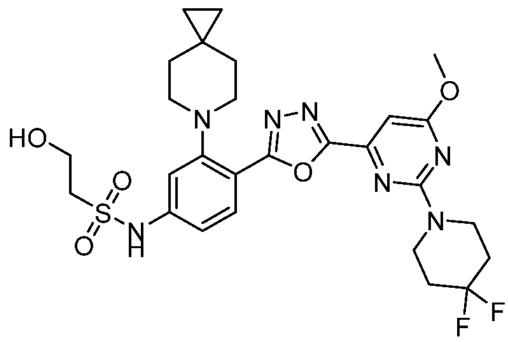

LCMS(ESI)m/z: $[M+H]^+$ =606.4, $^1H$ NMR (400 MHz, DMSO) δ 10.19 (s, 1H), 7.89 (d, *J* = 8.5 Hz, 1H), 7.06 (d, *J* = 2.0 Hz, 1H), 6.95 (dd, *J* = 8.5, 2.0 Hz, 1H), 6.83 (s, 1H), 4.98 (s, 1H), 4.02 (t, *J* = 5.8 Hz, 4H), 3.96 (s, 3H), 3.76 (t, *J* = 6.5 Hz, 2H), 3.35 (t, *J* = 6.5 Hz, 2H), 2.91 (t, *J* = 5.2 Hz, 4H), 2.08 (q, *J* = 7.2, 5.8 Hz, 4H), 1.52 (s, 4H), 0.31 (s, 4H).

Example 21:

**N-(4-(5-(2-(4,4-difluoropiperidin-1-yl)-6-hydroxypyrimidin-4-yl)-1,3,4-oxadiazol-2-yl)-3-(6-azaspiro [2.5] octan-6-yl)phenyl)-2-hydroxyethane-1-sulfonamide**

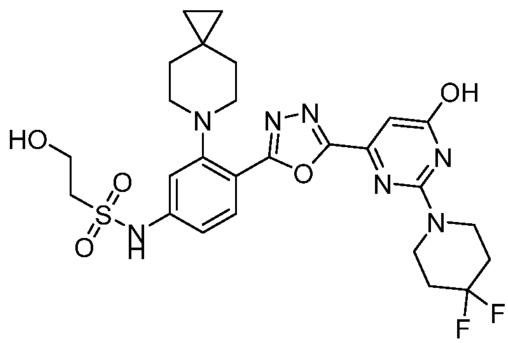

LCMS(ESI)m/z: $[M+H]^+$ =592.6, $^1H$ NMR (400 MHz, DMSO) δ 10.32 (s, 1H), 7.85 (d, J = 8.5 Hz, 1H), 7.06 (d, J = 2.1 Hz, 1H), 6.95 (dd, J = 8.5, 2.0 Hz, 1H), 6.51 (s, 1H), 3.92 (q, J = 4.0, 3.0 Hz, 4H), 3.78 (t, J = 6.4 Hz, 2H), 3.40 (s, 2H), 2.92 (t, J = 5.2 Hz, 4H), 2.03 (d, J = 15.6 Hz, 4H), 1.53 (s, 4H), 0.33 (s, 4H).

**Example 22:**

**N-(4-(5-(2-(4,4-difluoropiperidin-1-yl)-6-methoxypyridin-4-yl)-1,3,4-oxadiazol-2-yl)-3-(6-azaspiro[2.5]octan-6-yl) phenyl)-2-hydroxyethane-1-sulfonamide**

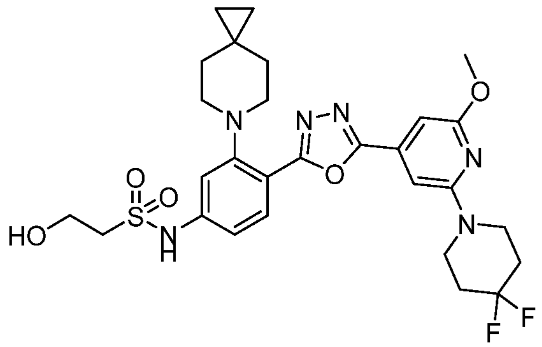

LCMS (ESI): $[M+H]^+$ =605.5, $^1$H NMR (400 MHz, DMSO-$d_6$) δ 10.17 (s, 1H), 7.91 (d, *J*= 8.5 Hz, 1H), 7.10 (d, *J*= 2.1 Hz, 1H), 7.01 - 6.95 (m, 2H), 6.65 (d, *J* = 0.8 Hz, 1H), 4.95 (t, *J* = 5.7 Hz, 1H), 3.88 (s, 3H), 3.77 (dt, *J* = 12.3, 5.9 Hz, 6H), 3.36 (t, *J* = 6.5 Hz, 2H), 2.93 (t, *J* = 5.2 Hz, 4H), 2.06 (td, *J* = 13.7, 6.7 Hz, 4H), 1.47 (t, *J* = 5.1 Hz, 4H), 0.32 (s, 4H).

**Example 23:**

**N-(4-(5-(2-(4,4-difluoropiperidin-1-yl)-3-fluoro-6-methylpyridin-4-yl)-1,3,4-oxadi azol-2-yl)-3-(6-azaspiro[2.5]octan-6-yl)phenyl)-2-hydroxyethane-1-sulfonamide**

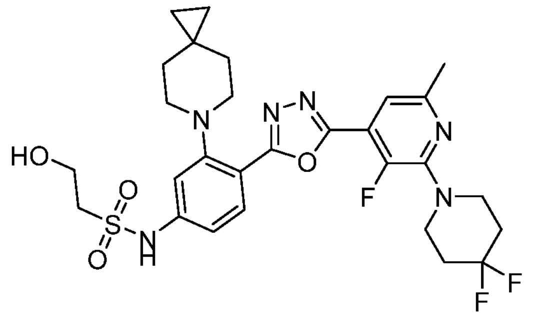

LCMS (ESI): $[M+H]^+$ = 607.3; $^1$H NMR (400 MHz, DMSO-$d_6$) δ ppm 10.75 - 9.64 (m, 1H), 7.88 (d, *J* = 8.8 Hz, 1H), 7.35 (d, *J* = 3.2 Hz, 1H), 7.09 (s, 1H), 6.98 (br d, *J* = 8.8 Hz, 1H), 5.31 - 4.55 (m, 1H), 3.76 (t, *J* = 6.4 Hz, 2H), 3.62 (br t, *J* = 4.8 Hz, 4H), 3.39 - 3.35 (m, 2H), 2.93 (br d, *J* = 4.8 Hz, 4H), 2.43 (s, 3H), 2.18 - 2.05 (m, 4H), 1.50 - 1.40 (m, 4H), 0.30 (s, 4H)

Example 24:

**N-(4-(5-(2-(4,4-difluoropiperidin-1-yl)-6-methylpyridin-4-yl)-1,3,4-oxadiazol-2-yl) -3-(6-azaspiro[2.5]octan-6-yl) phenyl)-2-hydroxyethane-1-sulfonamide**

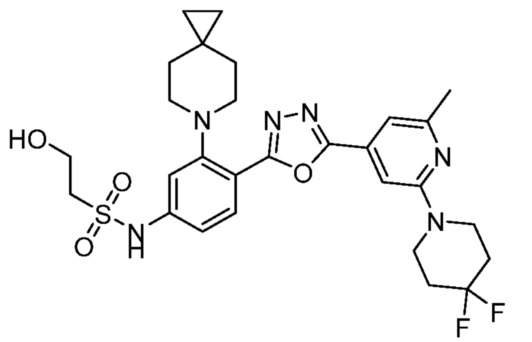

LCMS (ESI): $[M+H]^+$ = 589.9. $^1$H NMR (400 MHz, DMSO) δ 10.17 (s, 1H), 7.92 (d, J = 8.4 Hz, 1H), 7.25 (s, 1H),

7.18 (s, 1H), 7.10 (d, J = 2.0 Hz, 1H), 6.99 (dd, J = 8.4, 2.0 Hz, 1H), 4.94 (t, J = 5.6 Hz, 1H), 3.84-3.71 (m, 6H), 3.37 (t, J = 6.4 Hz, 2H), 3.00-2.89 (m, 4H), 2.44 (s, 3H), 2.08 - 1.97 (m, 4H), 1.54-1.43 (m, 4H), 0.32 (s, 4H).

**Example 25:**

**N-(4-(5-(6-(4,4-difluoropiperidin-1-yl)-4-methylpyridin-2-yl)-1,3,4-oxadiazol-2-yl) -3-(6-azaspiro[2.5]octan-6-yl) phenyl)-2-hydroxyethane-1-sulfonamide**

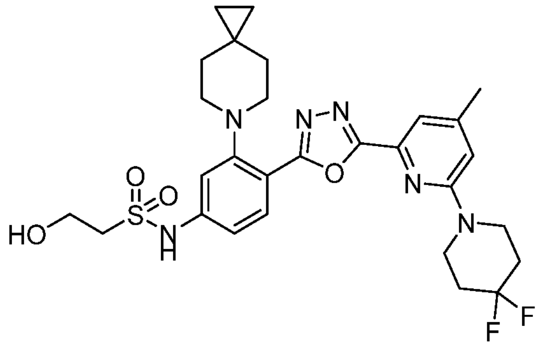

LCMS(ESI): $[M+H]^+$ =589.4. $^1$HNMR (400MHz, DMSO-$d_6$) δ 10.15 (s, 1H), 7.86 (d, *J* = 8.5 Hz, 1H), 7.43 (s, 1H), 7.06 (d, *J* = 1.9 Hz, 2H), 6.96 (dd, *J* = 8.5, 2.0 Hz, 1H), 4.96 (t, *J* = 5.6 Hz, 1H), 3.80 (dt, *J* = 28.5, 5.8 Hz, 6H), 3.30 (s, 2H), 2.92 (t, *J*= 5.2 Hz, 4H), 2.37 (s, 3H), 2.11-1.98 (m, 4H), 1.51 (s, 4H), 0.31 (s, 4H).

**Example 26:**

**N-(4-(5-(3-chloro-6-(4,4-difluoropiperidin-1-yl)-4-methylpyridin-2-yl)-1,3,4-oxadi azol-2-yl)-3-(6-azaspiro[2.5]octan-6-yl)phenyl)-2-hydroxyethane-1-sulfonamide**

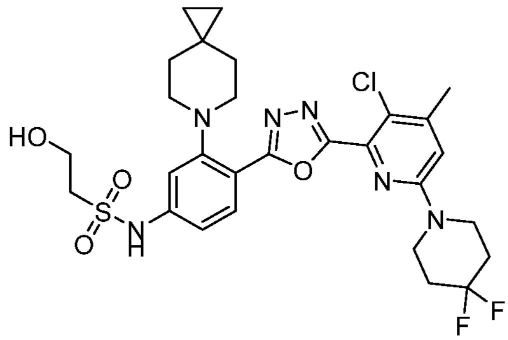

LCMS (ESI): $[M+H]^+$ =623.3. $^1$H NMR (400 MHz, DMSO-$d_6$) δ 10.16 (s, 1H), 7.82 (d, *J* = 8.4 Hz, 1H), 7.37 - 7.28 (m, 1H), 7.08 (d, *J* = 2.1 Hz, 1H), 6.97 (dd, *J* = 8.5, 2.1 Hz, 1H), 4.98 (s, 1H), 3.79 (q, *J* = 7.3, 6.7 Hz, 6H), 3.37 (d, *J* = 6.4 Hz, 2H), 2.93 (t, *J*= 5.2 Hz, 4H), 2.41 (s, 3H), 2.04 (dd, *J* = 13.0, 7.1 Hz, 4H), 1.46 (s, 4H), 0.30 (s, 4H).

**Example 27:**

**N-(4-(5-(4-(4,4-difluoropiperidin-1-yl)-6-methylpyrimidin-2-yl)-1,3,4-oxadiazol-2 -yl)-3-(6-azaspiro[2.5]octan-6-yl) phenyl)-2-hydroxyethanesulfonamide**

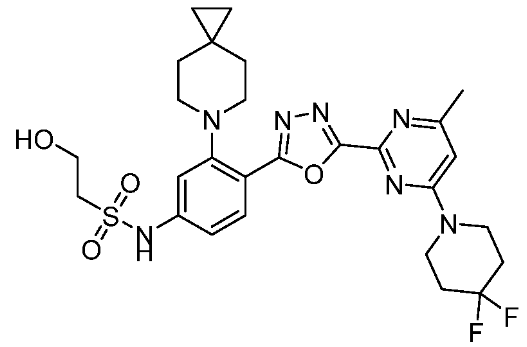

LCMS (ESI): $[M+H]^+$ = 590.4. $^1H$ NMR (400 MHz, DMSO-$d_6$) δ ppm 11.04 - 8.85 (m, 1H), 7.88 (d, *J* = 8.8 Hz, 1H), 7.10 - 7.02 (m, 2H), 6.96 (dd, *J* = 2.0, 8.4 Hz, 1H), 5.52 - 4.34 (m, 1H), 3.96 - 3.82 (m, 4H), 3.77 (t, *J* = 6.8 Hz, 2H), 3.36 (t, *J* = 6.8 Hz, 2H), 2.92 (br t, *J* = 4.8 Hz, 4H), 2.41 (s, 3H), 2.15 - 2.00 (m, 4H), 1.62 - 1.44 (m, 4H), 0.31 (s, 4H)

**Example 28:**

**2-(4-(5-(2-(4,4-difluoropiperidin-1-yl)-6-methylpyrimidin-4-yl)-1,3,4-oxadiazol-2-yl)-3-(6-azaspiro[2.5]octan-6-yl) phenyl)isothiazolidine 1,1-dioxide**

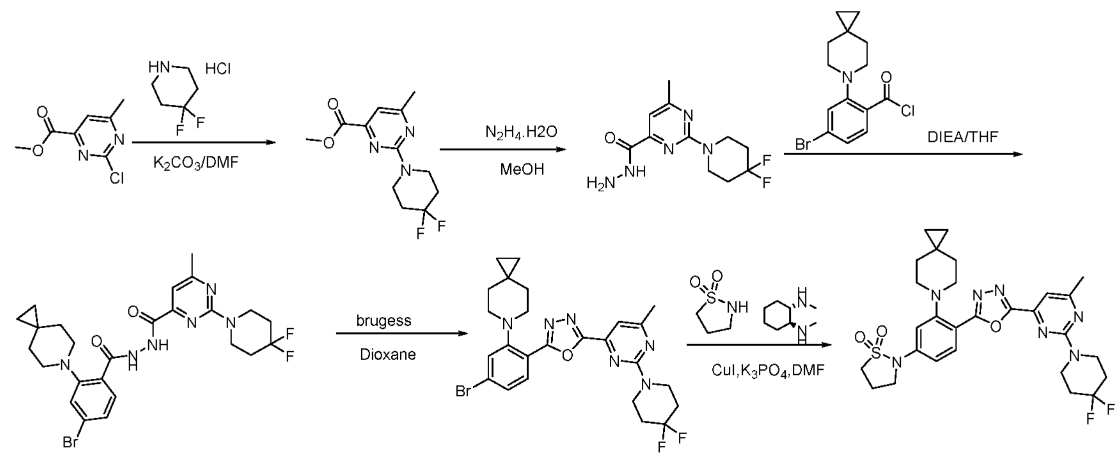


Step 1: Potassium carbonate (13.6 g, 96.4 mmol) was added to a solution of 4,4-difluoropiperidine hydrochloride (20.47 g, 128.62 mmol) and methyl 2-chloro-6-methylpyrimidin-4-carboxylate (6.0 g, 32.15 mmol) in N,N-dimethylacetamide (60 mL), and the mixture was heated to 120°C and stirred for 12 h. After completion of the reaction, water (120 mL) was added to the reaction mixture, which was extracted with ethyl acetate (120 mL × 3). The organic phases were combined, dried over anhydrous sodium sulfate, and filtered. The filtrate was concentrated under reduced pressure to give the residue. The residue was purified by flash column chromatography (silica gel, 0-25% gradient of ethyl acetate/petroleum ether) to give methyl 2-(4,4-difluoropiperidin-1-yl)-6-methylpyrimidin-4-carboxylate (4.7 g, 17.32 mmol, yield: 53.89%) as a yellow solid. LCMS (ESI): $[M+H]^+$ = 272

Step 2: Methyl 2-(4,4-difluoropiperidin-1-yl)-6-methylpyrimidin-4-carboxylate (2.0 g, 7.37 mmol) was added to a solution of methanol (20 mL). After being stirred at -10°C for 5 min, the hydrazine hydrate (1.15 g, 18.43 mmol) solution was slowly added dropwise to the mixture, which was continued stirring for 4 h. After completion of the reaction, the mixture was concentrated under reduced pressure into a solid mixture, acetonitrile (4 mL) was added for slurrying, and the solid was filtered. The cake was rinsed 2 times with acetonitrile (1 mL), collected, and dried to give 2-(4,4-difluoropiperidin-1-yl)-6-methylpyrimidin-4-formylhydrazine (1.8 g, 6.61 mmol, yield: 90%) as a white solid. LCMS (ESI): $[M+H]^+$ = 272

Step 3: A solution of N,N-diisopropylethylamine (2.37 g, 18.38 mmol) was added into a solution of 2-(4,4-difluoropiperidin-1-yl)-6-methylpyrimidin-4-formylhydrazine (1.0 g, 3.6 mmol) and 4-bromo-2-(6-azaspiro[2.5]octan-6-yl)

benzoyl chloride (1.0 g, 3.6 mmol) in THF (20 mL). The mixture was stirred at ambient temperature for 4 h. The stirring was stopped after solid compounds were precipitated, the solids were filtered, and the cake was rinsed with THF (1 mL), collected, and dried to give 4-bromo-2-(6-azaspiro[2.5]octane-6-ylbenzoyl)-2-(4,4-difluoropiperidin-1-yl)-6-meth ylpyrimidin-4-formylhydrazine (1.1 g, 1.95 mmol, yield: 53.4%) as a yellow solid. LCMS (ESI): $[M+H]^+$ = 563

Step 4: A solution of Burgess reagent (1.69 g, 7.1 mmol) was added to a solution of 4-bromo-2-(6-azaspiro[2.5] octane-6-ylbenzoyl)-2-(4,4-difluoropiperidin-1-yl)-6-meth ylpyrimidin-4-formylhydrazine (1.0 g, 1.77 mmol) in dioxane (10 mL), and the mixture was heated to 120°C and stirred for 12 h. After completion of the reaction, water (20 mL) was added to the reaction mixture, which was extracted with ethyl acetate (40 mL × 3). The organic phases were combined, dried over anhydrous sodium sulfate, and filtered, and The filtrate was concentrated under reduced pressure to give the residue. The residue was purified by flash column chromatography (silica gel, 0-50% gradient of ethyl acetate/petroleum ether) to give 2-(4-bromo-2-6-azaspiro[2.5]octan-6-phenyl)-5-(4,4-difluoropiperidin-1-yl)-6-methyl pyrimidin-4-yl-1,3,4-oxadiazole (400 mg, 0.73 mmol, yield: 41.3%) as a yellow solid. LCMS (ESI): $[M+H]^+$ = 545

Step 5: Under nitrogen protection, 2-hydroxyethane-1-sulfonamide (277 mg, 2.17 mmol), cuprous iodide (105 mg, 0.54 mmol), potassium phosphate (471 mg, 2.2 mmol) and (1R, 2R)-(-)-N,N'-dimethyl-1,2-cyclohexanediamine (316.1 mg, 2.2 mmol) were added in a vial. Then, the reaction vial was added with N,N-dimethylformamide (6 mL), and the mixture was stirred at 50°C for 0.5 h. After that, 2-(4-bromo-2-6-azaspiro[2.5]octan-6-phenyl)-5-(4,4-difluoropiperidin-1-yl)-6-methyl pyrimidin-4-yl-1,3,4-oxadiazole (300 mg, 0.55 mmol) was added, and the mixture was stirred at 50°C for 5 min. The reaction system was heated to 120°C and stirred for 12 h. After the completion of the reaction, the system was cooled to ambient temperature, poured into water (5 mL), and extracted with ethyl acetate (30 mL × 3), the organic phases were dried over anhydrous sodium sulfate, filtered, washed with the saturated aqueous sodium chloride solution (50 mL), and concentrated under reduced pressure, and the crude was purified with preparative liquid phase (column: BRIX-2860 AQ- C18, 20 × 250 mm 10 um; mobile phase A: water; mobile phase B: acetonitrile; flow rate: 20 mL/min; elution gradient: increase from 15% to 65% in 30 min for mobile phase B; detection wavelength: 220 nm; retention time: 40 min) to give 2-(4-(5-(2-(4,4-difluoropiperidin-1-yl)-6-methylpyrimidin-4-yl)-1,3,4-oxadiazol-2-yl) -3-(6-azaspiro[2.5]octan-6-yl)phenyl)isothiazolidine 1,1-dioxide (10 mg. 0.544 mmol, yield: 3.04%) as a yellow solid. LCMS (ESI)m/z: $[M+H]^+$= 586. $^1$H NMR (400 MHz, DMSO) δ 8.00 - 7.95 (m, 1H), 7.36 (s, 1H), 7.00 - 6.89 (m, 2H), 4.20 (s, 2H), 4.03 (t, *J = 5.6* Hz, 4H), 3.85 (t, *J* = 6.4 Hz, 2H), 3.60 (t, *J* = 7.2 Hz, 2H), 2.44 (d, *J* = 4.0 Hz, 5H), 2.07 (td, *J* = 13.6, 6.4 Hz, 4H), 1.53 (d, *J* = 6.4 Hz, 4H), 0.32 (s, 4H).

**Example 29:**

**N-(4-(5-(8-(4,4-difluoropiperidin-1-yl)-1,7-naphthyridin-6-yl)-1,3,4-oxadiazol-2-y 1)-3-(6-azaspiro[2.5]octan-6-yl) phenyl)-2-hydroxyethanesulfonamide**

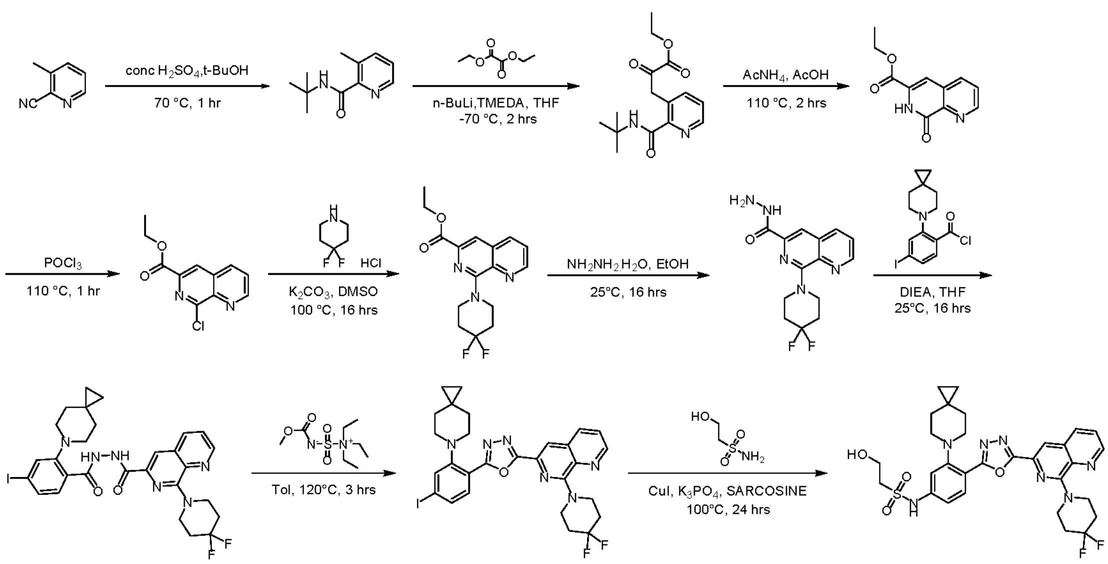

Step 1: In tert-butanol (30.0 mL) was dissolved 3-methylcyanopyridine (10.0 g, 84.6 mmol), and the mixture was stirred at 70°C for 20 min, added with concentrated sulfuric acid (10.0 mL, 178 mmol), and stirred at 70°C for 30 min. The reaction solution was poured into 150 mL of water (adding 2 mL of liquor ammonia) and extracted with ethyl acetate (100 mL × 2), and the organic phase was dried, filtered, and concentrated. The residue was purified by flash column chromatography (silica gel, 0-8% gradient THF/petroleum ether) to give N-(tert-butyl)-3-methylmethylpyridineamide(14.5 g, 72.8 mmol, yield: 86%) as a yellow oil. LCMS (ESI): $[M+H]^+$ = 193.1.

Step 2: N-(tert-butyl)-3-methylmethylpyridineamide (14.5 g, 75.4 mmol) was dissolved in THF (400 mL), n-butyl lithium (10.6 g, 166 mmol) and [2-(dimethylamino)ethyl]dimethylamine (8.85 g, 75.4 mmol) were added dropwise under nitrogen protection at -70 °C. The reaction solution was stirred at -70 °C for 30 min, diethyl oxalate (22.0 g, 151 mmol) dissolved in THF (200 mL) was added dropwise, and the reaction solution was stirred at -70 °C for 1 h. The reaction solution was quenched with the saturated ammonium chloride solution (500 mL) and extracted with ethyl acetate (400 mL × 2), and the organic phase was dried, filtered, and concentrated to give ethyl 3-(2-(tert-butylaminocarbonyl)pyridin-3-yl)-2-oxopropylidene (29.1 g, 39.2 mmol, yield: 52%) as a brown oil crude for direct use in the next step. LCMS (ESI): $[M+H]^+$ = 293.1

Step 3: Ethyl 3-(2-(tert-butylaminocarbonyl)pyridin-3-yl)-2-oxopropylidene (29.0 g, 99.2 mmol) was dissolved in acetic acid (290.0 mL), ammonium acetate (15.3 g, 198 mmol) was added, and the mixture was stirred at 110°C for 2 h. The reaction solution was concentrated under reduced pressure. The residue was purified by flash column chromatography (silica gel, 0-4% gradient of THF/petroleum ether) to give ethyl 8-oxo-7,8-dihydro-1,7-naphthyridine-6-carboxylate (3.30 g, 14.9 mmol, yield: 15%) as a brown solid. LCMS (ESI): $[M+H]^+$ = 219.1.

Step 4: Ethyl 8-oxo-7,8-dihydro-1,7-naphthyridine-6-carboxylate (3.30 g, 15.1 mmol) was added to phosphorus oxychloride (35.0 mL), and the mixture was stirred at 110°C for 1 h. The reaction solution was concentrated under reduced pressure, and dichloromethane (100 mL) was added. The diluent was slowly added into water (500 mL) in a stirring state, and the saturated sodium bicarbonate solution was used to adjust the pH to 8. The aqueous phase was extracted with dichloromethane (500 mL × 2), and the organic phase was dried, filtered, and concentrated to give ethyl 8-chloro-1,7-naphthyridine-6-carboxylate (3.30 g, 12.5 mmol, yield: 83%) as a brown solid crude for direct use in the next step. LCMS (ESI): $[M+H]^+$ = 237.0

Step 5: Ethyl 8-chloro-1,7-naphthyridine-6-carboxylate (1.00 g, 4.23 mmol) and 4,4-difluoropiperidine hydrochloride (1.20 g, 7.61 mmol) was dissolved in DMSO (20.0 mL), potassium carbonate (1.75 g, 12.67 mmol) was added, and the mixture was stirred at 100°C for 16 h. The reaction solution was diluted with water (100 mL) and extracted with ethyl acetate 400 mL (200 mL × 2), and the organic phase was washed with saturated saline (300 mL), dried, filtered, and concentrated. The residue was purified by flash column chromatography (silica gel, 0-14% gradient of THF/petroleum ether) to give ethyl 8-(4,4-difluoropiperidin-1-yl)-1,7-naphthyridine-6-carboxylate (0.72 g, 2.19 mmol, yield: 52%) as a yellow solid. LCMS (ESI): $[M+H]^+$ = 322.1. $^1H$ NMR (400 MHz, DMSO-$d_6$) δ ppm 8.97 (dd, *J* = 2.0, 4.4 Hz, 1H), 8.48 (dd, *J* = 2.0, 8.4 Hz, 1H), 8.02 (s, 1H), 7.77 (dd, *J* = 4.0, 8.4 Hz, 1H), 4.35 (q, *J* = 7.2 Hz, 2H), 4.20 - 4.12 (m, 4H), 2.23 - 2.09 (m, 4H), 1.34 (t, *J* = 7.2 Hz, 3H)

Step 6: Ethyl 8-(4,4-difluoropiperidin-1-yl)-1,7-naphthyridine-6-carboxylate (900 mg, 2.80 mmol) was dissolved in anhydrous ethanol (18.0 mL), hydrazine hydrate (1200 mg, 23.5 mmol) was added, and the mixture was stirred at 25°C for 16 h. The reaction solution was concentrated under reduced pressure to give 8-(4,4-difluoropiperidin-1-yl)-1,7-naphthyridine-6-formylhydrazine (840 mg, 2.68 mmol, yield: 96%) as a white solid crude for direct use in the next step. LCMS (ESI): $[M+H]^+$ = 308.1. $^1H$ NMR (400 MHz, DMSO-$d_6$) δ ppm 9.70 (br s, 1H), 8.93 (dd, *J* = 1.6, 4.4 Hz, 1H), 8.46 (dd, *J* = 1.6, 8.4 Hz, 1H), 7.91 (s, 1H), 7.74 (dd, *J* = 4.0, 8.0 Hz, 1H), 4.61 (br d, *J*= 2.8 Hz, 2H), 4.28 - 4.18 (m, 4H), 2.20 - 2.06 (m, 4H)

Step 7: In dichloromethane (30.0 mL) was dissolved 4-iodo-2-(6-azaspiro[2.5]octane-6-yl)benzoic acid (2.00 g, 133%), thionyl chloride (0.99 g, 8.40 mmol) and N,N-dimethylformamide (0.03 g, 0.42 mmol) were added, and the mixture was stirred at 25°C for 16 h. The reaction solution was concentrated under reduced pressure to give 4-iodo-2-(6-azaspiro[2.5]octane-6-yl)benzoyl chloride (2.00 g, 4.20 mmol, crude) as a brown solid crude for direct use in the next step. LCMS (ESI): $[M+H]^+$ = 371.9

Step 8: In THF (36.00 mL) was dissolved 8-(4,4-difluoropiperidin-1-yl)-1,7-naphthyridine-6-formylhydrazine (0.89 g, 2.90 mmol), diisopropylethylamine (3.74 g, 28.9 mmol) and 4-iodo-2-(6-azaspiro[2.5]octane-6-yl)benzoyl chloride (1.31 g, 3.48 mmol) were added, and the mixture was stirred at 25°C for 16 h. The reaction solution was concentrated under reduced pressure and purified by slurrying with dichloromethane to give 8-(4,4-difluoropiperidin-1-yl)-N'-(4-iodo-2-(6-azaspiro[2.5]octane-6-yl)benzoyl)-1,7-naphthyridine-6-formylhydrazine (1.50 g, 2.31 mmol, yield: 80%) as a brown solid. LCMS (ESI): $[M+H]^+$ = 647.1

Step 9: In toluene (14.00 mL) was dissolved 8-(4,4-difluoropiperidin-1-yl)-N'-(4-iodo-2-(6-azaspiro[2.5]octane-6-yl) benzoyl)-1,7-naphthyridine-6-formylhydrazine (700 mg, 1.08 mmol), Burgess reagent (789 mg, 3.25 mmol) was added, and the mixture was stirred at 120°C for 3 h. The reaction solution was purified by flash column chromatography (silica gel, 0-18% gradient of THF/petroleum ether) to give 2-(8-(4,4-difluoropiperidin-1-yl)-1,7-naphthyridin-6-yl)-5-(4-iodo-2-(6-azaspiro[2.5]o ctan-6-yl)phenyl)-1,3,4-oxadiazole (700 mg, 1.08 mmol, yield: 100%) as a yellow solid. LCMS (ESI): $[M+H]^+$ = 629.1. $^1$H NMR (400 MHz, DMSO-$d_6$) δ ppm 8.74 (dd, $J$= 1.6, 4.0 Hz, 1H), 8.27 (dd, $J$ = 1.6, 8.0 Hz, 1H), 7.97 (s, 1H), 7.56 (dd, $J$ = 4.0, 8.0 Hz, 1H), 7.41 (d, $J$ = 8.0 Hz, 1H), 7.31 - 7.25 (m, 2H), 4.05 - 3.98 (m, 4H), 2.26 - 2.24 (m, 4H), 2.02 - 1.92 (m, 4H), 1.34 - 1.20 (m, 4H), 0.05 (s, 4H)

Step 10: In N,N-dimethylformamide (14.0 mL) was dissolved 2-(8-(4,4-difluoropiperidin-1-yl)-1,7-naphthyridin-6-yl)-5-(4-iodo-2-(6-azaspiro[2.5]o ctan-6-yl)phenyl)-1,3,4-oxadiazole (700 mg, 1.11 mmol) and 2-hydroxyethanesulfonamide (278 mg, 2.23 mmol). Potassium phosphate (709 mg, 3.34 mmol), cuprous iodide (212 mg, 1.11 mmol) and 2-(methylamino)acetic acid (99.2 mg, 1.11 mmol) were added, and the mixture was stirred at 100 °C for 24 h under nitrogen protection. The reaction solution was diluted with 100 mL of water and 1 mL $NH_3H_2O$, and extracted with ethyl acetate 400 mL (200 mL × 2). The organic phase was dried, filtered, and concentrated. The residue was purified by normal phase separation (NP-5, 30-80% gradient of n-hexane/ethanol (0.1% liquor ammonia)) to give N-(4-(5-(8-(4,4-difluoropiperidin-1-yl)-1,7-naphthyridin-6-yl)-1,3,4-oxadiazol-2-yl)-3 -(6-azaspiro[2.5]octan-6-yl) phenyl)-2-hydroxyethanesulfonamide (38.0 mg, 0.05 mmol, yield: 5%) as a yellow solid. LCMS (ESI): $[M+H]^+$ = 626.3. $^1$H NMR (400 MHz, DMSO-$d_6$) δ ppm 10.64 - 9.66 (m, 1H), 8.98 (dd, $J$ = 1.6, 4.0 Hz, 1H), 8.52 (dd, $J$= 1.6, 8.4 Hz, 1H), 8.21 (s, 1H), 7.87 (d, $J$ = 8.4 Hz, 1H), 7.80 (dd, $J$ = 4.0, 8.4 Hz, 1H), 7.06 (d, $J$ = 1.6 Hz, 1H), 6.96 (dd, $J$ = 1.6, 8.4 Hz, 1H), 4.26 (br d, $J$ = 4.8 Hz, 4H), 3.77 (t, $J$ = 6.4 Hz, 2H), 3.37 (t, $J$ = 6.4 Hz, 2H), 2.94 (br d, $J$ = 4.4 Hz, 4H), 2.28 - 2.16 (m, 4H), 1.58 - 1.48 (m, 4H), 0.30 (s, 4H)

**Examples 30** to **33** were prepared with reference to the synthetic route of **Example 29:**

**Example 30:**

**N-(4-(5-(8-(4,4-difluoropiperidin-1-yl)-7-fluoroquinolin-6-yl)-1,3,4-oxadiazol-2-yl) -3-(6-azaspiro[2.5]octan-6-yl) phenyl)-2-hydroxyethane-1-sulfonamide**

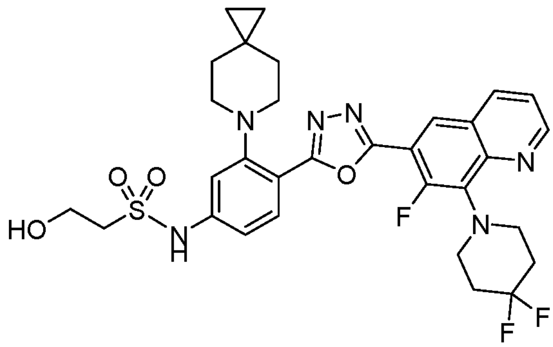

LCMS (ESI): $[M+H]^+$ = 643.2; $^1$H NMR (400 MHz, DMSO-$d_6$) δ ppm 10.48 - 9.84 (m, 1H), 9.12 - 8.90 (m, 1H), 8.69 - 8.55 (m, 1H), 8.54 - 8.43 (m, 1H), 7.93 (br d, $J$ = 8.0 Hz, 1H), 7.64 (br d, $J$ = 2.4 Hz, 1H), 7.17 - 7.08 (m, 1H), 7.01 (br d, $J$ = 7.6 Hz, 1H), 5.26 - 4.70 (m, 1H), 3.87 - 3.73 (m, 2H), 3.69 - 3.54 (m, 4H), 3.44 - 3.37 (m, 2H), 3.06 - 2.88 (m, 4H), 2.30 - 2.11 (m, 4H), 1.49 (br s, 4H), 0.30 (s, 4H)

**Example 31:**

**N-(4-(5-(6-(4,4-difluoropiperidin-1-yl)-5-(1-methyl-1H-pyrazol-4-yl)pyridin-2-yl) -1,3,4-oxadiazol-2-yl)-3-(6-azaspiro[2.5]octan-6-yl)phenyl)-2-hydroxyethane-1-su lfonamide**

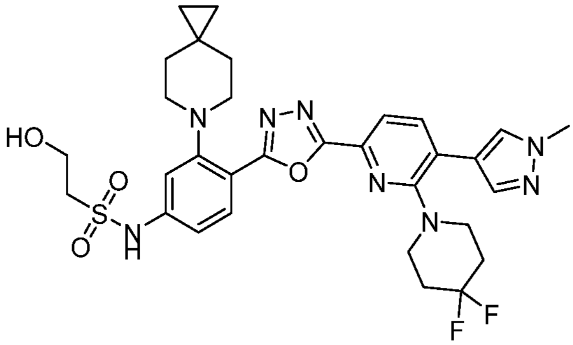

LCMS (ESI): $[M+H]^+$ = 655.4. $^1H$ NMR (600 MHz, DMSO) δ 10.14 (s, 1H), 8.36 (s, 1H), 8.06 (s, 1H), 8.01 (d, *J* = 7.6 Hz, 1H), 7.89 (d, *J* = 7.6 Hz, 1H), 7.87 (d, *J* = 8.4 Hz, 1H), 7.06 (d, *J* = 2.0 Hz, 1H), 6.95 (dd, *J* = 8.4, 2.0 Hz, 1H), 4.94 (s, 1H), 3.93 (s, 3H), 3.77 (t, *J* = 6.4 Hz, 2H), 3.37 (t, *J* = 6.4 Hz, 2H), 3.27 (t, *J* = 5.6 Hz, 4H), 2.93 (t, *J*= 5.2 Hz, 4H), 2.22-2.09 (m, 4H), 1.54 (s, 4H), 0.30 (s, 4H).

**Example 32:**

**N-(4-(5-(1-(4,4-difluoropiperidin-1-yl)pyrrolo[1,2-c])pyrimidin-3-yl)-1,3,4-oxadia zol-2-yl)-3-(6-azaspiro[2.5]octan-6-yl) phenyl)-2-hydroxyethanesulfonamide**

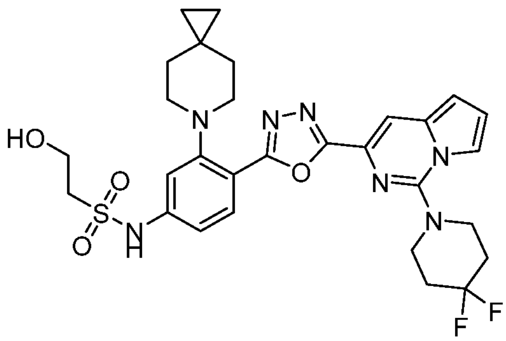

LCMS (ESI): $[M+H]^+$ = 614.3. $^1H$ NMR (400 MHz, DMSO-$d_6$) 10.14 (s, 1H), 8.11 (s, 1H), 7.87 (d, *J* = 8.4 Hz, 1H), 7.70 (d, *J* = 2.8 Hz, 1H), 7.11 - 7.03 (m, 2H), 6.96 (dd, *J* = 2.0, 8.4 Hz, 1H), 6.89 - 6.87 (m, 1H), 4.95 (t, *J* = 5.6 Hz, 1H), 3.77 (q, *J* = 6.4 Hz, 2H), 3.63 - 3.50 (m, 4H), 3.41 - 3.34 (m, 2H), 3.00 - 2.88 (m, 4H), 2.38 - 2.23 (m, 4H), 1.70 - 1.41 (m, 4H), 0.31 (s, 4H)

**Example 33:**

**N-(4-(5-(7-(4,4-difluoropiperidin-1-yl)furano[2,3-c])pyridin-5-yl)-1,3,4-oxadiazol-2-yl)-3-(6-azaspiro[2.5]octan-6-yl) phenyl)-2-hydroxyethanesulfonamide**

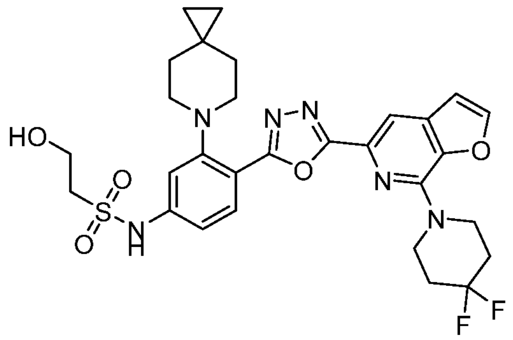

LCMS (ESI): $[M+H]^+$ = 615.7. $^1H$ NMR (400 MHz, DMSO-$d_6$) δ ppm 12.72 (br s, 1H), 10.14 (s, 1H), 8.26 (d, *J* = 2.0 Hz, 1H), 8.14 (s, 1H), 8.01 (s, 1H), 7.84 (d, *J* = 8.4 Hz, 1H), 7.16 (d, *J* = 2.0 Hz, 1H), 7.06 (d, *J* = 2.0 Hz, 1H), 6.96 (dd, *J* = 2.0, 8.4 Hz, 1H), 4.95 (br s, 1H), 4.15 - 4.03 (m, 4H), 3.83 - 3.73 (m, 2H), 3.42 - 3.36 (m, 2H), 3.02 - 2.85 (m, 4H), 2.24 - 2.10 (m, 4H), 1.62 - 1.42 (m, 4H), 0.30 (s, 4H)

**Example 34:**

**N-(4-(4-(2-(4,4-difluoropiperidin-1-yl)-6-methylpyrimidin-4-yl)-1H-1,2,3-triazol-1-yl)-3-(6-azaspiro[2.5]octan-6-yl) phenyl)-2-hydroxyethanesulfonamide**

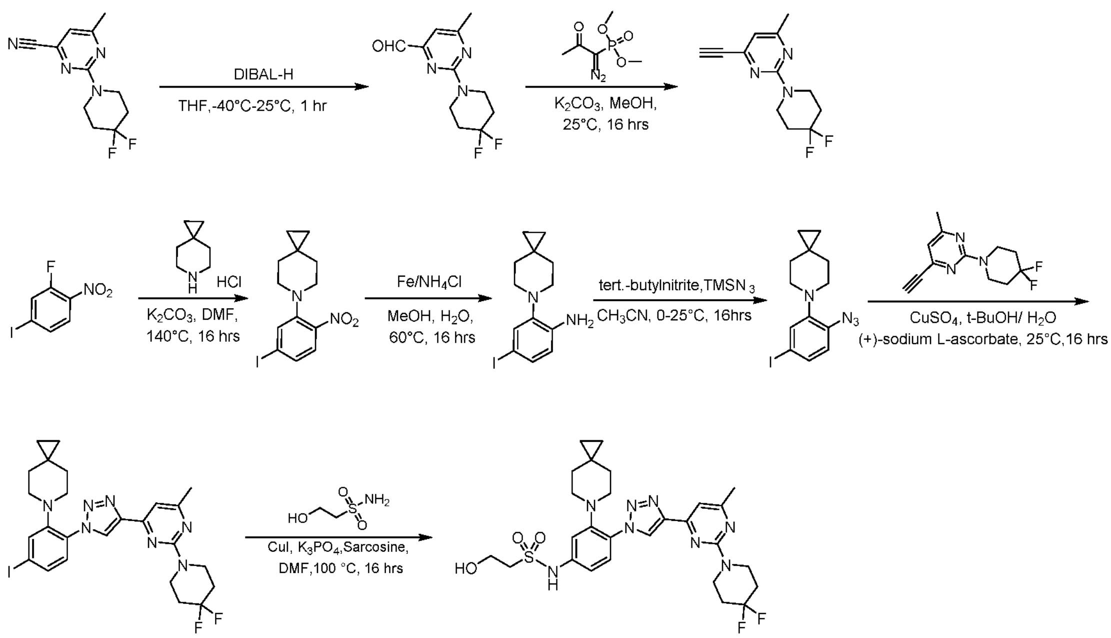


Step 1: A solution of diisobutylaluminium hydride 1 M in toluene (25.2 mL, 25.2 mmol) was added to a solution of 2-(4,4-difluoropiperidin-1-yl)-6-methylpyrimidin-4-carbonitrile (2.00 g, 8.39 mmol) in THF at -40°C. The mixture was slowly heated to 25°C and stirred for 1 h. The mixture was quenched with ice water (20 mL) and extracted with ethyl acetate (20 mL × 2). The organic phases were combined, dried over anhydrous sodium sulfate, and filtered, and The filtrate was concentrated under reduced pressure to give the residue. The residue was purified by flash column chromatography (silica gel, 0-8% gradient of ethyl acetate/petroleum ether) to give 2-(4,4-difluoropiperidin-1-yl)-6-methylpyrimidin-4-carbaldehyde (0.35 g, 1.45 mmol, yield: 17%) as a yellow oil. LCMS (ESI): $[M+H]^+$ =241.9.

Step 2: Dimethyl (1-diazo-2-oxopropylidene) phosphonate (0.33 g, 1.74 mmol) was added to a solution of 2-(4,4-difluoropiperidin-1-yl)-6-methylpyrimidin-4-carbaldehyde (0.35 g, 1.45 mmol) and potassium carbonate (0.40 g, 2.90 mmol) in methanol (7 mL). The mixture was stirred at 25°C for 16 h. The reaction mixture was diluted with water (20 mL) and extracted with ethyl acetate (20 mL×2). The organic phases were combined, dried over anhydrous sodium sulfate, and filtered, and The filtrate was concentrated under reduced pressure to give 2-(4,4-difluoropiperidin-1-yl)-4-ethynyl-6-methylpyrimidine (0.30 g, 1.26 mmol, yield: 87%) as a yellow oil. LCMS (ESI): $[M+H]^+$ = 237.9.

Step 3: To a solution of 2-fluoro-4-iodo-1-nitrobenzene (3.00 g, 11.2 mmol) and potassium carbonate (4.65 g, 33.7 mmol) in DMSO (45 mL) was added 6-azaspiro[2.5]octane hydrochloride (1.82 g, 12.3 mmol). The mixture was heated to 140°C and stirred for 16 h. Water (100 mL) was poured into the reaction solution, which was extracted with ethyl acetate (100 mL × 3). The organic phases were combined, dried over anhydrous sodium sulfate, and filtered, and The filtrate was concentrated under reduced pressure to give the residue. The residue was purified by flash column chromatography (silica gel, 0-10% gradient of ethyl acetate/petroleum ether) to give 6-(5-iodo-2-nitrophenyl)-6-azaspiro[2.5]octane (3.00 g, 8.37 mmol, yield: 75%) as a yellow solid. LCMS (ESI): $[M+H]^+$ = 358.8.

Step 4: Ammonium chloride (1.36 g, 25.1 mmol) and iron powder (4.73 g, 83.7 mmol) were added to a solution of 6-(5-iodo-2-nitrophenyl)-6-azaspiro[2.5]octane (3.00 g, 8.37 mmol) in methanol (60 mL) and water (20 mL). The mixture was heated to 60°C and stirred for 16 h. The reaction solution was filtered with diatomite and the filtrate was extracted with ethyl acetate (100 mL × 3). The organic phases were combined, dried over anhydrous sodium sulfate, and filtered. The filtrate was concentrated under reduced pressure to give the residue. The residue was purified by flash column chromatography (silica gel, 0-10% gradient of ethyl acetate/petroleum ether) to give 4-iodo-2-(6-azaspiro[2.5]octan-6-yl)aniline (2.20 g, 6.69 mmol, yield: 80%) as a yellow solid. LCMS (ESI): $[M+H]^+$ =329.0.

Step 5: Tert-butyl nitrite (1.09 g, 10.3 mmol) was added to a solution of 4-iodo-2-(6-azaspiro[2.5]octan-6-yl) aniline (2.26 g, 6.89 mmol) in acetonitrile (40 mL) at 0°C. Then, azidotrimethylsilane (1.19 g, 10.3 mmol) was added to the reaction solution and the mixture was stirred at 25°C for 16 h. The reaction solution was poured into water (40 mL) and extracted with ethyl acetate (40 mL×3). The organic phases were combined, dried over anhydrous sodium sulfate, and filtered. The filtrate was concentrated under reduced pressure to give the residue. The residue was purified by flash column chromatography (silica gel, 0-10% gradient of ethyl acetate/petroleum ether) to give 6-(2-azido-5-iodophenyl)-6-azaspiro [2.5]octane (1.75 g, 4.95 mmol, yield: 72%) as a yellow solid. LCMS (ESI): $[M+H]^+$ =354.9.

Step 6: Copper sulfate (225 mg, 1.41 mmol) and sodium ascorbate (279 mg, 1.41 mmol) were added to a solution of 6-(2-azido-5-iodophenyl)-6-azaspiro[2.5]octane (500 mg, 1.41 mmol) and 2-(4,4-difluoropiperidin-1-yl)-4-ethynyl-6-methylpyrimidine (334 mg, 1.41 mmol) in tert-butanol (5 mL) and water (5 mL). The mixture was stirred at 25°C for 16 h. The reaction solution was diluted with water (20 mL) and extracted with ethyl acetate (20 mL×3). The organic phases were combined, dried over anhydrous sodium sulfate, and filtered. The filtrate was concentrated under reduced pressure to give the residue. The residue was purified by flash column chromatography (silica gel, 0-5% gradient of ethyl acetate/petroleum ether) to give 6-(2-(4-(2-(4,4-difluoropiperidin-1-yl)-6-methylpyrimidin-4-yl)-1H-1,2,3-triazol-1-yl) -5-iodophenyl)-6-azaspiro[2.5]octane (350 mg, 0.59 mmol, yield: 42%) as a yellow solid. LCMS (ESI): $[M+H]^+$ =592.5.

Step 7: Cuprous iodide (49.2 mg, 0.25 mmol) was added to a solution of 2-hydroxyethanesulfonamide (82.5 mg, 0.66 mmol), 2-(methylamino)acetic acid (45.2 mg, 0.51 mmol), and potassium phosphate (439 mg, 2.03 mmol) in N,N-dimethylformamide (6 mL). The mixture was stirred at 50°C for 5 min. Then, 6-(2-(4-(2-(4,4-difluoropiperidin-1-yl)-6-methylpyrimidin-4-yl)-1H-1,2,3-triazol-1-yl) -5-iodophenyl)-6-azaspiro[2.5]octane (300 mg, 0.51 mmol) was added to the reaction mixture at 50°C, and the mixture was heated to 100°C under nitrogen protection and stirred for 16 h. Water (10 mL) was added to the reaction solution and extracted with ethyl acetate (10 mL × 2). The organic phases were combined, dried over anhydrous sodium sulfate, and filtered. The filtrate was concentrated under reduced pressure to give the residue. The residue was purified by preparative HPLC (C18, 44-84% gradient of water (formic acid)/acetonitrile) to give N-(4-(4-(2-(4,4-difluoropiperidin-1-yl)-6-methylpyrimidin-4-yl)-1H-1,2,3-triazol-1-yl) -3-(6-azaspiro[2.5]octan-6-yl)phenyl)-2-hydroxyethanesulfonamide (108 mg, 0.18 mmol, yield: 36%) as a white solid compound. LCMS (ESI): $[M+H]^+$ = 589.5. $^1H$ NMR (400 MHz, DMSO-$d_6$) δ ppm 10.04 (s, 1H), 9.22 (s, 1H), 7.58 (d, J = 8.4 Hz, 1H), 7.22 (s, 1H), 7.14 (d, J = 2.0 Hz, 1H), 7.04 (dd, J = 2.0, 8.4 Hz, 1H), 5.13 - 4.77 (m, 1H), 4.15 - 3.88 (m, 4H), 3.79 (t, J = 6.4 Hz, 2H), 3.42 - 3.35 (m, 2H), 2.74 - 2.63 (m, 4H), 2.41 (s, 3H), 2.08 - 1.93 (m, 4H), 1.41 - 1.18 (m, 4H), 0.25 (s, 4H)

**Example 35:**

**N-(4-(4-(2-(3,6-dihydro-2H-pyran-4-yl)-6-methylpyrimidin-4-yl)-1H-1,2,3-triazol -1-yl)-3-(6-azaspiro[2.5]octan-6-yl)phenyl)-2-hydroxyethane-1-sulfonamide**

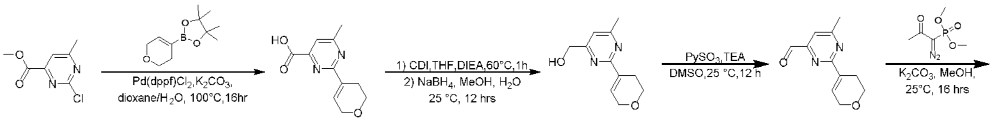


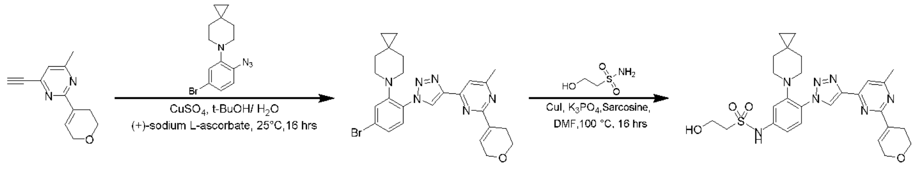


Step 1: To a solution of methyl 2-chloro-6-methylpyrimidin-4-carboxylate (2.00 g, 10.7 mmol), 2-(3,6-dihydro-2H-pyran-4-yl)-4,4,5,5-tetramethyl-1,3,2-dioxolane (3.37 g, 16.1 mmol) and potassium carbonate (4.53 g, 32.2 mmol) in water (8.00 mL) and dioxane (40.0 mL) was added 1,1-bis(diphenylphosphine)ferrocene palladium chloride (0.81 g, 1.07 mmol). Nitrogen replacement was performed 3 times and the mixture was heated to 100°C with stirring for 16 h. The reaction solution was diluted with water (100 mL) and extracted with ethyl acetate (100 mL). The pH of the aqueous phase was adjusted to 3 with 1 mol/L diluted hydrochloric acid solution to precipitate the brown solids. The solid was collected, washed twice with water (40 mL), then filtered and dried by an oil pump to give 2-(3,6-dihydro-2H-pyran-4-yl)-6-methylpyrimidin-4-carboxylic acid (2.50 g, 10.7 mmol, yield: 100%) as a brown solid. LCMS (ESI): $[M+H]^+$ = 221.0

Step 2: N,N-diisopropylethylamine (3.52 g, 27.2 mmol) was added to a solution of 2-(3,6-dihydro-2H-pyran-4-yl)-6-methylpyrimidin-4-carboxylic acid (2.00 g, 9.08 mmol) and 1,1-carbonyldiimidazole (2.25 g, 13.6 mmol) in THF (30.0 mL). The reaction mixture was heated to 60°C and stirred for 1 h. Methanol (10 mL) was added to the reaction mixture. After stirring at 60°C for half an hour, sodium borohydride (1.37 g, 36.3 mmol) and water (4 mL) were added, and the mixture was stirred at 25°C for 16 h. The reaction solution was diluted with water (100 mL) and extracted with a mixed solvent of ethyl acetate and THF (1:1) (200 mL × 2), the organic phases were combined, dried over anhydrous magnesium sulfate, and filtered. The filtrate was concentrated under reduced pressure to give the residue. The residue was purified by flash column chromatography (silica gel, 0-12% gradient of THF/petroleum ether) to give [2-(3,6-dihydro-2H-pyran-4-yl)-6-methylpyrimidin-4-yl]methanol (1.20 g, 5.81 mmol, yield: 64%) as a yellow solid. LCMS (ESI): $[M+H]^+$ =206.9

Step 3: Pyridine sulfur trioxide (1.99 g, 12.5 mmol) was added to a solution of [2-(3,6-dihydro-2H-pyran-4-yl)-6-methylpyrimidin-4-yl]methanol (860 mg, 4.17 mmol) and triethylamine (2.11 g, 20.9 mmol) in DMSO (8 mL), and the mixture was stirred at 25°C for 12 h. The reaction solution was diluted with water (80 mL) and extracted with a mixed solvent (chloroform: isopropanol = 3:1) (80 mL × 2), and the organic layer was dried over magnesium sulfate, filtered, and concentrated under reduced pressure to give 2-(3,6-dihydro-2H-pyran-4-yl)-6-methylpyrimidin-4-carbaldehyde (0.56 g, 2.75 mmol, yield: 66%) as the crude. LCMS (ESI): $[M+H]^+$ =200.9.

Step 4: In methanol (10.0 mL) was dissolved 2-(3,6-dihydro-2H-pyran-4-yl)-6-methylpyrimidin-4-carbaldehyde (0.50 g, 2.45 mmol) and dimethyl (1-diazo-2-oxopropylidene) phosphonate (564 mg, 2.94 mmol), potassium carbonate (1.02 g, 7.34 mmol) was added, and the mixture was stirred for 12 h at 25°C. The reaction solution was diluted with water (30 mL) and extracted with ethyl acetate (40 mL × 2), and the organic phase was dried, filtered, and concentrated. The residue was purified by flash column chromatography (silica gel, 0-12% gradient of THF/petroleum ether) to give 2-(3,6-dihydro-2H-pyran-4-yl)-4-ethynyl-6-methylpyrimidine (180 mg, 0.91 mmol, yield: 37%) as a yellow solid. LCMS (ESI): $[M+H]^+$ = 201.0

Step 5: In a mixture of tert-butanol (1.50 mL), THF (1.50 mL), and pure water (1.50 mL) was dissolved 2-(3,6-dihydro-2H-pyran-4-yl)-4-ethynyl-6-methylpyrimidine (150 mg, 0.75 mmol) and 6-(2-azido-5-bromophenyl)-6-azaspiro[2.5]octane (184 mg, 0.43 mmol), sodium ascorbate (148 mg, 0.75 mmol) and anhydrous copper sulfate (120 mg, 0.75 mmol) were added, and the mixture was stirred at 25°C for 4 h. The reaction solution was diluted with water (20 mL), extracted with ethyl acetate (20 mL×2), and the organic phase was dried, filtered and concentrated. The residue was purified by flash column chromatography (silica gel, 0-12% gradient of THF/petroleum ether) to give 6-(5-bromo-2-{4-[2-(3,6-dihydro-2H-pyran-4-yl)-6-methylpyrimidin-4-yl]-1H-1,2,3-t riazol-1-yl}phenyl)-6-azaspiro[2.5] octane (150 mg, 0.29 mmol, yield: 39%) as a yellow solid. LCMS (ESI): [M+H]+ = 508.9

Step 6: Tris(dibenzylideneacetone)dipalladium (33.2 mg, 0.04 mmol) was added to a solution of 6-(5-bromo-2-{4-[2-(3,6-dihydro-2H-pyran-4-yl)-6-methylpyrimidin-4-yl]-1H-1,2,3-t riazol-1-yl}phenyl)-6-azaspiro[2.5]octane (180. mg, 0.35 mmol), 2-hydroxyethanesulfonamide (54.4 mg, 0.43 mmol), di-tert-butyl-[2-(2,4,6-triisopropylphenyl) phenyl]phosphin (30.1 mg, 0.07 mmol) and potassium phosphate (307 mg, 1.42 mmol) in dioxane (4.00 mL). The The reaction solution was replaced with nitrogen for 3 times.. The mixture was heated to 90°C and stirred for 16 h.

The reaction solution was diluted with water (10 mL) and extracted with ethyl acetate (20 mL×2). The organic phases were combined, dried over anhydrous sodium sulfate, and filtered. The filtrate was concentrated under reduced pressure to give the residue. The residue was purified by preparative HPLC (C18, 26-66% gradient of water (ammonia + ammonium bicarbonate)/acetonitrile) to give N-(3-{6-azaspiro[2.5]octan-6-yl}-4-{4-[2-(3,6-dihydro-2H-pyran-4-yl)-6-methylpyri midin-4-yl]-1H-1,2,3-triazol-1-yl}phenyl)-2-hydroxyethane-1-sulfonamide as a white solid (111 mg, 0.20 mmol, yield: 57%). LCMS (ESI): $[M+H]^+$ = 552.2, $^1H$ NMR (400 MHz, DMSO-d6) δ ppm 9.89 (s, 1H), 9.29 (s, 1H), 7.81 (s, 1H), 7.60 (d, *J* = 8.4 Hz, 1H), 7.31 (br s, 1H), 7.14 (d, *J* = 2.4 Hz, 1H), 7.04 (dd, *J* = 2.0, 8.4 Hz, 1H), 4.99 (s, 1H), 4.34 (br d, *J* = 2.4 Hz, 2H), 3.84 (t, *J* = 5.2 Hz, 2H), 3.79 (t, *J* = 6.4 Hz, 2H), 3.36 (br s, 2H), 2.75 - 2.65 (m, 6H), 2.56 (s, 3H), 1.31 (br s, 4H), 0.25 (s, 4H)

**Example 36:**

**2-hydroxy-N-(4-(4-(6-methyl-2-(tetrahydro-2H-pyran-4-yl)pyrimidin-4-yl)-1H-1, 2,3-triazol-1-yl)-3-(6-azaspiro [2.5]octan-6-yl)phenyl)ethane-1-sulfonamide**

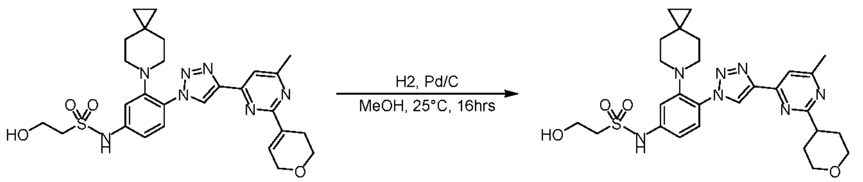


Step 1: Wet palladium/carbon (5 mg, mass fraction: 20%) was added to a solution of N-(3-{6-azaspiro[2.5] octan-6-yl}-4-{4-[2-(3,6-dihydro-2H-pyran-4-yl)-6-methylpyri midin-4-yl]-1H-1,2,3-triazol-1-yl}phenyl)-2-hydroxyethane-1-sulfonamide (80 mg, 0.15 mmol) in methanol (2 mL) at 25°C. The reaction mixture was subjected to argon atmosphere for 3 times followed by three hydrogen replacements and finally stirred under hydrogen (50 Psi) for 16 h. The reaction mixture was filtered with suction under vacuum to remove palladium/carbon and then concentrated in vacuum to give a residue. The residue was diluted with N,N-dimethylformamide (3 mL), and the dilution was purified by preparative HPLC (C18, 24-64% gradient of water (formic acid)/acetonitrile) to give N-(3-{6-azaspiro[2.5]octan-6-yl}-4-{4-[6-methyl-2-(oxan-4-yl)pyrimidin-4-yl]-1H-1, 2,3-triazol-1-yl}phenyl)-2-hydroxyethane-1-sulfonamide as a yellow solid (60.00 mg, 0.11 mmol, yield: 75%). LCMS (ESI): [M+H]+ = 554.3, $^1H$ NMR (400 MHz, DMSO-d6) δ ppm 10.27 - 9.75 (m, 1H), 9.23 (s, 1H), 7.81 (s, 1H), 7.58 (d, *J* = 8.4 Hz, 1H), 7.13 (d, *J* = 2.0 Hz, 1H), 7.03 (dd, *J* = 2.0, 8.8 Hz, 1H), 5.14 - 4.85 (m, 1H), 4.02 - 3.89 (m, 2H), 3.78 (br t, *J* = 6.4 Hz, 2H), 3.48 (dt, *J* = 3.2, 10.8 Hz, 2H), 3.36 - 3.34 (m, 2H), 3.11 - 3.04 (m, 1H), 2.71 - 2.65 (m, 4H), 2.53 (br s, 3H), 1.93 - 1.85 (m, 4H), 1.29 (br s, 4H), 0.23 (s, 4H)

**Examples 37 to 39** were prepared with reference to the synthetic route of **Example 35:**

**Example 37:**

**N-(4-(4-(2-(4,4-difluorocyclohex-1-en-1-yl)-6-methylpyrimidin-4-yl)-1H-1,2,3-tria zol-1-yl)-3-(6-azaspiro[2.5]octan-6-yl)phenyl)-2-hydroxyethane-1-sulfonamide**

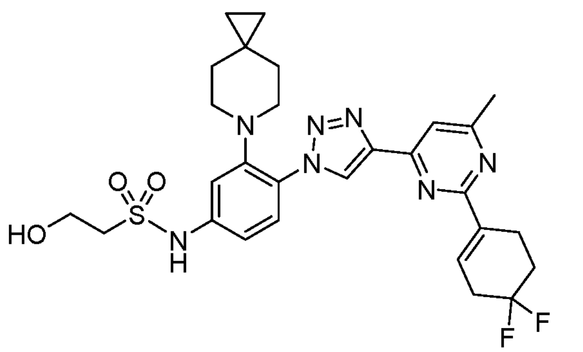

LCMS (ESI): [M+H]+ = 586.3, $^1$H NMR (400 MHz, DMSO-d6) δ ppm 9.28 (s, 1H), 7.81 (s, 1H), 7.58 (d, *J* = 8.4 Hz, 1H), 7.24 - 7.10 (m, 2H), 7.04 (br d, *J* = 8.4 Hz, 1H), 3.78 (t, *J* = 6.4 Hz, 2H), 3.32 - 3.29 (m, 2H), 2.94 - 2.77 (m, 4H), 2.73 - 2.62 (m, 4H), 2.59 - 2.52 (m, 3H), 2.26 - 2.13 (m, 2H), 1.30 (br s, 4H), 0.24 (s, 4H)

**Example 38:**

**N-(4-(4-(2-(5,6-dihydro-1,4-dioxin-2-yl)-6-methylpyrimidin-4-yl)-1H-1,2,3-triazol -1-yl)-3-(6-azaspiro[2.5]octan-6-yl)phenyl)-2-hydroxyethane-1-sulfonamide**

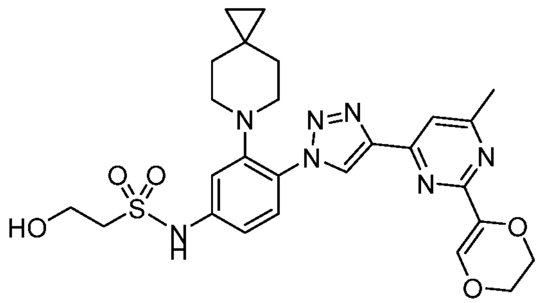

LCMS (ESI): [M+H]+ = 554.2, $^1$H NMR (400 MHz, DMSO-d6) δ ppm 9.89 (s, 1H), 8.94 (s, 1H), 7.49 (s, 1H), 7.40 (s, 1H), 7.28 (d, *J* = 8.8 Hz, 1H), 6.89 (d, *J* = 2.0 Hz, 1H), 6.78 (dd, *J* = 2.4, 8.8 Hz, 1H), 4.99 (s, 1H), 3.96 (br d, *J* = 3.2 Hz, 4H), 3.54 (t, *J* = 6.4 Hz, 2H), 3.13 - 3.09 (m, 2H), 2.50 - 2.41 (m, 4H), 2.26 (s, 3H), 1.02 (br s, 4H), 0.23 (s, 4H)

**Example 39:**

**2-hydroxy-N-(4-(4-(2-methyl-6-(1-methyl-1H-pyrazol-4-yl)pyridin-4-yl)-1H-1,2,3 -triazol-1-yl)-3-(6-azaspiro[2.5] octan-6-yl)phenyl)ethane-1-sulfonamide**

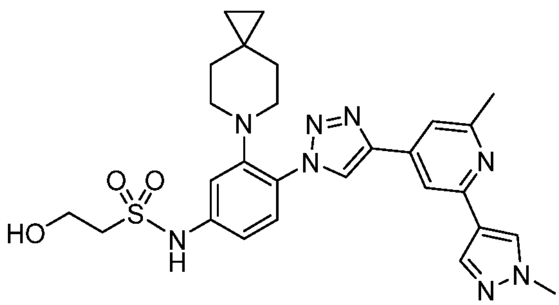

LCMS (ESI): [M+H]+ = 549.3; $^1$H NMR (400 MHz, DMSO-d6) δ ppm 10.06 (s, 1H), 9.20 (s, 1H), 8.31 (s, 1H), 8.03 (s, 1H), 7.98 (s, 1H), 7.59 (s, 1H), 7.50 (d, *J* = 8.4 Hz, 1H), 7.14 (d, *J* = 2.0 Hz, 1H), 7.02 (dd, *J* = 2.0, 8.4 Hz, 1H), 4.98 (br s, 1H), 3.90 (s, 3H), 3.78 (br t, *J* = 6.4 Hz, 2H), 3.38 - 3.34 (m, 2H), 2.72 - 2.64 (m, 4H), 2.54 (s, 3H), 1.25 (br s, 4H), 0.23 (s, 4H)

**Example 40:**

**N-(4-(4-(2-(1,4-dioxan-2-yl)-6-methylpyrimidin-4-yl)-1H-1,2,3-triazol-1-yl)-3-(6-a zaspiro[2.5]octan-6-yl)phenyl)-2-hydroxyethane-1-sulfonamide**

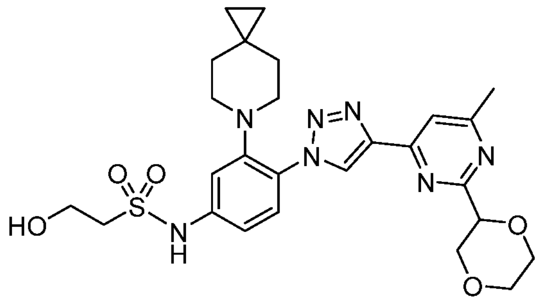

**Example 40** was prepared with reference to the synthetic route of **Example 36;** LCMS (ESI): [M+H]+ = 554.3, $^1$H NMR (400 MHz, DMSO-d6) δ ppm 10.27-9.75 (m, 1H), 9.23 (s, 1H), 7.81 (s, 1H), 7.58 (d, *J* = 8.4 Hz, 1H), 7.13 (d, *J*= 2.0 Hz, 1H), 7.03 (dd, *J* = 2.0, 8.8 Hz, 1H), 5.14-4.85 (m, 1H), 4.02-3.89 (m, 2H), 3.78 (br t, *J* = 6.4 Hz, 2H), 3.48 (dt, *J* = 3.2, 10.8 Hz, 2H), 3.36-3.34 (m, 2H), 3.11-3.04 (m, 1H), 2.71-2.65 (m, 4H), 2.53 (br s, 3H), 1.93-1.85 (m, 4H), 1.29 (br s, 4H), 0.23 (s, 4H)

**Example 41:**

**2-hydroxy-N-(4-(4-(6-methyl-2-(piperidin-1-yl)pyrimidin-4-yl)-1H-1,2,3-triazol-1 -yl)-3-(6-azaspiro[2.5]octan-6-yl) phenyl)ethanesulfonamide**

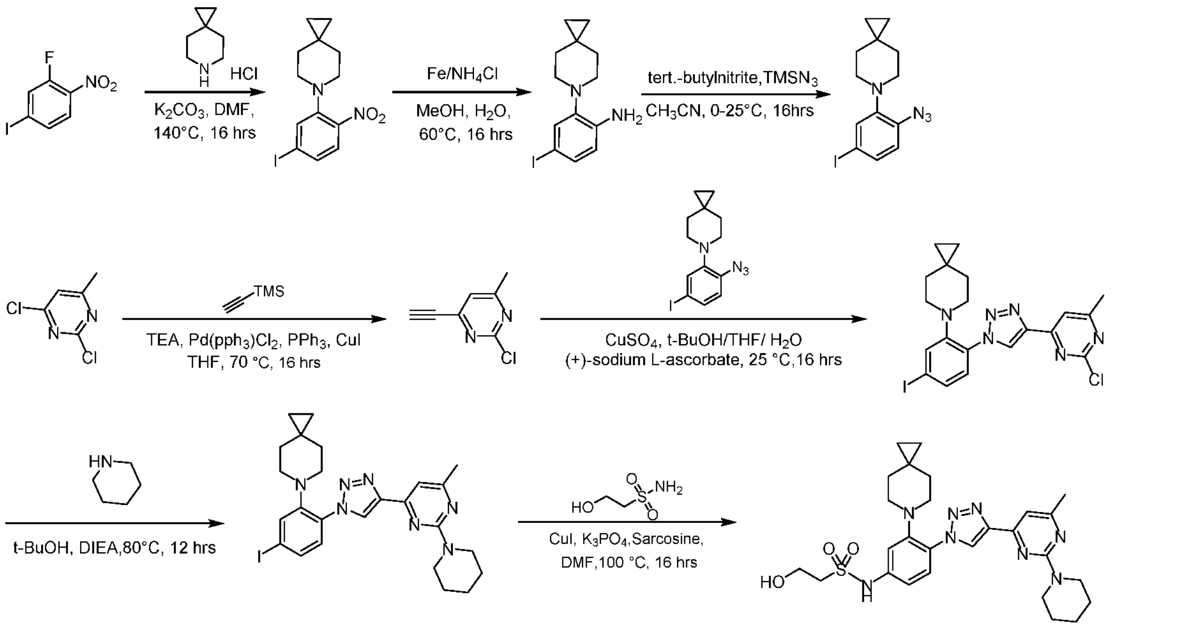


Step 1: To a solution of 2-fluoro-4-iodo-1-nitrobenzene (3.00 g, 11.2 mmol) and potassium carbonate (4.65 g, 33.7 mmol) in DMSO (45 mL) was added 6-azaspiro[2.5]octane hydrochloride (1.82 g, 12.3 mmol). The mixture was heated to 140°C and stirred for 16 h. Water (100 mL) was poured into the reaction solution, which was extracted with ethyl acetate (100 mL × 3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 6-(5-iodo-2-nitrophenyl)-6-azaspiro[2.5]octane (3.00 g, 8.37 mmol, yield: 75%) as a yellow solid. LCMS (ESI): $[M+H]^+$ = 358.8.

Step 2: Ammonium chloride (1.36 g, 25.1 mmol) and iron powder (4.73 g, 83.7 mmol) were added to a solution of 6-(5-iodo-2-nitrophenyl)-6-azaspiro[2.5]octane (3.00 g, 8.37 mmol) in methanol (60 mL) and water (20 mL). The mixture was heated to 60°C and stirred for 16 h. The reaction solution was filtered with diatomite and the filtrate was extracted with ethyl acetate (100 mL × 3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 4-iodo-2-(6-azaspiro[2.5]octan-6-yl)aniline (2.20 g, 6.69 mmol, yield: 80%) as a yellow solid. LCMS (ESI): $[M+H]^+$ =329.0.

Step 3: Tert-butyl nitrite (1.09 g, 10.3 mmol) was added to a solution of 4-iodo-2-(6-azaspiro[2.5]octan-6-yl) aniline (2.26 g, 6.89 mmol) in acetonitrile (40 mL) at 0°C. Then, azidotrimethylsilane (1.19 g, 10.3 mmol) was added to the reaction solution and the mixture was stirred at 25°C for 16 h. The reaction solution was poured into water (40 mL) and extracted with ethyl acetate (40 mL×3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 6-(2-azido-5-iodophenyl)-6-azaspiro[2.5]octane (1.75 g, 4.95 mmol, yield: 72%) as a yellow solid. LCMS (ESI): $[M+H]^+$ =354.9.

Step 4: To a solution of THF (100 mL) and triethylamine (9.31 g, 92.02 mmol), bis(triphenylphosphine) palladium dichloride (0.11 g, 0.15 mmol) and triphenylphosphine (0.08 g, 0.31 mmol) were added. Then, 2,4-dichloro-6-methylpyrimidine (5.00 g, 30.7 mmol), cuprous iodide (0.06 g, 0.31 mmol) and ethynyltrimethylsilane (3.31 g, 33.74 mmol) were added under nitrogen atmosphere. The solution was stirred at 70°C for 16 h under nitrogen atmosphere. After the reaction solution was filtered, the filtrate was poured into water (100 mL), extracted with ethyl acetate (200 mL x 2); the organic phase was dried, filtered, and concentrated. The residue was purified by flash column chromatography (silica gel, 0-2% gradient of THF/petroleum ether) to give 2-chloro-4-methyl-6-((trimethylsilyl)ethynyl)pyrimidine as a yellow oil (5.60 g, 15.95 mmol, yield: 52%). LCMS (ESI): $[M+H]^+$ = 225.0

Step 5: 2-chloro-4-methyl-6-((trimethylsilyl)ethynyl)pyrimidine (500 mg, 2.22 mmol) and 6-(2-azido-5-iodophenyl)-6-azaspiro[2.5]octane (787 mg, 2.22 mmol) were added into tert-butanol (3.50 mL), THF (5.00 mL) and pure water (3.50 mL). Sodium ascorbate (440 mg, 2.22 mmol) and copper sulfate (355 mg, 2.22 mmol) were added and the mixture was stirred at 25°C for 16 h. The reaction solution was poured into water (50 mL) and extracted with ethyl acetate (100 mL × 2); the organic phase was dried, filtered, and concentrated. The residue was purified by flash column chromatography (silica gel, 0-9% gradient of THF/petroleum ether) to give 6-(2-(4-(2-chloro-6-methylpyrimidin-4-yl)-1H-1,2,3-triazol-1-yl)-5-iodophenyl)-6-aza spiro[2.5]octane as a yellow solid (760 mg, 1.38 mmol, yield: 62%). LCMS (ESI): $[M+H]^+$ = 507.0.

Step 6: N,N-diisopropylethylamine (191 mg, 1.48 mmol) and piperidine (84 mg, 0.99 mmol) was added to a solution of 1-[2-(6-azaspiro[2.5]oct-6-yl)-4-iodophenyl]-4-(2-chloro-6-methylpyrimidin-4-yl)-1,2, 3-triazole (250 mg, 0.49 mmol) in tert-butanol (5 mL) at 25°C. The resulting mixture was stirred at 80 °C for 12 h. The reaction solution was diluted with water (20 mL) and extracted with ethyl acetate (40 mL × 3), and the organic phase was washed with saturated sodium chloride (40 mL). The organic phase was dried over anhydrous magnesium sulfate and filtered. concentrated under reduced pressure to give a residue. The residue was purified by flash column chromatography (silica gel, 0-10% gradient of THF/petroleum ether) to give 1-[2-(6-azaspiro[2.5]oct-6-yl)-4-iodophenyl]-4-(6-methyl-2-piperidin-4-yl)-1,2,3-triaz ole as a yellow solid (0.10 g, 0.18 mmol, yield: 36%). LCMS (ESI): [M+H]+ = 556.4 Step 7: To a solution of 2-hydroxyethanesulfonamide (40.6 mg, 0.32 mmol) and (1R,2R)-N1,N2-dimethylcyclohexylamin-1,2-diamine (23.2 mg, 0.16 mmol) in N,N-dimethylformamide (2 mL), cuprous iodide (15.4 mg, 0.08 mmol) and potassium phosphate (138 mg, 0.65 mmol) were added at 25°C. The reaction mixture was heated to 50°C, and stirred for 5 min under nitrogen blanketing. 1-[2-(6-azaspiro[2.5]oct-6-yl)-4-iodophenyl]-4-(6-methyl-2-piperidin-4-yl)-1,2,3-triaz ole (90 mg, 0.16 mmol) was added at 50°C. Nitrogen replacement was performed.

The reaction solution was stirred at 100°C for 16 h. The reaction mixture was added with liquor ammonia (5 mL) and water (15 mL) and extracted with dichloromethane (20 mL × 3). The organic phases were combined and washed once with saturated saline (20 mL), dried over anhydrous magnesium sulfate and filtered, and concentrated under reduced pressure to give a residue. The residue was diluted with N,N-dimethylformamide (5 mL). The diluent was purified by preparative HPLC (C18, 46-86% gradient of water (formic acid)/acetonitrile) to give 2-hydroxy-N-(4-(4-(6-methyl-2-(piperidin-1-yl)pyrimidin-4-yl)-1H-1,2,3-triazol-1-yl) -3-(6-azaspiro[2.5]octan-6-yl)phenyl)ethanesulfonamide as a yellow solid (43 mg, 0.08 mmol, yield: 48%). LCMS (ESI): $[M+H]^+$ = 553.3. $^1H$ NMR (400 MHz, DMSO-d6) $\delta$ ppm 10.06 (s, 1H), 9.18 (s, 1H), 7.60 (d, *J* = 8.4 Hz, 1H), 7.14 (d, *J* = 2.0 Hz, 1H), 7.11 (s, 1H), 7.04(dd, *J* = 2.0, 8.8 Hz, 1H), 3.87 - 3.80 (m, 4H), 3.78 (t, *J* = 6.4 Hz, 2H),3.34 (t, *J* = 6.4 Hz, 2H), 2.68 (br t, *J* = 4.8 Hz, 4H), 2.37 (s, 3H), 1.65 (br d, *J= 4.4* Hz, 2H), 1.54 (br d, *J* = 4.0 Hz, 4H), 1.39 - 1.26 (m, 4H), 0.25 (s, 4H)

**Examples 42 to 50** were prepared with reference to the synthetic route of **Example 41:**

**Example 42:**

**2-hydroxy-N-(4-(4-(6-methyl-2-morpholinopyrimidin-4-yl)-1H-1,2,3-triazol-1-yl)-3-(6-azaspiro[2.5]octan-6-yl) phenyl)ethanesulfonamide**

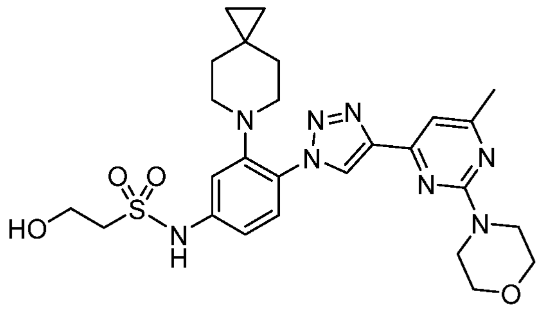

LCMS (ESI): $[M+H]^+$ = 554.9. $^1H$ NMR (400 MHz, DMSO-d6) δ ppm 10.06 (s, 1H), 9.19 (s, 1H), 7.58 (d, *J* = 8.8 Hz, 1H), 7.20 (s, 1H), 7.13 (d, *J* = 2.0 Hz, 1H), 7.03(dd, *J* = 2.0, 6.4 Hz, 1H), 4.98 *(t, J* = 5.6 Hz, 1H), 3.89 - 3.73 (m, 6H), 3.72 - 3.63 (m, 4H), 3.37 - 3.34 (m, 2H), 2.68 (br t, *J* = 4.8 Hz, 4H), 2.39 (s, 3H), 1.29 (br s, 4H), 0.25 (s, 4H)

**Example 43:**

**(S)-2-hydroxy-N-(4-(4-(6-methyl-2-(2-methylmorpholino)pyrimidin-4-yl)-1H-1,2, 3-triazol-1-yl)-3-(6-azaspiro[2.5]octan-6-yl)phenyl)ethane-1-sulfonamide**

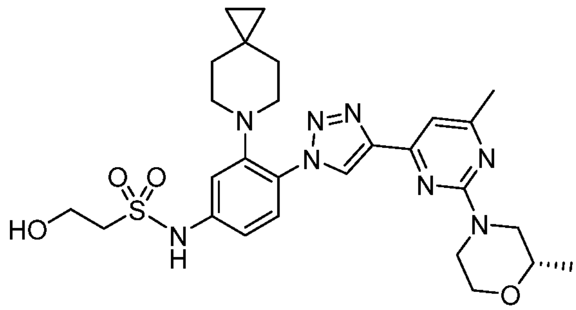

LCMS (ESI): $[M+H]^+$ = 569.3. $^1H$ NMR (400 MHz, DMSO-d6) δ ppm 10.06 (s, 1H), 9.22 (s, 1H), 7.59 (d, *J* = 8.4 Hz, 1H), 7.18 (s, 1H), 7.13 (d, *J* = 2.0 Hz, 1H), 7.03(dd, *J* = 2.0, 8.8 Hz, 1H), 5.45 - 4.74 (m, 1H), 4.70 - 4.45 (m, 2H), 3.90 (dd, *J* = 2.4, 11.2 Hz, 1H), 3.78 (t, *J* = 6.4 Hz, 2H), 3.54 - 3.47 (m, 2H), 3.33 - 3.31 (m, 2H), 3.01 - 2.91 (m, 1H), 2.75 - 2.64 (m, 5H), 2.39 (s, 3H), 1.30 (br s, 4H), 1.17 (d, *J* = 6.0 Hz, 3H), 0.25 (s, 4H)

**Example 44:**

**N-(4-(4-(2-((2R,6S)-2,6-dimethylmorpholino)-6-methylpyrimidin-4-yl)-1H-1,2,3-t riazol-1-yl)-3-(6-azaspiro[2.5]octan-6-yl)phenyl)-2-hydroxyethane-1-sulfonamide**

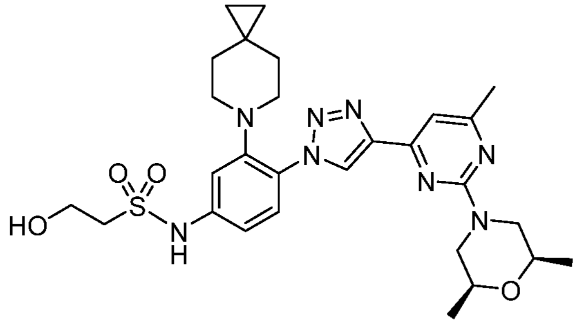

LCMS (ESI): $[M+H]^+$ = 583.3 , $^1H$ NMR (400 MHz, DMSO-d6) δ ppm 10.05 (s, 1H), 9.23 (s, 1H), 7.59 (d, *J* = 8.4 Hz, 1H), 7.17 (s, 1H), 7.13 (d, *J* = 2.0 Hz, 1H), 7.03 (dd, J= 2.0, 8.8 Hz, 1H), 5.33 - 4.77 (m, 1H), 4.64 (br d, J= 13.2 Hz, 2H), 3.78

(t, *J* = 6.4 Hz, 2H), 3.64 - 3.53 (m, 2H), 3.34 - 3.31 (m, 4H), 2.67 (br t, *J* = 4.4 Hz, 4H), 2.38 (s, 3H), 1.30 (br s, 4H), 1.17 (d, *J* = 6.4 Hz, 6H), 0.25 (s, 4H)

**Example 45:**

**2-hydroxy-N-(4-(4-(6-methyl-2-(3-methylmorpholinopyrimidin-4-yl)-1H-1,2,3-tri azol-1-yl)-3-(6-azaspiro[2.5]octan-6-yl)phenyl)ethanesulfonamide**

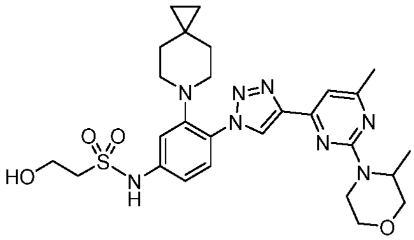

LCMS (ESI): $[M+H]^+$ = 569.5. $^1$H NMR (400 MHz, DMSO-$d_6$) δ ppm 10.05 (s, 1H), 9.19 (s, 1H), 7.60 (d, *J* = 8.4 Hz, 1H), 7.18 (s, 1H), 7.13 (d, *J* = 2.0 Hz, 1H), 7.03 (dd, *J = 2.0,* 8.8 Hz, 1H), 4.98 (br s, 1H), 4.75 (br dd, *J* = 1.6, 6.4 Hz, 1H), 4.38 (br d, *J* = 12.8 Hz, 1H), 3.92 (br dd, *J = 2.8,* 11.2 Hz, 1H), 3.81 - 3.69 (m, 3H), 3.60 (br dd, *J* = 2.8, 11.2 Hz, 1H), 3.49 - 3.40 (m, 1H), 3.35 (br s, 1H), 3.32 (s, 1H), 3.22 - 3.11 (m, 1H), 2.74 - 2.59 (m, 4H), 2.38 (s, 3H), 13.4 - 1.25 (m, 4H), 1.24 - 1.19 (m, 3H), 0.24 (s, 4H)

**Example 46:**

**N-(4-(4-(2-((3S,5R)-3,5-dimethylmorpholin)-6-methylpyrimidin-4-yl)-1H-1,2,3-tri azol-1-yl)-3-(6-azaspiro[2.5]octan-6-yl)phenyl)2-hydroxyethanesulfonamide**

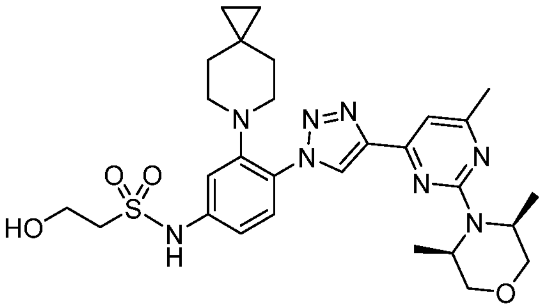

LCMS (ESI): [M+H]+ = 583.5. $^1$H NMR (400 MHz, DMSO-d6) δ ppm 10.05 (s, 1H), 9.18 (s, 1H), 7.62 (d, *J* = 8.4 Hz, 1H), 7.18 (s, 1H), 7.13 (d, *J* = 2.0 Hz, 1H), 7.04 (dd, *J* = 2.0, 8.8 Hz, 1H), 5.29 - 4.73 (m, 1H), 4.60 (br dd, *J* = 3.6, 6.8 Hz, 2H), 3.81 - 3.74 (m, 4H), 3.63 - 3.56 (m, 2H), 3.32 (br s, 2H), 2.71 - 2.61 (m, 4H), 2.39 (s, 3H), 1.39 - 1.22 (m, 10H), 0.23 (s, 4H)

**Example 47:**

**N-(4-(4-(2-(2-oxa-5-azabicyclo[2.2.1]heptan-5-yl)-6-methylpyrimidin-4-yl)-1H-1,2, 3-triazol-1-yl)-3-(6-azaspiro[2.5]octan-6-yl)phenyl)-2-hydroxyethane-1-sulfonami de**

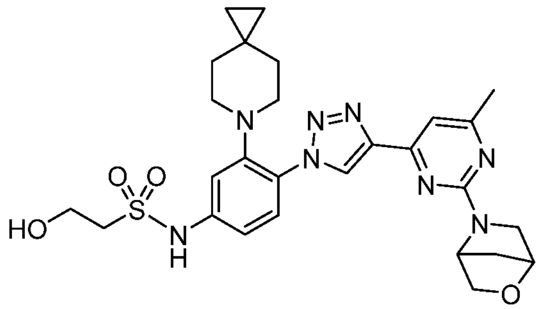

LCMS (ESI): $[M+H]^+$ = 567.3, $^1$H NMR (400 MHz, DMSO-$d_6$) δ ppm 10.05 (br s, 1H), 9.37 - 8.93 (m, 1H), 7.59 (d, *J* = 8.4 Hz, 1H), 7.18 (s, 1H), 7.14 (d, *J* = 2.0 Hz, 1H), 7.04 (dd, *J* = 2.0, 8.8 Hz, 1H), 5.16 - 4.90 (m, 2H), 4.68 (s, 1H), 3.91 - 3.63 (m, 4H), 3.55 - 3.43 (m, 2H), 3.38 - 3.34 (m, 2H), 2.74 - 2.63 (m, 4H), 2.38 (s, 3H), 2.02 - 1.84 (m, 2H), 1.30 (br s, 4H), 0.25 (s, 4H)

**Example 48:**

**N-(4-(4-(2-(3-oxa-8-azabicyclo[3.2.1]octan-8-yl)-6-methylpyrimidin-4-yl)-1H-1,2, 3-triazol-1-yl)-3-(6-azaspiro[2.5] octan-6-yl)phenyl)-2-hydroxyethane-1-sulfonami de**

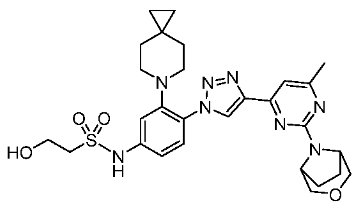

LCMS (ESI): $[M+H]^+$ = 581.3, $^1$H NMR (400 MHz, DMSO-d6) δ ppm 10.03 (s, 1H), 9.20 (s, 1H), 7.60 (d, *J* = 8.8 Hz, 1H), 7.19 (s, 1H), 7.12 (d, *J* = 2.0 Hz, 1H), 7.03 (dd, *J* = 2.0, 8.4 Hz, 1H), 5.11 - 4.83 (m, 1H), 4.78 - 4.59 (m, 2H), 3.77 (br t, *J* = 6.4 Hz, 2H), 3.69 - 3.53 (m, 4H), 3.33 (br s, 2H), 2.75 - 2.64 (m, 4H), 2.39 (s, 3H), 2.05 - 1.83 (m, 4H), 1.29 (br s, 4H), 0.24 (s, 4H)

**Example 49:**

**N-(4-(4-(2-(3-oxa-6-azabicyclo[3.1.1]heptan-6-yl)-6-methylpyrimidin-4-yl)-1H-1,2, 3-triazol-1-yl)-3-(6-azaspiro [2.5]octan-6-yl)phenyl)-2-hydroxyethane-1-sulfonami de**

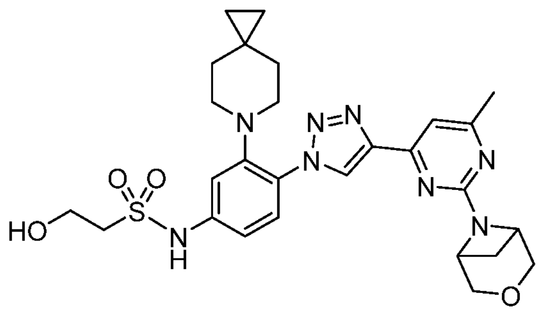

LCMS (ESI): $[M+H]^+$ = 567.2; $^1$H NMR (400 MHz, DMSO-d6) δ ppm 10.04 (s, 1H), 9.10 (s, 1H), 7.59 (d, *J* = 8.4 Hz, 1H), 7.30 (s, 1H), 7.12 (d, *J* = 2.0 Hz, 1H), 7.03 (dd, *J* = 2.0, 8.4 Hz, 1H), 5.20 - 4.76 (m, 1H), 4.37 (br d, *J* = 6.0 Hz, 2H), 4.29 - 4.05 (m, 2H), 3.86 - 3.63 (m, 4H), 3.45 - 3.34 (m, 2H), 2.83 - 2.61 (m, 5H), 2.40 (s, 3H), 1.77 (br d, *J* = 8.4 Hz, 1H), 1.27 (br s, 4H), 0.25 (s, 4H)

**Example 50:**

**N-(4-(4-(2-((2S,6R)-4,4-difluoro-2,6-dimethylpiperidin-1-yl)-6-methylpyrimidin-4 -yl)-1H-1,2,3-triazol-1-yl)-3-(6-azaspiro[2.5]octan-6-yl)phenyl)-2-hydroxyethane-1-sulfonamide**

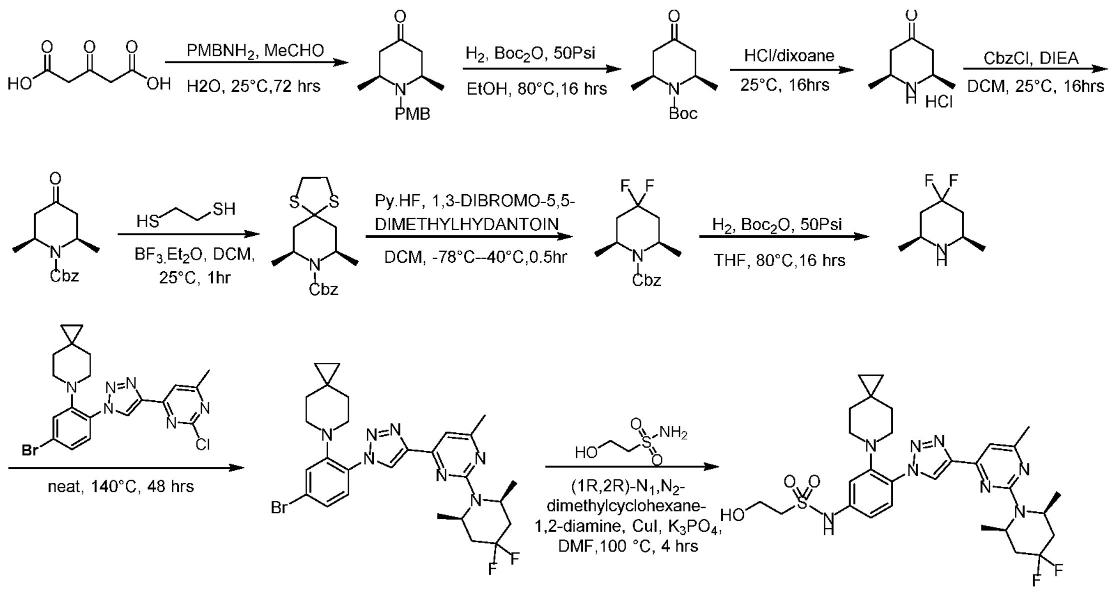


Step 1: A solution of 3-oxopentanedioic acid (100 g, 684 mmol), 1-(4-methoxyphenyl)methanamine (93.9 g, 684 mmol) and acetaldehyde (150 g, 1368 mmol, purity: 40%) in water (500 mL) was stirred at 25 °C for 72 h. The reaction solution was diluted with water (1000 mL) and extracted with ethyl acetate (1000 mL × 3); the organic phase was dried, filtered, and concentrated. The residue was purified by flash column chromatography (silica gel, 0-40% gradient of THF/petroleum ether) to give (2R,6S)-1-[(4-methoxyphenyl)methyl]-2,6-dimethylpiperidin-4-one as a colorless oil (77.0 g, 310 mmol, yield: 45.4%). LCMS (ESI): $[M+H]^+ = 287.0$; $^1H$ NMR (400 MHz, $CDCl_3$) δ ppm 7.32 (br d, $J$ = 8.4 Hz, 2H), 6.89 (br d, $J$ = 8.8 Hz, 2H), 3.95 - 3.84 (m, 1H), 3.82 (s, 3H), 3.62 - 3.51 (m, 1H), 3.34 - 3.21 (m, 2H), 2.56 - 2.41 (m, 2H), 2.26 - 2.12 (m, 2H), 1.11 (s, 3H), 1.09 (s, 3H)

Step 2: Palladium/carbon (10.0 g, purity: 10%) was added to a solution of (2R,6S)-1-[(4-methoxyphenyl)methyl]-2,6-dimethylpiperidin-4-one (70.0 g, 283 mmol) and di-tert-butyl dicarbonate (66.2 g, 303 mmol) in ethanol (500 mL). The mixture was stirred at 80°C for 16 h under hydrogen atmosphere (50 Psi). The reaction solution was cooled to ambient temperature and filtered by diatomite. The filtrate was concentrated to give t-butyl (2R,6S)-2,6-dimethyl-4-oxopiperidin-1-carboxylate as a yellow solid (70.0 g, crude). $^1H$ NMR (400 MHz, $CDCl_3$) δ ppm 4.45 - 4.33 (m, 2H), 2.93 - 2.78 (m, 2H), 2.44 - 2.34 (m, 2H), 1.50 (s, 9H), 1.26 (s, 3H), 1.24 (s, 3H)

Step 3: HCl in dioxane (400 mL, 4M) was added to a solution of t-butyl(2R, 6S)-2,6-dimethyl-4-oxopiperidin-1-carboxylate (40.0 g, 176 mmol) in dioxane (400 mL). The mixture was stirred at 25°C for 16 h. The reaction solution was concentrated to give (2R,6S)-2,6-dimethylpiperidin-4-one hydrochloride (28.0 g, crude). $^1H$ NMR (400 MHz, DMSO-d6) δ ppm 10.21 - 9.74 (m, 2H), 3.92 - 3.80 (m, 2H), 2.72 (dd, $J$= 4.4, 15.2 Hz, 2H), 2.49 - 2.39 (m, 2H), 1.31 (s, 3H), 1.28 (s, 3H)

Step 4: Benzyl chloride (31.2 g, 183 mmol) was added to a solution of (2R,6S)-2,6-dimethylpiperidin-4-one hydrochloride (30.0 g, 183 mmol) and N,N-diisopropylethylamine (96.7 g, 733 mmol) in dichloromethane (400 mL). The mixture was stirred at 25°C for 16 h. The reaction solution was diluted with water (400 mL) and extracted with dichloromethane (400 mL × 2); the organic phase was dried, filtered, and concentrated. The residue was purified by flash column chromatography (silica gel, 0-15% gradient of THF/petroleum ether) to give benzyl(2R,6S)-2,6-dimethyl-4-oxopiperidin-1-carboxylate as a yellow oil (30.0 g, 114 mmol, yield: 62.6%). $^1H$ NMR (400 MHz, $CDCl_3$) δ ppm 7.47 - 7.38 (m, 5H), 5.28 - 5.12 (m, 2H), 4.55 - 4.42 (m, 2H), 2.88 (dd, $J$ = 6.4, 18.0 Hz, 2H), 2.42 (dd, $J$ = 1.6, 18.0 Hz, 2H), 1.30 (s, 3H), 1.27 (s, 3H)

Step 5: Ethanedithiol (49.7 g, 528 mmol) was added to a solution of benzyl(2R,6S)-2,6-dimethyl-4-oxypiperidin-1-carboxylate (46.0 g, 176 mmol) and BF3.Etherate (150 g, 1056 mmol) in dichloromethane (1200 mL), and stirred at 25°C for 1 h. The reaction solution was diluted with water (1000 mL), extracted with dichloromethane (1000 mL×2), and the organic phase was dried, filtered and concentrated. The residue was purified by flash column chromatography

(silica gel, 0-8% gradient of THF/petroleum ether) to give benzyl (7R,9S)-7,9-dimethyl-1,4-dithia-8-azaspiro[4.5] decane-8-carboxylate as a yellow oil (46.0 g, 135 mmol, yield: 77.0%). $^1$H NMR (400 MHz, $CDCl_3$) δ ppm 7.39 - 7.20 (m, 5H), 5.13 - 4.97 (m, 2H), 4.16 - 3.99 (m, 2H), 3.36 - 3.26 (m, 2H), 3.23 - 3.08 (m, 2H), 2.53 (dd, *J* = 5.2, 15.2 Hz, 2H), 2.23 (dd, *J* = 4.0, 15.2 Hz, 2H), 1.29 (s, 3H), 1.27 (s, 3H)

Step 6: Pyridine hydrofluoride (41.9 g, 296 mmol, purity: 70%) was added to a solution of 1,3-dibromo-5,5-dimethyl hydantoin (25.4 g, 88.8 mmol) in dichloromethane (200 mL) at -70°C. The reaction solution was stirred at -40°C for 0.5 h. Subsequently, a solution of benzyl(7R,9S)-7,9-dimethyl-1,4-dithia-8-azaspiro[4.5]decane-8-carboxylate (10.0 g, 29.6 mmol) in dichloromethane (50 mL) was added to the reaction solution at -40°C, and stirred at -40°C for 10 min. The reaction solution was diluted with water (100.0 mL) and extracted with dichloromethane (100 mL × 2); the organic phase was dried, filtered, and concentrated. The residue was purified by flash column chromatography (silica gel, 0-8% gradient of THF/petroleum ether) to give benzyl (2R,6S)-4,4-difluoro-2,6-dimethylpiperidin-1-carboxylate as a yellow oil (1.20 g, 12.4 mmol, yield: 14.0%). LCMS (ESI): $[M+H]^+$ = 284.0; $^1$H NMR (400 MHz, $CDCl_3$) δ ppm 7.34 - 7.22 (m, 3H), 5.13 - 5.00 (m, 2H), 4.25 - 4.10 (m, 2H), 2.44 - 2.20 (m, 2H), 2.16 - 2.02 (m, 2H), 1.23 (s, 3H), 1.21 (s, 3H)

Step 7: Wet palladium/carbon (1.0 g, purity: 10%) was added to a solution of benzyl(2R,6S)-4,4-difluoro-2,6-dimethylpiperidin-1-carboxylate (3.60 g, 12.7 mmol) in THF (200 mL). The mixture was stirred at 80°C for 16 h under hydrogen atmosphere (50 Psi). After being filtered by diatomite, wet palladium/carbon (1.0 g, purity: 10%) was added again to the filtrate. The mixture was stirred at 80°C for 16 h under hydrogen atmosphere (50 Psi). Three times were repeated. After being filtered by diatomite, the filtrate was concentrated to give (2R,6S)-4,4-difluoro-2,6-dimethylpiperidine as a white solid (1.60 g, 10.6 mmol, yield: 84%). $^1$H NMR (400 MHz, DMSO-$d_6$) δ ppm 3.59 - 3.49 (m, 2H), 2.26 - 2.06 (m, 2H), 1.96 - 1.82 (m, 2H), 1.26 (s, 3H), 1.24 (s, 3H)

Step 8: (2R,6S)-4,4-difluoro-2,6-dimethylpiperidine (648 mg, 4.35 mmol) and 6-{5-bromo-2-[4-(2-chloro-6-methylpyrimidin-4-yl)-1H-1,2,3-triazol-1-yl]phenyl}-6-azaspiro[2.5]octane (200 mg, 0.435 mmol) were mixed stirred at 140°C for 48 h. The mixture was purified by flash column chromatography (silica gel, 0-5% gradient of THF/petroleum ether) to give 6-[5-bromo-2-(4-{2-[(2R,6S)-4,4-difluoro-2,6-dimethylpiperidin-1-yl]-6-methylpyrim idin-4-yl}-1H-1,2,3-triazol-1-yl)phenyl]-6-azaspiro[2.5]octane as a yellow solid (40.0 mg, 0.07 mmol, yield: 16%). LCMS (ESI): $[M+H]^+$ = 574.2

Step 9: At 25°C, 6-[5-bromo-2-(4-{2-[(2R,6S)-4,4-difluoro-2,6-dimethylpiperidin-1-yl]-6-methylpyrim idin-4-yl}-1H-1,2,3-triazol-1-yl)phenyl]-6-azaspiro [2.5]octane (40 mg, 0.07 mmol) and 2-hydroxyethanesulfonamide (17.4 mg, 0.14 mmol) were dissolved in N,N-dimethylformamide (10 mL); potassium phosphate (44.5 mg, 0.21 mmol), (1R,2R)-N1,N2-dimethylcyclohexylamin-1,2-diamine (10.0 mg, 0.07 mmol) and cuprous iodide (13.3 mg, 0.07 mmol) were added and stirred at 100 °C for 4 h under nitrogen blanketing. The reaction solution was diluted with water (20.0 mL) and + 0.50 mL of liquor ammonia and extracted with ethyl acetate (20 mL x 2); the organic phase was dried, filtered, and concentrated. The residue was purified by preparative HPLC (C18, 50-90% gradient of water (formic acid)/acetonitrile) to give N-(4-(4-(2-((2S,6R)-4,4-difluoro-2,6-dimethylpiperidin-1-yl)-6-methylpyrimidin-4-yl) -1H-1,2,3-triazol-1-yl)-3-(6-azaspiro[2.5]octan-6-yl)phenyl)-2-hydroxyethane-1-sulfo namide as a white solid (28.0 mg, 0.04 mmol, yield: 65.0%). LCMS (ESI): $[M+H]^+$ = 617.6; $^1$H NMR (400 MHz, DMSO-$d_6$) 10.04 (s, 1H), 9.14 (s, 1H), 7.61 (d, *J* = 8.4 Hz, 1H), 7.25 - 7.10 (m, 2H), 7.04 (dd, *J* = 2.0, 8.4 Hz, 1H), 4.97 (br t, *J* = 5.2 Hz, 1H), 4.71 (br s, 2H), 3.85 - 3.71 (m, 2H), 3.33 - 3.25 (m, 2H), 2.78 - 2.57 (m, 6H), 2.43 - 2.30 (m, 5H), 1.36 - 1.22 (m, 10H), 0.23 (s, 4H)

**Example 51:**

**N-(4-(4-(2-(4,4-difluoropiperidin-1-yl)-6-methoxypyrimidin-4-yl)-1H-1,2,3-triazol -1-yl)-3-(6-azaspiro[2.5]octan-6-yl)phenyl)-2-hydroxyethanesulfonamide**

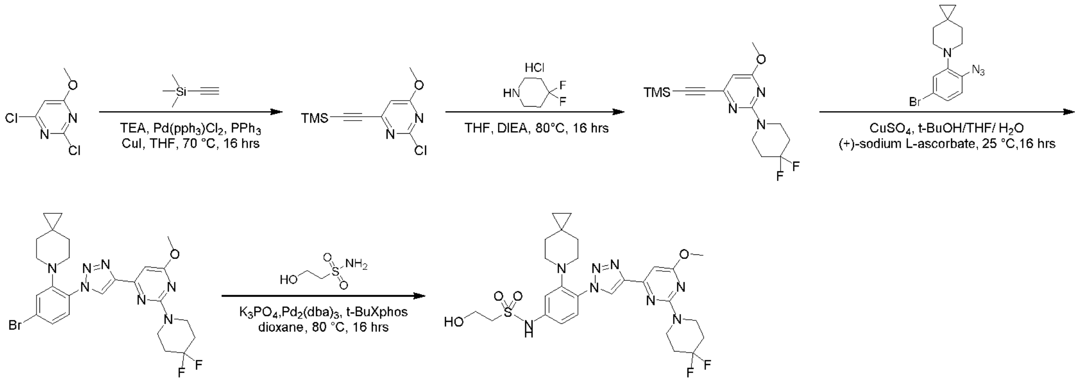


Step 1: Bis(triphenylphosphine) palladium dichloride (0.06 g, 0.08 mmol) and triphenylphosphine (0.04 g, 0.17 mmol) were added to a solution of triethylamine (5.09 g, 50.3 mmol) and THF (60 mL). Then, 2,4-dichloro-6-methoxypyrimidine (3.00 g, 16.7 mmol), cuprous iodide (0.03 g, 0.17 mmol) and ethynyltrimethylsilane (1.81 g, 18.4 mmol) were added, and stirred at 70 °C for 16 h under nitrogen atmosphere. The reaction solution was directly used in the next step.

Step 2: To the reaction solution prepared in the first step, HCl in 4,4-difluoropiperidine (3.14 g, 19.94 mmol) and diisopropylethylamine (6.44 g, 49.8 mmol) were added and stirred at 80°C for 16 h. The reaction solution was diluted with water (100 mL), extracted with ethyl acetate 400 mL (200 mL × 2), and the organic phase was washed with saturated saline (300 mL), dried, filtered and concentrated. The residue was purified by flash column chromatography (silica gel, 0-5% gradient of THF/petroleum ether) to give 2-(4,4-difluoropiperidin-1-yl)-4-methoxy-6-((trimethyl silyl) ethynyl)pyrimidine as a brown oil (2.00 g, 2.33 mmol, yield: 14%). LCMS (ESI): $[M+H]^+$ = 326.2

Step 3: 2-(4,4-difluoropiperidin-1-yl)-4-methoxy-6-((trimethylsilyl)ethynyl)pyrimidine (1.00 g, 3.07 mmol) and 6-(2-azido-5-bromophenyl)-6-azaspiro[2.5]octane (0.94 g, 3.07 mmol) were dissolved in tert-butanol (10 mL), THF (10 mL) and pure water (10.00 mL), and then sodium ascorbate (0.61 g, 3.07 mmol) and anhydrous copper sulfate (0.49 g, 3.07 mmol) were added. The mixture was stirred at 25°C for 16 h. The reaction solution was diluted with water (50 mL), extracted with 200 mL ethyl acetate (100 mL × 2), and the organic phase was dried, filtered and concentrated. The residue was purified by flash column chromatography (silica gel, 0-13% gradient of THF/petroleum ether) to give 6-(5-bromo-2-(4-(2-(4,4-difluoropiperidin-1-yl)-6-methoxypyrimidin-4-yl)-1H-1,2,3-t riazol-1-yl)phenyl)-6-azaspiro[2.5] octane as a yellow solid (0.56 g, 0.89 mmol, yield: 29%). LCMS (ESI): $[M+H]^+$ = 562.3. $^1H$ NMR (400 MHz, CHLOROFORM-d) δ ppm 8.92 (s, 1H), 7.50 (d, *J* = 8.4 Hz, 1H), 7.28 - 7.21 (m, 2H), 6.82 (s, 1H), 4.00 - 3.93 (m, 4H), 3.89 (s, 3H), 2.72 (t, *J* = 5.2 Hz, 4H), 2.03 - 1.85 (m, 4H), 1.35 - 1.25 (m, 4H), 0.23 (s, 4H)

Step 4: 6-(5-bromo-2-(4-(2-(4,4-difluoropiperidin-1-yl)-6-methoxypyrimidin-4-yl)-1H-1,2,3-t riazol-1-yl)phenyl)-6-azaspiro[2.5]octane (260 mg, 0.46 mmol) and 2-hydroxyethanesulfonamide (75.5 mg, 0.60 mmol) were dissolved in dioxane (5.00 mL). Potassium phosphate (295 mg, 1.39 mmol), 2-di-tert-butylphosphin-2',4',6'-triisopropylbiphenyl (39.4 mg, 0.09 mmol) and tris(dibenzylideneacetone)dipalladium (42.5 mg, 0.05 mmol) were added and stirred at 80°C for 16 h under nitrogen atmosphere. The reaction solution was diluted with water (50 mL), extracted with ethyl acetate 200 mL (100 mL×2), and the organic phase was dried, filtered and concentrated. The residue was purified by preparative HPLC (C18, 40 - 80 % water (liquor ammonia + ammonium bicarbonate)/acetonitrile) to give N-(4-(4-(2-(4,4-difluoropiperidin-1-yl)-6-methoxypyrimidin-4-yl)-1H-1,2,3-triazol-1-yl)-3-(6-azaspiro[2.5]octan-6-yl) phenyl)-2-hydroxyethanesulfonamide as a white solid (110.60 mg, 0.18 mmol, yield: 39%). LCMS (ESI): $[M+H]^+$ = 605.2. $^1H$ NMR (400 MHz, DMSO-$d_6$) δ ppm 9.17 (s, 1H), 7.57 (d, *J* = 8.4 Hz, 1H), 7.14 (d, *J* = 2.4 Hz, 1H), 7.04 (dd, *J* = 2.0, 8.4 Hz, 1H), 6.69 (s, 1H), 5.39 - 4.57 (m, 1H), 3.98 (br t, *J* = 5.2 Hz, 4H), 3.92 (s, 3H), 3.78 (t, *J* = 6.8 Hz, 2H), 3.34 - 3.30 (m, 2H), 2.67 (br t, *J* = 4.4 Hz, 4H), 2.08 - 1.94 (m, 4H), 1.36 - 1.24 (m, 4H), 0.25 (s, 4H)

Example 52:

**N-(4-(4-(2-(4,4-difluoropiperidin-1-yl)-6-hydroxypyrimidin-4-yl)-1H-1,2,3-triazol -1-yl)-3-(6-azaspiro[2.5]octan-6-yl)phenyl)-2-hydroxyethane-1-sulfonamide**

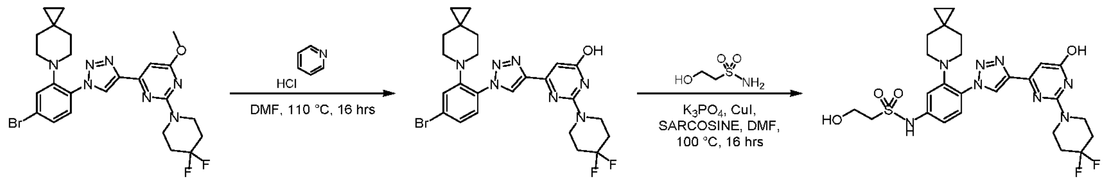


Step 1: 6-(5-bromo-2-{4-[2-(4,4-difluoropiperidin-1-yl)-6-methoxypyrimidin-4-yl]-1H-1,2,3-triazol-1-yl}phenyl)-6-azaspiro[2.5]octane (5.80 g, 10.3 mmol) was dissolved in N,N-dimethylformamide (120 mL); pyridine hydrochloride (23.9 g, 206 mmol) was added and stirred at 110°C for 48 h. The reaction solution was diluted with water (200 mL) and extracted with dichloromethane (200 mL × 3); the organic phase was dried, filtered, and concentrated. The residue was purified by flash column chromatography (silica gel, 0-8% gradient of THF/dichloromethane) to give 6-[1-(2-{6-azaspiro[2.5]octan-6-yl}-4-bromophenyl)-1H-1,2,3-triazol-4-yl]-2-(4,4-dif luoropiperidin-1-yl)pyrimidin-4-phenol as a yellow solid (4.67 g, 7.14 mmol, yield: 69%). LCMS (ESI): $[M+H]^+$ = 548.0, $^1H$ NMR (400 MHz, CHLOROFORM-d) δ ppm 13.07 - 11.51 (m, 1H), 8.87 (s, 1H), 7.50 (d, *J* = 8.0 Hz, 1H), 7.26 - 7.21 (m, 2H), 6.62 (s, 1H), 3.90 (br t, *J* = 5.2 Hz, 4H), 2.70 (t, *J* = 5.2 Hz, 4H), 2.10 - 1.96 (m, 4H), 1.32 - 1.25 (m, 4H), 0.22 (s, 4H)

Step 2: 6-[1-(2-{6-azaspiro[2.5]octan-6-yl}-4-bromophenyl)-1H-1,2,3-triazol-4-yl]-2-(4,4-dif luoropiperidin-1-yl)pyrimidin-4-phenol (500 mg, 0.92 mmol) and 2-hydroxyethane-1-sulfonamide (229 mg, 1.83 mmol) were dissolved in N,N-dimethylformamide (10.0 mL); potassium phosphate (582 mg, 2.75 mmol), (1R, 2R)-N1, N2-dimethylcyclohexane-1,2-diamine (131 mg, 0.92 mmol) and cuprous iodide (174 mg, 0.92 mmol) were added and stirred at 100°C for 16 h. The reaction solution was diluted with 50 mL of water and 0.5 mL of liquor ammonia) and extracted with dichloromethane (100 mL × 2); the organic phase was dried, filtered, and concentrated. The residue was purified by preparative HPLC (C18, 20-60% gradient of water (formic acid)/acetonitrile) to give N-(4-(4-(2-(4,4-difluoropiperidin-1-yl)-6-hydroxypyrimidin-4-yl)-1H-1,2,3-triazol-1-yl)-3-(6-azaspiro[2.5]octan-6-yl)phenyl)-2-hydroxyethane-1-sulfonamide (76.9 mg, 0.13 mmol, yield: 14%) as a white solid. LCMS (ESI): $[M+H]^+$ = 591.2, $^1H$ NMR (400 MHz, DMSO-$d_6$) δ ppm 9.10 (s, 1H), 7.57 (d, *J* = 8.8 Hz, 1H), 7.13 (d, *J*= 2.4 Hz, 1H), 7.03 (dd, *J* = 2.8, 9.2 Hz, 1H), 6.39 (br s, 1H), 3.92 - 3.85 (m, 4H), 3.78 (t, *J* = 6.8 Hz, 2H), 3.39 - 3.34 (m, 2H), 2.67 (br t, *J* = 4.8 Hz, 4H), 2.09 - 1.97 (m, 4H), 1.34 - 1.26 (m, 4H), 0.26 (s, 4H)

**Example 53:**

**N-(4-(4-(2-((3S,5R)-3,5-dimethylmorpholino)-6-methoxypyrimidin-4-yl)-1H-1,2,3 -triazol-1-yl)-3-(6-azaspiro[2.5] octan-6-yl)phenyl)-2-hydroxyethane-1-sulfonamid e**

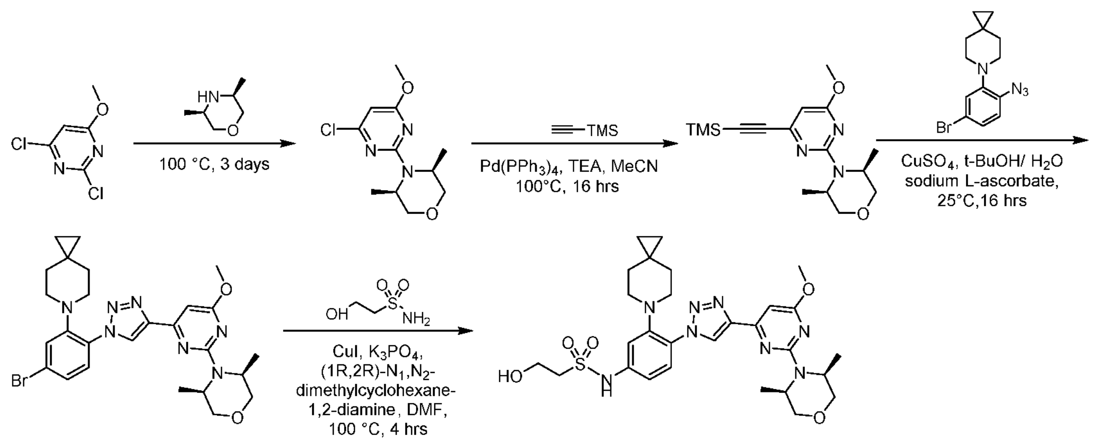


Step 1: 2,4-dichloro-6-methoxypyrimidine (1.00 g, 5.59 mmol) and (3R,5S)-3,5-dimethylmorpholine (0.64 g, 5.59 mmol) were stirred at 100°C for 3 d. The mixture was purified by flash column chromatography (silica gel, 0-4% gradient of THF/petroleum ether) to give (3R,5S)-4-(4-chloro-6-methoxypyrimidin-2-yl)-3,5-dimethylmorpholine (0.20 g, 0.71 mmol, yield: 12.7%). LCMS (ESI): $[M+H]^+$ = 258.1; $^1H$ NMR (400 MHz, CHLOROFORM-d) δ ppm 6.02 (s, 1H), 4.52 (dd, *J* = 4.0, 7.2 Hz, 2H), 3.89 (s, 3H), 3.82 (d, *J*= 11.6 Hz, 2H), 3.65 (dd, *J* = 3.6, 11.2 Hz, 2H), 1.36 (d, *J* = 6.8 Hz, 6H) Step 2: (3R,5S)-4-(4-chloro-6-methoxypyrimidin-2-yl)-3,5-dimethylmorpholine (150 mg, 0.58 mmol)

was dissolved in acetonitrile (3.00 mL). Triethylamine (235 mg, 2.33 mmol), ethynyltrimethylsilane (571 mg, 5.82 mmol) and tetrakis(triphenylphosphine)palladium (134 mg, 0.12 mmol) were added and stirred at 100°C for 16 h under nitrogen atmosphere. The reaction solution was diluted with water (50.0 mL) and extracted with ethyl acetate (100 mL x 2); the organic phase was dried, filtered, and concentrated. The residue was purified by flash column chromatography (silica gel, 0-4% gradient of THF/petroleum ether) to give (3R, 5S)-4-{4-methoxy-6-[2-(trimethylsilyl)ethynyl] pyrimidin-2-yl}-3,5-dimethylmorpholi ne as a white solid (120 mg, 0.28 mmol, yield: 49.0%). LCMS (ESI): $[M+H]^+$ = 320.1; $^1H$ NMR (400 MHz, CHLOROFORM-d) δ ppm 6.15 (s, 1H), 4.58 (br dd, *J* = 3.6, 6.8 Hz, 2H), 3.88 (s, 3H), 3.81 (d, *J* = 11.2 Hz, 2H), 3.64 (dd, *J* = 3.6, 11.2 Hz, 2H), 1.36 (s, 3H), 1.35 (s, 3H), 0.26 (s, 9H)

Step 3: (3R,5S)-4-{4-methoxy-6-[2-(trimethylsilyl)ethynyl]pyrimidin-2-yl}-3,5-dimethylmor pholine (100 mg, 0.31 mmol) and 6-(2-azido-5-bromophenyl)-6-azaspiro[2.5]octane (96.1 mg, 0.31 mmol) were dissolved in tert-butanol (1.00 mL), THF (1.00 mL) and pure water (1.00 mL). Sodium ascorbate (62.0 mg, 0.31 mmol) and anhydrous copper sulfate (49.9 mg, 0.31 mmol) were added and stirred at 25°C for 16 h. The reaction solution was diluted with water (50.0 mL), extracted with ethyl acetate (100 mL×2), and the organic phase was dried, filtered and concentrated. The residue was purified by flash column chromatography (silica gel, 0-7% gradient of THF/petroleum ether) to give 6-[5-bromo-2-(4-{2-[(3R,5S)-3,5-dimethylmorpholin-4-yl]-6-methoxypyrimidin-4-yl} -1H-1,2,3-triazol-1-yl)phenyl]-6-azaspiro[2.5]octane as a yellow solid (160 mg, 0.23 mmol, yield: 75.0%). LCMS (ESI): $[M+H]^+$ = 555.9; $^1H$ NMR (400 MHz, CHLOROFORM-d) δ ppm 8.97 (s, 1H), 7.56 (d, *J* = 8.0 Hz, 1H), 7.27 - 7.24 (m, 1H), 7.21 (s, 1H), 6.83 (s, 1H), 4.61 (br dd, *J* = 3.6, 6.8 Hz, 2H), 3.90 (s, 3H), 3.81 (d, *J* = 11.2 Hz, 2H), 3.66 (dd, *J* = 4.0, 11.6 Hz, 2H), 2.74 (t, *J* = 5.2 Hz, 4H), 1.39 - 1.30 (m, 10H), 0.23 (s, 4H)

Step 4: 6-[5-bromo-2-(4-{2-[(3R,5S)-3,5-dimethylmorpholin-4-yl]-6-methoxypyrimidin-4-yl} -1H-1,2,3-triazol-1-yl) phenyl]-6-azaspiro [2.5]octane (140 mg, 0.25 mmol) and 2-hydroxyethane-1-sulfonamide (41.1 mg, 0.33 mmol) were dissolved in N,N-dimethylformamide (3.00 mL). Potassium phosphate (160 mg, 0.76 mmol), (1R,2R)-N1,N2-dimethylcyclohexane-1,2-diamine (36.2 mg, 0.25 mmol) and cuprous iodide (48.1 mg, 0.25 mmol) were added and stirred at 100 °C for 4 h. The reaction solution was diluted with water (50.0 mL + 0.50 mL of liquor ammonia) and extracted with ethyl acetate (100 mL × 2); the organic phase was dried, filtered, and concentrated. The residue was purified by preparative HPLC (C18, 42-82% gradient of water (liquor ammonia + ammonium bicarbonate)/acetonitrile) to give N-(4-(4-(2-((3S,5R)-3,5-dimethylmorpholino)-6-methoxypyrimidin-4-yl)-1H-1,2,3-tri azol-1-yl)-3-(6-azaspiro [2.5]octan-6-yl)phenyl)-2-hydroxyethane-1-sulfonamide as a white solid (90.9 mg, 0.15 mmol, yield: 60.0%). LCMS (ESI): $[M+H]^+$ = 599.3; $^1H$ NMR (400 MHz, DMSO-$d_6$) δ ppm 10.60 - 9.47 (m, 1H), 9.13 (s, 1H), 7.60 (d, *J* = 8.8 Hz, 1H), 7.13 (d, *J* = 2.0 Hz, 1H), 7.04 (dd, *J* = 2.0, 8.4 Hz, 1H), 6.66 (s, 1H), 5.37 - 4.72 (m, 1H), 4.58 (br dd, *J* = 3.6, 6.4 Hz, 2H), 3.90 (s, 3H), 3.83 - 3.74 (m, 4H), 3.60 (br dd, *J* = 3.6, 11.6 Hz, 2H), 3.36 - 3.33 (m, 2H), 2.66 (br t, *J* = 4.4 Hz, 4H), 1.38 - 1.22 (m, 10H), 0.23 (s, 4H)

**Example 54:**

**N-(3-{6-azaspiro[2.5]octan-6-yl}-4-{4-[2-(4,4-difluoropiperidin-1-yl)-6-(dimethyla mino)pyrimidin-4-yl]-1H-1,2,3-triazol-1-yl}phenyl)-2-hydroxyethane-1-sulfonami de**

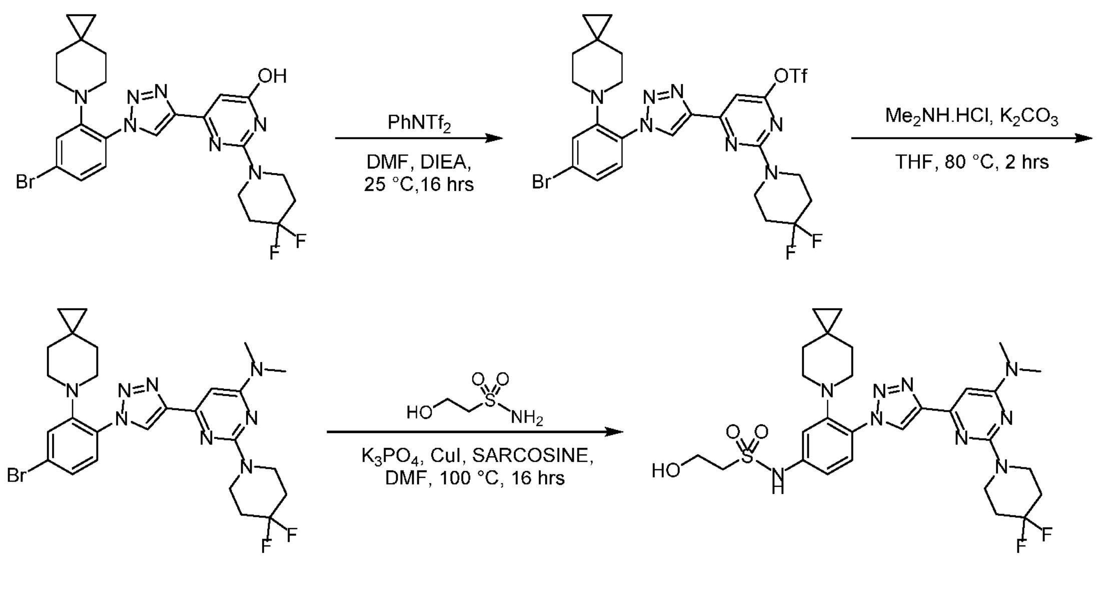

Step 1: 6-[1-(2-{6-azaspiro[2.5]octan-6-yl}-4-bromophenyl)-1H-1,2,3-triazol-4-yl]-2-(4,4-dif luoropiperidin-1-yl)pyrimidin-4-phenol (2.90 g, 5.31 mmol) was dissolved in N,N-dimethylformamide (60.0 mL). 1,1,1-trifluoro-N-phenyl-N-trifluoromethanesulfonamide (2.84 g, 7.96 mmol) and diisopropylethylamine (3.43 g, 26.5 mmol) were added and stirred at 25°C for 16 h. The reaction solution was diluted with water (100 mL) and extracted with ethyl acetate (200 mL × 2); the organic phase was dried, filtered, and concentrated. The residue was purified by flash column chromatography (silica gel, 0-2% gradient of THF/dichloromethane) to give 6-[1-(2-{6-azaspiro[2.5]octan-6-yl}-4-bromophenyl)-1H-1,2,3-triazol-4-yl]-2-(4,4-dif luoropiperidin-1-yl)pyrimidin-4-trifluoromethanesulfonate as a yellow oil (3.50 g, 5.10 mmol, yield: 96%). LCMS (ESI): $[M+H]^+$ = 679.8, $^1H$ NMR (400 MHz, CHLOROFORM-d) δ ppm 9.00 (s, 1H), 7.53 - 7.49 (m, 1H), 7.36 - 7.32 (m, 1H), 7.29 (s, 1H), 7.10 (s, 1H), 3.96 (br s, 4H), 2.73 (br t, *J* = 4.8 Hz, 4H), 2.05 - 1.91 (m, 4H), 1.34 - 1.25 (m, 4H), 0.24 (s, 4H)

Step 2: Dimethylamine hydrochloride (0.24 g, 2.95 mmol) was dissolved in THF (20. 0 mL). potassium carbonate (0.81 g, 5.90 mmol) and 6-[1-(2-{6-azaspiro[2.5]octan-6-yl}-4-bromophenyl)-1H-1,2,3-triazol-4-yl]-2-(4,4-dif luoropiperidin-1-yl)pyrimidin-4-yl trifluoromethanesulfonate (1.00 g, 1.47 mmol) were added and stirred at 80°C for 2 h. The reaction solution was diluted with water (50 mL) and extracted with ethyl acetate (100 mL × 2); the organic phase was dried, filtered, and concentrated. The residue was purified by flash column chromatography (silica gel, 0-11% gradient of THF/petroleum ether) to give 6-[1-(2-{6-azaspiro[2.5]octan-6-yl}-4-bromophenyl)-1H-1,2,3-triazol-4-yl]-2-(4,4-dif luoropiperidin-1-yl)-N,N-dimethylpyrimidin-4-amine as a white solid (0.68 g, 1.08 mmol, yield: 73%). LCMS (ESI): $[M+H]^+$ = 575.0, $^1H$ NMR (400 MHz, DMSO-$d_6$) δ ppm 9.14 (s, 1H), 7.58 (d, *J* = 8.4 Hz, 1H), 7.47 - 7.39 (m, 2H), 6.65 (s, 1H), 3.93 (br t, *J*= 5.2 Hz, 4H), 3.10 (s, 6H), 2.75 - 2.67 (m, 4H), 2.03 - 1.90 (m, 4H), 1.33 - 1.25 (m, 4H), 0.24 (s, 4H)

Step 3: 6-[1-(2-{6-azaspiro[2.5]octan-6-yl}-4-bromophenyl)-1H-1,2,3-triazol-4-yl]-2-(4,4-dif luoropiperidin-1-yl)-N,N-dimethylpyrimidin-4-amine (640 mg, 1.12 mmol) and 2-hydroxyethane-1-sulfonamide (279 mg, 2.23 mmol) was dissolved in N,N-dimethylformamide (12.0 mL), then potassium phosphate (710 mg, 3.35 mmol), (1R,2R)-N1,N2-dimethylcyclohexane-1,2-diamine (160 mg, 1.12 mmol), and cuprous iodide (212 mg, 1.12 mmol) were added and stirred at 100°C for 16 h. The reaction solution was diluted with water (100 mL + 1 mL of liquor ammonia), extracted with ethyl acetate (100 mL x 2), and the organic phase was dried, filtered and concentrated. Purification was performed using HPLC (C18, 26 - 66 % water (formic acid)/acetonitrile). The residue was purified by normal phase separation (XB-CN, 5 - 35 % n-heptane-ethanol (0.1% liquor ammonia)) to give N-(3-{6-azaspiro[2.5]octan-6-yl}-4-{4-[2-(4,4-difluoropiperidin-1-yl)-6-(dimethylami no)pyrimidin-4-yl]-1H-1,2,3-triazol-1-yl}phenyl)-2-hydroxyethane-1-sulfonamide as a white solid (244 mg, 0.39 mmol, yield: 35%). LCMS (ESI): $[M+H]^+$ = 618.3, $^1H$ NMR (400 MHz, DMSO-$d_6$) δ ppm 10.49 - 9.60 (m, 1H), 9.05 (s, 1H), 7.57 (d, *J* = 8.4 Hz, 1H), 7.12 (d, *J* = 2.0 Hz, 1H), 7.03 (dd, *J* = 2.0, 8.4 Hz, 1H), 6.64 (s, 1H), 5.17 - 4.76 (m, 1H), 3.94 - 3.86 (m, 4H), 3.78 (t, *J* = 6.4 Hz, 2H), 3.34 - 3.30 (m, 2H), 3.09 (s, 6H), 2.66 (br t, *J* = 4.4 Hz, 4H), 2.02 - 1.89 (m, 4H), 1.35 - 1.22 (m, 4H), 0.24 (s, 4H)

**Examples 55 to 61** were prepared with reference to the synthetic route of **Example 54:**

**Example 55:**

**N-(4-{4-[6-amino-2-(4,4-difluoropiperidin-1-yl)pyrimidin-4-yl]-1H-1,2,3-triazol-1 -yl}-3-{6-azaspiro[2.5]octan-6-yl}phenyl)-2-hydroxyethane-1-sulfonamide**

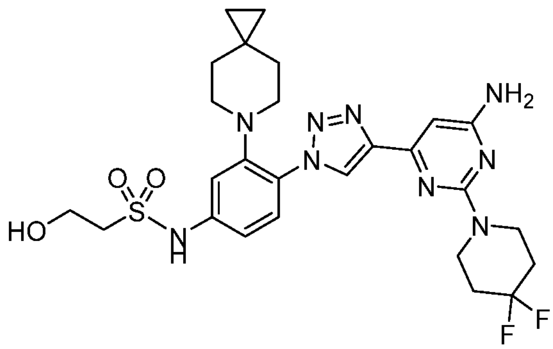

LCMS (ESI): $[M+H]^+$ = 590.3, $^1H$ NMR (400 MHz, DMSO-$d_6$) δ 10.02 (s, 1H), 9.02 (br s, 1H), 7.58 (br d, *J* = 8.0 Hz, 1H), 7.13 (br s, 1H), 7.03 (br d, *J* = 8.0 Hz, 1H), 6.70 (br s, 2H), 6.52 (br s, 1H), 5.19 - 4.73 (m, 1H), 3.90 (br s, 4H), 3.78 (br s, 2H), 3.40 - 3.37 (m, 2H), 2.67 (br s, 4H), 2.05 - 1.85 (br s, 4H), 1.40 - 1.24 (m, 4H), 0.26 (br s, 4H)

**Example 56:**

**N-(3-{6-azaspiro[2.5]octan-6-yl}-4-{4-[2-(4,4-difluoropiperidin-1-yl)-6-(methylam ino)pyrimidin-4-yl]-1H-1,2,3-triazol-1-yl}phenyl)-2-**hydroxyethane-1-sulfonamide

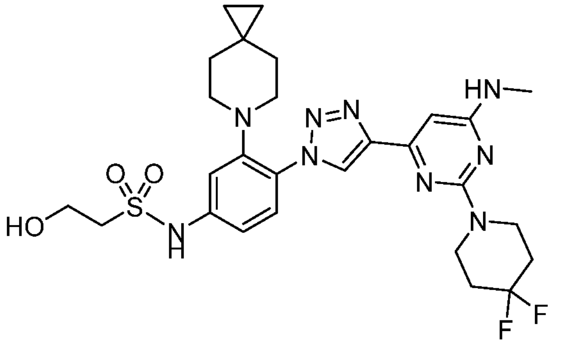

LCMS (ESI): $[M+H]^+ = 604.5$, $^1H$ NMR (400 MHz, DMSO-$d_6$) δ ppm 9.01 (s, 1H), 7.55 (d, *J* = 8.4 Hz, 1H), 7.26 (br s, 1H), 7.10 (d, *J* = 2.0 Hz, 1H), 7.01 (dd, *J* = 2.0, 8.4 Hz, 1H), 6.52 (s, 1H), 3.98 - 3.88 (m, 4H), 3.78 (t, *J* = 6.8 Hz, 2H), 3.33 - 3.30 (m, 2H), 2.84 (d, *J* = 4.4 Hz, 3H), 2.67 (br t, *J* = 4.8 Hz, 4H), 2.03 - 1.87 (m, 4H), 1.36 - 1.25 (m, 4H), 0.25 (s, 4H)

**Example 57:**

**N-(3-{6-azaspiro[2.5]octan-6-yl}-4-{4-[2-(4,4-difluoropiperidin-1-yl)-6-[(2-hydrox yethyl)amino]pyrimidin-4-yl]-1H-1,2,3-triazol-1-yl}phenyl)-2-hydroxyethane-1-s ulfonamide**

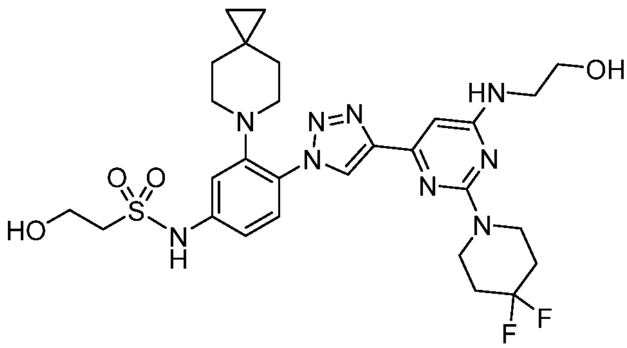

LCMS (ESI): $[M+H]^+ = 634.2$, $^1H$ NMR (400 MHz, DMSO-$d_6$) δ ppm 10.51 - 9.43 (m, 1H), 9.00 (s, 1H), 7.57 (d, *J* = 8.8 Hz, 1H), 7.36 (br s, 1H), 7.11 (d, *J* = 2.0 Hz, 1H), 7.02 (dd, *J* = 2.0, 8.4 Hz, 1H), 6.55 (s, 1H), 4.72 (br s, 2H), 3.95 - 3.86 (m, 4H), 3.77 (t, *J* = 6.4 Hz, 2H), 3.58 - 3.51 (m, 2H), 3.43 - 3.36 (m, 2H), 3.33 - 3.27 (m, 2H), 2.70 - 2.61 (m, 4H), 2.01 - 1.89 (m, 4H), 1.35 - 1.24 (m, 4H), 0.24 (s, 4H)

**Example 58:**

**N-(3-{6-azaspiro[2.5]octan-6-yl}-4-{4-|2-(4,4-difluoropiperidin-1-yl)-6-[(oxan-4-yl) amino]pyrimidin-4-yl]-1H-1,2,3-triazol-1-yl}phenyl)-2-hydroxyethane-1-sulfona mide**

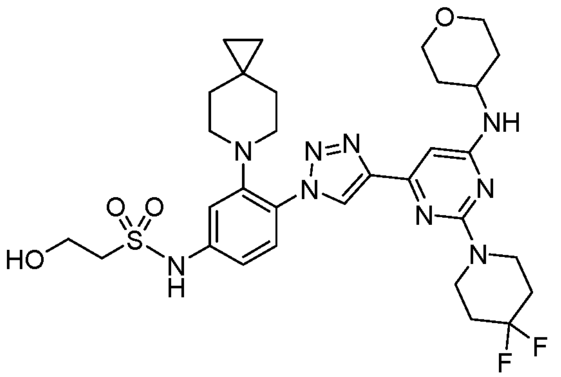

LCMS (ESI): $[M+H]^+$ = 674.7; $^1H$ NMR (400 MHz, DMSO-$d_6$) δ ppm 9.01 (s, 1H), 7.57 (d, *J* = 8.4 Hz, 1H), 7.38 - 7.23 (m, 1H), 7.12 (d, *J* = 1.6 Hz, 1H), 7.02 (dd, *J* = 1.6, 8.4 Hz, 1H), 6.55 (s, 1H), 4.12 - 3.95 (m, 1H), 3.95 - 3.83 (m, 6H), 3.78 (t, *J* = 6.4 Hz, 2H), 3.49 - 3.39 (m, 2H), 3.32 (br s, 2H), 2.66 (br t, *J* = 4.8 Hz, 4H), 2.54 - 2.52 (m, 1H), 2.02 - 1.85 (m, 6H), 1.54 - 1.40 (m, 2H), 1.34 - 1.26 (m, 4H), 0.25 (s, 4H)

**Example 59:**

**N-(3-{6-azaspiro[2.5]octan-6-yl}-4-{4-[2-(4,4-difluoropiperidin-1-yl)-6-{[(pyridine -2-yl)methyl]amino}pyrimidin-4-yl]-1H-1,2,3-triazol-1-yl}phenyl)-2-hydroxyetha ne-1-sulfonamide**

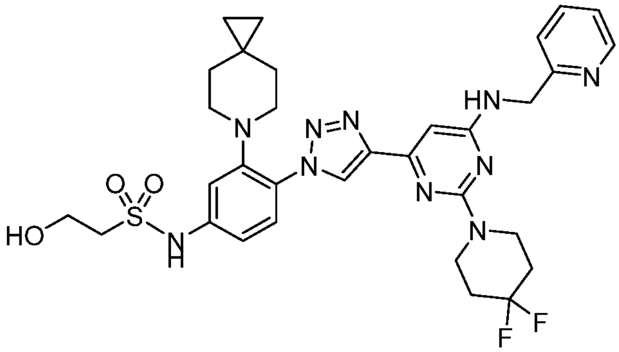

LCMS (ESI): $[M+H]^+$ = 681.3; $^1H$ NMR (400 MHz, DMSO-$d_6$) δ ppm 10.24 - 9.76 (m, 1H), 9.01 (s, 1H), 8.51 (br d, *J* = 4.4 Hz, 1H), 8.02 (br s, 1H), 7.74 (br t, *J* = 7.6 Hz, 1H), 7.57 (br d, *J* = 8.4 Hz, 1H), 7.35 (d, *J* = 7.6 Hz, 1H), 7.30 - 7.20 (m, 1H), 7.11 (s, 1H), 7.02 (br d, *J* = 8.8 Hz, 1H), 6.66 (br s, 1H), 4.98 (br d, *J* = 2.4 Hz, 1H), 4.61 (br d, *J* = 3.6 Hz, 2H), 3.90 - 3.70 (m, 6H), 3.34 - 3.30 (m, 2H), 2.70 - 2.60 (m, 4H), 1.90 - 1.70 (m, 4H), 1.34 - 1.24 (m, 4H), 0.24 (s, 4H)

**Example 60:**

**N-(3-{6-azaspiro[2.5]octan-6-yl}-4-{4-[2-(4,4-difluoropiperidin-1-yl)-6-{[(pyridine -3-yl)methyl]amino}pyrimidin-4-yl]-1H-1,2,3-triazol-1-yl}phenyl)-2-hydroxyetha ne-1-sulfonamide**

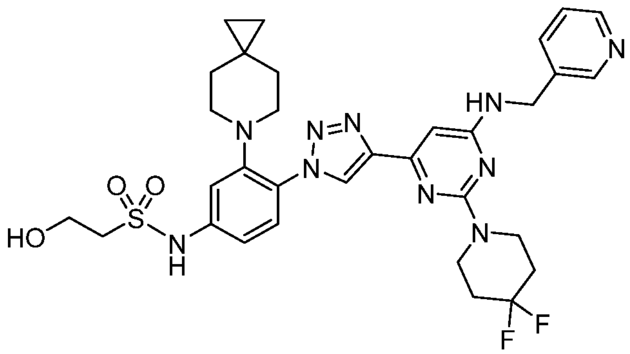

LCMS (ESI): $[M+H]^+$ = 681.3; $^1$H NMR (400 MHz, DMSO-$d_6$) δ ppm 10.03 (s, 1H), 9.02 (s, 1H), 8.76 - 8.39 (m, 2H), 7.96 (br d, *J* = 1.2 Hz, 1H), 7.78 (br d, *J* = 7.6 Hz, 1H), 7.57 (d, *J* = 8.4 Hz, 1H), 7.39 (br d, *J* = 2.0 Hz, 1H), 7.11 (d, *J* = 2.0 Hz, 1H), 7.02 (dd, *J* = 2.0, 8.4 Hz, 1H), 6.59 (br s, 1H), 4.97 (br s, 1H), 4.56 (br d, *J* = 5.6 Hz, 2H), 3.87 (br s, 4H), 3.77 (br t, *J* = 5.6 Hz, 2H), 2.68 - 2.62 (m, 4H), 2.52 (br d, *J* = 2.0 Hz, 2H), 1.98 - 1.78 (m, 4H), 1.35 - 1.22 (m, 4H), 0.24 (s, 4H)

**Example 61:**

**N-(3-{6-azaspiro[2.5]octan-6-yl}-4-{4-[2-(4,4-difluoropiperidin-1-yl)-6-{[(pyridine -2-yl)methyl]amino}pyrimidin-4-yl]-1H-1,2,3-triazol-1-yl}phenyl)-2-hydroxyetha ne-1-sulfonamide**

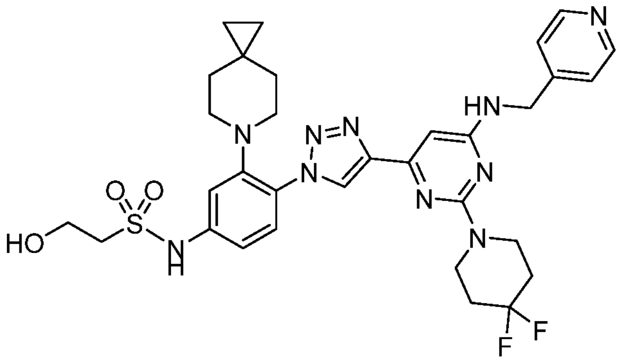

LCMS (ESI): $[M+H]^+$ = 681.2; $^1$H NMR (400 MHz, DMSO-$d_6$) δ ppm 10.03 (s, 1H), 9.02 (s, 1H), 8.54 (br s, 2H), 8.03 (br s, 1H), 7.57 (d, *J* = 8.8 Hz, 1H), 7.41 (br d, *J* = 4.8 Hz, 2H), 7.12 (d, *J* = 2.4 Hz, 1H), 7.02 (dd, *J* = 2.4, 8.8 Hz, 1H), 6.65 (br s, 1H), 4.96 (br s, 1H), 4.58 (br d, *J* = 4.8 Hz, 2H), 3.91 - 3.74 (m, 6H), 3.35 - 3.30 (m, 2H), 2.71 - 2.62 (m, 4H), 1.93 - 1.63 (m, 4H), 1.36 - 1.24 (m, 4H), 0.24 (s, 4H)

**Examples 62 to 65** were prepared with reference to the synthetic route of Example **34:**

**Example 62:**

**N-(4-(4-(2-(4,4-difluoropiperidin-1-yl)-6-methoxypyridin-4-yl)-1H-1,2,3-triazol-1 -yl)-3-(6-azaspiro[2.5]octan-6-yl) phenyl)-2-hydroxyethane-1-sulfonamide**

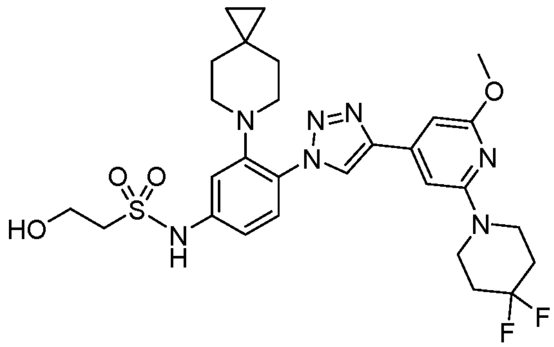

LCMS (ESI): $[M+H]^+$ = 604.0; $^1$H NMR (400 MHz, DMSO-$d_6$) δ ppm 10.04 (s, 1H), 9.17 (s, 1H), 7.48 (d, *J* = 8.4 Hz, 1H), 7.12 (d, *J* = 2.0 Hz, 1H), 7.05 - 6.98 (m, 2H), 6.64 (s, 1H), 5.21 - 4.51 (m, 1H), 3.84 (s, 3H), 3.81 - 3.72 (m, 6H), 3.34 (br t, *J*= 6.4 Hz, 2H), 2.66 (br t, *J* = 4.4 Hz, 4H), 2.11 - 1.95 (m, 4H), 1.24 (br s, 4H), 0.23 (s, 4H)

**Example 63:**

**N-(4-(4-(2-(4,4-difluoropiperidin-1-yl)-3-fluoro-6-methoxypyridin-4-yl)-1H-1,2,3-triazol-1-yl)-3-(6-azaspiro[2.5]octan-6-yl)phenyl)-2-hydroxyethane-1-sulfonamid e**

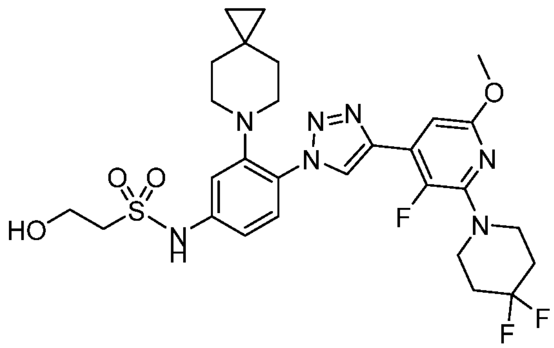

LCMS (ESI): [M+H]+ = 622.3; $^1$H NMR (400 MHz, DMSO-d6) δ ppm 10.07 (s, 1H), 8.93 (d, *J* = 3.6 Hz, 1H), 7.53 (d, *J* = 8.4 Hz, 1H), 7.15 (d, *J* = 2.0 Hz, 1H), 7.04 (dd, *J* = 2.0, 8.4 Hz, 1H), 6.91 (d, *J* = 2.8 Hz, 1H), 4.98 (t, *J* = 5.6 Hz, 1H), 3.86 (s, 3H), 3.79 (q, *J* = 6.4 Hz, 2H), 3.64 (br t, *J* = 5.2 Hz, 4H), 3.41 - 3.34 (m, 2H), 2.72 - 2.65 (m, 4H), 2.21 - 2.01 (m, 4H), 1.25 (br s, 4H), 0.25 (s, 4H)

**Example 64:**

**N-(4-(4-(2-(4,4-difluoropiperidin-1-yl)-6-methylpyridin-4-yl)-1H-1,2,3-triazol-1-y l)-3-(6-azaspiro[2.5]octan-6-yl)phenyl)-2-hydroxyethane-1-sulfonamide**

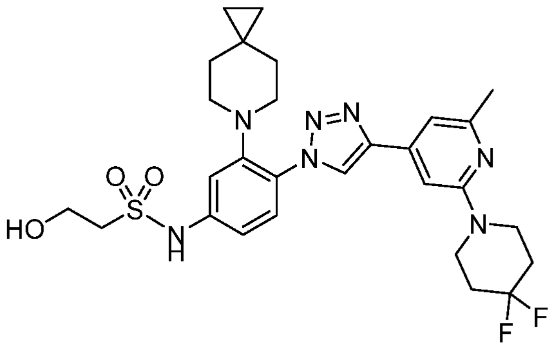

LCMS (ESI): $[M+H]^+$ = 588.3, $^1H$ NMR (400 MHz, DMSO-$d_6$) δ ppm 9.84 (s, 1H), 8.94 (s, 1H), 7.25 (d, *J* = 8.4 Hz, 1H), 7.03 (s, 1H), 6.90 (d, *J* = 2.8 Hz, 2H), 6.79 (dd, *J*= 2.0, 8.8 Hz, 1H), 4.77 (br s, 1H), 3.61 - 3.47 (m, 6H), 3.12 - 3.09 (m, 2H), 2.43 (br t, *J*= 4.4 Hz, 4H), 2.16 (s, 3H), 1.85 - 1.69 (m, 4H), 1.06 - 0.96 (m, 4H), 0.00 (s, 4H)

**Example 65:**

**N-(4-(4-(2-(4,4-difluoropiperidin-1-yl)-3-fluoro-6-methylpyridin-4-yl)-1H-1,2,3-tr iazol-1-yl)-3-(6-azaspiro[2.5]octan-6-yl)phenyl)-2-hydroxyethane-1-sulfonamide**

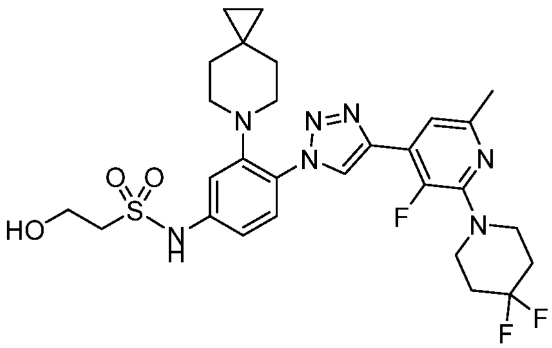

LCMS (ESI): $[M+H]^+$ = 606.3; $^1H$ NMR (400 MHz, DMSO-$d_6$) δ ppm 10.06 (br s, 1H), 8.91 (d, *J* = 3.6 Hz, 1H), 7.52 (d, *J* = 8.4 Hz, 1H), 7.48 (d, *J* = 3.6 Hz, 1H), 7.14 (d, *J*= *2.0* Hz, 1H), 7.03 (dd, *J* = 1.6, 8.4 Hz, 1H), 5.40 - 4.58 (m, 1H), 3.78 (t, *J* = 6.4 Hz, 2H), 3.65 - 3.50 (m, 4H), 3.42 - 3.33 (m, 2H), 2.66 (br d, *J* = 4.8 Hz, 4H), 2.42 (s, 3H), 2.20 - 2.04 (m, 4H), 1.35 - 1.15 (m, 4H), 0.24 (s, 4H)

**Example 66:**

**N-(6-{6-azaspiro[2.5]octan-6-yl}-5-{4-[2-(4,4-difluoropiperidin-1-yl)-6-methylpyr imidin-4-yl]-1H-1,2,3-triazol-1-yl}pyridin-2-yl)-2-hydroxyethane-1-sulfonamide**

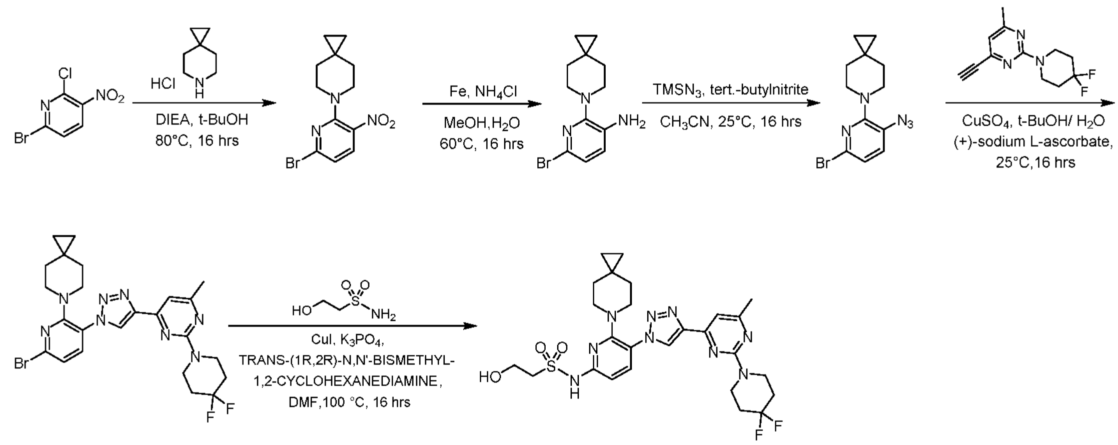


Step 1: To a solution of 6-bromo-2-chloro-3-nitropyridin (5.00 g, 21.0 mmol) and N,N-diisopropylethylamine (8.33 g, 63.2 mmol) in tert-butanol (100 mL), 6-azaspiro [2.5]octanoic hydrochloride (2.34 g, 21.0 mmol) was added. The mixture was heated to 80°C and stirred for 16 h. The reaction mixture was diluted with water (200 mL) and extracted with ethyl acetate (200 mL×2). The organic phases were combined, dried over anhydrous sodium sulfate, and filtered, and concentrated under reduced pressure to give the residue. The residue was purified by flash column chromatography (silica gel, 0-30% gradient of ethyl acetate/petroleum ether) to give 6-(6-bromo-3-nitropyridin-2-yl)-6-azaspiro[2.5]octane (4.60 g, 15.0 mmol, yield: 70%) as a yellow solid. LCMS (ESI): $[M+H]^+$ =311.9

Step 2: To a solution of 6-(6-bromo-3-nitropyridin-2-yl)-6-azaspiro[2.5]octane (2.00 g, 6.41 mmol) in methanol (60 mL) and water (20 mL) was added ammonium chloride (1.36 g, 25.1 mmol) and iron powder (4.73 g, 83.7 mmol). The mixture was heated to 60°C and stirred for 16 h. The reaction solution was filtered with diatomite and the filtrate was extracted with ethyl acetate (100 mL × 3). The organic phases were combined, dried over anhydrous sodium sulfate, and filtered, andconcentrated under reduced pressure to give the residue. The residue was purified by flash column chromatography (silica gel, 0-10% gradient of ethyl acetate/petroleum ether) to give 2-{6-azaspiro[2.5]octan-6-yl}-6-bromopyridin-3-amine as a yellow solid (1.50 g, 5.3 mmol, yield: 83%). LCMS (ESI): [M+H]+ =283.9.

Step 3: To a solution of 2-{6-azaspiro[2.5]octan-6-yl}-6-bromopyridine-3-amine (500 mg, 2.28 mmol) in acetonitrile (5.00 mL) was added tert-butyl nitrite (0.28 g, 2.66 mmol). Then, azidotrimethylsilane (0.31 g, 2.66 mmol) was added to the reaction solution and the mixture was stirred at 25°C for 16 h. The reaction solution was poured into water (10 mL) and extracted with ethyl acetate (10 mL×3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 6-(3-azido-6-bromopyridin-2-yl)-6-azaspiro[2.5]octane as a yellow oil (400 mg, 1.29 mmol, yield: 73%). LCMS (ESI): [M+H]+ =309.9.

Step 4: To a solution of 6-(3-azido-6-bromopyridin-2-yl)-6-azaspiro[2.5]octane (200 mg, 0.65 mmol), 2-(4,4-difluoropiperidin-1-yl)-4-ethynyl-6-methylpyrimidine (154 mg, 0.65 mmol) in tert-butanol (5 mL), THF (5 mL) and water (5 mL), copper sulfate (104 mg, 0.65 mmol) and sodium ascorbate (129 mg, 0.65 mmol) were added. The mixture was stirred at 25°C for 16 h. The reaction solution was diluted with water (20 mL) and extracted with ethyl acetate (20 mL×3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 6-(6-bromo-3-{4-[2-(4,4-difluoropiperidin-1-yl)-6-methylpyrimidin-4-yl]-1H-1,2,3-tri azol-1-yl}pyridin-2-yl)-6-azaspiro[2.5]octane (165 mg, 0.30 mmol, yield 46%) as a yellow solid. LCMS (ESI): [M+H]+ =547.2

Step 5: To a solution of 2-hydroxyethanesulfonamide (52.7 mg, 0.41 mmol), (1R,2R)-(-)-N,N-dimethylcyclohexane-1,2-diamine (39.5 mg, 0.27 mmol) and potassium phosphate (178 mg, 0.83 mmol) in N,N-dimethylformamide (5.00 mL), cuprous iodide (26.7 mg, 0.14 mmol) was added. The mixture was stirred at 50°C for 5 min. Then, 6-(6-bromo-3-{4-[2-(4,4-difluoropiperidin-1-yl)-6-methylpyrimidin-4-yl]-1H-1,2,3-tri azol-1-yl}pyridin-2-yl)-6-azaspiro[2.5]octane (150 mg, 0.28 mmol) was added to the reaction mixture at 50°C, and the mixture was heated to 100°C under nitrogen atmosphere and stirred for 16 h. Water (10 mL) was added to the reaction solution and extracted with ethyl acetate (10 mL × 2). The organic phases were combined, dried over anhydrous sodium sulfate, and filtered, and concentrated under reduced pressure to give the residue. The residue was purified by preparative HPLC (C18, 42-82% gradient of water (formic acid)/acetonitrile) to give N-(3-{6-azaspiro[2.5]octan-6-yl}-4-{1-[8-(4,4-difluoropiperidin-1-yl)quinolin-6-yl]-1 H-1,2,3-triazol-4-yl}phenyl)-2-hydroxyethane-1-sulfonamide as a white solid (53 mg, 0.12 mmol, yield: 44%). LCMS (ESI): [M+H]+ = 590.3, $^1$H NMR (400 MHz, DMSO-d6) $\delta$ ppm 10.74 (s, 1H), 9.12 (s, 1H), 7.75 (d, $J$ = 8.4 Hz, 1H), 7.21 (s, 1H), 6.47 (d, $J$ = 8.4 Hz, 1H), 5.13 - 4.74 (m, 1H), 3.97 (br t, $J$ = 5.6 Hz, 4H), 3.86 - 3.67 (m, 4H), 3.06 - 2.85 (m, 4H), 2.39 (s, 3H), 2.14 - 1.85 (m, 4H), 1.32 - 1.19 (m, 4H), 0.24 (s, 4H)

**Examples 67 to 68** were prepared with reference to the synthetic route of **Example 66:**

**Example 67:**

**N-(4-{6-azaspiro[2.5]octan-6-yl}-5-{4-[2-(4,4-difluoropiperidin-1-yl)-6-methylpyr imidin-4-yl]-1H-1,2,3-triazol-1-yl}pyridin-2-yl)-2-hydroxyethane-1-sulfonamide**

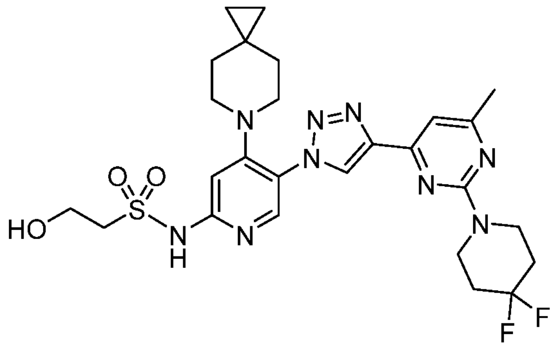

LCMS (ESI): [M+H]+ = 590.3. $^1$H NMR (400 MHz, DMSO-d6) δ ppm 10.72 (s, 1H), 9.11 (s, 1H), 8.20 (s, 1H), 7.22 (s, 1H), 6.66 (s, 1H), 4.97 (s, 1H), 3.97 (br t, *J* = 5.2 Hz, 4H), 3.84 - 3.75 (m, 2H), 3.63 (br d, *J* = 2.8 Hz, 2H), 2.83 - 2.71 (m, 4H), 2.39 (s, 3H), 2.06 - 1.91 (m, 4H), 1.29 - 1.22 (m, 4H), 0.24 (s, 4H)

**Example 68:**

**N-(4-{6-azaspiro[2.5]octan-6-yl}-5-{4-[2-(4,4-difluoropiperidin-1-yl)-6-methylpyr idin-4-yl]-1H-1,2,3-triazol-1-yl} pyridin-2-yl)-2-hydroxyethane-1-sulfonamide**

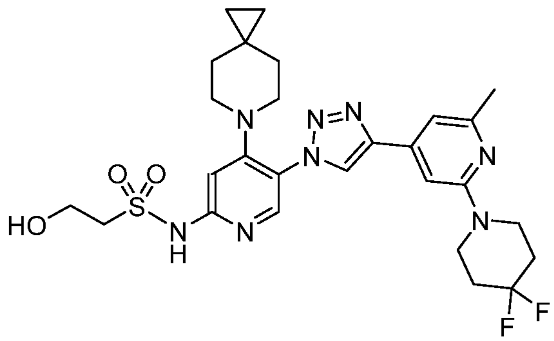

LCMS (ESI): [M+H]+ = 589.5, $^1$H NMR (400 MHz, DMSO-d6) δ ppm 10.77 (s, 1H), 9.10 (s, 1H), 8.15 (s, 1H), 7.24 (s, 1H), 7.12 (s, 1H), 6.67 (s, 1H), 4.98 (s, 1H), 3.83 - 3.78 (m, 2H), 3.78 - 3.71 (m, 4H), 3.63 (br d, J = 5.2 Hz, 2H), 2.84 - 2.72 (m, 4H), 2.39 (s, 3H), 2.12 - 1.92 (m, 4H), 1.32 - 1.20 (m, 4H), 0.25 (s, 4H)

**Example 69:**

**N-(3-{6-azaspiro[2.5]octan-6-yl}-4-{4-[2-(4,4-difluoropiperidin-1-yl)-6-methylpyr imidin-4-yl]-5-methyl-1H-1,2,3-triazol-1-yl}phenyl)-2-hydroxyethane-1-sulfonam ide**

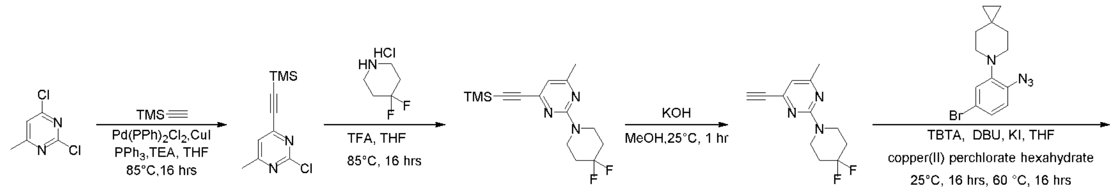


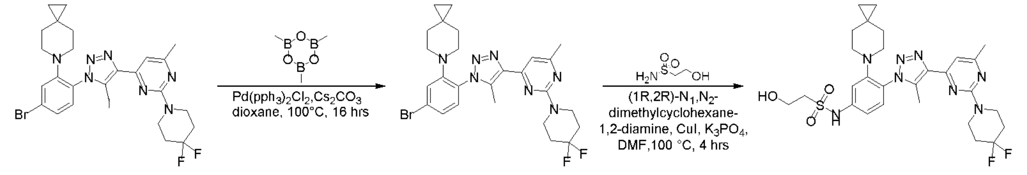


Step 1: To a solution of 2,4-dichloro-6-methylpyrimidine (25.0 g, 153 mmol), ethynyltrimethylsilane (15.1 g, 153 mmol), cuprous iodide (0.30 g, 1.53 mmol) and triphenylphosphine (0.40 g, 1.53 mmol) in triethylamine (39.6 g, 383 mmol) and THF (500 mL), bis(triphenylphosphine)palladium(II) dichloride (1.09 g, 1.53 mmol) was added. The reaction solution was replaced with nitrogen for 3 times. The mixture was heated to 85°C and stirred for 16 h. The reaction solution was filtered and the filtrate was used directly in the next step.

Step 2: To the filtrate of 2-chloro-4-methyl-6-[2-(trimethylsilyl)ethynyl]pyrimidine (30.0 g, 133 mmol), 4,4-difluoropiperidine hydrochloride (21.0 g, 133 mmol) and triethylamine (41.3 g, 400 mmol) were added. The mixture was heated to 80°C under nitrogen atmosphere and stirred for 16 h. The reaction solution was diluted with water (200 mL) and extracted with ethyl acetate (200 mL×3). The organic phases were combined, dried over anhydrous sodium sulfate, and filtered, andconcentrated under reduced pressure to give the residue. The residue was purified by silica gel flash chromatography (silica gel, 0-5% gradient of ethyl acetate/petroleum ether) to give 2-(4,4-difluoropiperidin-1-yl)-4-methyl-6-[2-(trimethylsilyl)ethynyl]pyrimidine as a yellow oil (21.0 g, 67.8 mmol, yield: 51%). LCMS (ESI): [M+H]+ =310.0

Step 3: To a solution of 2-(4,4-difluoropiperidin-1-yl)-4-methyl-6-[2-(trimethylsilyl)ethynyl]pyrimidine (17.5 g, 56.5 mmol) in methanol (200 mL), potassium hydroxide (6.35 g, 113 mmol) was added. The mixture was stirred at 25°C for 1 h. The reaction solution was diluted with water (200 mL) and extracted with ethyl acetate (200 mL×3). The organic phases were combined, dried over anhydrous sodium sulfate, and filtered, and concentrated under reduced pressure to give the residue. The residue was purified by silica gel flash chromatography (silica gel, 0-15% gradient of ethyl acetate/petroleum ether) to give 2-(4,4-difluoropiperidin-1-yl)-4-ethynyl-6-methylpyrimidine as a yellow solid (8.00 g, 1.30 mmol, yield: 60%). LCMS (ESI): [M+H]+ =238.0

Step 4: To a solution of 6-(2-azido-5-bromophenyl)-6-azaspiro[2.5]octane (2.07 g, 6.75 mmol) in THF (5 mL), copper (II) perchlorate hexahydrate (5.00 g, 13.5 mmol), TBTA (0.36 g, 0.67 mmol) and potassium iodide (4.57 g, 26.9 mmol) were added. The mixture was stirred at 25°C for 5 min. 2-(4,4-difluoropiperidin-1-yl)-4-ethynyl-6-methylpyrimidine (1.76 g, 7.42 mmol) and 1,8-diazabiclyclo[5.4.0]undec-7-ene (1.04 g, 6.75 mmol) were added to the reaction solution. The mixture was heated to 60°C and stirred for 16 h. The reaction solution was filtered to give 6-(5-bromo-2-{4-[2-(4,4-difluoropiperidin-1-yl)-6-methylpyrimidin-4-yl]-5-iodo-1H-1,2,3-triazol-1-yl}phenyl)-6-azaspiro[2.5]octane (3.80 g, 5.66 mmol, yield: 84%) as a brown solid. LCMS (ESI): [M+H]+ =672.0; $^1$H NMR (400 MHz, DMSO-$d_6$) δ ppm 7.54 - 7.36 (m, 3H), 7.29 (br s, 1H), 4.14 - 3.93 (m, 4H), 2.74 (br s, 4H), 2.42 - 2.37 (m, 3H), 2.12 - 1.97 (m, 4H), 1.25 - 0.99 (m, 4H), 0.19 (br s, 4H)

Step 5: To a solution of 6-(5-bromo-2-{4-[2-(4,4-difluoropiperidin-1-yl)-6-methylpyrimidin-4-yl]-5-iodo-1H-1,2,3-triazol-1-yl}phenyl)-6-azaspiro[2.5]octane (500.00 mg, 0.75 mmol), trimethyl-1,3,5,2,4,6-trimethylboroxine (28.09 mg, 0.22 mmol) and caesium carbonate (729.08 mg, 2.24 mmol) in THF (20.0 mL), bis(triphenylphosphine)palladium(II) dichloride (52.88 mg, 0.07 mmol) was added. The reaction solution was replaced with nitrogen for 3 times. The mixture was heated to 100°C and stirred for 16 h. The reaction solution was diluted with water (5 mL) and extracted with ethyl acetate (5 mL×3). The organic phases were combined, dried over anhydrous sodium sulfate, and filtered, and concentrated under reduced pressure to give the residue. The residue was purified by silica gel flash chromatography (silica gel, 0-8% gradient of ethyl acetate/petroleum ether) to give 6-(5-bromo-2-{4-[2-(4,4-difluoropiperidin-1-yl)-6-methylpyrimidin-4-yl]-5-methyl-1 H-1,2,3-triazol-1-yl}phenyl)-6-azaspiro[2.5]octane as a white solid (180 mg, 0.32 mmol, yield: 43%). LCMS (ESI): $[M+H]^+$ = 560.0.

Step 6: Cuprous iodide (27.8 mg, 0.14 mmol) was added to a solution of 2-hydroxyethanesulfonamide (71.7 mg, 0.57 mmol), trans-N,N-dimethyl-1,2-cyclohexanediamine (41.2 mg, 0.29 mmol), potassium phosphate (243 mg, 1.15 mmol) in N,N-dimethylformamide (4.00 mL). The mixture was stirred at 50°C for 5 min. Then, 6-(5-bromo-2-{4-[2-(4,4-difluoropiperidin-1-yl)-6-methylpyrimidin-4-yl]-5-methyl-1 H-1,2,3-triazol-1-yl}phenyl)-6-azaspiro[2.5]octane (160 mg, 0.29 mmol) was added to the reaction mixture at 50°C, and the mixture was heated to 100°C under nitrogen atmosphere and stirred for 4 h. The reaction solution was diluted with water (5 mL) and extracted with ethyl acetate (5 mL×2). The organic phases were combined and washed with liquor ammonia (5 mL × 2), dried over anhydrous magnesium sulfate and filtered. The filtrate was concentrated under reduced pressure to give a residue. The residue was purified by preparative HPLC (C18, 26-66% gradient of water (hydrochloric acid)/acetonitrile) to give N-(3-{6-azaspiro[2.5]octan-6-yl}-4-{4-[2-(4,4-difluoropiperidin-1-yl)-6-methylpyrim idin-4-yl]-5-methyl-1H-1,2,3-triazol-1-yl}phenyl)-2-hydroxyethane-1-sulfonamide as a white solid (17.0 mg, 0.03 mmol, yield: 10%). LCMS (ESI): $[M+H]^+$ =603.3; $^1$H NMR (400 MHz, DMSO-$d_6$) δ ppm 10.17 (s, 1H), 7.36 (d, $J$= 1.4 Hz, 2H), 7.13 (d, $J$ = 2.0 Hz, 1H), 7.04 (dd, $J$ = 2.0, 8.4 Hz, 1H), 4.02 - 3.92 (m, 4H), 3.79 (t, $J$ = 6.4 Hz, 2H), 3.37 (t, $J$ = 6.4 Hz, 2H), 2.77 - 2.61 (m, 4H), 2.52 (s, 3H), 2.43 (s, 3H), 2.14 - 1.97 (m, 4H), 1.19 - 1.02 (m, 4H), 0.20 (s, 4H)

**Example 70:**

**N-(4-(4-(6-(4,4-difluoropiperidin-1-yl)-5-(2-hydroxypropan-2-yl)pyridin-2-yl)-1H -1,2,3-triazol-1-yl)-3-(6-azaspiro [2.5]octan-6-yl)phenyl)-2-hydroxyethanesulfona mide**

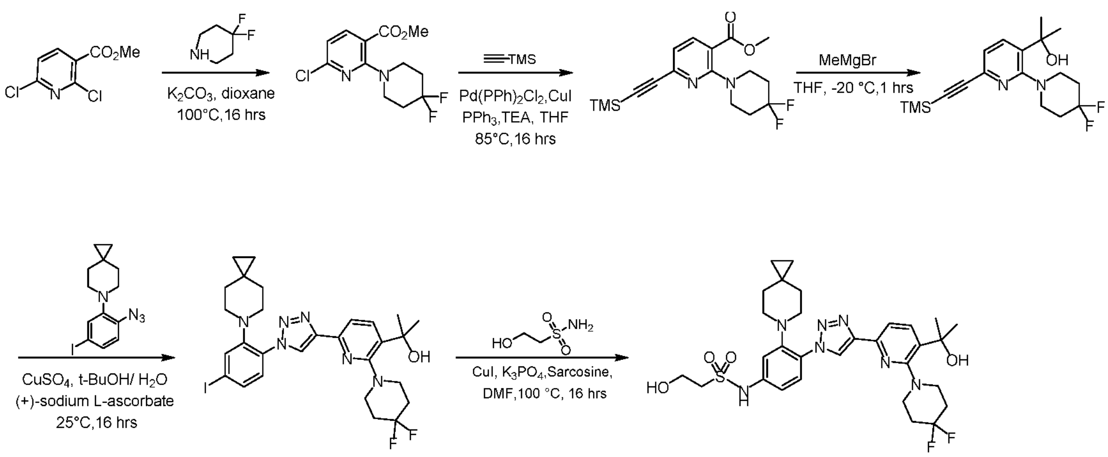


Step 1: To a solution of methyl-2,6-dichloronicotyl ester (10.0 g, 48.5 mmol) in dioxane (100 mL), HCl in 4,4-difluoropiperidine (11.5 g, 72.8 mmol) and potassium carbonate (20.5 g, 146 mmol) were added. The mixture was heated to 100°C and stirred for 16 h. The reaction solution was diluted with water (200 mL) and extracted with ethyl acetate (200 mL×3). The organic phases were combined, dried over anhydrous sodium sulfate, and filtered, and concentrated under reduced pressure to give the residue. The residue was purified by silica gel flash chromatography (silica gel, 0-10% gradient of ethyl acetate/petroleum ether) to give 6-chloro-2-(4,4-difluoropiperidin-1-yl)nicotyl ester as a yellow oil (11.0 g, 37.8 mmol, yield: 78%). LCMS (ESI): $[M+H]^+$ = 327.0.

Step 2: To a solution of methyl-6-chloro-2-(4,4-difluoropiperidin-1-yl) nicotyl ester (5.00 g, 17.2 mmol), ethynyltrimethylsilane (1.86 g, 18.9 mmol), cuprous iodide (0.33 g, 1.72 mmol) and triphenylphosphine (0.45 g, 1.72 mmol) in triethylamine (30.0 ml) and THF (20.0 mL), bis(triphenylphosphine)palladium(II) dichloride (1.86 g, 18.9 mmol) was added. The reaction solution was replaced with nitrogen for 3 times. The mixture was heated to 85°C and stirred for 16 h. The reaction solution was diluted with water (50 mL) and extracted with ethyl acetate (50 mL x 3). The organic phases were combined, dried over anhydrous sodium sulfate, and filtered, and concentrated under reduced pressure to give the residue. The residue was purified by silica gel flash chromatography (silica gel, 0-10% gradient of ethyl acetate/petroleum ether) to give methyl 2-(4,4-difluoropiperidin-1-yl)-6-((trimethylsilyl)ethynyl)nicotylate as a yellow oil (4.30 g, 15.3 mmol, yield: 89%). LCMS (ESI): $[M+H]^+$ = 353.1.

Step 3: To a solution of methyl-2-(4,4-difluoropiperidin-1-yl)-6-((trimethylsilyl)ethynyl)nicotine ester (1.00 g, 3.57 mmol) in THF (20.0 mL), methylmagnesium bromide (3.57 mL, 10.7 mmol) was added. The reaction solution was replaced with nitrogen for 3 times. The mixture was stirred at -20°C for 1 h. The reaction solution was diluted with water (20 mL) and extracted with ethyl acetate (30 mL×3). The organic phases were combined, dried over anhydrous sodium sulfate, and filtered, and concentrated under reduced pressure to give the residue. The residue was purified by silica gel flash chromatography (silica gel, 0-5% gradient of ethyl acetate/petroleum ether) to give (2-(4,4-difluoropiperidin-1-yl)-6-((trimethylsilyl)ethynyl)pyridin-3-yl)propan-2-ol as a yellow solid (600 mg, 2.13 mmol, yield: 60%).

LCMS (ESI): [M+H]+ =353.1

Step 4: Copper sulfate (225 mg, 1.41 mmol) and sodium ascorbate (279 mg, 1.41 mmol) were added to a solution of 6-(2-azido-5-iodophenyl)-6-azaspiro[2.5]octane (500 mg, 1.41 mmol), 2-(2-(4,4-difluoropiperidin-1-yl)-6-((trimethylsilyl)ethynyl)pyridin-3-yl)propan-2-ol (498 mg, 1.41 mmol) in tert-butanol (5 mL), THF (5 mL) and water (5 mL). The mixture was stirred at 25°C for 16 h. The reaction solution was diluted with water (20 mL) and extracted with ethyl acetate (20 mL×3). The organic phases were combined, dried over anhydrous sodium sulfate, and filtered, andconcentrated under reduced pressure to give the residue. The residue was purified by flash column chromatography (silica gel, 0-10% gradient of ethyl acetate/petroleum ether) to give 2-(2-(4,4-difluoropiperidin-1-yl)-6-(1-(4-iodo-2-(6-azaspiro[2.5]octan-6-yl)phenyl)-1 H-1,2,3-triazol-4-yl)pyridin-3-yl)propan-2-ol as a yellow solid (800 mg, 1.24 mmol, yield: 89%). LCMS (ESI): [M+H]+ =635.1

Step 5: To a solution of 2-hydroxyethanesulfonamide (118 mg, 0.95 mmol), sarcosine (43.0 mg, 0.47 mmol), and potassium phosphate (410 mg, 1.89 mmol) in N,N-dimethylformamide (6 mL) was added cuprous iodide (91.9 mg, 0.47 mmol). The mixture was stirred at 50°C for 5 min. 2-(2-(4,4-difluoropiperidin-1-yl)-6-(1-(4-iodo-2-(6-azaspiro[2.5]octan-6-yl)phenyl)-1 H-1,2,3-triazol-4-yl)pyridin-3-yl)propan-2-ol (300 mg, 0.47 mmol) was added to the reaction solution at 50°C. The mixture was heated to 100°C and stirred for 16 h under nitrogen atmosphere. Water (10 mL) was added to the reaction solution and extracted with ethyl acetate (10 mL × 2). The organic phases were combined, dried over anhydrous sodium sulfate, and filtered, andconcentrated under reduced pressure to give the residue. The residue was purified by preparative HPLC (C18, 32-72% gradient of water (formic acid)/acetonitrile) to give N-(4-(4-(6-(4,4-difluoropiperidin-1-yl)-5-(2-hydroxypropan-2-yl)pyridin-2-yl)-1H-1,2, 3-triazol-1-yl)-3-(6-azaspiro[2.5]octan-6-yl)phenyl)-2-hydroxyethanesulfonamide as a white solid (133 mg, 0.48 mmol, yield: 45%). LCMS (ESI): [M+H]+ = 632.3. $^1$H NMR (400 MHz, DMSO-d6) δ ppm 10.05 (br d, *J* = 1.2 Hz, 1H), 9.13 (s, 1H), 8.14 (d, *J= 8.0* Hz, 1H), 7.89 (d, *J*= 8.0 Hz, 1H), 7.63 (d, *J*= 8.8 Hz, 1H), 7.13 (d, *J*= 2.0 Hz, 1H), 7.04 (dd, *J* = 2.4, 8.6 Hz, 1H), 6.05 (s, 1H), 4.98 (br s, 1H), 3.78 (br t, *J*= 6.4 Hz, 2H), 3.37 - 3.34 (m, 2H), 3.17 - 3.06 (m, 4H), 2.74 - 2.61 (m, 4H), 2.23 - 2.07 (m, 4H), 1.58 (s, 6H), 1.43 - 1.30 (m, 4H), 0.25 (s, 4H)

Example 71:

N-(4-(4-(2-(4,4-difluoropiperidin-1-yl)-6-(2-hydroxypropan-2-yl)pyrimidin-4-yl)-**1H-1,2,3-triazol-1-yl)-3-(6-azaspiro [2.5]octan-6-yl)phenyl)-2-hydroxyethanesulfo** namide

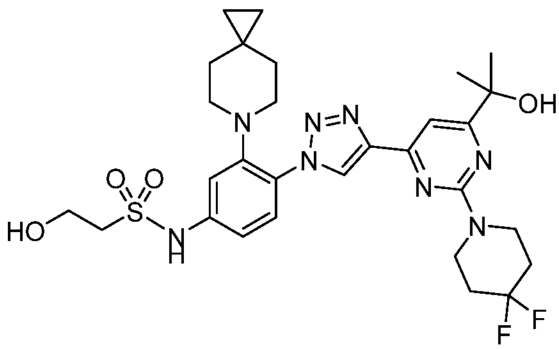

LCMS (ESI): $[M+H]^+$ = 633.5. $^1$H NMR (400 MHz, DMSO-$d_6$) δ ppm 10.05 (s, 1H), 9.24 (s, 1H), 7.69 - 7.50 (m, 2H), 7.13 (d, *J* = 2.4 Hz, 1H), 7.03 (dd, *J* = 2.4, 8.4 Hz, 1H), 5.36 (s, 1H), 5.14 - 4.82 (m, 1H), 4.05 - 3.92 (m, 4H), 3.83 - 3.72 (m, 2H), 3.38 - 3.33 (m, 2H), 2.71 - 2.64 (m, 4H), 2.07 - 1.94 (m, 4H), 1.44 (s, 6H), 1.35 - 1.24 (m, 4H), 0.25 (s, 4H)

**Example 72:**

**N-(4-(4-(8-(4,4-difluoropiperidin-1-yl)quinolin-6-yl)-1H-1,2,3-triazol-1-yl)-3-(6-az aspiro[2.5]octan-6-yl)phenyl)-2-hydroxyethane-1-sulfonamide**

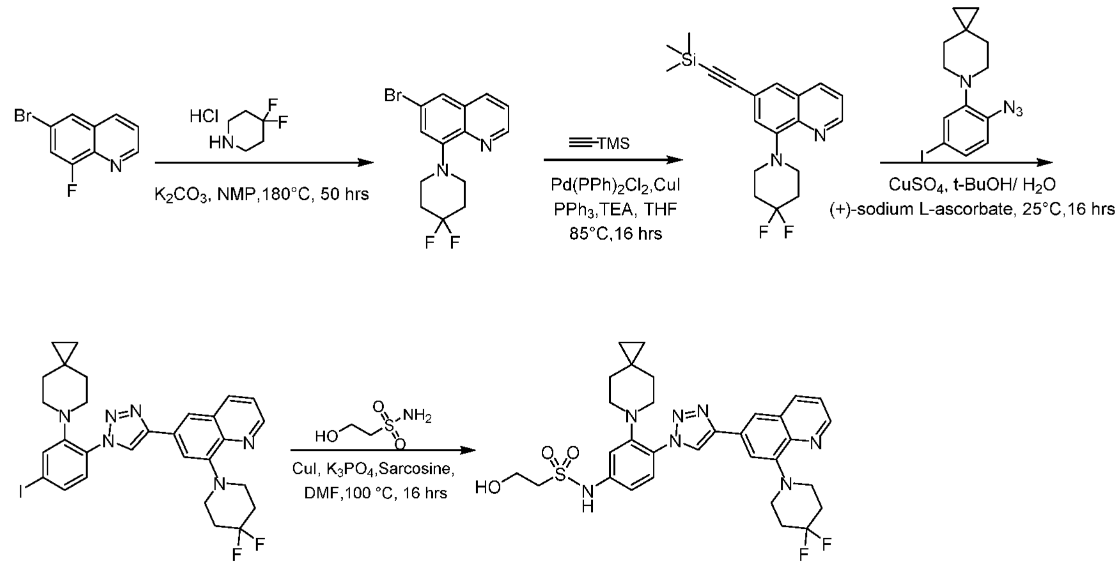


Step 1: HCl in 4,4-difluoropiperidine (5.22 g, 33.2 mmol) and potassium carbonate (9.36 g, 66.4 mmol) were added to a solution of 6-bromo-8-fluoroquinoline (5.00 g, 22.1 mmol) in 1-methyl-2-pyrrolidone (50.0 mL). The mixture was heated to 180°C and stirred for 50 h. The reaction solution was diluted with water (20 mL) and extracted with ethyl acetate (20 mL×3). The organic phases were combined, dried over anhydrous sodium sulfate, and filtered, and concentrated under reduced pressure to give the residue. The residue was purified by silica gel flash chromatography (silica gel, 0-1% gradient of ethyl acetate/petroleum ether) to give 6-bromo-8-(4,4-difluoropiperidin-1-yl)quinoline as a yellow solid (4.30 g, 13 mmol, yield: 59.4%). LCMS (ESI): $[M+H]^+$ = 327.0.

Step 2: To a solution of 6-bromo-8-(4,4-difluoropiperidin-1-yl)quinoline (4.00 g, 12.2 mmol), ethynyltrimethylsilane (1.32 g, 13.4 mmol), cuprous iodide (0.24 g, 1.22 mmol) and triphenylphosphine (0.32 g, 1.22 mmol) in triethylamine (30.0 mL) and THF (20.0 mL) was added bis(triphenylphosphine)palladium(II) dichloride (0.87 g, 1.22 mmol) and replaced with nitrogen 3 times. The mixture was heated to 85° and stirred for 16 h. The reaction solution was diluted with water (50 mL) and extracted with ethyl acetate (50 mL×3). The organic phases were combined, dried over anhydrous sodium sulfate, and filtered, and concentrated under reduced pressure to give the residue. The residue was purified by silica gel flash chromatography (silica gel, 0-1% gradient of ethyl acetate/petroleum ether) to give 8-(4,4-difluoropiperidin-1-yl)-6-((trimethylsilyl)ethynyl)quinoline as a white solid (2.5 g, 9.15 mmol, yield: 75%). LCMS (ESI): $[M+H]^+$ = 345.1.

Step 3: To a solution of 6-(2-azido-5-iodophenyl)-6-azaspiro[2.5]octane (500 mg, 1.41 mmol), 8-(4,4-difluoropiperidin-1-yl)-6-((trimethylsilyl)ethynyl)quinoline (490 mg, 1.41 mmol) in tert-butanol (5 mL), THF (5 mL) and water (5 mL) was added copper sulfate (225 mg, 1.41 mmol) and sodium ascorbate (279 mg, 1.41 mmol). The mixture was stirred at 25°C for 16 h. The reaction solution was diluted with water (20 mL) and extracted with ethyl acetate (20 mL×3). The organic phases were combined, dried over anhydrous sodium sulfate, and filtered, and concentrated under reduced pressure to give the residue. The residue was purified by flash column chromatography (silica gel, 0-5% gradient of ethyl acetate/petroleum ether) to give 8-(4,4-difluoropiperidin-1-yl)-6-(1-(4-iodo-2-(6-azaspiro[2.5]octan-6-yl)phenyl)-1H-1, 2,3-triazol-4-yl)quinoline (600 mg, 0.95 mmol, yield 68%) as a yellow solid. LCMS (ESI): [M+H]+ =627.0.

Step 4: To a solution of 2-hydroxyethanesulfonamide (120 mg, 0.96 mmol), sarcosine (43.5 mg, 0.48 mmol), and potassium phosphate (415 mg, 1.92 mmol) in N,N-dimethylformamide (6 mL) was added cuprous iodide (93.1 mg, 0.48 mmol). The mixture was stirred at 50°C for 5 min. Then, 8-(4,4-difluoropiperidin-1-yl)-6-(1-(4-iodo-2-(6-azaspiro[2.5]octan-6-yl)phenyl)-1H-1, 2,3-triazol-4-yl)quinoline (300 mg, 0.48 mmol) was added to the reaction mixture at 50°C, and the mixture was heated to 100°C under nitrogen atmosphere and stirred for 16 h. Water (10 mL) was added to the reaction solution and extracted with ethyl acetate (10 mL × 2). The organic phases were combined, dried over anhydrous sodium sulfate, and filtered, and concentrated under reduced pressure to give the residue. The residue was purified by preparative HPLC (C18, 32-72% gradient of water (formic acid)/acetonitrile) to give N-(4-(4-(8-(4,4-difluoropiperidin-1-yl)quinolin-6-yl)-1H-1,2,3-triazol-1-yl)-3-(6-azas piro[2.5]octan-6-yl)phenyl)-2-hydroxyethane-1-sulfonamide (133 mg, 0.48 mmol, yield 45%) as a white solid compound. LCMS (ESI): [M+H]+ = 648.3. $^1H$ NMR (400 MHz, DMSO-d6) δ ppm 10.06 (br s, 1H), 9.22 (s, 1H), 8.88 (br s, 1H), 8.40 (br d, $J$ = 2.4 Hz, 1H), 8.15 (br s, 1H), 7.76 (br s, 1H), 7.66 - 7.44 (m, 2H), 7.14 (br s, 1H), 7.03 (br d, $J$ = 8.8 Hz, 1H), 5.23 - 4.72 (m, 1H), 3.88 - 3.72 (m, 2H), 3.67 - 3.45 (m, 4H), 3.39 - 3.34 (m, 2H), 2.82 - 2.61 (m, 4H), 2.39 - 2.12 (m, 4H), 1.37 - 1.18 (m, 4H), 0.23 (s, 4H)

**Examples 73 to 82** were prepared with reference to the synthetic route of Example **72:**

**Example 73:**

**N-(3-{6-azaspiro [2.5]octan-6-yl}-4-{4-[8-(4,4-difluoropiperidin-1-yl)-7-fluoroquin olin-6-yl]-1H-1,2,3-triazol-1-yl} phenyl)-2-hydroxyethane-1-sulfonamide**

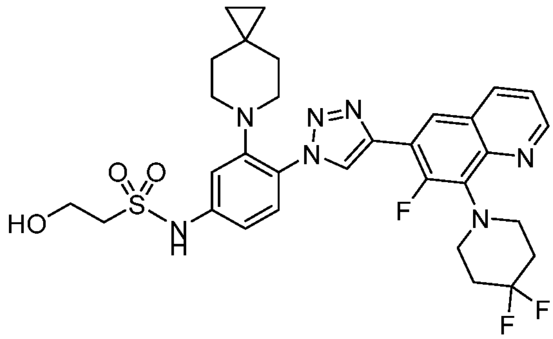

LCMS (ESI): $[M+H]^+$ = 642.6, $^1H$ NMR (400 MHz, DMSO-$d_6$) δ ppm 10.17 (s, 1H), 9.30 (br d, *J* = 4.4 Hz, 2H), 9.07 (d, *J* = 4.0 Hz, 1H), 9.01 (br d, *J* = 6.4 Hz, 1H), 8.14 - 8.05 (m, 1H), 7.55 (d, *J* = 8.4 Hz, 1H), 7.20 (d, *J* = 2.0 Hz, 1H), 7.07 (dd, *J* = 2.0, 8.4 Hz, 1H), 3.79 (br t, *J* = 6.4 Hz, 2H), 3.43 - 3.32 (m, 6H), 2.76 - 2.67 (m, 4H), 2.47 - 2.30 (m, 4H), 1.33 - 1.19 (m, 4H), 0.23 (s, 4H)

**Example 74:**

**N-(3-{6-azaspiro[2.5]octan-6-yl}-4-{4-[8-(4,4-difluoropiperidin-1-yl)-2-methylqui nolin-6-yl]-1H-1,2,3-triazol-1-yl} phenyl)-2-hydroxyethane-1-sulfonamide**

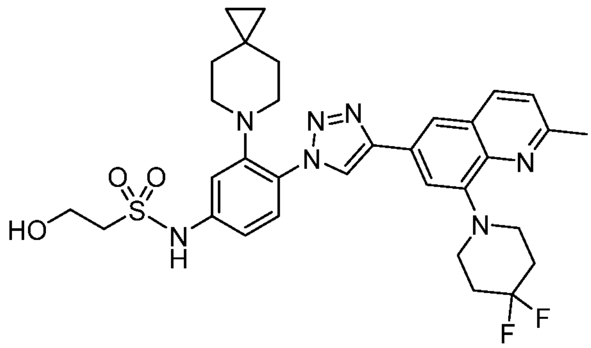

LCMS (ESI): $[M+H]^+$ = 638.3, $^1H$ NMR (400 MHz, DMSO-$d_6$) δ ppm 10.26 - 9.83 (m, 1H), 9.16 (s, 1H), 8.26 (d, *J* = 8.4 Hz, 1H), 8.06 (d, *J* = 1.2 Hz, 1H), 7.67 (d, *J* = 1.6 Hz, 1H), 7.51 (d, *J* = 8.4 Hz, 1H), 7.43 (d, *J* = 8.4 Hz, 1H), 7.14 (d, *J* = 2.0 Hz, 1H), 7.03 (dd, *J* = 2.4, 8.8 Hz, 1H), 5.00 (br s, 1H), 3.79 (br t, *J* = 6.0 Hz, 2H), 3.62 - 3.50 (m, 4H), 3.40 - 3.35 (m, 2H), 2.74 - 2.65 (m, 7H), 2.32 - 2.19 (m, 4H), 1.35- 1.20 (m, 4H), 0.22 (s, 4H)

**Example 75:**

**N-(3-{6-azaspiro[2.5]octan-6-yl}-4-{4-[8-(4,4-difluoropiperidin-l-yl)-7-fluoro-2-m ethylquinolin-6-yl]-1H-1,2,3-triazol-1-yl}phenyl)-2-hydroxyethane-1-sulfonamide**

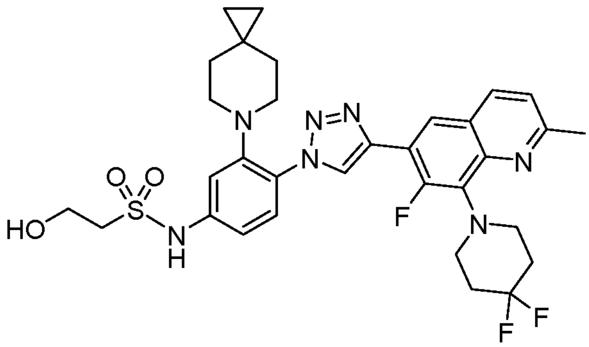

LCMS (ESI): [M+H]+ = 656.3, $^{1}$H NMR (400 MHz, DMSO-$d_6$) δ ppm 10.68 - 9.47 (m, 1H), 8.87 (d, *J* = 4.4 Hz, 1H), 8.38 (dd, *J* = 3.2, 8.0 Hz, 2H), 7.61 - 7.38 (m, 2H), 7.15 (d, *J* = 1.6 Hz, 1H), 7.04 (dd, *J* = 2.0, 8.8 Hz, 1H), 5.30 - 4.65 (m, 1H), 3.79 (t, *J* = 6.4 Hz, 2H), 3.64 - 3.56 (m, 4H), 3.39 - 3.36 (m, 2H), 2.67 (br s, 7H), 2.28 - 2.13 (m, 4H), 1.35 - 1.24 (m, 4H), 0.24 (s, 4H)

**Example 76:**

**N-(4-(4-(8-(4,4-difluoropiperidin-1-yl)cinnolin-6-yl)-1H-1,2,3-triazol-1-yl)-3-(6-az aspiro[2.5]octan-6-yl)phenyl)-2-hydroxyethane-1-sulfonamide**

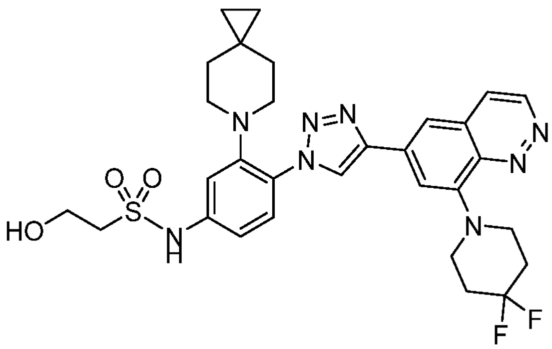

LCMS (ESI): [M+H]$^{+}$ = 625.2, $^{1}$H NMR (400 MHz, DMSO-$d_6$) δ ppm 10.39 - 9.65 (m, 1H), 9.45 - 9.24 (m, 2H), 8.18 (d, *J* = 6.0 Hz, 1H), 8.13 (d, *J* = 1.2 Hz, 1H), 7.79 (d, *J* = 1.2 Hz, 1H), 7.52 (d, *J* = 8.8 Hz, 1H), 7.15 (d, *J* = 2.0 Hz, 1H), 7.04 (dd, *J* = 2.4, 8.4 Hz, 1H), 5.45 - 4.52 (m, 1H), 3.79 (t, *J* = 6.4 Hz, 2H), 3.74 (br d, *J* = 4.4 Hz, 4H), 3.35 (t, *J* = 6.4 Hz, 2H), 2.77 - 2.66 (m, 4H), 2.36 - 2.21 (m, 4H), 1.32 - 1.2 (br s, 4H), 0.22 (s, 4H)

**Example 77:**

**N-(4-(4-(3-amino-8-(4,4-difluoropiperidin-1-yl)cinnolin-6-yl)-1H-1,2,3-triazol-1-y l)-3-(6-azaspiro[2.5]octan-6-yl) phenyl)-2-hydroxyethane-1-sulfonamide**

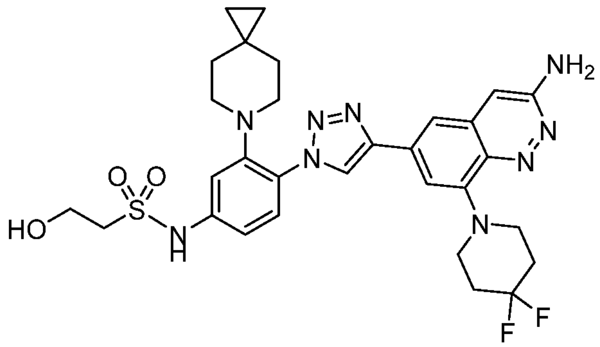

LCMS (ESI): $[M+H]^+$ =640.3, $^1$H NMR (400 MHz, DMSO-d6) $\delta$ 10.14 - 9.53 (m, 1H), 9.00 (s, 1H), 7.52 (br s, 1H), 7.28 (br d, $J$ = 8.4 Hz, 1H), 7.09 (br s, 1H), 6.91 (br s, 1H), 6.87 - 6.61 (m, 2H), 6.36 - 6.16 (m, 2H), 4.86 - 4.64 (m, 1H), 3.67 - 3.52 (m, 2H), 3.50 - 3.30 (m, 4H), 3.11 (br s, 2H), 2.53 - 2.42 (m, 4H), 2.05 - 1.90 (m, 4H), 1.05 (br s, 4H), 0.00 (s, 4H)

**Example 78:**

**N-(3-{6-azaspiro[2.5]octan-6-yl}-4-{4-[3-(4,4-difluoropiperidin-1-yl)-5-methyl-4-n itrophenyl]-1H-1,2,3-triazol-1-yl}phenyl)-2-hydroxyethane-1-sulfonamide**

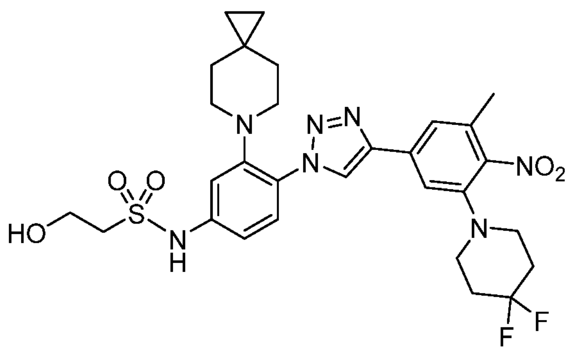

LCMS (ESI): [M+H]+ = 632.3, $^1$H NMR (400 MHz, DMSO-$d_6$) $\delta$ ppm 10.05 (s, 1H), 9.18 (s, 1H), 7.92 - 7.77 (m, 2H), 7.48 (d, $J$ = 8.4 Hz, 1H), 7.13 (d, $J$ = 2.0 Hz, 1H), 7.02 (dd, $J$= *2.0,* 8.4 Hz, 1H), 5.19 - 4.75 (m, 1H), 3.78 (t, $J$ = 6.4 Hz, 2H), 3.36 (br s, 2H), 3.11 (br t, $J$ = 5.2 Hz, 4H), 2.74 - 2.61 (m, 4H), 2.31 (s, 3H), 2.11 - 2.00 (m, 4H), 1.32 - 1.19 (m, 4H), 0.24 (s, 4H)

**Example 79:**

**N-(4-{4-[5-amino-6-(4,4-difluoropiperidin-1-yl)pyrazin-2-yl]-1H-1,2,3-triazol-1-yl} -3-{6-azaspiro[2.5]octan-6-yl} phenyl)-2-hydroxyethane-1-sulfonamide**

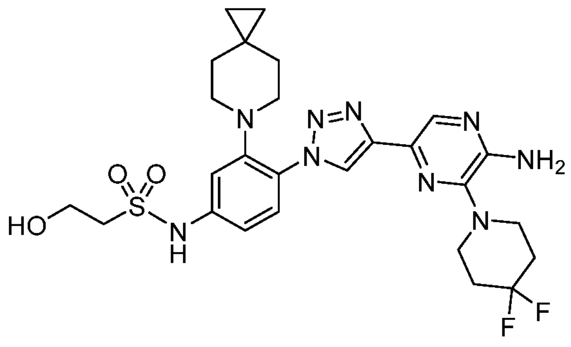

LCMS (ESI): $[M+H]^+$ =590.3, $^1$H NMR (400 MHz, DMSO-d6) δ 10.20 - 9.84 (m, 1H), 8.87 (s, 1H), 8.60 - 8.09 (m, 1H), 7.58 (br d, *J* = 8.4 Hz, 1H), 7.12 (br s, 1H), 7.03 (br d, *J*= 8.4 Hz, 1H), 6.36 (br s, 2H), 5.11 - 4.84 (m, 1H), 3.78 (br t, *J*= 5.6 Hz, 2H), 3.33 - 3.22 (m, 6H), 2.68 (br t, *J* = 4.4 Hz, 4H), 2.27 - 2.13 (m, 4H), 1.34 (br s, 4H), 0.25 (s, 4H)

**Example 80:**

**N-(4-{4-[2-amino-8-(4,4-difluoropiperidin-1-yl)-7-fluoroquinolin-6-yl]-1H-1,2,3-t riazol-1-yl}-3-{6-azaspiro[2.5]octan-6-yl}phenyl)-2-hydroxyethane-1-sulfonamide**

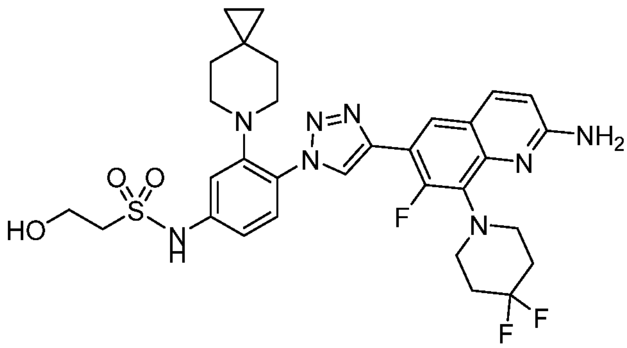

LCMS (ESI): $[M+H]^+$ =657.3; $^1$H NMR (400 MHz, DMSO-d6) δ 13.17 - 11.36 (m, 1H), 10.05 (s, 1H), 8.92 - 8.66 (m, 1H), 8.42 - 7.92 (m, 2H), 7.53 (d, *J* = 8.4 Hz, 1H), 7.14 (d, *J= 2.0* Hz, 1H), 7.03 (dd, *J* = 2.0, 8.8 Hz, 1H), 6.95 - 6.54 (m, 2H), 4.99 (t, *J* = 5.6 Hz, 1H), 3.78 (q, *J* = 6.4 Hz, 2H), 3.52 - 3.41 (m, 4H), 3.31 - 3.24 (m, 2H), 2.73 - 2.66 (m, 4H), 2.39 - 2.07 (m, 4H), 1.28 (br s, 4H), 0.24 (s, 4H)

**Example 81:**

**N-(4-{4-[2-amino-4-(4,4-difluoropiperidin-1-yl)-5-fluoro-1,3-benzothiazol-6-yl]-1 H-1,2,3-triazol-1-yl}-3-{6-azaspiro[2.5]octan-6-yl}phenyl)-2-hydroxyethane-1-sul fonamide**

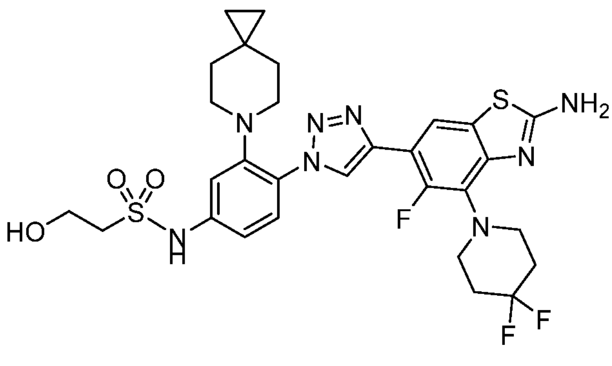

LCMS (ESI): $[M+H]^+$ =663.2; $^1$H NMR (400 MHz, DMSO-d6) δ 10.05 (s, 1H), 8.68 (d, *J* = 3.9 Hz, 1H), 8.16 - 8.06 (m, 1H), 7.77 (br s, 2H), 7.51 (d, *J* = 8.5 Hz, 1H), 7.13 (d, *J* = 2.1 Hz, 1H), 7.02 (dd, *J* = 2.2, 8.6 Hz, 1H), 3.78 (t, *J* = 6.6 Hz, 2H), 3.48 - 3.42 (m, 4H), 3.28 - 3.24 (m, 2H), 2.72 - 2.64 (m, 4H), 2.18 - 2.04 (m, 4H), 1.27 (br s, 4H), 0.24 (s, 4H)

**Example 82:**

**N-(3-{6-azaspiro[2.5]octan-6-yl}-4-{4-[2-(4,4-difluoropiperidin-1-yl)-[3,3'-bipyrid yl]-6-yl]-1H-1,2,3-triazol-1-yl} phenyl)-2-hydroxyethane-1-sulfonamide**

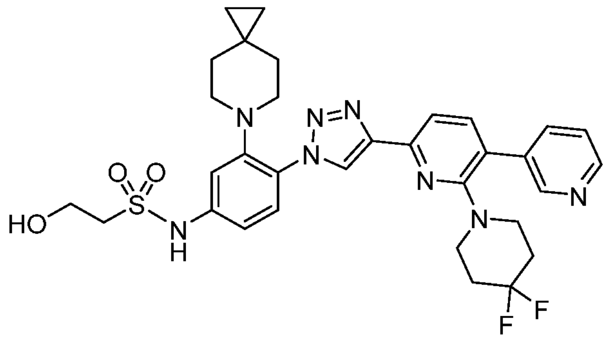

LCMS (ESI): $[M+H]^+$ = 651.3, $^1H$ NMR (400 MHz, DMSO-d6) δ ppm 10.06 (br s, 1H), 9.16 (s, 1H), 8.87 (d, *J= 2.0* Hz, 1H), 8.59 (dd, *J* = 1.6, 4.8 Hz, 1H), 8.13 (td, *J* = 2.0, 8.0 Hz, 1H), 7.81 (d, *J* = 7.6 Hz, 1H), 7.73 (d, *J* = 7.6 Hz, 1H), 7.63 (d, *J* = 8.4 Hz, 1H), 7.52 (dd, *J* = 5.2, 8.0 Hz, 1H), 7.14 (d, *J* = 2.0 Hz, 1H), 7.05 (dd, *J* = 2.0, 8.4 Hz, 1H), 5.00 (t, *J* = 5.6 Hz, 1H), 3.79 (q, *J* = 6.4 Hz, 2H), 3.33 (br s, 2H), 3.28 - 3.22 (m, 4H), 2.74 - 2.65 (m, 4H), 2.09 - 1.87 (m, 4H), 1.37 (br s, 4H), 0.26 (s, 4H)

**Example 83:**

**(2R)-N-(3-{6-azaspiro[2.5]octan-6-yl}-4-{4-[2-(4,4-difluoropiperidin-1-yl)-6-meth ylpyrimidin-4-yl]-1H-1,2,3-triazol-1-yl}phenyl)-1-hydroxypropane-2-sulfonamide**

**Example 84:**

**(2S)-N-(3-{6-azaspiro[2.5]octan-6-yl}-4-{4-[2-(4,4-difluoropiperidin-1-yl)-6-meth ylpyrimidin-4-yl]-1H-1,2,3-triazol-1-yl} phenyl)-1-hydroxypropane-2-sulfonamide**

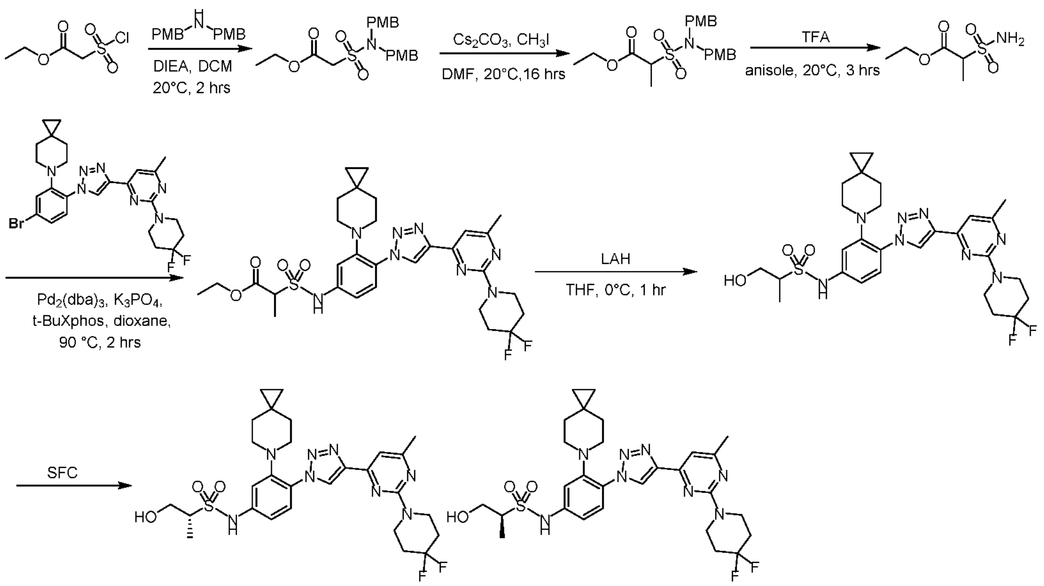


Step 1: Ethyl 2-(chlorosulfonyl)ethyl acetate (3.63 g, 19.4 mmol) was added to a solution of bis(4-methoxybenzyl) amine (5.00 g, 19.4 mmol) and N,N-diisopropylethylamine (5.12 g, 38.9 mmol) in dichloromethane (50 mL). The mixture was stirred at 20°C for 2 h. The reaction solution was extracted with water (100 mL) and dichloromethane (100 mL×3). The combined organic layer was washed with saturated saline (100 mL), dried over anhydrous magnesium sulfate, and filtered. The filtrate was concentrated under reduced pressure to give a residue. The residue was purified by flash column chromatography (silica gel, 0-15% gradient of THF/petroleum ether) to give 2-{bis[(4-methoxyphenyl) methyl]sulfamoyl}ethyl acetate (3.10 g, 7.60 mmol, yield: 39%) as a pale yellow oil. $^1H$ NMR (400 MHz, DMSO-d6) δ ppm 7.14 (d, *J* = 8.4 Hz, 4H), 6.86 (d, J=8.4 Hz, 4H), 4.29 (s, 2H), 4.25 (s, 4H), 3.73 (s, 8H), 1.21 (t, *J* = 7.2 Hz, 3H)

Step 2: Cesium carbonate (3.79 g, 11.4 mmol) was added to a solution of 2-{bis[(4-methoxyphenyl)methyl]sulfamoyl} ethyl acetate (3.10 g, 7.60 mmol) in N,N-dimethylformamide (40 mL), and then methyl iodide (1.32 g, 9.13 mmol) was slowly added dropwise. The mixture was stirred at 20°C for 16 h. The reaction solution was extracted with water (100 mL) and ethyl acetate (100 mL×3). The organic phases were combined and washed with saturated saline (100 mL×2), dried over anhydrous magnesium sulfate, and filtered. The filtrate was concentrated under reduced pressure to give a residue. The residue was purified by flash column chromatography (silica gel, 0-15% gradient of THF/petroleum ether) to give ethyl 2-{bis[(4-methoxyphenyl)methyl]sulfamoyl}propyl ester (2.60 g, 7.60 mmol, yield: 81%) as a colorless oil. $^1$H NMR (400 MHz, DMSO-d6) δ ppm 7.13 (d, *J* = 8.4 Hz, 4H), 6.85 (d, *J* = 8.4 Hz, 4H), 4.36 - 4.23 (m, 3H), 4.23 - 4.11 (m, 4H), 3.73 (s, 6H), 1.46 (d, *J*= 7.2 Hz, 3H), 1.21 (t, *J*= 7.2 Hz, 3H)

Step 3: Anisole (2 mL) was added to a solution of ethyl 2-{bis[(4-methoxyphenyl)methyl]sulfamoyl}propyl ester (1.20 g, 2.85 mmol) in trifluoroacetic acid (10 mL). The mixture was stirred at 20°C for 3 h. The reaction solution was concentrated, adjusted to pH 7 by adding the saturated aqueous sodium bicarbonate solution, and extracted with ethyl acetate (25 mL × 3). The organic phases were combined, dried over anhydrous magnesium sulfate, and filtered. The filtrate was concentrated under reduced pressure to give a residue. The residue was purified by flash column chromatography (silica gel, 0-25% gradient of THF/petroleum ether) to give ethyl 2-sulfamoylpropyl ester (0.42 g, 1.92 mmol, yield: 81%) as a white solid. $^1$H NMR (400 MHz, DMSO-d6) δ ppm 7.13 (s, 2H), 4.14 (q, *J* = 7.2 Hz, 2H), 3.97 (d, *J= 6.8 Hz,* 1H), 1.44 (d, J= 6.8 Hz, 3H), 1.21 (t, *J* = 7.2 Hz, 3H)

Step 4: To a solution of 6-(5-bromo-2-{4-[2-(4,4-difluoropiperidin-1-yl)-6-methylpyrimidin-4-yl]-1H-1,2,3-tri azol-1-yl} phenyl)-6-azaspiro[2.5]octane (800 mg, 1.47 mmol) and ethyl 2-sulfamoylpropyl ester (399 mg, 2.20 mmol) in dioxane (15 mL) was added 2-di-tert-butylphosphine-2',4',6'-triisopropylbiphenyl (125 mg, 0.29 mmol), potassium phosphate (955 mg, 4.41 mmol), and tris(dibenzylideneacetone) dipalladium (137 mg, 0.15 mmol). The reaction solution was replaced with nitrogen three times, heated to 90°C under a nitrogen atmosphere and stirred for 2 h. The reaction solution was cooled to ambient temperature and extracted with water (30 mL) and ethyl acetate (30 mL × 3). The combined organic layer was washed with saturated saline (30 mL), dried over anhydrous magnesium sulfate, and filtered. The filtrate was concentrated under reduced pressure to give a residue. The residue was purified by flash column chromatography (silica gel, 0-33% gradient of ethyl acetate/petroleum ether) to give ethyl 2-[(3-{6-azaspiro [2.5]octan-6-yl}-4-{4-[2-(4,4-difluoropiperidin-1-yl)-6-methylpyrim idin-4-yl]-1H-1,2,3-triazol-1-yl}phenyl)sulfamoyl] propyl ester (830 mg, 1.29 mmol, yield: 88%) as a white solid. LCMS (ESI): [M+H]+ = 645.4; $^1$H NMR (400 MHz, DMSO-d6) δ ppm 10.53 (s, 1H), 9.22 (s, 1H), 7.59 (d, *J* = 8.8 Hz, 1H), 7.21 (s, 1H), 7.15 (s, 1H), 7.05 (br d, *J* = 8.8 Hz, 1H), 4.27 (q, *J*= 6.8 Hz, 1H), 4.14 - 4.06 (m, 2H), 3.98 (br t, *J*= 5.6 Hz, 4H), 2.71 - 2.61 (m, 4H), 2.40 (s, 3H), 2.05 - 1.97 (m, 4H), 1.48 (d, *J* = 6.8 Hz, 3H), 1.28 (br s, 4H), 1.19 - 1.15 (m, 3H), 0.24 (s, 4H)

Step 5: Ethyl 2-[(3-{6-azaspiro[2.5]octan-6-yl}-4-{4-[2-(4,4-difluoropiperidin-1-yl)-6-methylpyrim idin-4-yl]-1H-1,2,3-triazol-1-yl}phenyl)sulfamoyl]propyl ester (590 mg, 0.92 mmol) was added to THF solution (12 mL), and lithium tetrahydridoaluminate (49.0 mg, 1.37 mmol) was added portionwise at 0 °C. The mixture was stirred at 0°C for 1 h. The reaction solution was quenched with water (0.05 mL) and 10% aqueous sodium hydroxide solution (0.10 mL) and filtered. The filtrate was concentrated under reduced pressure to give a residue. The residue was purified by preparative HPLC (C18, 48-88% gradient of water (formic acid)/acetonitrile) and resolved by chiral SFC (IG, system: carbon dioxide/methanol (0.1% liquor ammonia)) to give the white solid compounds (2R)-N-(3-{6-azaspiro[2.5] octan-6-yl}-4-{4-[2-(4,4-difluoropiperidin-1-yl)-6-methyl pyrimidin-4-yl]-1H-1,2,3-triazol-1-yl}phenyl)-1-hydroxypropane-2-sulfonamide (107 mg, 0.17 mmol, yield: 19%) (Example 83) and (2S)-N-(3-{6-azaspiro[2.5]octan-6-yl}-4-{4-[2-(4,4-difluoropiperidin-1-yl)-6-methylp yrimidin-4-yl]-1H-1,2,3-triazol-1-yl}phenyl)-1-hydroxypropane-2-sulfonamide (91.0 mg, 0.15 mmol, yield: 17%) **(Example 84).**

**Example 83:** LCMS (ESI): [M+H]+ = 603.3; $^1$H NMR (400 MHz, DMSO-d6) δ ppm 10.08 (s, 1H), 9.22 (s, 1H), 7.57 (d, *J* = 8.8 Hz, 1H), 7.21 (s, 1H), 7.15 (d, *J* = 2.4 Hz, 1H), 7.05 (dd, *J* = 2.4, 8.4 Hz, 1H), 5.04 (br t, *J* = 5.6 Hz, 1H), 3.98 (br t, *J* = 5.6 Hz, 4H), 3.90 - 3.83 (m, 1H), 3.49 (td, *J* = 6.8, 11.2 Hz, 1H), 3.30 - 3.21 (m, 1H), 2.66 (br t, *J* = 5.2 Hz, 4H), 2.40 (s, 3H), 2.07 - 1.93 (m, 4H), 1.36 - 1.24 (m, 7H), 0.24 (s, 4H)

Example 84: LCMS (ESI): [M+H]+ = 603.3; $^1$H NMR (400 MHz, DMSO-d6) δ ppm 10.03 (s, 1H), 9.22 (s, 1H), 7.56 (d, *J* = 8.8 Hz, 1H), 7.21 (s, 1H), 7.14 (d, *J* = 2.4 Hz, 1H), 7.04 (dd, *J* = 2.4, 8.4 Hz, 1H), 5.09 (s, 1H), 3.98 (br t, *J* = 5.6 Hz, 4H), 3.86 (dd, *J* = 4.4, 11.2 Hz, 1H), 3.49 (dd, *J* = 7.6, 11.2 Hz, 1H), 3.29 - 3.19 (m, 1H), 2.66 (br t, *J* = 5.2 Hz, 4H), 2.40 (s, 3H), 2.07 - 1.92 (m, 4H), 1.37 - 1.21 (m, 7H), 0.24 (s, 4H)

**Examples 85 to 87** were prepared with reference to the synthetic route of Example **84:**

**Example 85:**

**N-(3-{6-azaspiro[2.5]octan-6-yl}-4-{4-[2-(4,4-difluoropiperidin-1-yl)-6-methylpyr imidin-4-yl]-1H-1,2,3-triazol-1-yl}phenyl)-1-(hydroxymethyl)cyclopropane-1-sulf onamide**

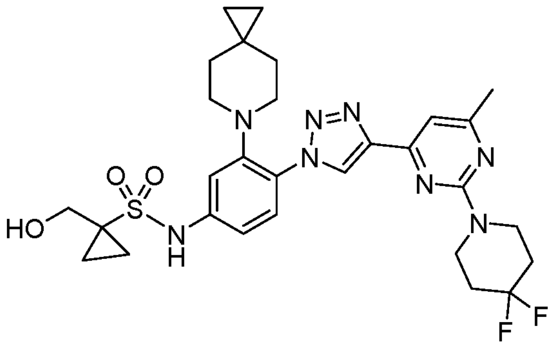

LCMS (ESI): [M+H]+ = 603.3; $^1$H NMR (400 MHz, DMSO-d6) δ ppm 9.21 (s, 1H), 7.50 (d, *J* = 8.8 Hz, 1H), 7.22 (s, 1H), 7.13 (d, *J* = 2.0 Hz, 1H), 6.99 (dd, *J* = 2.0, 8.8 Hz, 1H), 3.98 (br t, *J* = 5.2 Hz, 4H), 3.76 (s, 2H), 2.65 (br t, *J* = 5.2 Hz, 4H), 2.40 (s, 3H), 2.06 - 1.88 (m, 4H), 1.28 (br s, 4H), 1.14 - 1.07 (m, 2H), 0.99 - 0.90 (m, 2H), 0.24 (s, 4H)

**Example 86:**

**N-(3-{6-azaspiro[2.5]octan-6-yl}-4-{4-[2-(4,4-difluoropiperidin-1-yl)-6-methylpyr imidin-4-yl]-1H-1,2,3-triazol-1-yl}phenyl)-1-hydroxy-2-methylpropane-2-sulfona mide**

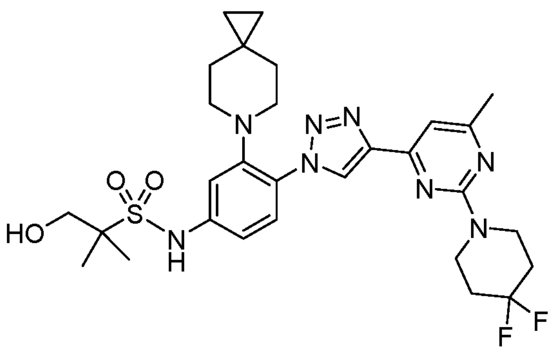

LCMS (ESI): [M+H]+ = 617.5; $^1$H NMR (400 MHz, DMSO-$d_6$) δ ppm 10.05 - 9.69 (m, 1H), 9.23 (s, 1H), 7.54 (d, *J* = 8.4 Hz, 1H), 7.25 (d, *J* = 2.0 Hz, 1H), 7.21 (s, 1H), 7.08 (dd, *J* = 2.4, 8.8 Hz, 1H), 5.21 - 5.03 (m, 1H), 3.98 (br t, *J* = 5.6 Hz, 4H), 3.57 (s, 2H), 2.70 - 2.62 (m, 4H), 2.40 (s, 3H), 2.15 - 1.91 (m, 4H), 1.35 - 1.18 (m, 10H), 0.24 (s, 4H)

**Example 87A:**

**(2R)-N-(3-{6-azaspiro[2.5]octan-6-yl}-4-{4-[8-(4,4-difluoropiperidin-1-yl)-7-fluor oquinolin-6-yl]-1H-1,2,3-triazol-1-yl}phenyl)-1-hydroxypropane-2-sulfonamide**

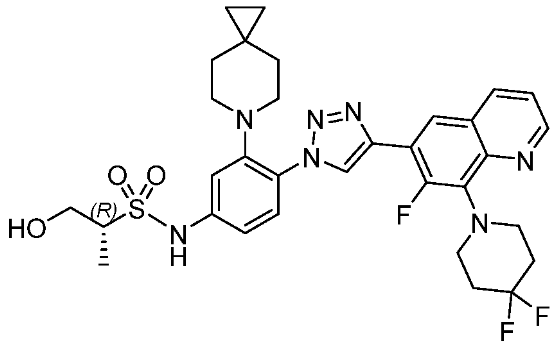

LCMS (ESI): [M+H]+ = 656.1; $^{1}$H NMR (400 MHz, DMSO-$d_6$) δ ppm 10.07 (br d, *J* = 3.6 Hz, 1H), 9.04 - 8.84 (m, 2H), 8.65 - 8.31 (m, 2H), 7.88 - 7.37 (m, 2H), 7.17 (d, *J* = 2.0 Hz, 1H), 7.06 (dd, *J* = 2.0, 8.8 Hz, 1H), 5.04 (br s, 1H), 3.87 (br d, *J* = 7.2 Hz, 1H), 3.59 (br t, *J* = 4.8 Hz, 4H), 3.53 - 3.43 (m, 1H), 3.30 - 3.26 (m, 1H), 2.70 (br t, *J* = 4.8 Hz, 4H), 2.25 - 2.10 (m, 4H), 1.35 - 1.23 (m, 7H), 0.24 (s, 4H)

**Example 87B:**

**(2S)-N-(3-{6-azaspiro[2.5]octan-6-yl}-4-{4-[8-(4,4-difluoropiperidin-1-yl)-7-fluoro quinolin-6-yl]-1H-1,2,3-triazol-1-yl}phenyl)-1-hydroxypropane-2-sulfonamide**

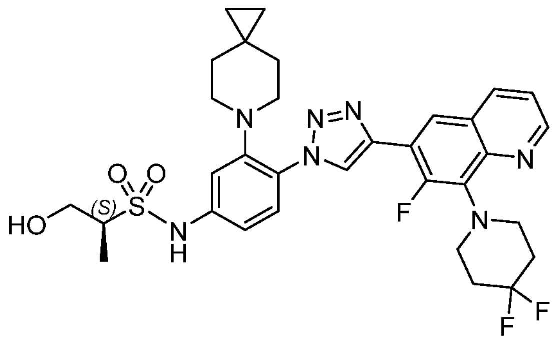

LCMS (ESI): [M+H]+ = 656.1; $^{1}$H NMR (400 MHz, DMSO-$d_6$) δ ppm 10.08 (s, 1H), 9.02 - 8.81 (m, 2H), 8.49 (dd, *J* = 7.6, 18.4 Hz, 2H), 7.66 - 7.41 (m, 2H), 7.17 (d, *J* = 2.0Hz, 1H), 7.06 (dd, *J* = 2.0, 8.8 Hz, 1H), 5.05 (br s, 1H), 3.93 - 3.81 (m, 1H), 3.59 (br s, 4H), 3.53 - 3.44 (m, 1H), 3.29 - 3.24 (m, 1H), 2.70 (br t, *J* = 4.8 Hz, 4H), 2.26 - 2.10 (m, 4H), 1.36 - 1.23 (m, 7H), 0.24 (s, 4H)

**Example 88:**

**N-{3-[4-(difluoromethylidene)piperidin-1-yl]-4-{4-[2-(4,4-difluoropiperidin-1-yl)-6-methylpyrimidin-4-yl]-1H-1,2,3-triazol-1-yl}phenyl}-2-hydroxyethane-1-sulfon amide**

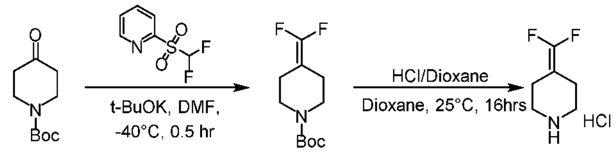


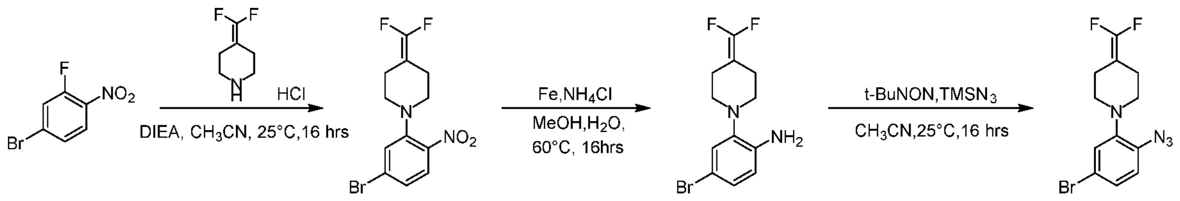


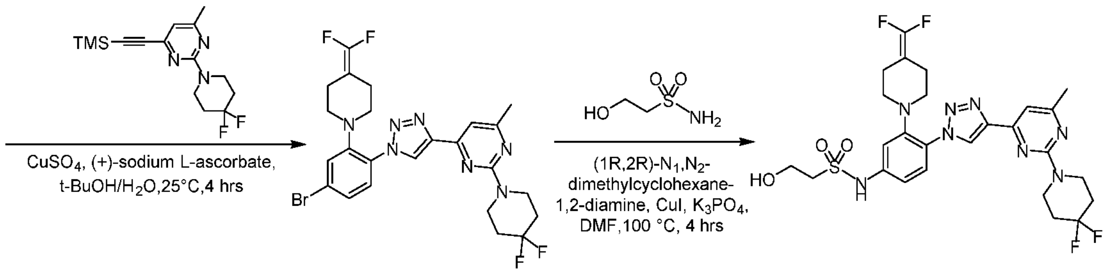


Step 1: A solution of potassium tert-butoxide (4.27 g, 37.6 mmol) in N,N-dimethylformamide (100 mL) was slowly added dropwise to a solution of tert-butyl 4-oxypiperidine-1-carboxylate (5.00 g, 25.1 mmol) and 2-difluoromethanesulfonylpyridine (4.85 g, 25.1 mmol) in N,N-dimethylformamide (100 mL) at -40°C. The mixture was stirred at -40°C for 0.5 h. Then, saturated ammonium chloride (12 mL) and 3 M hydrochloric acid (30 mL) were added to the reaction solution. Then, the solution was slowly heated to ambient temperature, and extracted with ethyl acetate (100 mL × 2), the organic phases were combined, dried over anhydrous sodium sulfate, and filtered, and concentrated under reduced pressure to give the residue. The residue was purified by flash column chromatography (silica gel, 0-8% gradient of ethyl acetate/petroleum ether) to give tert-butyl 4-(difluoromethylidene)piperidin-1-carboxylate (3.50 g, 15.0 mmol, yield: 60%) as a colorless oil. $^1$H NMR (400 MHz, CDCl3) δ ppm 3.42 - 3.28 (m, 4H), 2.08 (br t, J = 5.6 Hz, 4H), 1.46 - 1.32 (m, 9H)

Step 2: Hydrochloric acid/dioxane (35 mL, 4M) was added to a solution of tert-butyl 4-(difluoromethylidene) piperidin-1-carboxylate (3.50 g, 15.0 mmol) in dioxane (35 mL). The mixture was stirred at 25°C for 16 h. The reaction solution was concentrated and concentrated under reduced pressure to give 4-(difluoromethylidene) piperidine hydrochloride (2.50 g, 14.7 mmol, yield: 98.2%) as a white solid. $^1$H NMR (400 MHz, DMSO-$d_6$) δ ppm 9.52 - 9.06 (m, 2H), 3.14 - 3.02 (m, 4H), 2.45 - 2.32 (m, 4H)

Step 3: To a solution of 4-bromo-2-fluoro-1-nitrobenzene (2.70 g, 12.3 mmol) and N,N-diisopropylethylamine (4.86 g, 36.8 mmol) in acetonitrile (54 mL) was added 4-(difluoromethylidene)piperidine hydrochloride (2.08 g, 12.3 mmol). The mixture was stirred at 25°C for 16 h. The reaction solution was concentrated under reduced pressure to give 1-(5-bromo-2-nitrophenyl)-4-(difluoromethylidene)piperidine (3.80 g, 11.4 mmol, yield: 93%) as a yellow solid. LCMS (ESI): $[M+H]^+$ = 332.9; $^1$H NMR (400 MHz, DMSO-$d_6$) δ ppm 7.80 (d, *J* = 8.8 Hz, 1H), 7.48 (d, *J* = 2.0 Hz, 1H), 7.29 (dd, J= 2.0, 8.4 Hz, 1H), 3.06 (t, *J* = 5.6 Hz, 4H), 2.31 - 2.21 (m, 4H)

Step 4: Ammonium chloride (1.80 g, 33.3 mmol) and iron powder (3.13 g, 55.5 mmol) were added to a solution of 1-(5-bromo-2-nitrophenyl)-4-(difluoromethylidene)piperidine (3.70 g, 11.1 mmol) in methanol (80 mL) and water (20 mL). The mixture was heated to 60°C and stirred for 16 h. The reaction solution was filtered with diatomite and the filtrate was extracted with ethyl acetate (100 mL × 3). The organic phases were combined, dried over anhydrous sodium sulfate, and filtered, and concentrated under reduced pressure to give 4-bromo-2-[4-(difluoromethylidene)piperidin-1-yl]aniline (3.35 g, 10.9 mmol, yield: 99%) as a brown solid. LCMS (ESI): $[M+H]^+$ =304.9; $^1$H NMR (400 MHz, DMSO-$d_6$) δ ppm 7.02 - 6.95 (m, 2H), 6.69 (d, *J* = 8.4 Hz, 1H), 5.02 (s, 2H), 2.83 (br t, *J* = 4.8 Hz, 4H), 2.36 (br t, *J* = 5.2 Hz, 4H)

Step 5: Tert-butyl nitrite (1.71 g, 16.3 mmol) was added to a solution of 4-bromo-2-[4-(difluoromethylidene)piperidin-1-yl]aniline (3.30 g, 10.9 mmol) in acetonitrile (60 mL) at 0°C. Then, azidotrimethylsilane (1.88 g, 16.3 mmol) was added to the reaction solution and the mixture was stirred at 25°C for 16 h. The reaction solution was poured into water (100 mL) and extracted with ethyl acetate (100 mL×3). The organic phases were combined, dried over anhydrous sodium sulfate, and filtered, and concentrated under reduced pressure to give the residue. The residue was purified by flash

column chromatography (silica gel, 0-10% gradient of ethyl acetate/petroleum ether) to give 1-(2-azido-5-bromophenyl)-4-(difluoromethylidene)piperidine (3.00 g, 9.15 mmol, yield: 84%) as a yellow solid. LCMS (ESI): $[M+H]^+$ =330.9; $^1H$ NMR (400 MHz, DMSO-$d_6$) δ ppm 7.31 (dd, *J* = 2.0, 8.4 Hz, 1H), 7.24 (d, *J= 2.0* Hz, 1H), 7.16 (d, *J* = 8.4 Hz, 1H), 3.04 (br t, *J* = 5.6 Hz, 4H), 2.35 (br t, *J* = 5.4 Hz, 4H)

Step 6: Copper sulfate (242 mg, 1.52 mmol) and sodium ascorbate (301 mg, 1.51 mmol) were added to a solution of 1-(2-azido-5-bromophenyl)-4-(difluoromethylidene)piperidine (500 mg, 1.52 mmol) and 2-(4,4-difluoropiperidin-1-yl)-4-methyl-6-[2-(trimethylsilyl)ethynyl]pyrimidine (470 mg, 1.52 mmol) in tert-butanol (5 mL) and water (5 mL). The mixture was stirred at 25°C for 16 h. The reaction solution was diluted with water (20 mL) and extracted with ethyl acetate (20 mL×3). The organic phases were combined, dried over anhydrous sodium sulfate, and filtered, and concentrated under reduced pressure to give the residue. The residue was purified by flash column chromatography (silica gel, 0-15% gradient of ethyl acetate/petroleum ether) to give 4-(1-{4-bromo-2-[4-(difluoromethylidene)piperidin-1-yl]phenyl}-1H-1,2,3-triazol-4-yl)-2-(4,4-difluoropiperidin-1-yl)-6-methylpyrimidine (800 mg, 1.41 mmol, yield: 93%) as a yellow solid. LCMS (ESI): $[M+H]^+$ =568.0; $^1H$ NMR (400 MHz, DMSO-$d_6$) δ ppm 8.78 (s, 1H), 7.48 (d, *J* = 8.4 Hz, 1H), 7.31 (dd, *J* = 2.0, 8.4 Hz, 1H), 7.24 (d, *J* = 2.0 Hz, 1H), 7.22 (s, 1H), 4.01 - 3.96 (m, 4H), 2.73 (t, *J* = 5.6 Hz, 4H), 2.40 (s, 3H), 2.13 (br t, *J* = 5.6 Hz, 4H), 2.00 - 1.90 (m, 4H)

Step 7: To a solution of 2-hydroxyethanesulfonamide (177 mg, 1.41 mmol), (1R,2R)-N1,N2-dimethylcyclohexane-1,2-diamine (101 mg, 0.71 mmol), and potassium phosphate (450 mg, 2.12 mmol) in N,N-dimethylformamide (8 mL) was added cuprous iodide (134 mg, 0.71 mmol). The mixture was stirred at 50°C for 5 min. Then, 4-(1-{4-bromo-2-[4-(difluoromethylidene)piperidin-1-yl]phenyl}-1H-1,2,3-triazol-4-yl)-2-(4,4-difluoropiperidin-1-yl)-6-methylpyrimidine (400 mg, 0.71 mmol) was added to the reaction mixture at 50°C, the mixture was heated to 100°C under nitrogen protection, and stirred for 4 h. Water (10 mL) was added to the reaction solution and extracted with ethyl acetate (10 mL × 2). The organic phases were combined, dried over anhydrous sodium sulfate, and filtered, and concentrated under reduced pressure to give the residue. The residue was purified by preparative HPLC (C18, 44-84% gradient of water (formic acid)/acetonitrile) to give N-{3-[4-(difluoromethylidene)piperidin-1-yl]-4-{4-[2-(4,4-difluoropiperidin-1-yl)-6-methylpyrimidin-4-yl]-1H-1,2,3-triazol-1-yl}phenyl}-2-hydroxyethane-1-sulfonamid e (210 mg, 0.35 mmol, yield: 49%) as a white solid compound. LCMS (ESI): $[M+H]^+$ = 611.1; $^1H$ NMR (400 MHz, DMSO-$d_6$) δ ppm 10.10 (br s, 1H), 9.22 (s, 1H), 7.57 (d, *J* = 8.4 Hz, 1H), 7.23 (s, 1H), 7.12 - 7.02 (m, 2H), 4.99 (br t, *J* = 5.6 Hz, 1H), 3.96 (br t, *J* = 5.2 Hz, 4H), 3.82 - 3.74 (m, 2H), 3.38 - 3.35 (m, 2H), 2.69 (br t, *J* = 5.2 Hz, 4H), 2.40 (s, 3H), 2.12 - 2.04 (m, 4H), 2.03 - 1.90 (m, 4H)

**Example 89:**

**N-(4-(1-(2-(4,4-difluoropiperidin-1-yl)-6-methylpyrimidin-4-yl)-1H-1,2,3-triazol-4-yl)-3-(6-azaspiro[2.5]octan-6-yl) phenyl)-2-hydroxyethanesulfonamide**

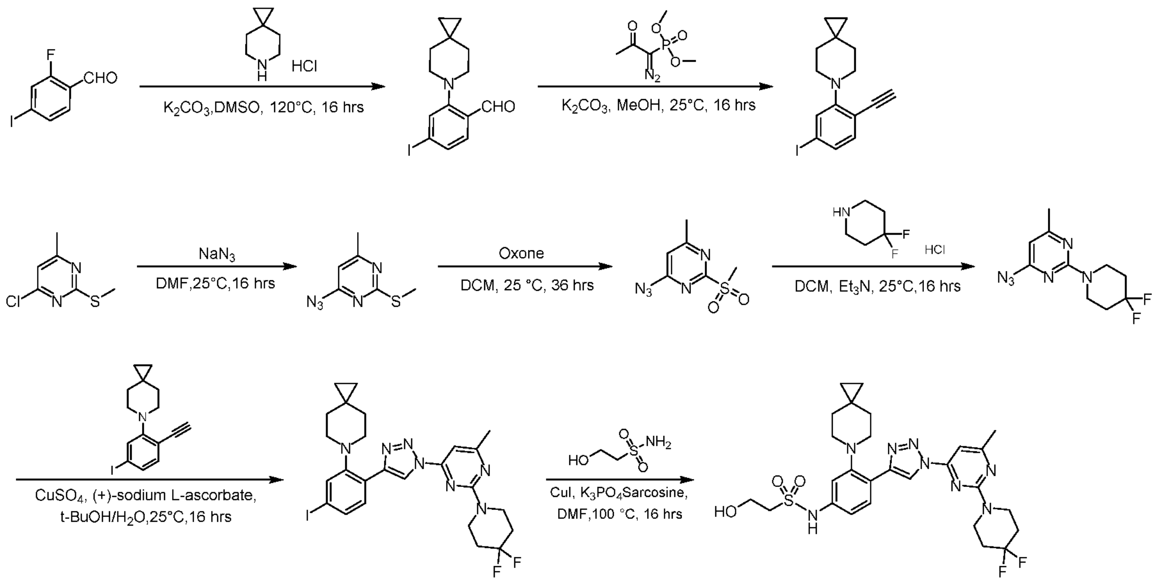


Step 1: To a solution of 2-fluoro-4-iodobenzaldehyde (1.00 g, 4.00 mmol) and potassium carbonate (1.65 g, 12.00

mmol) in DMSO (20 mL) was added 6-azaspiro[2.5]octane hydrochloride ((0.49 g, 4.40 mmol). The mixture was heated to 120°C and stirred for 16 h. The reaction mixture was diluted with water (20 mL) and extracted with ethyl acetate (20 mL×2). The organic phases were combined, dried over anhydrous sodium sulfate, and filtered, and concentrated under reduced pressure to give the residue. The residue was purified by flash column chromatography (silica gel, 0-8% gradient of ethyl acetate/petroleum ether) to give 4-iodo-2-(6-azaspiro[2.5]octan-6-yl)benzaldehyde (1.00 g, 2.93 mmol, yield: 73%) as a yellow solid. LCMS (ESI): $[M+H]^+$ =341.9.

Step 2: Dimethyl (1-diazo-2-oxopropylidene) phosphonate (0.68 g, 3.52 mmol) was added to a solution of 4-iodo-2-(6-azaspiro[2.5]octan-6-yl)benzaldehyde (1.00 g, 2.93 mmol) and potassium carbonate (0.81 g, 5.86 mmol) in methanol (20 mL). The mixture was stirred at 25°C for 16 h. The reaction mixture was diluted with water (20 mL) and extracted with ethyl acetate (20 mL×2). The organic phases were combined, dried over anhydrous sodium sulfate, and filtered, and concentrated under reduced pressure to give the residue. The residue was purified by flash column chromatography (silica gel, 0-5% gradient of ethyl acetate/petroleum ether) to give 6-(2-ethynyl-5-iodophenyl)-6-azaspiro[2.5]octane (0.90 g, 2.67 mmol, yield: 91%) as a yellow solid. LCMS (ESI): $[M+H]^+$ = 338.0.

Step 3: Sodium azide (2.54 g, 39.1 mmol) was added to a solution of 4-chloro-6-methyl-2-(methylthio)pyrimidine (4.50 g, 25.7 mmol) in N,N-dimethylformamide (45 mL). The mixture was stirred at 25°C for 16 h. The reaction solution was diluted with water (100 mL) and extracted with ethyl acetate (100 mL×3). The organic phases were combined, dried over anhydrous sodium sulfate, and filtered, andconcentrated under reduced pressure to give the residue. The residue was purified by flash column chromatography (silica gel, 0-7% gradient of ethyl acetate/petroleum ether) to give 4-azido-6-methyl-2-(methylthio)pyrimidine (3.00 g, 16.5 mmol, yield: 64%) as a yellow solid. LCMS (ESI): $[M+H]^+$ = 181.9.

Steps 4 to 5: Potassium monopersulfate (13.9 g, 82.8 mmol) was added portionwise to a solution of 4-azido-6-methyl-2-(methylthio)pyrimidine (1.50 g, 8.28 mmol) in dichloromethane (45 mL). The mixture was stirred at 25°C for 36 h. The mixture was filtered. To this filtrate were added 4,4-difluoropiperidine hydrochloride (1.66 g, 10.5 mmol) and triethylamine (2.13 g, 21.1 mmol). The mixture was stirred at 25°C for 16 h. The reaction solution was diluted with water (50 mL) and extracted with ethyl acetate (50 mL×3). The organic phases were combined, dried over anhydrous sodium sulfate, and filtered, and concentrated under reduced pressure to give the residue. The residue was purified by flash column chromatography (silica gel, 0-12% gradient of ethyl acetate/petroleum ether) to give 4-azido-2-(4,4-difluoropiperidin-1-yl)-6-methylpyrimidine (1.00 g, 3.93 mmol, yield: 56%) as a yellow solid. LCMS (ESI): $[M+H]^+$ = 254.9.

Step 6: Copper sulfate (360 mg, 2.26 mmol) and sodium ascorbate (448 mg, 2.26 mmol) were added to a solution of 4-azido-2-(4,4-difluoropiperidin-1-yl)-6-methylpyrimidine (575 mg, 2.26 mmol), 6-(2-ethynyl-5-iodophenyl)-6-azaspiro [2.5]octane (762 mg, 2.26 mmol) in tert-butanol (5 mL), THF (5 mL) and water (5 mL). The mixture was stirred at 25°C for 16 h. The reaction solution was diluted with water (10 mL) and extracted with ethyl acetate (10 mL×3). The organic phases were combined, dried over anhydrous sodium sulfate, and filtered, and concentrated under reduced pressure to give the residue. The residue was purified by flash column chromatography (silica gel, 0-12% gradient of ethyl acetate/petroleum ether) to give 6-(2-(1-(2-(4,4-difluoropiperidin-1-yl)-6-methylpyrimidin-4-yl)-1H-1,2,3-triazol-4-yl) -5-iodophenyl)-6-azaspiro[2.5]octane (500 mg, 0.84 mmol, yield: 37%) as a yellow solid. LCMS (ESI): $[M+H]^+$ =592.0.

Step 7: To a solution of 2-hydroxyethanesulfonamide (253 mg, 2.02 mmol), 2-(methylamino)acetic acid (90.3 mg, 1.01 mmol), and potassium phosphate (878 mg, 4.05 mmol) in N,N-dimethylformamide (12 mL) was added cuprous iodide (98.5 mg, 0.50 mmol). The mixture was stirred at 50°C for 5 min. Then, 6-(2-(1-(2-(4,4-difluoropiperidin-1-yl)-6-methylpyrimidin-4-yl)-1H-1,2,3-triazol-4-yl) -5-iodophenyl)-6-azaspiro[2.5]octane (600 mg, 1.01 mmol) was added to the reaction mixture at 50°C, and the mixture was heated to 100°C under nitrogen atmosphere and stirred for 16 h. Water (10 mL) was added to the reaction solution and extracted with ethyl acetate (10 mL × 2). The organic phases were combined, dried over anhydrous sodium sulfate, and filtered, and concentrated under reduced pressure to give the residue. The residue was purified by preparative HPLC (C18, 48-88% gradient of water (formic acid)/acetonitrile) to give N-(4-(1-(2-(4,4-difluoropiperidin-1-yl)-6-methylpyrimidin-4-yl)-1H-1,2,3-triazol-4-yl) -3-(6-azaspiro[2.5]octan-6-yl)phenyl)-2-hydroxyethanesulfonamide (324.6 mg, 0.54 mmol, yield 54%) as a white solid compound. LCMS (ESI): $[M+H]^+$ = 589.5. $^1H$ NMR (400 MHz, DMSO-$d_6$) $\delta$ ppm 9.80 (s, 1H), 9.56 (s, 1H), 8.09 (d, *J* = 8.4 Hz, 1H), 7.29 (s, 1H), 7.18 (d, *J* = 2.0 Hz, 1H), 7.07 (dd, *J* = 2.0, 8.4 Hz, 1H), 4.95 (t, *J* = 5.6 Hz, 1H), 4.01 (br t, *J* = 5.2 Hz, 4H), 3.76 (q, *J* = 6.4 Hz, 2H), 3.32 - 3.28 (m, 2H), 2.84 (br t, *J* = 4.8 Hz, 4H), 2.47 (s, 3H), 2.11 - 2.01 (m, 4H), 1.78 - 1.30 (m, 4H), 0.36 (s, 4H)

**Examples 90 to 91** were prepared with reference to the synthetic route of Example **89:**

**Example 90:**

**N-(3-{6-azaspiro[2.5]octan-6-y=-difluoropiperidin-1-yl)-6-methoxyp yrimidin-4-yl]-1H-1,2,3-triazol-4-yl}phenyl)-2-hydroxyethane-1-sulfonamide**

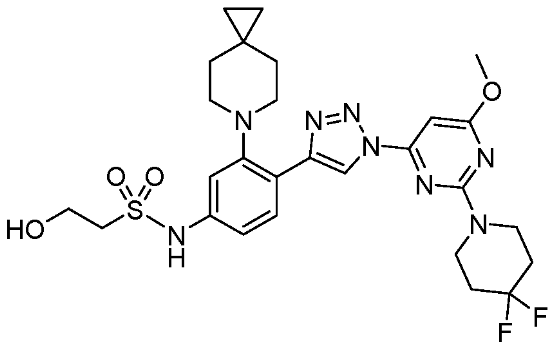

LCMS (ESI): $[M+H]^+$ = 605.1; $^1$H NMR (400 MHz, DMSO-$d_6$) δ ppm 9.89 (s, 1H), 9.43 (s, 1H), 8.08 (d, *J* = 8.4 Hz, 1H), 7.18 (d, *J* = 2.0 Hz, 1H), 7.07 (dd, *J* = 2.0, 8.4 Hz, 1H), 6.72 (s, 1H), 4.95 (s, 1H), 4.01 (br t, *J* = 5.6 Hz, 4H), 3.98 (s, 3H), 3.76 (t, *J* = 6.8 Hz, 2H), 3.31 - 3.26 (m, 2H), 2.85 (br t, *J* = 4.8 Hz, 4H), 2.21 - 2.00 (m, 4H), 1.75 - 1.31 (m, 4H), 0.37 (s, 4H)

**Example 91:**

**N-(3-{6-azaspiro[2.5]octan-6-yl}-4-{2-[2-(4,4-difluoropiperidin-1-yl)-6-methylpyr idin-4-yl]-2H-1,2,3,4-tetrazol-5-yl]phenyl)-2-hydroxyethane-1-sulfonamide**

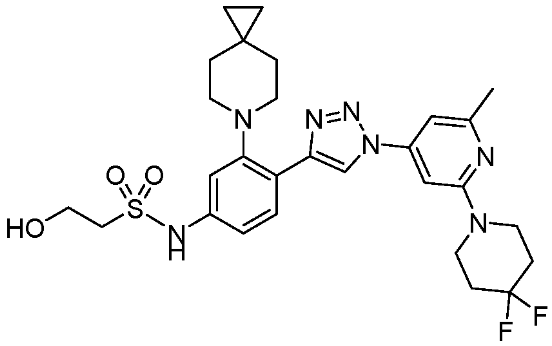

LCMS (ESI): $[M+H]^+$ =588.3, $^1$H NMR (400 MHz, DMSO-d6) δ 9.62 (s, 1H), 8.95 (s, 1H), 7.56 (d, *J* = 8.4 Hz, 1H), 7.01 (s, 1H), 6.94 (s, 1H), 6.88 (d, *J* = 2.0 Hz, 1H), 6.76 (dd, *J* = 2.0, 8.4 Hz, 1H), 5.04 - 4.38 (m, 1H), 3.60 - 3.49 (m, 6H), 3.05 - 3.03 (m, 2H), 2.58 (br t, *J* = 4.8 Hz, 4H), 2.20 (s, 3H), 1.85 - 1.72 (m, 4H), 1.25 - 1.15 (m, 4H), 0.06 (s, 4H)

**Example 92:**

**(2R)-N-(3-{6-azaspiro[2.5]octan-6-yl}-4-{1-[2-(4,4-difluoropiperidin-1-yl)-6-meth ylpyrimidin-4-yl]-1H-1,2,3-triazol-4-yl} phenyl)-1-hydroxypropane-2-sulfonamide**

**Example 93:**

**(2S)-N-(3-{6-azaspiro[2.5]octan-6-yl}-4-{1-[2-(4,4-difluoropiperidin-1-yl)-6-meth ylpyrimidin-4-yl]-1H-1,2,3-triazol-4-yl} phenyl)-1-hydroxypropane-2-sulfonamide**

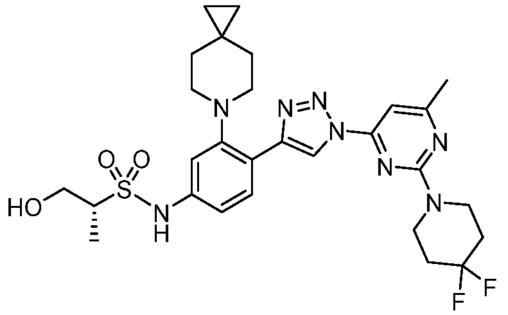

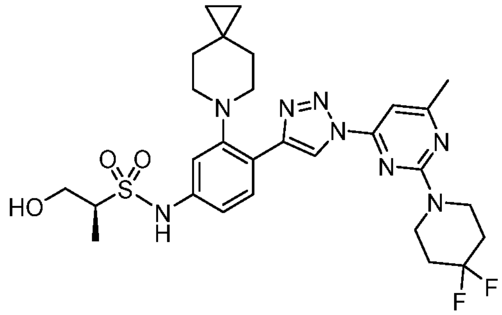

**Example 92:** LCMS (ESI): [M+H]+ = 603.3; $^1$H NMR (400 MHz, DMSO-d6) $\delta$ ppm 9.92 (s, 1H), 9.46 (s, 1H), 8.08 (d, *J* = 8.4 Hz, 1H), 7.30 (s, 1H), 7.20 (d, *J* = 2.0 Hz, 1H), 7.08 (dd, *J* = 2.0, 8.4 Hz, 1H), 4.99 (s, 1H), 4.01 (br t, *J* = 5.6 Hz, 4H), 3.85 (dd, *J*= 4.4, 10.8 Hz, 1H), 3.43 (m, 1H), 3.22 (m, 1H), 2.84 (br t, *J* = 4.8 Hz, 4H), 2.47 (s, 3H), 2.14 - 1.96 (m, 4H), 1.55 (br s, 4H), 1.29 (d, J = 6.8 Hz, 3H), 0.36 (s, 4H)

**Example 93:** LCMS (ESI): [M+H]+ = 603.1; $^1$H NMR (400 MHz, DMSO-d6) $\delta$ ppm 9.91 (s, 1H), 9.46 (s, 1H), 8.08 (d, *J* = 8.4 Hz, 1H), 7.29 (s, 1H), 7.20 (d, *J* = 2.0 Hz, 1H), 7.08 (dd, *J* = 2.0, 8.4 Hz, 1H), 4.99 (s, 1H), 4.01 (br t, *J* = 5.6 Hz, 4H), 3.85 (dd, *J* = 3.6, 10.8 Hz, 1H), 3.45 (br t, *J* = 8.4 Hz, 1H), 3.22 (m, 1H), 2.84 (br t, *J* = 4.8 Hz, 4H), 2.47 (s, 3H), 2.14 - 1.96 (m, 4H), 1.55 (br s, 4H), 1.29 (d, *J* = 6.8 Hz, 3H), 0.36 (s, 4H)

**Example 94:**

**N-(4-(1-(8-(4,4-difluoropiperidin-1-yl)quinolin-6-yl)-1H-1,2,3-triazol-4-yl)-3-(6-az aspiro[2.5]octan-6-yl)phenyl)-2-hydroxyethane-1-sulfonamide**

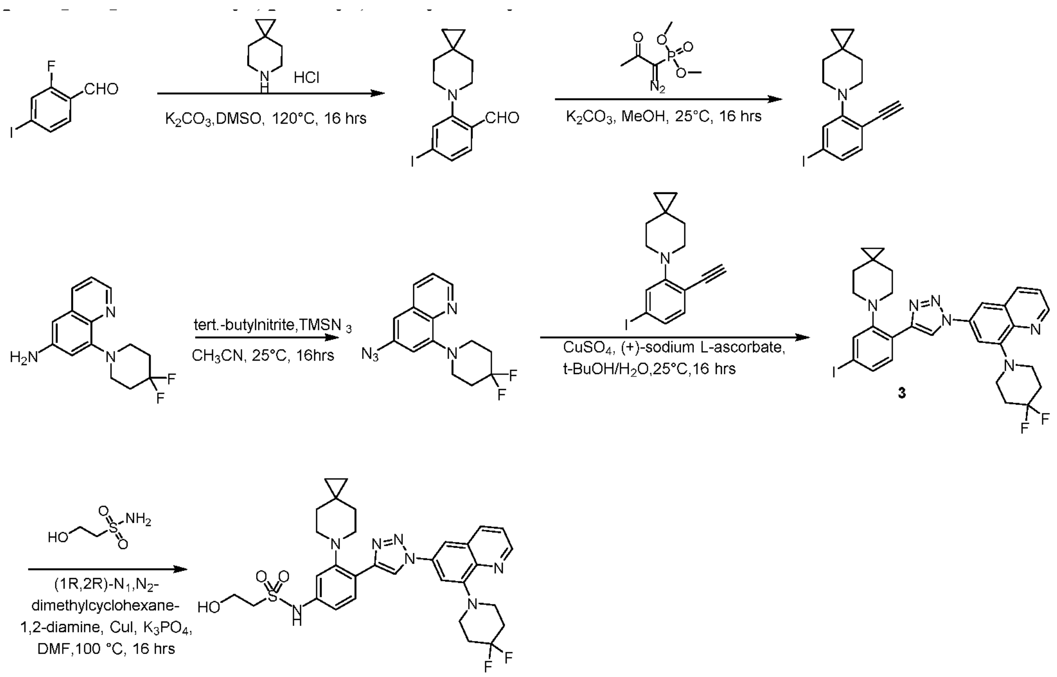


Step 1: To a solution of 2-fluoro-4-iodobenzaldehyde (2.00 g, 8.00 mmol) and potassium carbonate (3.31 g, 24.0 mmol) in DMSO (20.0 mL) was added 6-azaspiro[2.5]octane hydrochloride (1.33 g, 12.0 mmol). The mixture was heated to 120°C and stirred for 16 h. The reaction mixture was diluted with water (20 mL) and extracted with ethyl acetate (20 mL$\times$2). The organic phases were combined, dried over anhydrous sodium sulfate, and filtered, and concentrated under reduced pressure to give the residue. The residue was purified by flash column chromatography (silica gel, 0-8% gradient of ethyl acetate/petroleum ether) to give 4-iodo-2-(6-azaspiro[2.5]octan-6-yl)benzaldehyde (1.70 g, 4.95 mmol, yield: 62%) as a yellow solid. LCMS (ESI): $[M+H]^+$ =342.0

Step 2: To a solution of 4-iodo-2-(6-azaspiro[2.5]octan-6-yl)benzaldehyde (1.70 g, 4.98 mmol) and potassium carbonate (1.37 g, 9.97 mmol) in methanol (20.0 mL) was added dimethyl (1-diazo-2-oxopropylidene)phosphonate (1.15 g, 5.98 mmol). The mixture was stirred at 25°C for 16 h. The reaction mixture was diluted with water (20 mL) and extracted with ethyl acetate (20 mL×2). The organic phases were combined, dried over anhydrous sodium sulfate, and filtered, and concentrated under reduced pressure to give the residue. The residue was purified by flash column chromatography (silica gel, 0-5% gradient of ethyl acetate/petroleum ether) to give 6-(2-ethynyl-5-iodophenyl)-6-azaspiro[2.5]octane (1.50 g, 4.43 mmol, yield: 89%) as a yellow solid. LCMS (ESI): $[M+H]^+$ = 338.4.

Step 3: Tert-butyl nitrite (599 mg, 5.70 mmol) was added to a solution of 8-(4,4-difluoropiperidin-1-yl)quinolin-6-amine (600 mg, 2.28 mmol) in acetonitrile (5.00 mL). Then, azidotrimethylsilane (525 g, 4.56 mmol) was added to the reaction solution and the mixture was stirred at 25°C for 16 h. The reaction solution was poured into water (10 mL) and extracted with ethyl acetate (10 mL×3). The organic phases were combined, dried over anhydrous sodium sulfate, and filtered, and concentrated under reduced pressure to give the residue. The residue was purified by flash column chromatography (silica gel, 0-10% gradient of ethyl acetate/petroleum ether) to give 6-azido-8-(4,4-difluoropiperidin-1-yl)quinoline (240 mg, 0.83 mmol, yield: 36%) as a white solid. LCMS (ESI): [M+H]+ =290.0.

Step 4: Copper sulfate (88.3 mg, 0.55 mmol) and sodium ascorbate (110 mg, 0.55 mmol) were added to a solution of 6-azido-8-(4,4-difluoropiperidin-1-yl)quinoline (160 mg, 0.55 mmol) and 6-(2-ethynyl-5-iodophenyl)-6-azaspiro[2.5] octane (224 mg, 0.66 mmol) in tert-butanol (5.00 mL) and water (5.00 mL). The mixture was stirred at 25°C for 16 h. The reaction solution was diluted with water (20 mL) and extracted with ethyl acetate (20 mL×3). The organic phases were combined, dried over anhydrous sodium sulfate, and filtered, and concentrated under reduced pressure to give the residue. The residue was purified by flash column chromatography (silica gel, 0-5% gradient of ethyl acetate/petroleum ether) to give 6-[4-(2-{6-azaspiro[2.5]octan-6-yl}-4-iodophenyl)-1H-1,2,3-triazol-1-yl]-8-(4,4-diflu oropiperidin-1-yl)quinoline (240 mg, 0.38 mmol, yield 69%) as a brown solid. LCMS (ESI): [M+H]+ =627.2.

Step 5: To a solution of 2-hydroxyethanesulfonamide (47.9 mg, 0.38 mmol), (1R,2R)-(-)-N,N-dimethylcyclohexane-1,2-diamine (27.5 mg, 0.19 mmol), and potassium phosphate (163 mg, 0.77 mmol) in N,N-dimethylformamide (5.0 mL) was added cuprous iodide (18.6 mg, 0.10 mmol). The mixture was stirred at 50°C for 5 min. Then, 6-[4-(2-{6-azaspiro [2.5]octan-6-yl}-4-iodophenyl)-1H-1,2,3-triazol-1-yl]-8-(4,4-diflu oropiperidin-1-yl)quinoline (120 mg, 0.19 mmol) was added to the reaction mixture at 50°C, the mixture was heated to 100°C under nitrogen protection, and stirred for 16 h. Water (10 mL) was added to the reaction solution and extracted with ethyl acetate (10 mL × 2). The organic phases were combined, dried over anhydrous sodium sulfate, and filtered, and concentrated under reduced pressure to give the residue. The residue was purified by preparative HPLC (C18, 36-76% gradient of water (formic acid)/acetonitrile) to give N-(4-(1-(8-(4,4-difluoropiperidin-1-yl)quinolin-6-yl)-1H-1,2,3-triazol-4-yl)-3-(6-azas piro[2.5]octan-6-yl)phenyl)-2-hydroxyethane-1-sulfonamide (53 mg, 0.084 mmol, yield: 44%) as a white solid compound. LCMS (ESI): [M+H]+ = 624.3. $^1$H NMR (400 MHz, DMSO-d6) δ ppm 9.88 (s, 1H), 9.26 (s, 1H), 8.96 (br d, *J*= 2.8 Hz, 1H), 8.51 (d, *J* = 0.8 Hz, 1H), 8.15 (d, *J* = 1.6 Hz, 1H), 7.91 (d, *J* = 8.4 Hz, 1H), 7.71 - 7.58 (m, 2H), 7.16 (d, *J* = 1.6 Hz, 1H), 7.04 (dd, *J* = 2.0, 8.4 Hz, 1H), 5.73 - 3.98 (m, 1H), 3.77 (t, *J* = 6.8 Hz, 2H), 3.70 - 3.62 (m, 4H), 3.30 (t, *J* = 1.2 Hz, 2H), 2.92 - 2.82 (m, 4H), 2.32 - 2.21 (m, 4H), 1.60 - 1.43 (m, 4H), 0.33 (s, 4H)

**Example 95:**

**N-(3-{6-azaspiro[2.5]octan-6-yl}-4-{4-[2-(4,4-difluoropiperidin-1-yl)-6-(2-hydrox yethoxy)pyrimidin-4-yl]-1H-1,2,3-triazol-1-yl}phenyl)-2-hydroxyethane-1-sulfonamide**

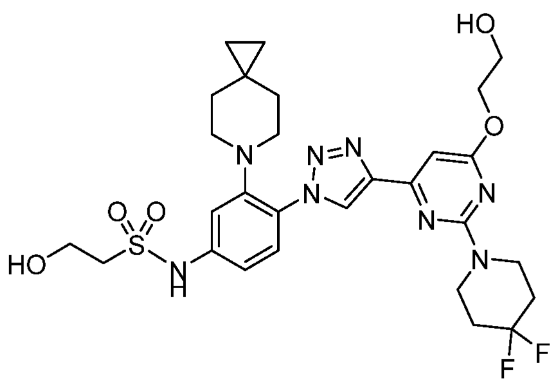

**Example 95** was prepared with reference to the synthetic route of Example 54;

LCMS (ESI): [M+H]+ = 635.3; $^{1}$H NMR (400 MHz, DMSO-d6) δ ppm 10.13 (s, 1H), 9.16 (s, 1H), 7.55 (d, *J* = 8.4 Hz, 1H), 7.11 (d, *J* = 1.6 Hz, 1H), 7.01 (dd, *J* = 2.0, 8.8 Hz, 1H), 6.68 (s, 1H), 5.29 (s, 1H), 4.88 (t, *J* = 5.6 Hz, 1H), 4.36 (t, *J* = 5.2 Hz, 2H), 3.96 (br s, 4H), 3.81 - 3.67 (m, 4H), 3.32 - 3.29 (m, 2H), 2.66 (br t, *J*=4.8 Hz, 4H), 2.11 - 1.90 (m, 4H), 1.29 (br s, 4H), 0.24 (s, 4H)

**Example 96:**

**N-(3-{6-azaspiro[2.5]octan-6-yl}-4-{4-[2-(4,4-difluoropiperidin-1-yl)-6-(oxolan-3-yloxy)pyrimidin-4-yl]-1H-1,2,3-triazol-1-yl}phenyl)-2-hydroxyethane-1-sulfonam ide**

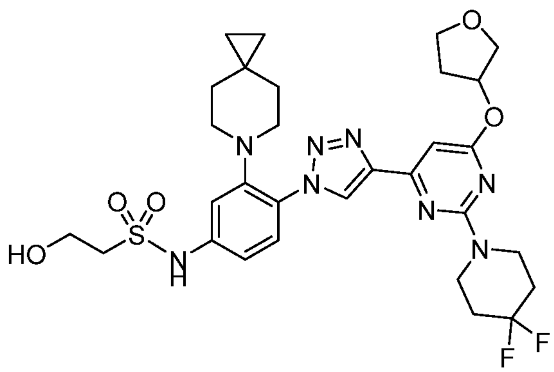

**Example 96** was prepared with reference to the synthetic route of Example 54;

LCMS (ESI): [M+H]+ = 661.2; $^{1}$H NMR (400 MHz, DMSO-d6) δ ppm 10.13 (s, 1H), 9.17 (s, 1H), 7.56 (d, *J* = 8.4 Hz, 1H), 7.13 (d, *J* = 2.4 Hz, 1H), 7.03 (dd, *J* = 2.0, 8.8 Hz, 1H), 6.67 (s, 1H), 5.56 (tt, *J* = 2.4, 4.8 Hz, 1H), 5.14 - 4.79 (m, 1H), 4.01 - 3.92 (m, 5H), 3.90 - 3.80 (m, 2H), 3.80 - 3.71 (m, 3H), 3.35 - 3.33 (m, 2H), 2.66 (br t, *J* = 5.2 Hz, 4H), 2.32 - 2.22 (m, 1H), 2.11 - 1.95 (m, 5H), 1.29 (br s, 4H), 0.25 (s, 4H)

**Example 97:**

**N-(3-{6-azaspiro[2.5]octan-6-yl}-4-{4-[2-(4,4-difluoropiperidin-1-yl)-6-[(oxolan-3-yl)methoxy]pyrimidin-4-yl]-1H-1,2,3-triazol-1-yl}phenyl)-2-hydroxyethane-1-sulf onamide**

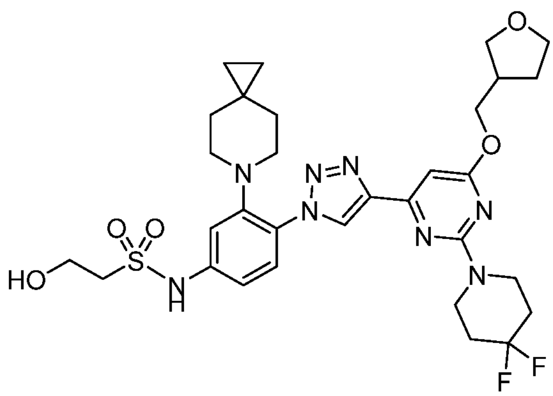

**Example 97** was prepared with reference to the synthetic route of Example 54;

LCMS (ESI): [M+H]+ = 675.2; $^{1}$H NMR (400 MHz, DMSO-$d_6$) δ ppm 10.54 - 9.69 (m, 1H), 9.16 (s, 1H), 7.57 (d, *J* =

8.4 Hz, 1H), 7.21 - 6.92 (m, 2H), 6.68 (s, 1H), 5.19 - 4.78 (m, 1H), 4.49 - 4.13 (m, 2H), 3.97 (br s, 4H), 3.86 - 3.74 (m, 4H), 3.67 (q, *J* = 7.6 Hz, 1H), 3.55 (br dd, *J* = 5.6, 8.4 Hz, 2H), 2.67 (br s, 6H), 2.02 (br t, *J* = 12.8 Hz, 5H), 1.69 (td, *J* = 6.8, 12.8 Hz, 1H), 1.29 (br s, 4H), 0.25 (s, 4H)

**Example 98:**

**N-(3-{6-azaspiro[2.5]octan-6-yl}-4-{4-[2-(4,4-difluoropiperidin-1-yl)-6-methylpyr imidin-4-yl]-1H-1,2,3-triazol-1-yl}phenyl)-1-hydroxybutane-2-sulfonamide**

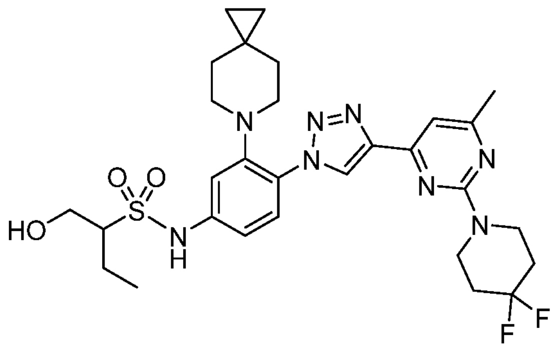

LCMS (ESI): [M+H]+ = 617.3; $^{1}$H NMR (400 MHz, DMSO-d6) δ ppm 9.99 (m, 1H), 9.21 (s, 1H), 7.56 (d, *J*= 8.4 Hz, 1H), 7.21 (s, 1H), 7.14 (d, *J* = 2.4 Hz, 1H), 7.03 (dd, *J* = 2.4, 8.4 Hz, 1H), 5.35 - 4.67 (m, 1H), 3.98 (br t, *J* = 5.6 Hz, 4H), 3.86 (dd, *J* = 4.8, 11.2 Hz, 1H), 3.67 (dd, *J* = 6.4, 11.6 Hz, 1H), 3.07 (br t, *J* = 5.6 Hz, 1H), 2.66 (br t, *J* = 5.2 Hz, 4H), 2.40 (s, 3H), 2.06 - 1.93 (m, 4H), 1.90 - 1.73 (m, 2H), 1.28 (br s, 4H), 1.00 (t, *J* = 7.2 Hz, 3H), 0.24 (s, 4H)

**Example 99:**

**(2R)-N-(3-{6-azaspiro[2.5]octan-6-yl}-4-{4-[8-(4,4-difluoropiperidin-1-yl)quinolin -6-yl]-1H-1,2,3-triazol-1-yl}phenyl)-1-hydroxypropane-2-sulfonamide**

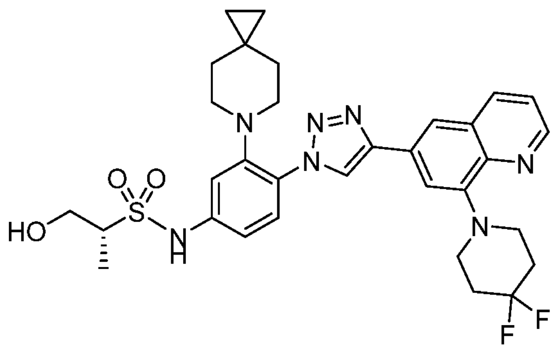

LCMS (ESI): [M+H]+ = 638.1. $^{1}$H NMR (400 MHz, DMSO-$d_6$) δ ppm 10.56 - 9.63 (m, 1H), 9.20 (s, 1H), 8.96 - 8.79 (m, 1H), 8.39 (br d, *J* = 7.2 Hz, 1H), 8.13 (s, 1H), 7.73 (s, 1H), 7.62 - 7.44 (m, 2H), 7.16 (d, *J* = 1.6 Hz, 1H), 7.04 (dd, *J* = 2.0, 8.4 Hz, 1H), 5.45 - 4.58 (m, 1H), 3.88 (dd, *J* = 4.4, 11.2 Hz, 1H), 3.58 (br s, 4H), 3.50 (br dd, *J* = 8.0, 10.8 Hz, 1H), 3.29 - 3.22 (m, 1H), 2.70 (br s, 4H), 2.25 (br t, *J* = 13.2 Hz, 4H), 1.35 - 1.22 (m, 7H), 0.23 (s, 4H)

**Example 100:**

**(2S)-N-(3-{6-azaspiro[2.5]octan-6-yl}-4-{4-[8-(4,4-difluoropiperidin-1-yl)quinolin -6-yl]-1H-1,2,3-triazol-1-yl}phenyl)-1-hydroxypropane-2-sulfonamide**

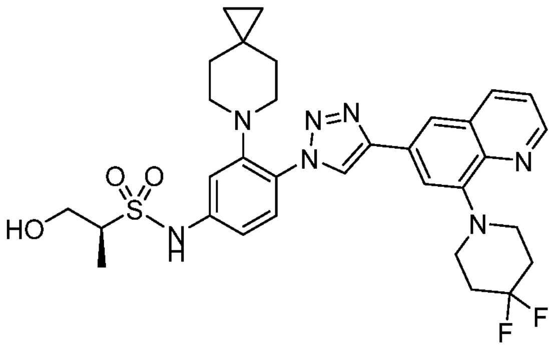

LCMS (ESI): [M+H]+ = 638.1. $^1$H NMR (400 MHz, DMSO-$d_6$) δ ppm 10.85 - 9.40 (m, 1H), 9.20 (s, 1H), 8.87 (dd, *J* = 2.0, 4.0 Hz, 1H), 8.38 (dd, *J* = 1.6, 8.4 Hz, 1H), 8.12 (d, *J* = 1.2 Hz, 1H), 7.72 (d, *J* = 1.2 Hz, 1H), 7.58 - 7.44 (m, 2H), 7.15 (d, *J* = 2.0 Hz, 1H), 7.03 (dd, *J* = 2.0, 8.4 Hz, 1H), 5.64 - 4.36 (m, 1H), 3.87 (dd, *J* = 4.4, 11.2 Hz, 1H), 3.58 (br s, 4H), 3.49 (dd, *J* = 8.0, 11.2 Hz, 1H), 3.30 - 3.23 (m, 1H), 2.69 (br t, *J* = 4.8 Hz, 4H), 2.32 - 2.16 (m, 4H), 1.37 - 1.24 (m, 7H), 0.22 (s, 4H)

**Example 101:**

**N-(4-(4-(8-(4,4-difluoropiperidin-1-yl)quinolin-6-yl)-1H-1,2,3-triazol-1-yl)-3-(6-az aspiro[2.5]octan-6-yl)phenyl)-1-hydroxy-2-methylpropane-2-sulfonamide**

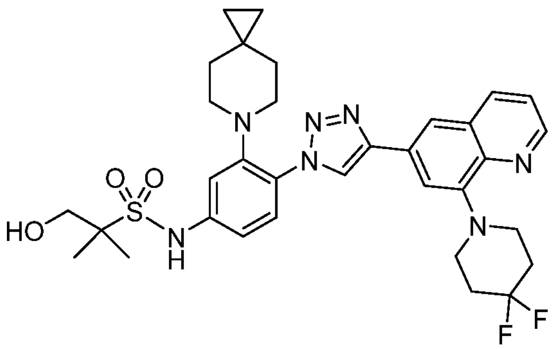

LCMS (ESI): [M+H]+ = 652.1; $^1$H NMR (400 MHz, DMSO-$d_6$) δ ppm 10.20 - 9.55 (m, 1H), 9.22 (s, 1H), 8.87 (br d, *J* = 2.8 Hz, 1H), 8.38 (br d, *J*= 7.6 Hz, 1H), 8.14 (s, 1H), 7.73 (s, 1H), 7.61 - 7.40 (m, 2H), 7.27 (s, 1H), 7.07 (dd, *J* = 1.2, 8.8 Hz, 1H), 5.63 - 4.61 (m, 1H), 3.58 (br s, 6H), 2.69 (br s, 4H), 2.25 (br t, *J* = 13.6 Hz, 4H), 1.27 (s, 10H), 0.22 (s, 4H)

**Example 102:**

**N-(4-(5-(2-(4,4-difluoropiperidin-1-yl)-6-methoxypyridin-4-yl)-1,3,4-oxadiazol-2-yl)-3-(6-azaspiro[2.5]octan-6-yl) phenyl)-1-hydroxypropane-2-sulfonamide**

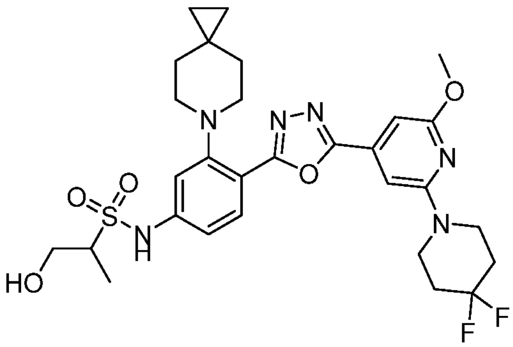

LCMS (ESI): $[M+H]^+$ =619.4; $^1H$ NMR (400 MHz, DMSO) δ 10.17 (s, 1H), 7.90 (d, J = 8.5 Hz, 1H), 7.12 (d, J = 2.1 Hz, 1H), 7.00 (d, J = 10.7 Hz, 2H), 6.65 (s, 1H), 5.04 (s, 1H), 3.95-3.82 (m, 4H), 3.79 (t, J = 5.7 Hz, 4H), 3.49 (dd, J = 11.1, 7.6 Hz, 1H), 3.28 (dd, J = 7.2, 4.5 Hz, 1H), 2.93 (t, J = 5.2 Hz, 4H), 2.05 (tt, J = 13.4, 5.2 Hz, 4H), 1.47 (t, J = 5.2 Hz, 4H), 1.30 (d, J = 6.8 Hz, 3H), 0.32 (s, 4H).

**Example 103:**

**N-(4-(5-(2-(4,4-difluoropiperidin-1-yl)-6-methylpyridin-4-yl)-1,3,4-oxadiazol-2-yl) -3-(6-azaspiro[2.5]octan-6-yl) phenyl)-1-hydroxypropane-2-sulfonamide**

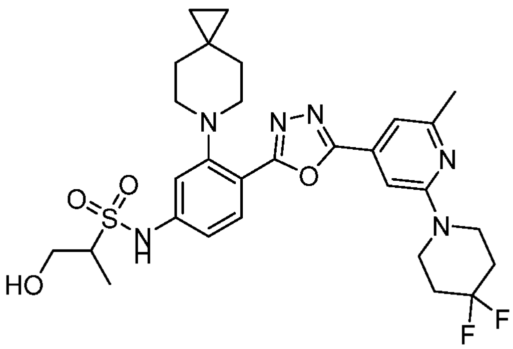

LCMS (ESI): $[M+H]^+$ =603.5; $^1H$ NMR (400 MHz, DMSO) δ 10.18 (s, 1H), 7.91 (d, J = 8.5 Hz, 1H), 7.25 (s, 1H), 7.18 (s, 1H), 7.12 (d, J = 2.1 Hz, 1H), 7.01 (dd, J = 8.5, 2.1 Hz, 1H), 5.04 (s, 1H), 3.85 (dd, J = 11.1, 4.4 Hz, 1H), 3.79 (t, J = 5.8 Hz, 4H), 3.49 (dd, J = 11.1, 7.7 Hz, 1H), 3.29 (dt, J = 7.2, 3.6 Hz, 1H), 2.93 (t, J = 5.2 Hz, 4H), 2.44 (s, 3H), 2.08-1.95 (m, 4H), 1.48 (t, J = 5.3 Hz, 4H), 1.30 (d, J = 6.8 Hz, 3H), 0.32 (s, 4H).

**Example 104:**

**N-(4-(5-(2-(4,4-difluoropiperidin-1-yl)-6-methylpyridin-4-yl)-1,3,4-oxadiazol-2-yl) -3-(6-azaspiro[2.5]octan-6-yl) phenyl)-1-hydroxy-2-methylpropane-2-sulfonamide**

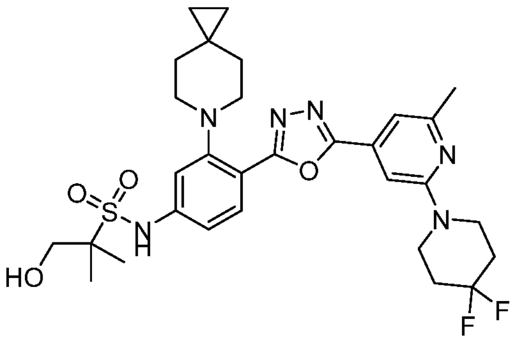

LCMS (ESI): $[M+H]^+$ =617.4; $^1H$ NMR (400 MHz, DMSO) δ 9.95 (s, 1H), 7.89 (d, J = 8.6 Hz, 1H), 7.28-7.11 (m, 3H), 7.05 (dd, J = 8.6, 2.1 Hz, 1H), 5.12 (s, 1H), 3.79 (t, J = 5.7 Hz, 4H), 3.56 (s, 2H), 2.92 (t, J = 5.2 Hz, 4H), 2.44 (s, 3H), 2.04 (td, J = 14.4, 13.6, 7.2 Hz, 4H), 1.48 (t, J = 5.3 Hz, 4H), 1.26 (s, 6H), 0.32 (s, 4H).

**Example 105:**

**N-(4-(5-(2-(4,4-difluoropiperidin-1-yl)-6-methoxypyridin-4-yl)-1,3,4-oxadiazol-2-yl)-3-(6-azaspiro[2.5]octan-6-yl) phenyl)-1-hydroxy-2-methylpropane-2-sulfonami de**

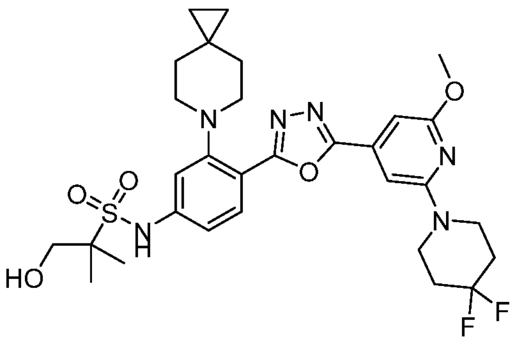

LCMS (ESI): $[M+H]^+$ =633.4; $^1H$ NMR (400 MHz, DMSO) δ 9.94 (s, 1H), 7.88 (d, J = 8.6 Hz, 1H), 7.23 (d, J = 2.0 Hz, 1H), 7.09-6.94 (m, 2H), 6.65 (d, J = 0.8 Hz, 1H), 5.12 (s, 1H), 3.88 (s, 3H), 3.79 (t, J = 5.6 Hz, 4H), 3.56 (s, 2H), 2.92 (t, J = 5.2 Hz, 4H), 2.06 (ddt, J = 20.0, 14.0, 5.6 Hz, 4H), 1.47 (t, J = 5.2 Hz, 4H), 1.26 (s, 6H), 0.32 (s, 4H).

**Example 106:**

**N-(4-(4-(4,4-difluoropiperidin-1-yl)-6-methyl-3H-imidazo[4,5-c]pyridin-2-yl)-3-(6 -azaspiro[2.5]octan-6-yl)phenyl)-2-hydroxyethane-1-sulfonamide**

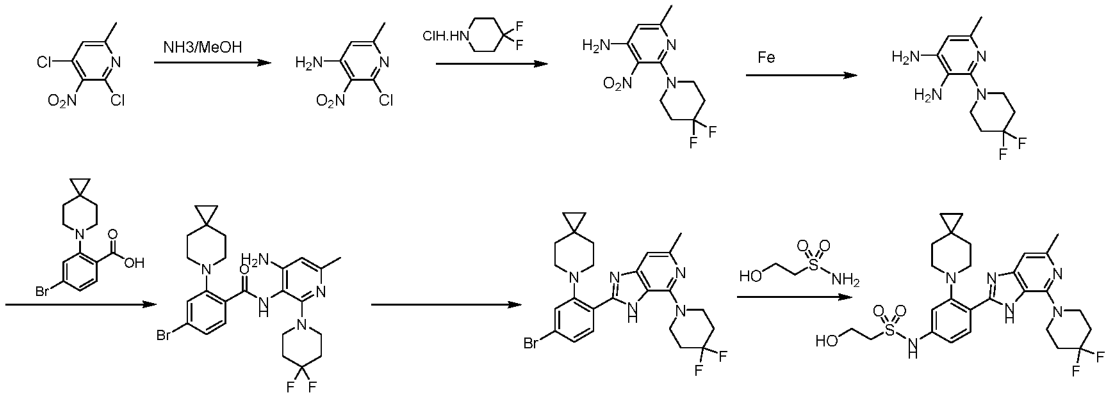


Step 1: The mixed solution containing 2,4-dichloro-6-methyl-3-nitropyridine (5.00 g, 24.15 mmol), ammonia methanol solution (25 mL, 7M), and THF (50 mL) was stirred at 50°C for 12 h. The reaction solution was poured into water (30 mL) and extracted with ethyl acetate (30 mL × 3), and the organic phase was dried over anhydrous sodium sulfate, filtered, concentrated, and purified by column chromatography (silica gel, 0-100% gradient of ethyl acetate/petroleum ether) to give 2-chloro-6-methyl-3-nitropyridine-4-amine (1.90 g, 10.13 mmol, yield: 41.9%) as a pale yellow solid compound. LCMS (ESI): $[M+H]^+$ = 188.2. $^1$H NMR (400 MHz, DMSO) δ 7.26 (s, 2H), 6.66-6.62 (m, 1H), 2.33 -2.23 (m, 3H).

Step 2: The mixed solution containing 2-chloro-6-methyl-3-nitro-4-pyridinamine (1.90 g, 10.13 mmol), 4,4-difluoropiperidine hydrochloride (1.90 g, 12.16 mmol), potassium carbonate (4.30 g, 30.39 mmol), and DMSO (20 mL) was stirred at 100°C for 12 h. The reaction solution was poured into water (30 mL) and extracted with ethyl acetate (30 mL x 3), and the organic phase was dried over anhydrous sodium sulfate, filtered, concentrated, and purified by column chromatography (silica gel, 0-100% gradient of ethyl acetate/petroleum ether) to give 2-(4,4-difluoropiperidin-1-yl)-6-methyl-3-nitropyridine-4-amine (2.30 g, 8.45 mmol, yield: 83.4%) as a pale yellow oil compound. LCMS (ESI): $[M+H]^+$ =273.1

Step 3 : The mixed solution containing 2-(4,4-difluoropiperidyl)-6-methyl-3-nitro-4-pyridinamine (2.30 g, 8.45 mmol), iron powder (1.40 g, 25.34 mmol), ammonium chloride (1.40 g, 25.34 mmol), ethanol (20 mL) and water (4 mL) was stirred at 60°C for 1 h. The hot reaction solution was filtered. After concentration, the filtrate was extracted with ethyl acetate (30 mL x 3), and the organic phase was dried over anhydrous sodium sulfate, filtered, concentrated, and purified by column chromatography (silica gel, 0-30% gradient of methanol/dichloromethane) to give 2-(4,4-difluoropiperidin-1-yl)-6-methylpyridine-3,4-diamine (1.80 g, 7.43 mmol, yield: 87.9%) as a gray solid compound. LCMS (ESI): $[M+H]^+$ = 243.2.

Step 4: The mixed solution containing 2-(6-azaspiro[2.5]oct-6-yl)-4-bromobenzoic acid (2.80 g, 8.92 mmol), 2-(4,4-difluoropiperidinyl)-6-methylpyridine-3.4- diamine (1.80 g, 7.43 mmol), 2-(7-azobenzotriazole)-N,N,N',N'-tetramethylurea hexafluorophosphate (4.32 g, 11.15 mmol), N,N-diisopropylethylamine (2.90 g, 22.29 mmol), and N,N-dimethylformamide (20 mL) was stirred at 20°C for 12 h. The reaction solution was poured into water (30 mL) and extracted with ethyl acetate (30 mL x 3), and the organic phase was dried over anhydrous sodium sulfate, filtered, concentrated, and purified by column chromatography (silica gel, 0-100% gradient of ethyl acetate/petroleum ether to give N-(4-amino-2-(4,4-difluoropiperidin-1-yl)-6-methylpyridin-3-yl)-4-bromo-2-(6-azasp iro[2.5]octan-6-yl) benzamide (1.30 g, 2.43 mmol, yield: 32.7%) as a yellow solid compound. LCMS (ESI): $[M+H]^+$ = 534.3; $^1$H NMR (400 MHz, DMSO) δ 11.29 (s, 1H), 7.91 (d, J = 8.4 Hz, 1H), 7.65 (d, J = 2.0 Hz, 1H), 7.51 (dd, J = 8.4, 2.0 Hz, 1H), 6.29 (s, 1H), 5.62 (s, 2H), 3.14-3.02 (m, 8H), 2.21 (s, 3H), 1.96-1.85 (m, 4H), 1.44-1.39 (m, 4H), 0.33 (s, 4H).

Step 5 : The solution containing N-[4-amino-2-(4,4-difluoropiperidinyl)-6-methyl(3-pyridyl)] [2-(6-azaspiro[2.5] oct-6-yl)-4-bromophenyl]formamide (1.3 g, 2.43 mmol) and acetic acid (15 mL) was stirred at 120°C for 48 h. The reaction solution was poured into water (100 mL) and the pH was adjusted to about 8 by adding aqueous sodium bicarbonate solution (500 mL). Then, it was extracted with ethyl acetate (100 mL x 3) and the organic phase was dried over anhydrous sodium sulfate, filtered, concentrated, and purified by column chromatography (silica gel, 0-100% gradient of ethyl acetate/petroleum ether) to give 2-(4-bromo-2-(6-azaspiro[2.5]oct-6-yl)phenyl)-4-(4,4-difluoropiperidin-1-yl)-6-methy l-3H-imidazo[4,5-c]pyridine (630 mg, 1.22 mmol, yield: 50.2%) as a yellow solid compound. LCMS (ESI): $[M+H]^+$ = 516.3

Step 6: Under nitrogen protection, the mixed solution containing 2-(4-bromo-2-(6-azaspiro[2.5]oct-6-yl)phenyl)-4-(4,4-difluoropiperidin-1-yl)-6-methy l-3H-imidazo[4,5-c]pyridine (300 mg, 0.58 mmol), 2-hydroxyethanesulfonamide (148 mg, 1.16 mmol), potassium phosphate (377 mg, 1.74 mmol), 2-di-tert-butylphosphino-2',4',6'-triisopropylbiphenyl (25 mg, 0.058 mmol), tris(dibenzylideneacetone) dipalladium (54 mg, 0.058 mmol) and dioxane (5 mL) was stirred at 100°C for 8 h. The reaction solution was poured into water (30 mL) and extracted with ethyl acetate (30 mL x 3), and the organic phase was dried over anhydrous sodium sulfate, filtered, concentrated, and purified by column chromatography (silica gel, 0-100% gradient of ethyl acetate/petroleum ether) to give N-(4-(4-(4,4-difluoropiperidin-1-yl)-6-methyl-3H-imidazo[4,5-c]pyridin-2-yl)-3-(6-az aspiro[2.5]octan-6-yl)phenyl)-2-hydroxyethane-1-sulfonamide (141.25 mg, 0.25 mmol, yield: 43.4%) as a white solid compound. LCMS (ESI): $[M+H]^+$ = 561.0; $^1H$ NMR (400 MHz, DMSO) δ 12.22 (s, 1H), 9.94 (s, 1H), 7.79 (d, J = 8.4 Hz, 1H), 7.10 (d, J = 2.0 Hz, 1H), 6.99 (dd, J = 8.4, 2.0 Hz, 1H), 6.83 (s, 1H), 4.95 (s, 1H), 4.39-4.16 (m, 4H), 3.76 (t, J = 6.8 Hz, 2H), 3.32-3.25 (m, 2H), 2.81 (t, J = 5.2 Hz, 4H), 2.38 (s, 3H), 2.12-2.00 (m, 4H), 1.47 (s, 4H), 0.30 (s, 4H).

**Effect Example 1: Enzyme Activity Test Experiment**

**Materials:** Human KIF18A (amino acid sequence 1-417) was obtained from Viva Biotech (Shanghai) Ltd., and the ADP-Glo™ protein kinase kit was acquired from Promega, USA. Tubulin was sourced from Cytoskeleton, USA, while a 384-well assay plate and the Envision multifunctional microplate reader were obtained from PerkinElmer, USA.

**Enzyme activity Test:** To perform the enzymatic activity test, the compound powder in DMSO was firstly dissolved to prepare a 10 mM stock solution. Next, gradient dilutions were prepared in the microplate to achieve final concentrations of 0-10 μM. Sequentially, 2.5 μL of tubulin (60 μg/mL), the compound, ATP (25 μM), and KIF18A protein (2.5 nM) were added to each well, and the reaction was incubated at ambient temperature for 120 minutes. After the reaction, 10 μL of ADP-Glo™ reagent was added to each well and incubated at ambient temperature for 30 minutes. Subsequently, 20 μL of detection reagent was added to each well and incubated for an additional 30 minutes in the dark. Finally, chemiluminescence detection was performed using the PerkinElmer Envision multifunctional microplate reader.

**Table 1: Enzymatic Activity Biological Data for Compounds of the Present Invention**

| Example | Enzyme Activity (nM) | Example | Enzyme Activity (nM) | Example | Enzyme Activity (nM) |
|---|---|---|---|---|---|
| 1 | >3000 | 37 | 253 | 73 | 271 |
| 2 | >3000 | 38 | 452 | 74 | 807 |
| 3 | 852 | 39 | 414 | 75 | >3000 |
| 4 | 38 | 40 | >3000 | 76 | 75 |
| 5 | 1051 | 41 | 44 | 77 | 64 |
| 6 | 1089 | 42 | 19 | 78 | >3000 |
| 7 | 102 | 43 | 44 | 79 | 24 |
| 8 | 775 | 44 | 22 | 80 | 475 |
| 9 | 312 | 45 | 88 | 81 | 710 |
| 10 | 251 | 46 | 151 | 82 | 283 |
| 11 | 252 | 47 | 64 | 83 | 117 |
| 12 | >3000 | 48 | 22 | 84 | 125 |
| 13 | 1276 | 49 | 61 | 85 | 188 |
| 14 | 158 | 50 | 1050 | 86 | 162 |
| 15 | 60 | 51 | 131 | 87 | NT |
| 16 | 243 | 52 | 25 | 88 | 118 |
| 17 | 46 | 53 | 296 | 89 | 61 |
| 18 | 52 | 54 | 277 | 90 | 16 |
| 19 | 630 | 55 | 40 | 91 | 278 |
| 20 | 61 | 56 | 110 | 92 | 232 |
| 21 | 67 | 57 | 72 | 93 | 315 |
| 22 | 46 | 58 | 779 | 94 | 227 |
| 23 | 153 | 59 | 437 | 95 | 73 |
| 24 | 51 | 60 | 433 | 96 | 117 |
| 25 | 46 | 61 | 377 | 97 | 234 |
| 26 | 287 | 62 | 272 | 98 | 154 |

(continued)

| Example | Enzyme Activity (nM) | Example | Enzyme Activity (nM) | Example | Enzyme Activity (nM) |
|---|---|---|---|---|---|
| 27 | 124 | 63 | 437 | 99 | 214 |
| 28 | 293 | 64 | 177 | 100 | 187 |
| 29 | 48 | 65 | 383 | 101 | 320 |
| 30 | 255 | 66 | 62 | 102 | 60 |
| 31 | 161 | 67 | 12 | 103 | 73 |
| 32 | 113 | 68 | 112 | 104 | 83 |
| 33 | 61 | 69 | >3000 | 105 | 62 |
| 34 | 56 | 70 | 1229 | 106 | 210 |
| 35 | 42 | 71 | 165 | | |
| 36 | 50 | 72 | 190 | | |
| NT means no test | | | | | |

**Example 2: Cell Proliferation Activity Test**

**Materials and Cells:** OVCAR3 cells were purchased from Nanjing Cobioer Biosciences Co., Ltd. RPMI-1640 medium, fetal bovine serum, and the CyQUANT Direct Cell Proliferation Assay kit were purchased from Thermo Fisher Scientific, USA; 96-well cell culture plates were sourced from Coming, USA.

**Cell Culture:** Culture OVCAR3 cells with RPMI-1640 medium containing 10% fetal bovine serum and incubate them in a 5% $CO_2$ incubator at 37°C. Only cells in the logarithmic growth phase can be used for the test.

**Cell Proliferation Activity Test:**

Inoculate OVCAR3 cells into a 96-well cell culture plate at 90 μL per well and incubate overnight in a 5% $CO_2$ incubator at 37°C. Dissolve the compound powder in DMSO to prepare a stock solution of 10 mM. Then perform gradient dilution for the compounds in the microplate to achieve a final concentration of 0-10 μM. Add 10 μL of cell culture medium containing compounds to each well to make the final DMSO content 0.2%. Incubate the cell plates in a 5% $CO_2$ incubator at 37°C for 3 days. Add 100 μL of CyQUANT detection reagent to each well, react at 37°C for 60 min, and perform fluorescence detection using the PerkinElmer Envision.

**Table 2: OVCAR-3 Cell Viability Biological Data for Compounds of the Present Invention**

| Examp le | OVCAR-3 Cell Viability_IC50_ nM | Examp le | OVCAR-3 Cell Viability_IC50_ nM | Examp le | OVCAR-3 Cell Viability_IC50_ nM |
|---|---|---|---|---|---|
| 1 | 474 | 37 | 87 | 73 | 34 |
| 2 | 766 | 38 | 424 | 74 | 119 |
| 3 | 181 | 39 | 1333 | 75 | 300 |
| 4 | 62 | 40 | 411 | 76 | 12 |
| 5 | 389 | 41 | 15 | 77 | 13 |
| 6 | 257 | 42 | 12 | 78 | >3000 |
| 7 | 1185 | 43 | 16 | 79 | 11 |
| 8 | 496 | 44 | 14 | 80 | 21 |
| 9 | 107 | 45 | 22 | 81 | 20 |
| 10 | 46 | 46 | 14 | 82 | 15 |
| 11 | 164 | 47 | 37 | 83 | 13 |
| 12 | 464 | 48 | 17 | 84 | 21 |
| 13 | 472 | 49 | 54 | 85 | 25 |

(continued)

| Examp le | OVCAR-3 Cell Viability_IC50_ nM | Examp le | OVCAR-3 Cell Viability_IC50_ nM | Examp le | OVCAR-3 Cell Viability_IC50_ nM |
|---|---|---|---|---|---|
| 14 | 145 | 50 | 222 | 86 | 22 |
| 15 | 61 | 51 | 24 | 87 | NT |
| 16 | 717 | 52 | 110 | 88 | 29 |
| 17 | 74 | 53 | 35 | 89 | 36 |
| 18 | 30 | 54 | 84 | 90 | 44 |
| 19 | 1401 | 55 | 3 | 91 | 87 |
| 20 | 139 | 56 | 9 | 92 | 93 |
| 21 | 1615 | 57 | 8 | 93 | 85 |
| 22 | 34 | 58 | 224 | 94 | 92 |
| 23 | 115 | 59 | 26 | 95 | 7 |
| 24 | 16 | 60 | 29 | | |
| 25 | 91 | 61 | 22 | | |
| 26 | 767 | 62 | 59 | | |
| 27 | 875 | 63 | 108 | | |
| 28 | 118 | 64 | 27 | | |
| 29 | 37 | 65 | 116 | | |
| 30 | 55 | 66 | 52.7 | | |
| 31 | 16 | 67 | 25 | | |
| 32 | 187 | 68 | 159 | | |
| 33 | 34 | 69 | 1505 | | |
| 34 | 12 | 70 | 582 | | |
| 35 | 23 | 71 | 94 | | |
| 36 | 30 | 72 | 20 | | |
| NT means no test | | | | | |

**Example 3:** CyQuant Cell Proliferation Activity Test

**Materials and Cells:** HT29 cells were purchased from Nanjing Cobioer Biosciences Co., Ltd.; RPMI-1640 medium, fetal bovine serum, Trypsin-EDTA (0.25%), DMSO, 96-well plates, and CyQuant reagents were purchased from ThermoFisher (USA).

**Cell Culture:** Culture HT29 cells with RPMI-1640 containing 10% fetal bovine serum under the condition of 37°C and 5% $CO_2$. Only cells in the logarithmic growth phase can be used for the test.

**Cell Proliferation Activity Test:** Use the CyQuant reagent to detect the proliferation inhibitory activity of compounds on HT29 cells. Adjust the cell density and inoculate 100 μL per well into the 96-well plate (HT29 at 2000 cells per well), then incubate overnight under the condition of 37°C and 5% $CO_2$. Add compounds of various target concentrations (initial concentration of 3000 nM, 3-fold dilution, 9 concentration gradients), and make the DMSO content 0.2%. Incubate the cell plates at 37°C and 5% $CO_2$ for 3 days. Incubate with CyQuant reagent for 1 h, read the plate by Envision, and calculate $IC_{50}$ using XLFIT.

**Table 3: HT-29 Cell Viability Biological Data for Compounds of the Present Invention**

| Examp le | HT-29 Cell Viability_IC50-nM | Examp le | HT-29 Cell Viability_IC50_ nM | Examp le | HT-29 Cell Viability_IC50_ nM |
|---|---|---|---|---|---|
| 1 | 629 | 37 | 161 | 73 | 63 |

(continued)

| Examp le | HT-29 Cell Viability_IC50-nM | Examp le | HT-29 Cell Viability_IC50_ nM | Examp le | HT-29 Cell Viability_IC50_ nM |
|---|---|---|---|---|---|
| 2 | 811 | 38 | NT | 74 | 127 |
| 3 | 210 | 39 | 1571 | 75 | NT |
| 4 | 77 | 40 | NT | 76 | 16 |
| 5 | NT | 41 | 14 | 77 | 12 |
| 6 | NT | 42 | 15 | 78 | >3000 |
| 7 | >3000 | 43 | 18 | 79 | 7 |
| 8 | 250 | 44 | 12 | 80 | 26 |
| 9 | 165 | 45 | 13 | 81 | 30 |
| 10 | 56 | 46 | 39 | 82 | 23 |
| 11 | 319 | 47 | NT | 83 | 16 |
| 12 | 568 | 48 | 14 | 84 | 25 |
| 13 | 444 | 49 | 31 | 85 | 34 |
| 14 | 88 | 50 | 245 | 86 | 21 |
| 15 | 60 | 51 | 22 | 87 | NT |
| 16 | 1523 | 52 | 299 | 88 | 52 |
| 17 | 337 | 53 | 47 | 89 | 40 |
| 18 | 26 | 54 | 73 | 90 | 62 |
| 19 | >3000 | 55 | 3 | 91 | 126 |
| 20 | 55 | 56 | 8 | 92 | 50 |
| 21 | >3000 | 57 | 4.5 | 93 | 83 |
| 22 | 23 | 58 | 286 | 94 | 57 |
| 23 | 118 | 59 | 34 | | |
| 24 | 20 | 60 | 41 | | |
| 25 | 122 | 61 | 34 | | |
| 26 | 1272 | 62 | 60 | | |
| 27 | 24587 | 63 | 153 | | |
| 28 | 192 | 64 | 27 | | |
| 29 | 45 | 65 | 127 | | |
| 30 | 61 | 66 | 54 | | |
| 31 | 11 | 67 | 19 | | |
| 32 | 251 | 68 | 250 | | |
| 33 | 65 | 69 | >3000 | | |
| 34 | 15 | 70 | 700 | | |
| 35 | 21 | 71 | 131 | | |
| 36 | 39 | 72 | 25 | | |
| NT means no test | | | | | |

**Example 4:** Experimental testing of cytotoxicity in human primary hepatocytes.

**Materials:** One donor for cytotoxicity testing. Information on human primary hepatocytes is provided in the table below.

| Donor | Race | Source |
|---|---|---|
| 1 | Caucasian | BioIVT |

Note: Hepatocytes of other species may be used, and specific information will be reflected in the test report.

1) CellTiter-Glo Luminescent Cell Viability Assay System was purchased from Promega (Madison, WI).

**Instruments:**

| Instruments | Supplier | Cat# or Model |
|---|---|---|
| 96 Plate | Corning | 354407 |
| Centrifuge | Eppendorf | 5804R&5810R |
| $CO_2$ incubator | Thermo Scientific | 371 |
| Cell counter | Nexcelom Bioscience LLC | Cellmeter K2 |
| Oscillator | IKA | MS3 BASIC |
| Microplate reader | TECAN | INFINITE 200 PRO |

**Experimental Design:**

Warm the following media to 37°C in a water bath: Prepare hepatocyte recovery medium, inoculation medium, and incubation medium according to the table below.

**Recovery Medium**

| Reagent | Initial Concentration | Final Concentration | Volume |
|---|---|---|---|
| Williams' Medium E | - | - | 31.2 mL |
| Isotonic Percoll | - | 30% | 13.5 mL |
| DPBS (10×) | - | - | 1.5 mL |
| GlutaMAX™-1 (100×) | 200 mM/100× | 2 mM | 500 μL |
| HEPES | 1 M | 15 mM | 750 μL |
| FBS | - | 5% | 2.5 mL |
| Human recombination insulin | 4 mg/mL | 4 μg/mL | 50 μL |
| Dexamethasone | 10 mM | 1 μM | 5 μL |

**Inoculation Medium**

| Reagent | Initial Concentration | Final Concentration | Volume |
|---|---|---|---|
| Williams' Medium E | - | - | 45.7 mL |
| FBS | - | 5% | 2.5 mL |

(continued)

| Reagent | Initial Concentration | Final Concentration | Volume |
|---|---|---|---|
| Dexamethasone | 10 mM | 1 μM | 5 μL |
| Penicillin-streptomycin mixed solution | Penicillin: 10,000 u/mL<br>Streptomycin: 10,000 μg/mL | Penicillin: 100 u/mL<br>Streptomycin: 100 μg/mL | 500 μL |
| Human recombination insulin | 4 mg/mL | 4 μg/mL | 50 μL |
| GlutaMAX™-1 (100×) | 200 mM/100× | 2 mM | 500 μL |
| HEPES | 1 M | 15 mM | 750 μL |

**Incubation Medium**

| Reagent | Initial Concentration | Final Concentration | Volume |
|---|---|---|---|
| Williams' Medium E | - | - | 48 mL |
| Dexamethasone | 10mM | 0.1 μM | 0.5 μL |
| ITS (100×) | - | - | 500 μL |
| Penicillin-streptomycin mixed solution | Penicillin: 10,000 u/mL<br>Streptomycin: 10,000 μg/mL | Penicillin: 50 u/mL<br>Streptomycin: 50 μg/mL | 250 μL |
| GlutaMAX™-1 (100×) | 200 mM/100× | 2 mM | 500 μL |
| HEPES | 1M | 15 mM | 750 μL |

1) Take one tube of cryopreserved hepatocytes and ensure that the hepatocytes are cryogenically frozen until recovery. Quickly place the hepatocytes in a 37°C water bath and gently shake until all ice crystals are completely dispersed, spray with 75% ethanol and transfer to a biosafety cabinet.

2) Transfer the contents of the hepatocyte tube (1 mL, approximately $5 \times 10^6$ cells) into a 50 mL centrifuge tube containing 50 mL of recovery medium and centrifuge at 100 g for 10 min. After centrifugation, aspirate the recovery medium and add sufficient inoculation medium to obtain a cell suspension with a cell density of approximately $1.0 \times 10^6$ cells/mL. Count hepatocytes and determine viable cell density with the cell counter Cellometer®. Make sure that the viability of hepatocytes is greater than 80%. Adjust the cell density to $0.2 \times 10^6$ cells/mL with inoculation medium, and inoculate into 96-well plates coated with collagen I at 100 μL per well. Incubate the plates at 37°C for 4-6 h in a 5% $CO_2$ incubator with 95% relative humidity.

3) Prepare a 200X stock solution of the test compound in DMSO (the stock concentration of the test compound can be reduced depending on solubility). Make the final concentration of DMSO 0.5%

4) Prepare the working solutions on sterile 96-well plates by adding 2.5 μL of the test compound stock solution to 497.5 μL of hepatocyte culture medium.

5) Remove the hepatocyte culture medium from the cell plate, and add 125 μL of working solution to the corresponding wells in triplicate. Incubate the plate at 37°C and 5% $CO_2$ for 72 h. After every 24 h of treatment, replace the medium in cell plates with freshly diluted test and positive control compounds from hepatocyte culture media. Return the plate to the incubator, and add 50 μL of CellTiter-Glo reagent directly into each well of the 96-well plate. Shake on a shaker for 10 min at ambient temperature. After 10 min, transfer 100 μL of the above incubation to a new white and opaque flat-bottom 96-well plate and record the fluorescence.

**Data Analysis:**

All calculations are performed using Microsoft Excel.

The viability of the test compound can be calculated using the following formula:

$$\% \text{Vehicle} = \frac{\text{Read}_{\text{Compound}} - \text{Read}_{\text{Blank}}}{\text{Read}_{\text{Vehicle}} - \text{Read}_{\text{Blank}}} \times 100\%$$

The % Vehicle is fitted to the concentration of the test compound, and then GraphPad Prism 5.0 is used to model the data as a sigmoidal dose-response curve with a variable slope. The $IC_{50}$ of this compound is calculated from the curve using the following formula

$$Y=Bottom + (Top-Bottom)/(1+10^((LogIC50-X)*HillSlope)).$$

**Example** 5: Experimental testing of the microsphere model for human primary hepatocytes

Cells: Donor information: Male, Caucasian, 36 years old (Lot No. NFX, BioIVT)

**Instruments:**

| Instruments | Brand | Cat. No./Model |
|---|---|---|
| 96-well ultra-low adsorption culture plate | Corning | 4515 |
| Centrifuge | Eppendorf | 5804R |
| Cell incubator | Thermo Scientific | 371 |
| Cell counter | Invitrogen | Countess II |
| Oscillator | IKA | MS 3 |
| Microplate reader | Tecan | Infinite M200 |

**Cell Inoculation and Culture:**

1) One tube of cryopreserved hepatocytes was taken, ensuring the hepatocytes were cryogenically frozen until recovery. The hepatocytes were quickly placed in a 37°C water bath and gently shaken until all ice crystals were completely dissolved. After spraying with 70% ethanol, the tube was transferred to a biosafety cabinet.

2) Transfer the contents of the hepatocyte tube into a 50 mL centrifuge tube containing 50 mL of recovery medium and centrifuge at 100 g for 3 min. After centrifugation, the recovery medium was aspirated, and sufficient incubation medium was added to obtain a cell suspension with a cell density of approximately $1.0 \times 10^6$ cells/mL.

3) Count hepatocytes and determine viable cell density with Cellometer Vision. Make sure that the viability of hepatocytes is greater than 80%. Adjust the cell density to 4000 cells/well with inoculation medium, and inoculate 100 μL per well.

4) Incubate in a cell incubator at 37°C for 7-9 days until microspheres are formed.

**Compound Preparation and Administration:**

1) Dilute the compound with the cell culture medium according to the three-fold dilution method, and the dilution gradient is shown in the table below. The maximum concentration of the control compound is 200 μM, the maximum concentration of the test compound (according to customer requirement) is 5 μM and the final concentration of DMSO is 0.1%.

2) Perform administration after the microspheres are formed. Aspirate the medium in the cell culture plate wells and add 100 μL of the compound working solution before each administration. Perform administration every three days until the fourteenth day after microsphere formation.

**Test Indicator Detection:**

1) After 14 days of culture, remove the cell culture plates from the incubator and transfer 80 μL of supernatant to determine the albumin and lactate dehydrogenase content.

2) Thaw CellTiter-Fluor™ Cell Viability Kit at ambient temperature, add 10 μL of GF-AFC substrate to 10 mL of 2× working solution, and dilute into 1× working solution with an equal volume of PBS.

3) Add 100 μL of working solution to each well of the cell culture plate and incubate at 37°C for 30 min, then pipette 80 μL of supernatant into a 96-well black plate and detect the absorbance at an excitation wavelength of 400 nm and an emission wavelength of 505 nm.

**Data Analysis:**

1) The cell viability (% Vehicle) is calculated using the following formula:

$$\% \text{Vehicle} = \frac{\text{Read}_{\text{Compound}} - \text{Read}_{\text{Blank}}}{\text{Read}_{\text{Vehicle}} - \text{Read}_{\text{Blank}}} \times 100\%$$

2) The $IC_{50}$ is calculated using GraphPad Prism 8.0.2 with the following software calculation formula:

$$Y=\text{Bottom} + (\text{Top}-\text{Bottom})/(1+10\text{^}((\text{LogIC}_{50}-X)*\text{HillSlope})).$$

## Claims

**1.** A compound having the structure of formula (I), or its pharmaceutically acceptable salts, stereoisomers, isotope isomers, prodrugs, hydrates, or solvates:

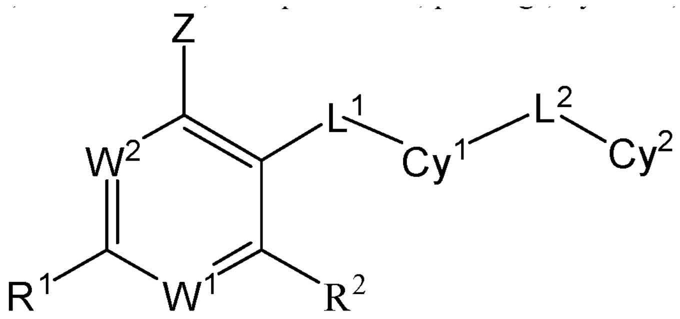

Formula (I)

wherein, $W^1$ represents $CR^{W1}$ or N;
wherein, $W^2$ represents $CR^{W2}$ or N;
wherein, Z represents $-CR^TR^{T'}$ or $-NR^SR^{S'}$;
wherein, $R^T$ and $R^{T'}$, together with the C atom they are attached to, form a ring having a structure selected from the following:

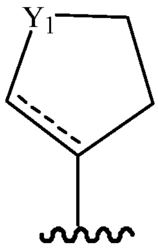,
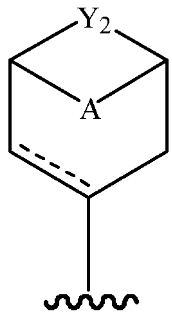 and/or
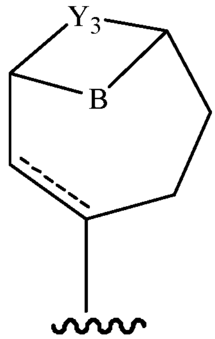;

wherein, $R^S$ and $R^{S'}$, together with the N atom they are attached to, form a ring having a structure selected from the following:

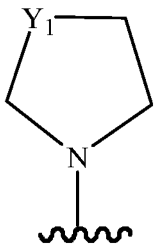,
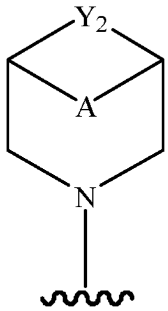 and/or
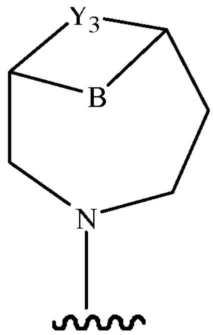

wherein, $Y^1$, $Y^2$ and $Y^3$ each independently represent $-(CR^aR^b)_o-(NR^a)_p-(CR^{a'}R^{b'})_q-$;
wherein, A and B each independently represent $-(CR^aR^b)m-$;
wherein, $L^1$ represents 5-6-membered heteroaryl,

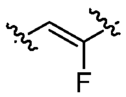,
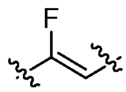,
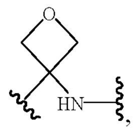,

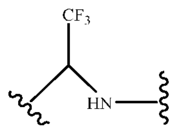,
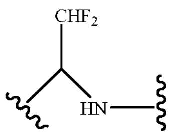 and
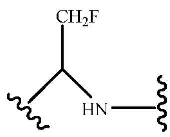;

wherein, $L^2$ represents absence, $-C_1-C_6$ alkylene-, $-NR^a-$, $-NR^a(C_1-C_6$ alkylene)-, $-C(O)NR^a(C_1-C_6$ alkylene)-, -O-, $-O-(C_1-C_6$ alkylene), -S-, -S(O)-, $-S(O)_2-$, $-S(O)_2NR^a-$ or $-S(O)(NR^a)-$;
wherein, $R^1$ represents $L^3-R^3$ or

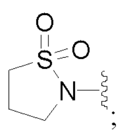;

wherein, $R^2$ represents hydrogen, halogen, cyano, nitro, hydroxy($C_1-C_6$ alkyl), $C_1-C_6$ alkyl, $C_3-C_8$ cycloalkyl, $C_1-C_6$ haloalkyl, $-OR^a$, $-SO_3R^a$, $-SR^a$, $-SF_5$, $-S(O)R^a$, -O-, $C_1-C_6$ haloalkyl or $-NR^aR^b$;
wherein, $L^3$ represents absence, $C_1-C_6$ alkylene, $-NR^a-$, $-NR^aSO_2-$, $-SO_2NR^a-$, $-S(=O)(NR^a)-$, $-P(O)(OR^a)_2$, $-NR^aP(O)(OR^a)_2$ or $-NR^aP(O)(R^a)_2$;
wherein, $R^3$ represents absence, hydrogen, or $C_1-C_6$ alkyl or $C_3-C_6$ cycloalkyl, wherein the $C_1-C_6$ alkyl or $C_3-C_6$ can be optionally independently substituted with 0-3 substituents selected from halogen, $-OR^a$, $-NR^aR^b$, cyano and $-O-C_1-C_6$ haloalkyl;

wherein, $R^{W1}$ and $R^{W2}$ each independently represent hydrogen, halogen, cyano, nitro, hydroxy($C_1$-$C_6$ alkyl), $C_1$-$C_6$ alkyl, $C_3$-$C_8$ cycloalkyl, $C_1$-$C_6$ haloalkyl, -$OR^a$, -$SO_3R^a$, -S(O)$R^a$, -O-, $C_1$-$C_6$ haloalkyl, -$SR^a$, -$SF_5$ or -$NR^aR^b$;

wherein, $Cy^1$ represents 6-12-membered aryl or 5-12-membered heteroaryl; the $Cy^1$ may be optionally substituted with 0-3 substituents selected from the following: halogen, $C_1$-$C_6$ alkyl, $C_1$-$C_6$ haloalkyl, hydroxy($C_1$-$C_6$ alkyl), $OR^a$, -O-($C_1$-$C_6$ haloalkyl), 5-6-membered heteroaryl, phenyl, -$SR^a$, -$SF_5$, cyano, nitro, -$NR^aR^b$, -NH-($C_0$-$C_6$ alkyl)-$R^M$, -$NR^aC(O)R^b$, -C(O)$NR^aR^b$, -OC(O)$R^a$, -C(O)$R^a$, -P(O)$R^aR^b$, -C(O)$OR^a$, -S(O)$R^a$, -$S(O)_2R^a$ and -$S(O)_2NR^aR^b$;

wherein, $Cy^2$ represents a 3-12-membered saturated or unsaturated monocyclic or bicyclic ring which may optionally contain 0-3 heteroatoms selected from O, N and S; the $Cy^2$ may be optionally substituted with 0-3 substituents selected from the following: halogen, $C_1$-$C_6$ haloalkyl, $C_1$-$C_6$ haloalkyl, hydroxy($C_1$-$C_6$ alkyl), -$OR^a$, -O-($C_1$-$C_6$ haloalkyl), -$SR^a$, -$SF_5$, cyano, nitro, -$NR^aR^b$, -$NR^aC(O)R^b$, -C(O)$NR^aR^b$, -OC(O)$R^a$, -C(O)$OR^a$, -S(O)$R^a$, -$S(O)_2R^a$ and -$S(O)_2NR^aR^b$;

wherein, $R^M$ represents -$OR^a$, -$NR^aR^b$, 5-6-membered heterocyclyl, 5-10-membered heteroaryl and 6-10-membered aryl;

wherein, $R^a$, $R^b$, $R^{a'}$ and $R^{b'}$ each independently represent hydrogen, halogen, $C_1$-$C_6$ alkyl, $C_3$-$C_6$ cycloalkyl, $C_1$-$C_6$ haloalkyl or hydroxy($C_1$-$C_6$ alkyl); OR $R^a$ and $R^b$, together with the atom they are attached to, form a 3-6-membered saturated or unsaturated ring which may optionally contain 0-2 heteroatoms selected from O, S and N; OR $R^{a'}$ and $R^{b'}$, together with the atom they are attached to, form a 3-6-membered saturated or unsaturated ring which may optionally contain 0-2 heteroatoms selected from O, S and N;

wherein, the dashed line represents a single or double bond;

wherein, m, o, p and q independently represent integers from 0 to 3.

**2.** A compound according to claim 1 or its pharmaceutically acceptable salts, stereoisomers, isotope isomers, prodrugs, hydrates, or solvates, wherein $W^1$ represents CH or N.

**3.** A compound according to claim 1 or claim 2, or its pharmaceutically acceptable salts, stereoisomers, isotope isomers, prodrugs, hydrates, or solvates, wherein $W^2$ represents CH or N.

**4.** A compound according to any one of claims 1-3 or its pharmaceutically acceptable salts, stereoisomers, isotope isomers, prodrugs, hydrates, or solvates, wherein $L^3$ represents -$NR^aSO_2$-, -$SO_2NR^a$- or -S(=O)($NR^a$)-.

**5.** A compound according to any one of claims 1-4 or its pharmaceutically acceptable salts, stereoisomers, isotope isomers, prodrugs, hydrates, or solvates, wherein $L^3$ represents -$NR^aSO_2$-.

**6.** A compound according to any one of claims 1-5 or its pharmaceutically acceptable salts, stereoisomers, isotope isomers, prodrugs, hydrates, or solvates, wherein $R^3$ represents hydrogen, or $C_1$-$C_6$ alkyl or $C_3$-$C_6$ cycloalkyl substituted with 0-3 substituents selected from halogen, -$OR^a$, -$NR^aR^b$, cyano and -O- $C_1$-$C_6$ haloalkyl.

**7.** A compound according to any one of claims 1-6 or its pharmaceutically acceptable salts, stereoisomers, isotope isomers, prodrugs, hydrates, or solvates, wherein $R^3$ represents $C_1$-$C_6$ alkyl substituted with 0-3 substituents selected from halogen, -ORa, -NRaRb, cyano and -O-$C_1$-$C_6$ haloalkyl.

**8.** A compound according to any one of claims 1-7 or its pharmaceutically acceptable salts, stereoisomers, isotope isomers, prodrugs, hydrates, or solvates, wherein Z represents -$NR^SR^{S'}$.

**9.** A compound according to any one of claims 1-8 or its pharmaceutically acceptable salts, stereoisomers, isotope isomers, prodrugs, hydrates, or solvates, wherein Z represents:

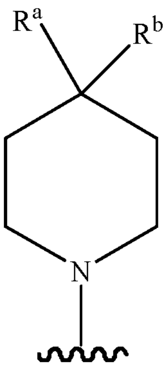 , 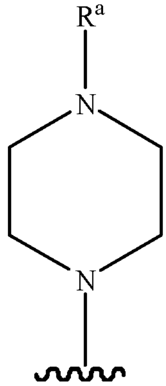 , 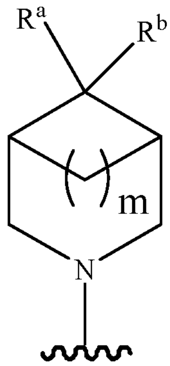 and/or 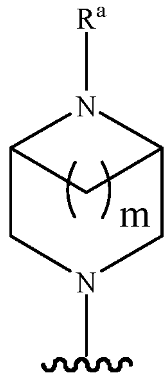 .

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy($C_1$-$C_6$ alkyl); OR $R^a$ and $R^b$, together with the atom they are attached to, form a 3-6-membered saturated or unsaturated ring which may optionally contain 0-2 heteroatoms selected from O, S and N.

**10.** A compound according to any one of claims 1-9 or its pharmaceutically acceptable salts, stereoisomers, isotope isomers, prodrugs, hydrates, or solvates, wherein, Z represents:

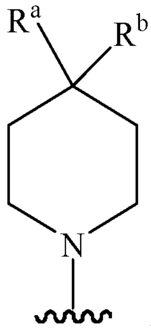 ,

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N.

**11.** A compound according to any one of claims 1-10 or its pharmaceutically acceptable salts, stereoisomers, isotope isomers, prodrugs, hydrates, or solvates, wherein Z represents:

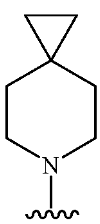 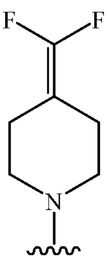 .

**12.** A compound according to any one of claims 1-11 or its pharmaceutically acceptable salts, stereoisomers, isotope isomers, prodrugs, hydrates, or solvates, wherein $L^1$ represents 5-6-membered heteroaryl,

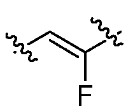 , 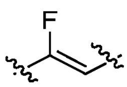 , 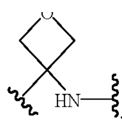 ,

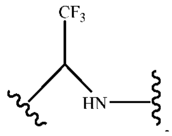 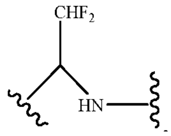 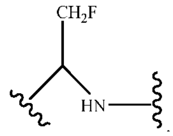

13. A compound according to claim 12 or its pharmaceutically acceptable salts, stereoisomers, isotope isomers, prodrugs, hydrates, or solvates, wherein, $Cy^1$ represents any one of the following groups substituted with 0-3 substituents selected from halogen, $C_1$-$C_6$ alkyl, $C_1$-$C_6$ haloalkyl, hydroxy $C_1$-$C_6$ alkyl, $OR^a$, -O- $C_1$-$C_6$ haloalkyl, 5-6 membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$, and $-S(O)_2NR^aR^b$:

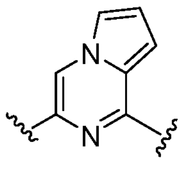 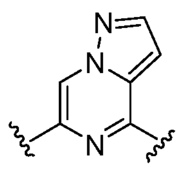 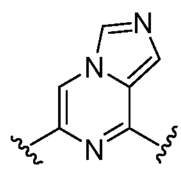 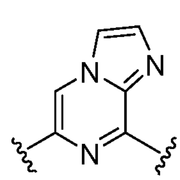

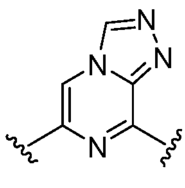 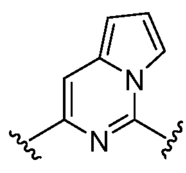 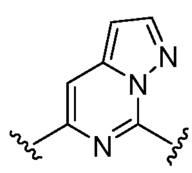 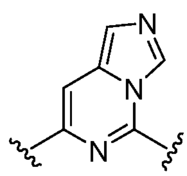

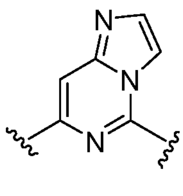 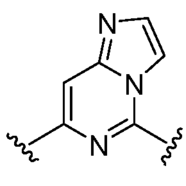 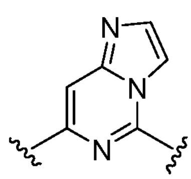 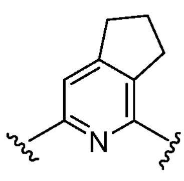

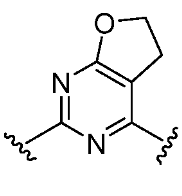 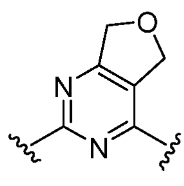 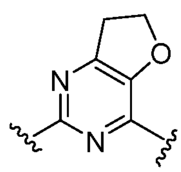 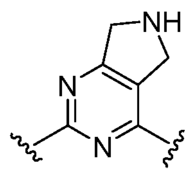

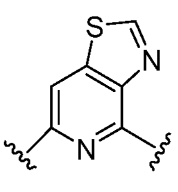 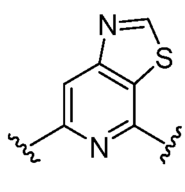 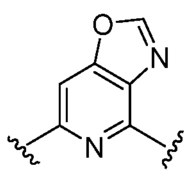 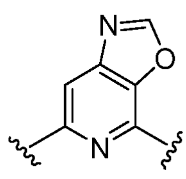

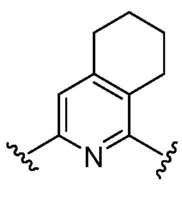 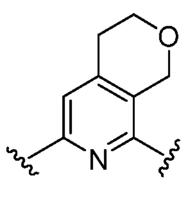 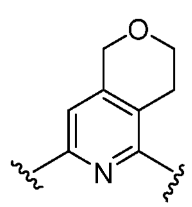 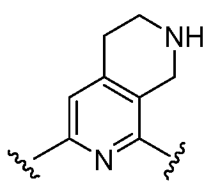

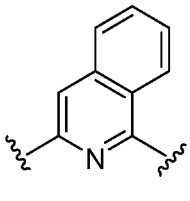 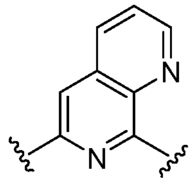 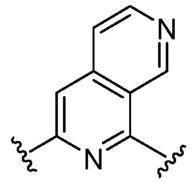 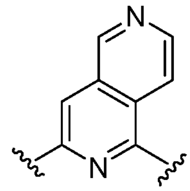

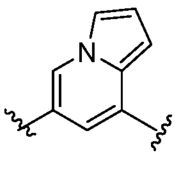 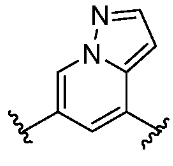 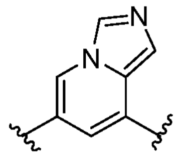 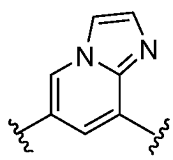

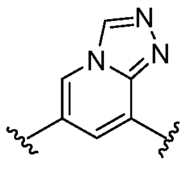 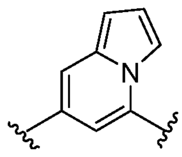 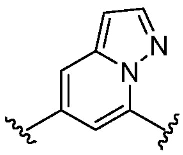 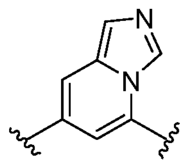

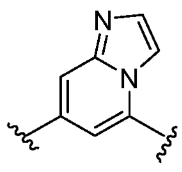 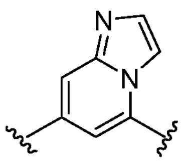 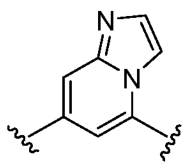 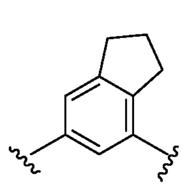

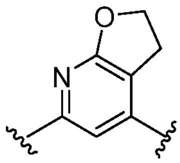 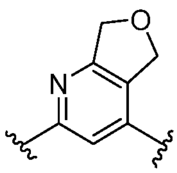 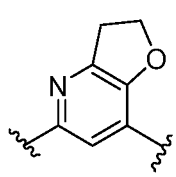 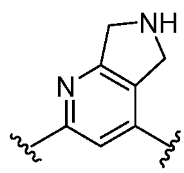

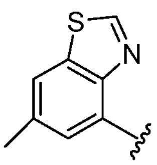 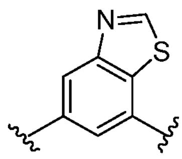 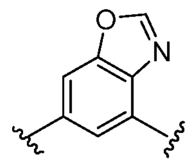 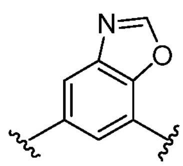

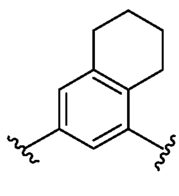 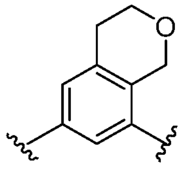 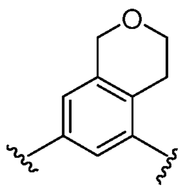 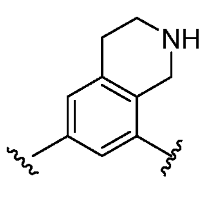

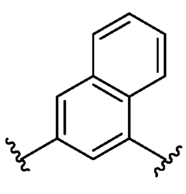 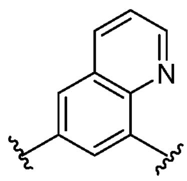 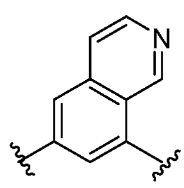 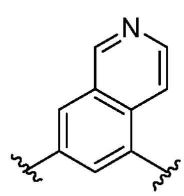

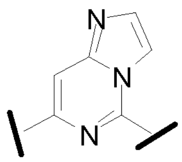 、 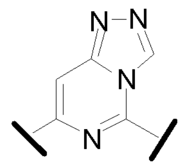 、 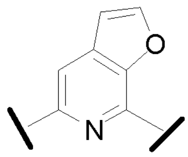 、 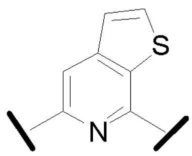

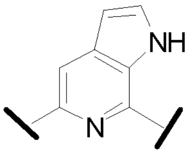 、 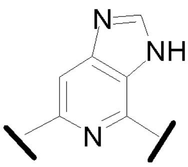 、 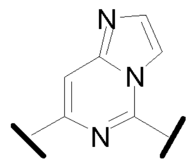 、 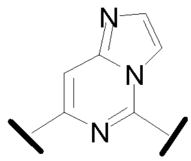

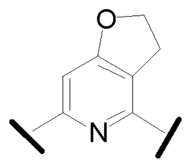 、 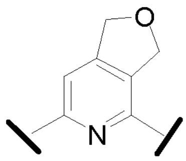 、 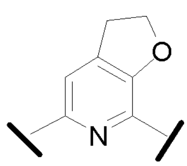 、 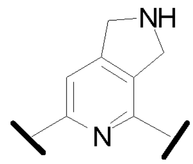

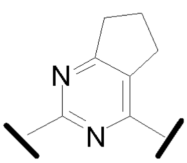 、 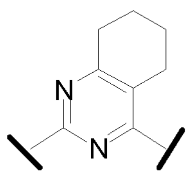 、 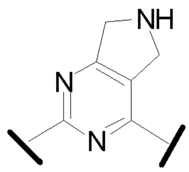 、 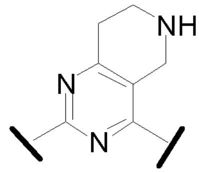

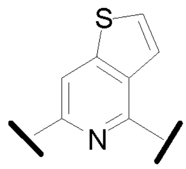 、 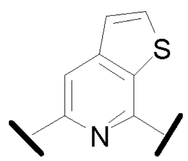 、 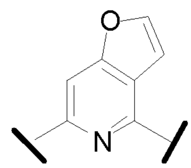 、 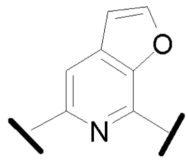

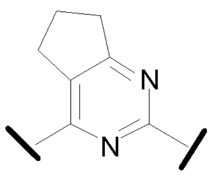 、 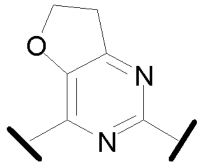 、 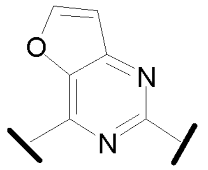 、 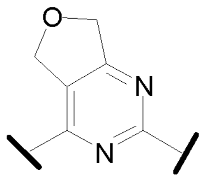

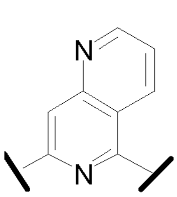 、 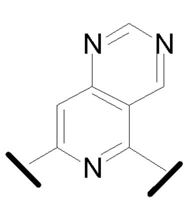 、 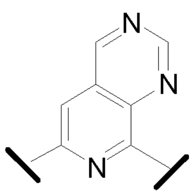 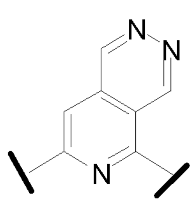

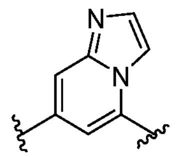 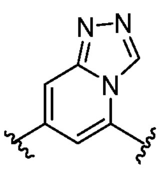 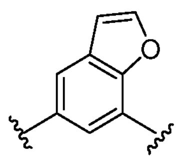 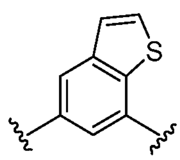

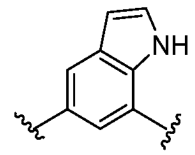
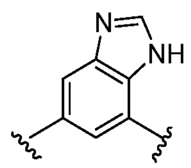
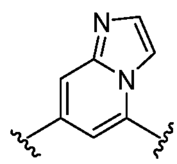
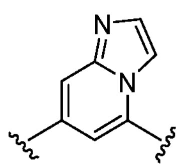
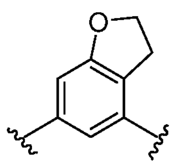
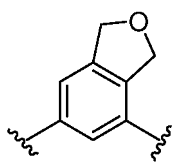
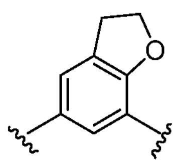
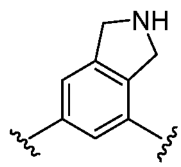
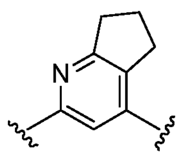
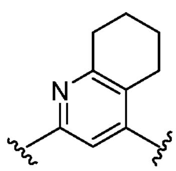
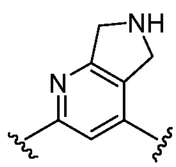
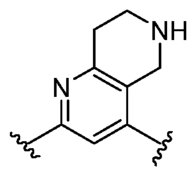
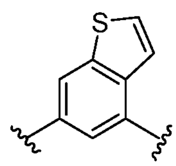
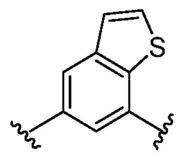
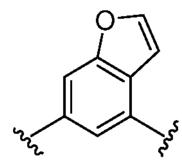
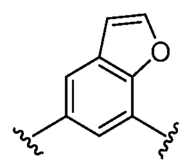
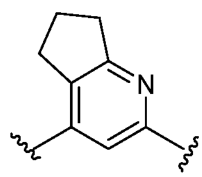
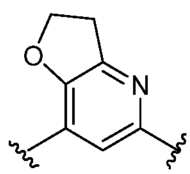
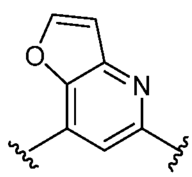
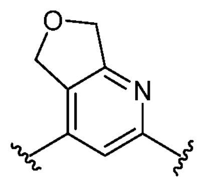
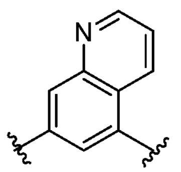
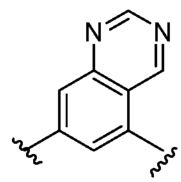
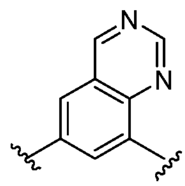
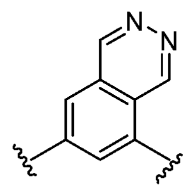
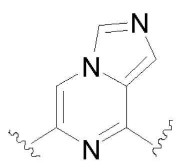
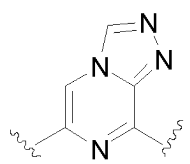
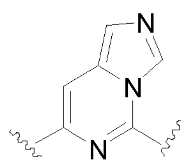
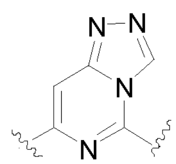
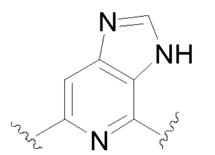
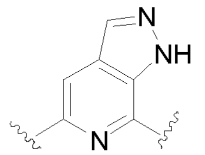
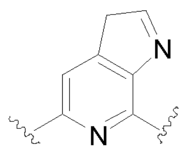
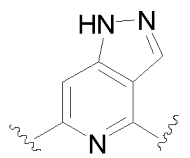

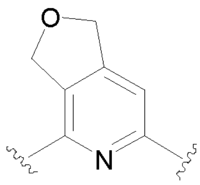 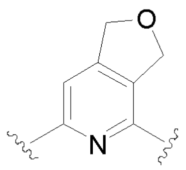 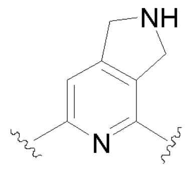 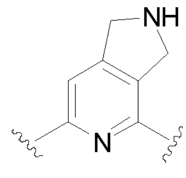

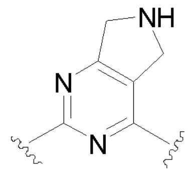 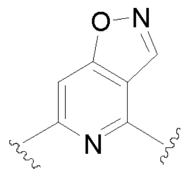 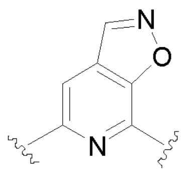 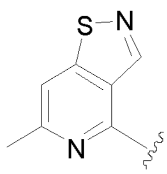

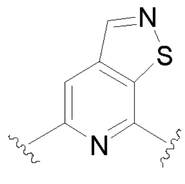 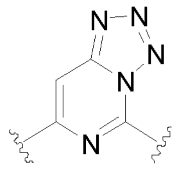 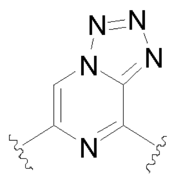 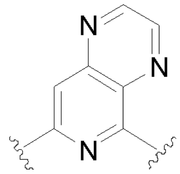

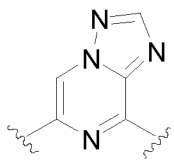 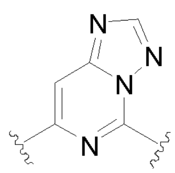 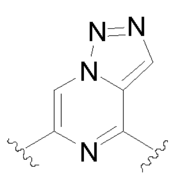 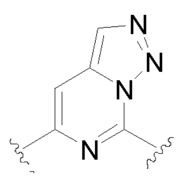

and/or

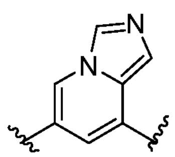 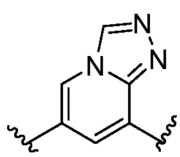 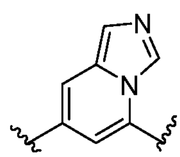 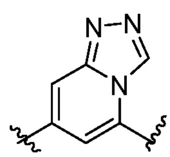

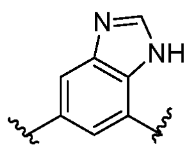 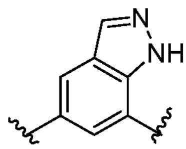 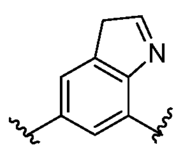 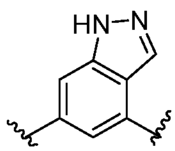

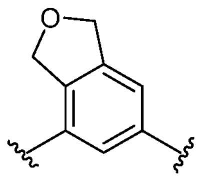 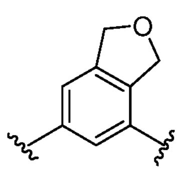 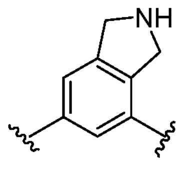 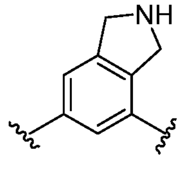

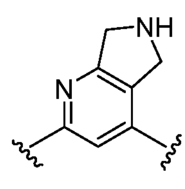 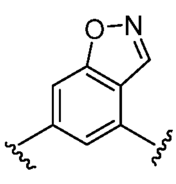 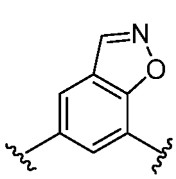 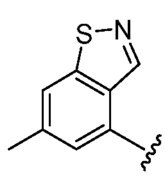

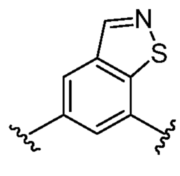 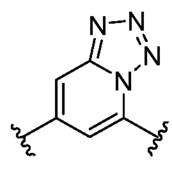 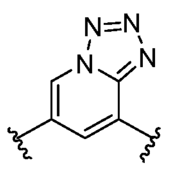 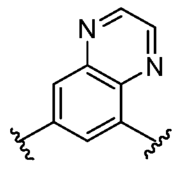

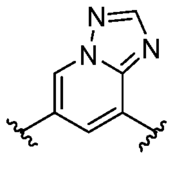 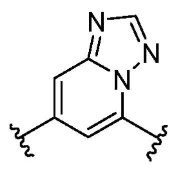 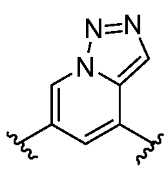 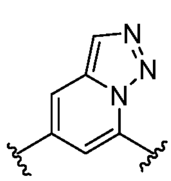

For example:

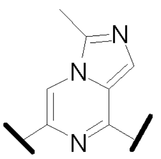 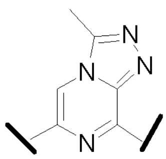 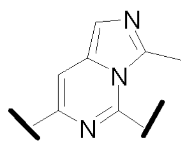 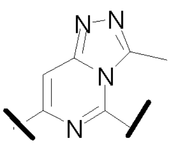

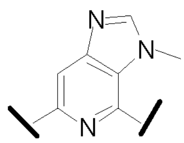 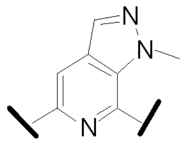 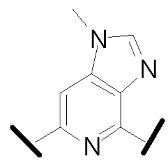 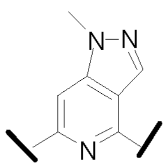

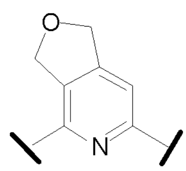 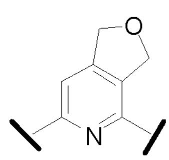 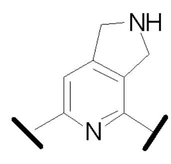 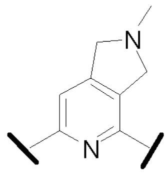

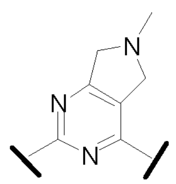 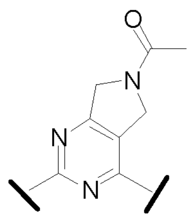 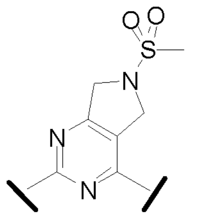 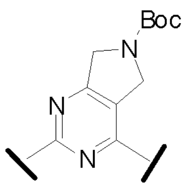

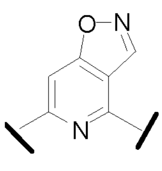 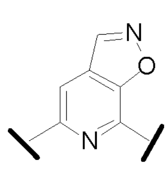 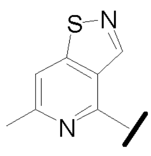 and/or 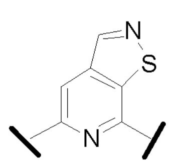

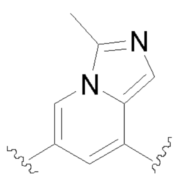 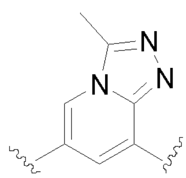 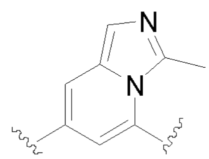 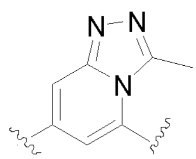

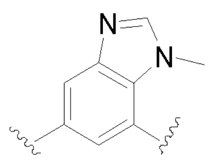
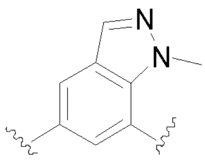
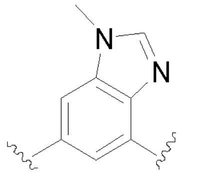
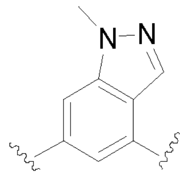

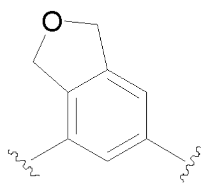
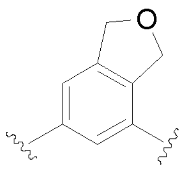
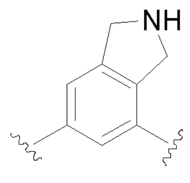
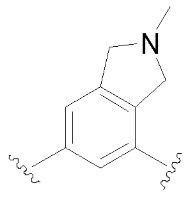

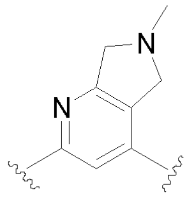
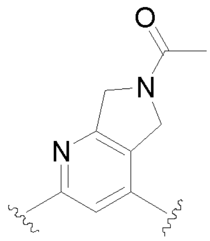
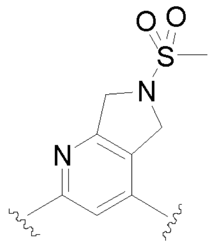
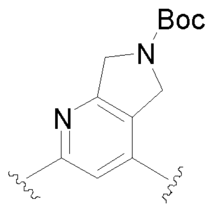

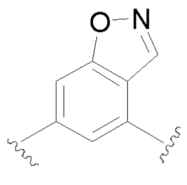
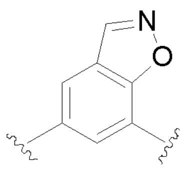
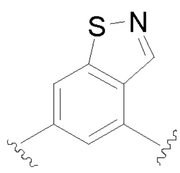
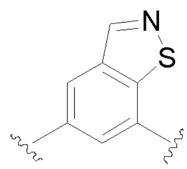
;

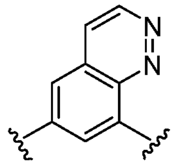
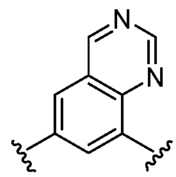
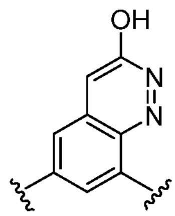
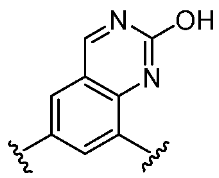

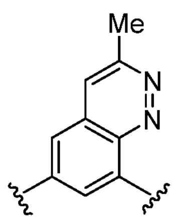
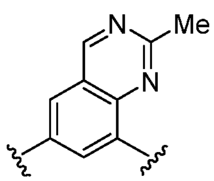
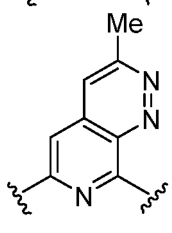
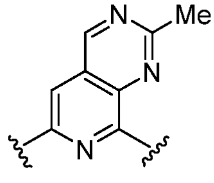

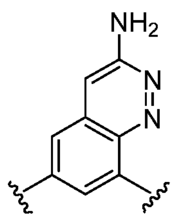
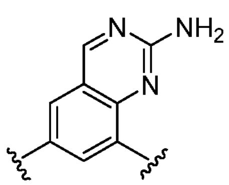
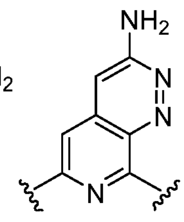
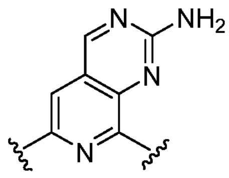

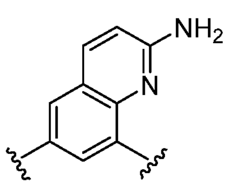
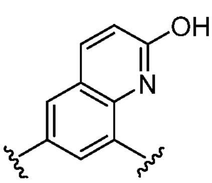
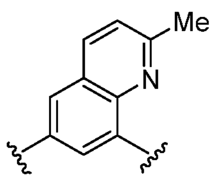
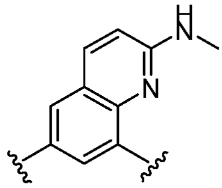

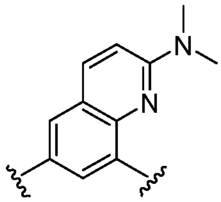
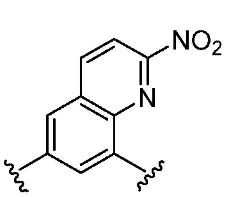
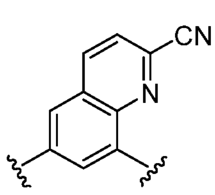
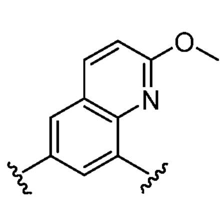

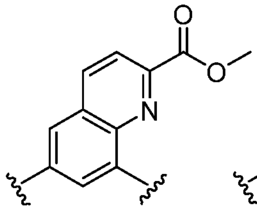
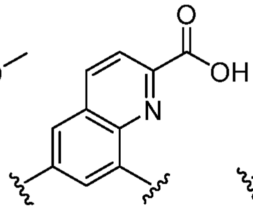
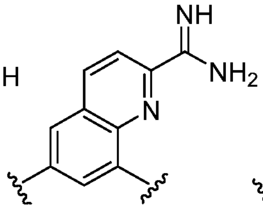
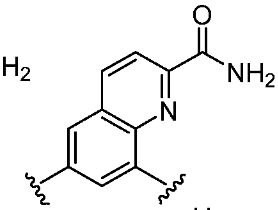

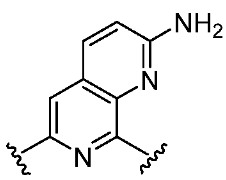
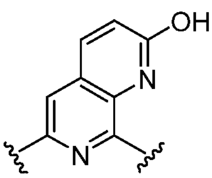
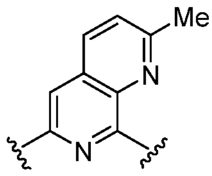
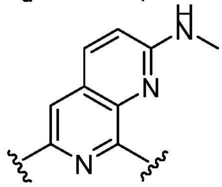

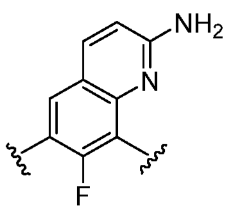
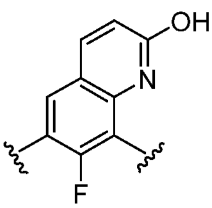
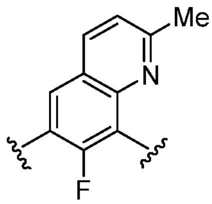
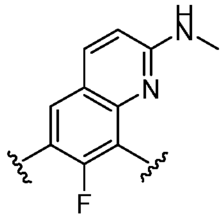

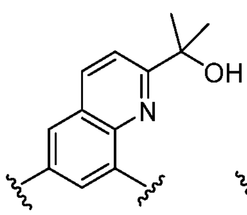
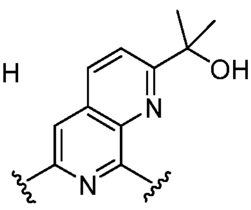
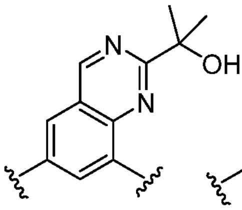
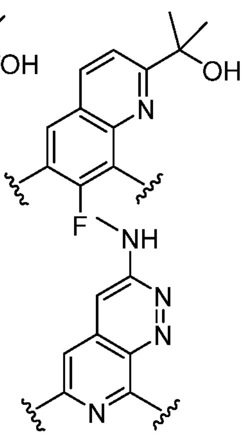

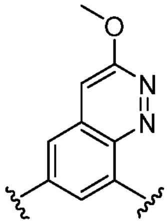
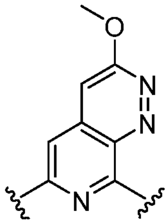
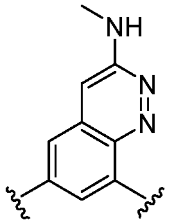

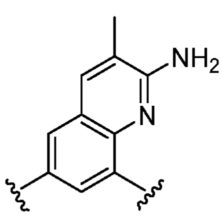
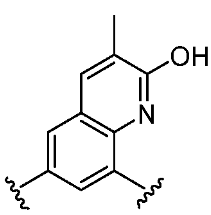
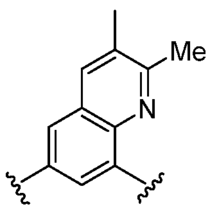
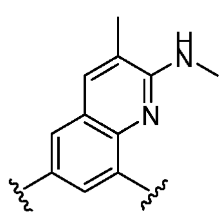

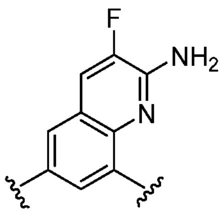 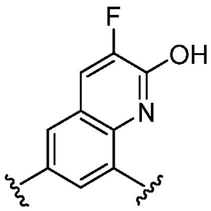 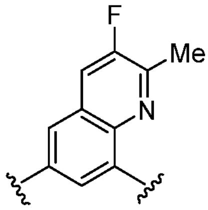 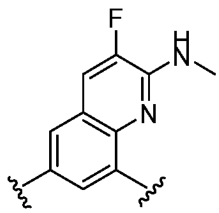

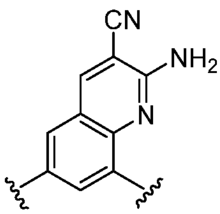 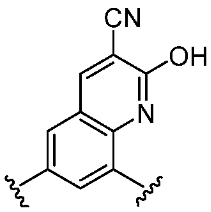 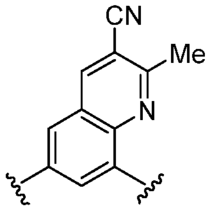 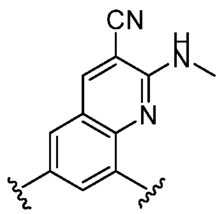

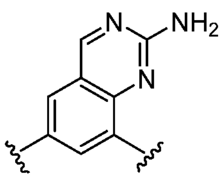 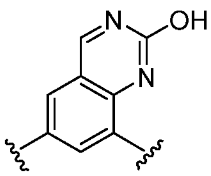 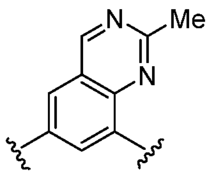 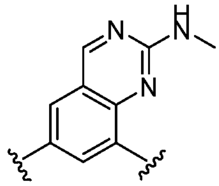

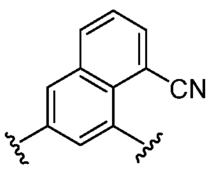 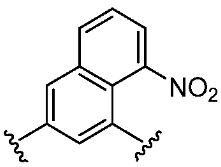 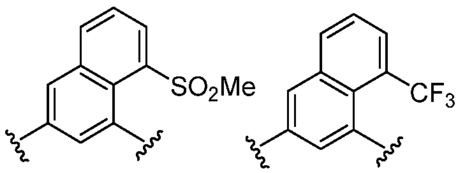

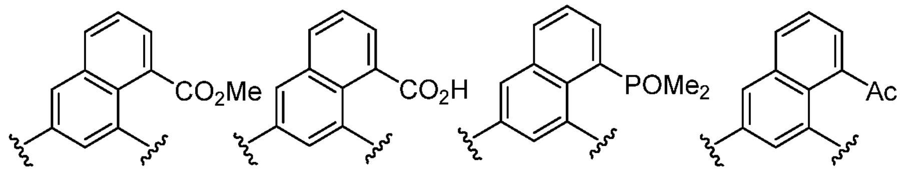

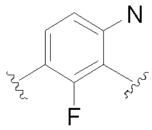 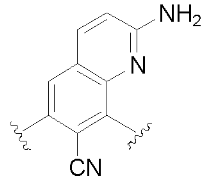 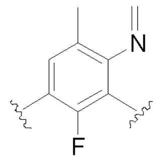 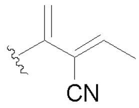

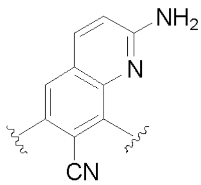 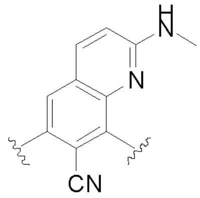 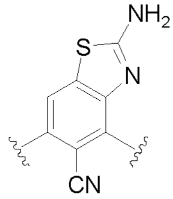 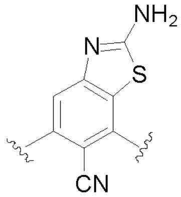

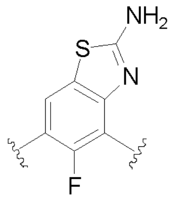 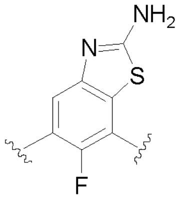 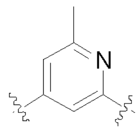 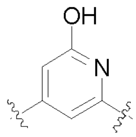

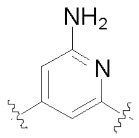 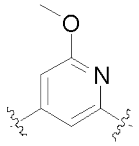 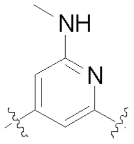 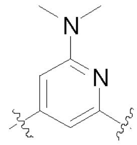

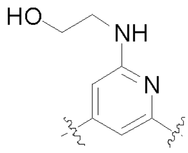 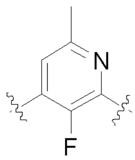 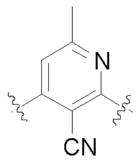 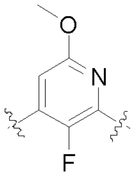

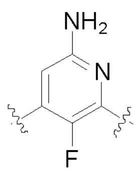 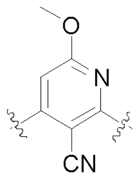 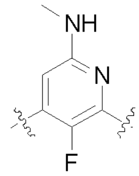 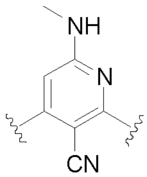

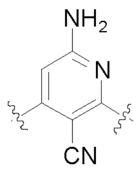 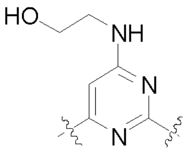 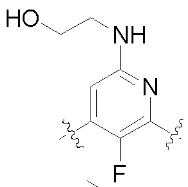 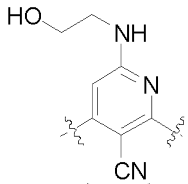

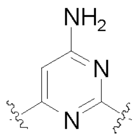 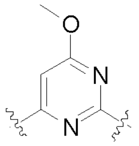 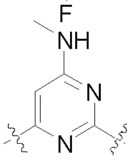 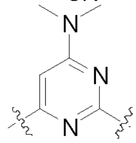 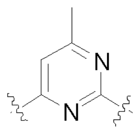 .

**14.** A compound according to any one of claims 1-13 or its pharmaceutically acceptable salts, stereoisomers, isotope isomers, prodrugs, hydrates, or solvates, wherein $L^1$ represents: 5-6-membered heteroaryl,

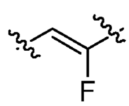 , 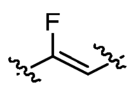 ,

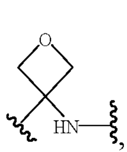, 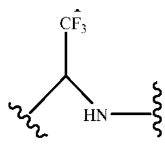, 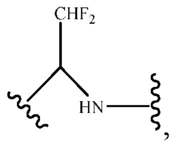, 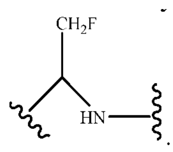.

**15.** A compound according to claim 14 or its pharmaceutically acceptable salts, stereoisomers, isotope isomers, prodrugs, hydrates, or solvates, wherein $L^1$ represents any one of the following groups:

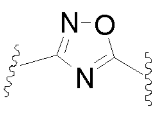、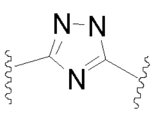、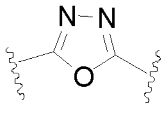、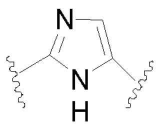、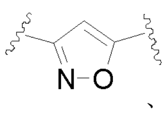、

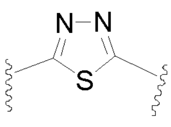、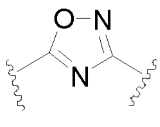、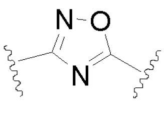、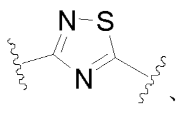、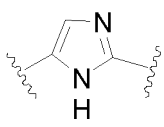

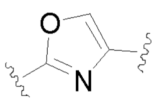 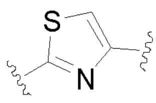 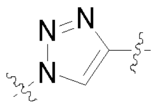 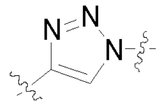 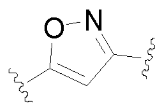

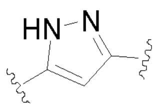 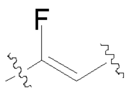 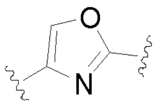 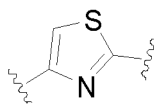 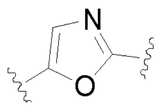

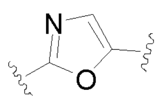 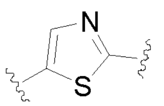 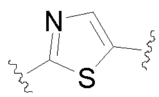 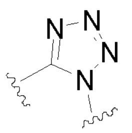 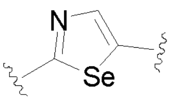

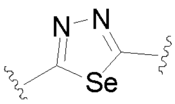 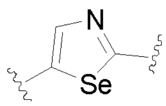 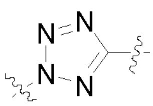 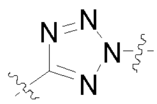 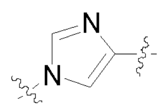

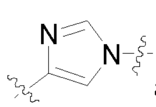 and/or 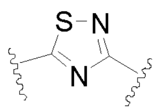

**16.** A compound according to claim 14 or claim 15, or its pharmaceutically acceptable salts, stereoisomers, isotope isomers, prodrugs, hydrates, or solvates, wherein, $Cy^1$ represents any one of the following groups substituted with 0-3 substituents selected from halogen, $C_1$-$C_6$ alkyl, $C_1$-$C_6$ haloalkyl, hydroxy $C_1$-$C_6$ alkyl, $OR^a$, $-O-C_1$-$C_6$ haloalkyl, 5-6-membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$, and $-S(O)_2NR^aR^b$:

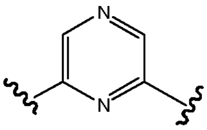 , 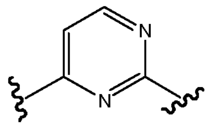 , 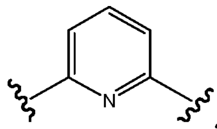 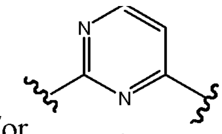 and/or

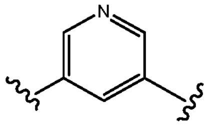 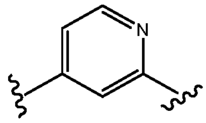 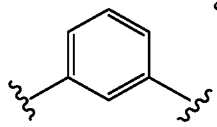 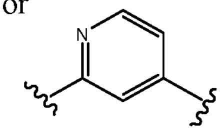 .

**17.** A compound according to claim 16 or its pharmaceutically acceptable salts, stereoisomers, isotope isomers, prodrugs, hydrates, or solvates, wherein, $Cy^1$ represents

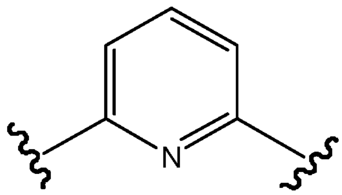

substituted with 0-3 substituents selected from halogen, $C_1$-$C_6$ alkyl, $C_1$-$C_6$ haloalkyl, hydroxy $C_1$-$C_6$ alkyl, $OR^a$, -O-$C_1$-$C_6$ haloalkyl, 5-6-membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$, and $-S(O)_2NR^aR^b$.

**18.** A compound according to claim 17 or its pharmaceutically acceptable salts, stereoisomers, isotope isomers, prodrugs, hydrates, or solvates, wherein $Cy^1$ represents

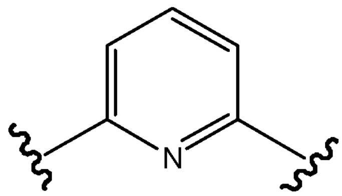

substituted with phenyl, pyridinyl, thiazolyl, oxazolyl, pyrazolyl, imidazolyl, and N-methylpyrazolyl.

**19.** A compound according to claim 18 or its pharmaceutically acceptable salts, stereoisomers, isotope isomers, prodrugs, hydrates, or solvates, wherein $Cy^1$ represents the following groups:

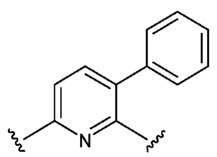 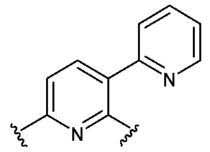 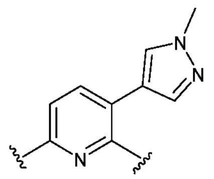

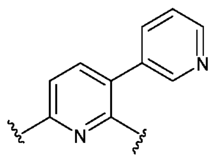 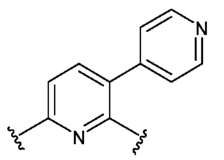 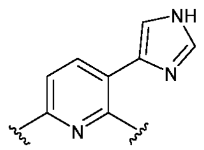

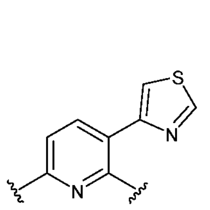 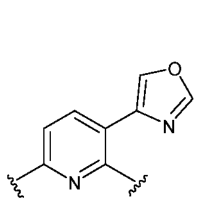 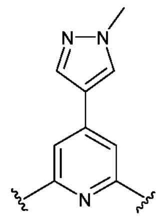 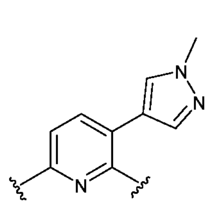

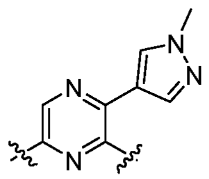 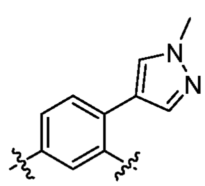 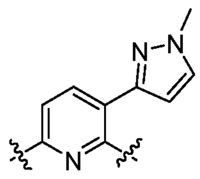 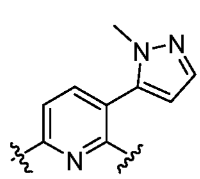

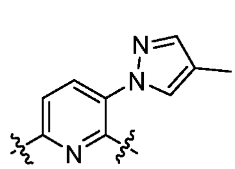 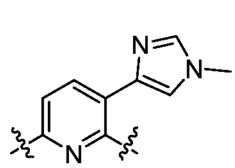 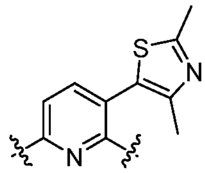 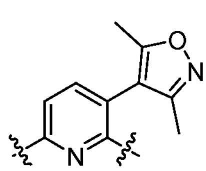

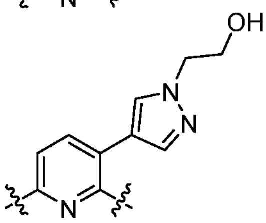 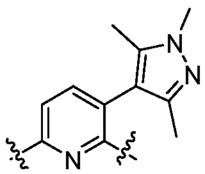 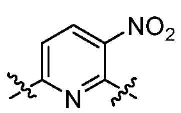 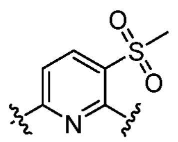

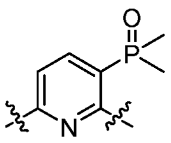 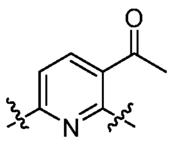 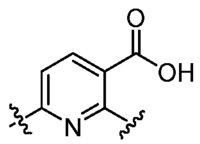 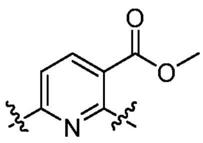

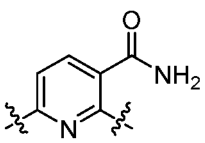 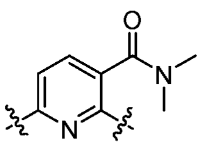 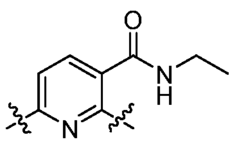 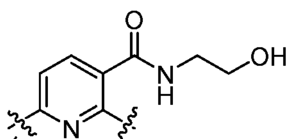

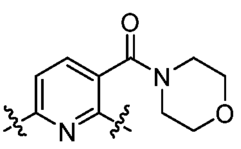 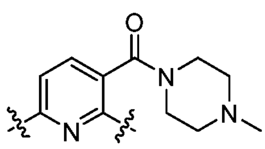 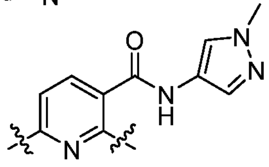 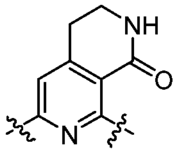

.

**20.** A compound according to any one of claims 1-19 or its pharmaceutically acceptable salts, stereoisomers, isotope isomers, prodrugs, hydrates, or solvates, wherein $L^2$ represents absence, $-C_1-C_6$ alkylene- or -NH-.

**21.** A compound according to any one of claims 1-20 or its pharmaceutically acceptable salts, stereoisomers, isotope isomers, prodrugs, hydrates, or solvates, wherein $L^2$ represents absence.

**22.** A compound according to any one of claims 1-21 or its pharmaceutically acceptable salts, stereoisomers, isotope isomers, prodrugs, hydrates, or solvates, wherein $Cy^2$ represents a saturated, partially saturated or unsaturated 3-, 4-, 5-, 6-, 7-membered monocyclic ring, or a 3-, 4-, 5-, 6-, 7-membered fused ring, which contains 0-3 N heteroatoms and 0-2 O or S heteroatoms and is substituted with 0-3 substituents selected from the following: halogen, cyano, nitro, hydroxy $C_1$-$C_6$ alkyl, $C_1$-$C_6$ alkyl, $C_3$-$C_8$ cycloalkyl, $C_1$-$C_6$ haloalkyl, $-OR^a$, $-SO_3R^a$, $-S(O)R^a$, -O $C_1$-$C_6$ haloalkyl, $-SR^a$, $-SF_5$ or $NR^aR^b$-substituted morpholinyl, piperidinyl, azetidine, pyrrolidinyl, cyclopropyl, cyclobutyl, cyclopentyl, cyclohexyl, piperazinyl or THF; wherein, $R^a$ and $R^b$ each independently represent hydrogen, halogen, $C_1$-$C_6$ alkyl,

$C_3$-$C_8$ cycloalkyl, $C_1$-$C_6$ haloalkyl or hydroxy $C_1$-$C_6$ alkyl; or $R^a$ and $R^b$, together with the atom they are attached to, form a 3-6 membered ring which contains 0-2 heteroatoms selected from O, N and S.

**23.** A compound according to any one of claims 1-22 or its pharmaceutically acceptable salts, stereoisomers, isotope isomers, prodrugs, hydrates, or solvates, wherein $Cy^2$ represents

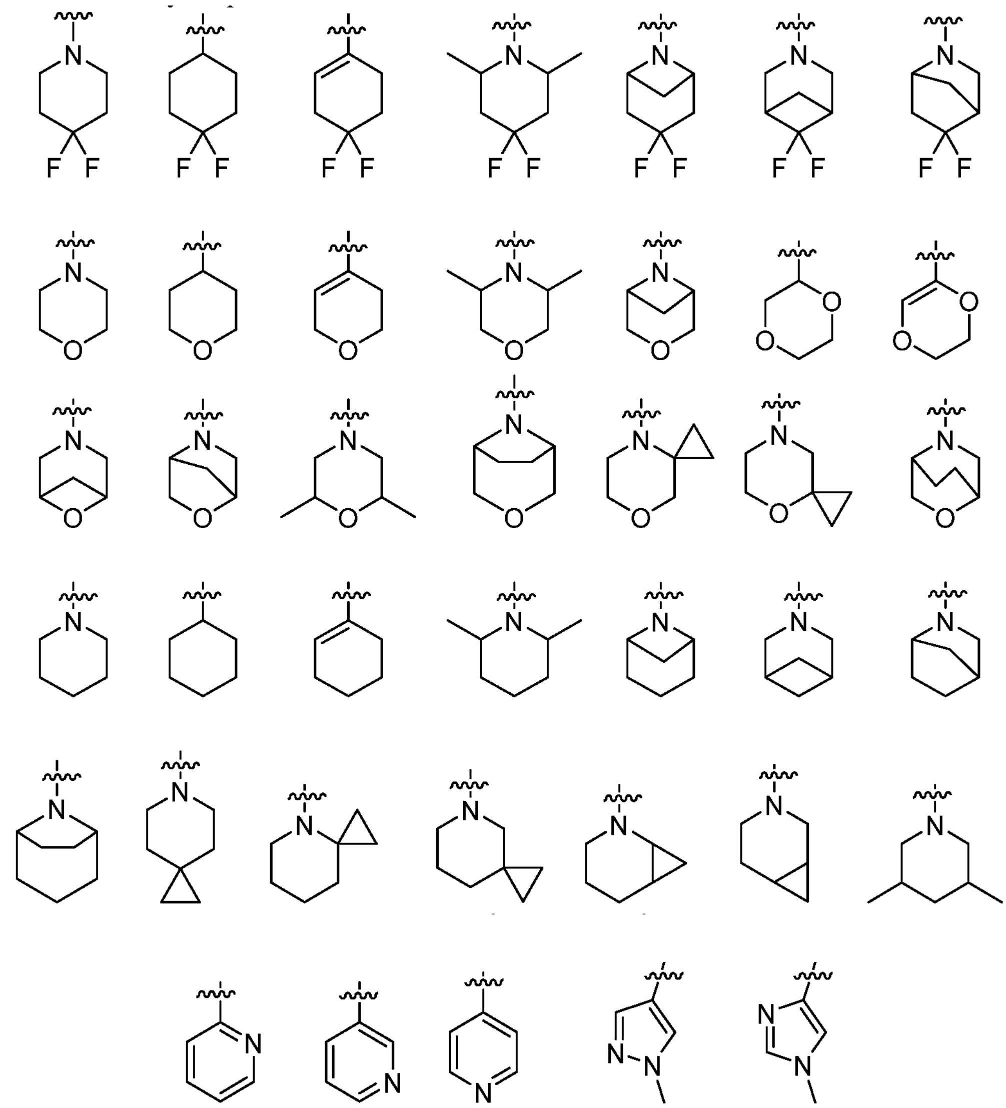

**24.** Compounds with the following structures:

| | | | |
|---|---|---|---|
| 1 | 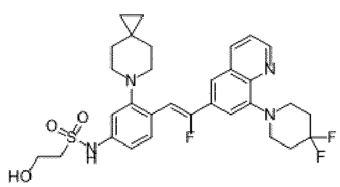 | 2 | 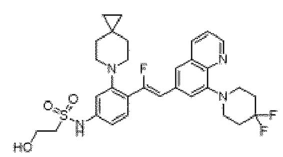 |
| 3 | 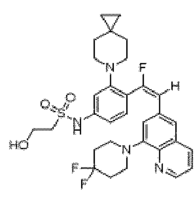 | 4 | 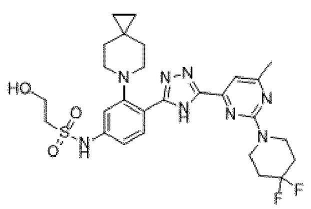 |

(continued)

| | | | |
|---|---|---|---|
| 5 | 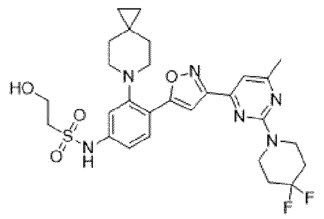 | 6 | 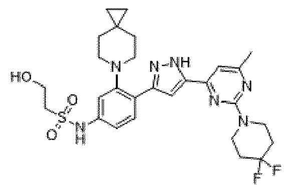 |
| 7 | 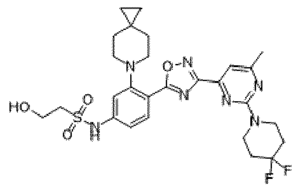 | 8 | 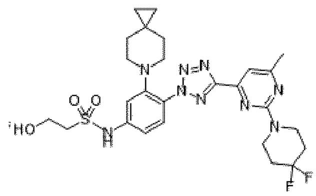 |
| 9 | 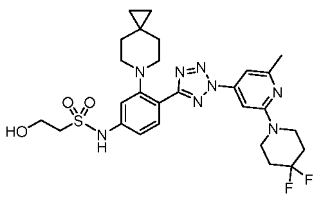 | 10 | 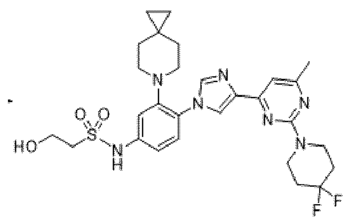 |
| 11 | 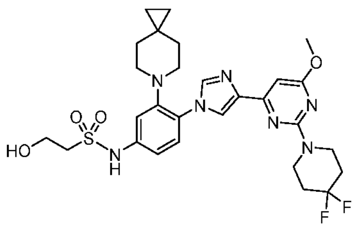 | 12 | 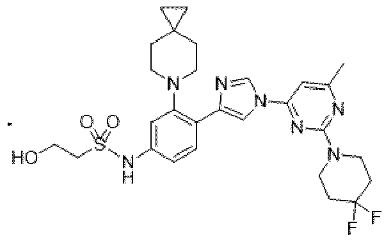 |
| 13 | 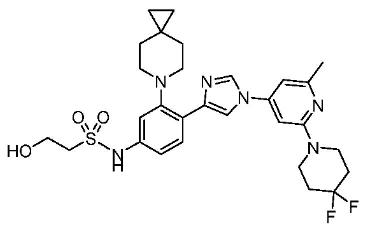 | 14 | 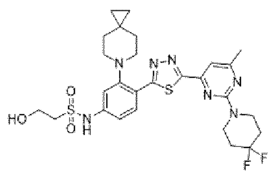 |
| 15 | 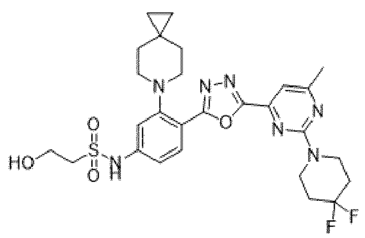 | 16 | 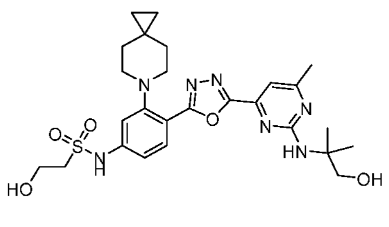 |
| 17 | 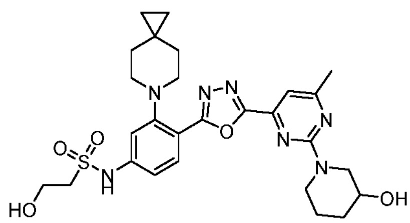 | 18 | 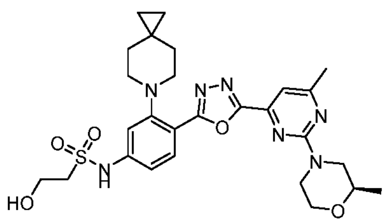 |

(continued)

| | | | |
|---|---|---|---|
| 19 | 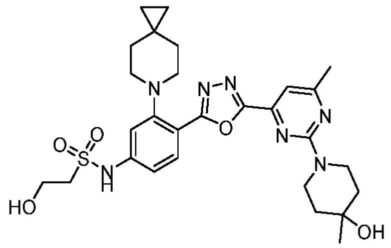 | 20 | 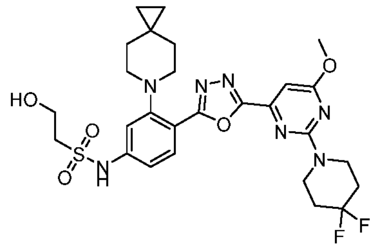 |
| 21 | 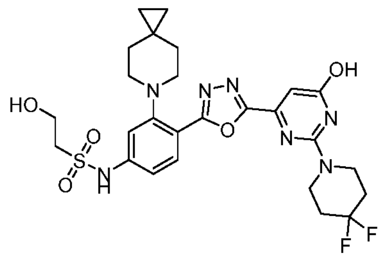 | 22 | 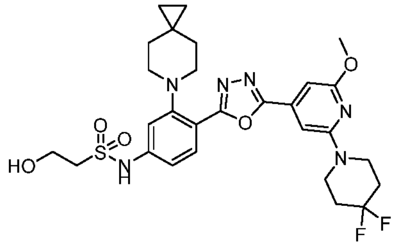 |
| 23 | 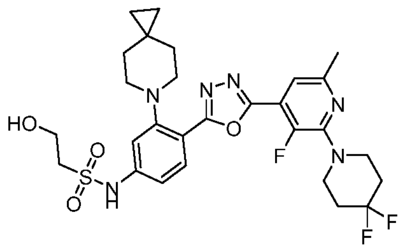 | 24 | 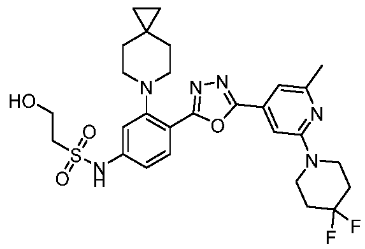 |
| 25 | 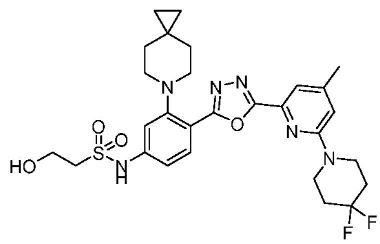 | 26 | 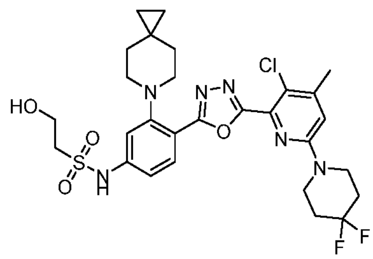 |
| 27 | 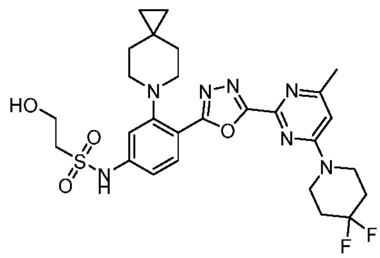 | 28 | 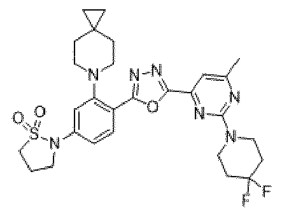 |
| 29 | 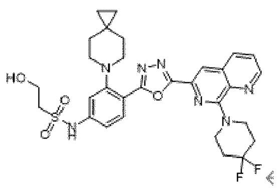 | 30 | 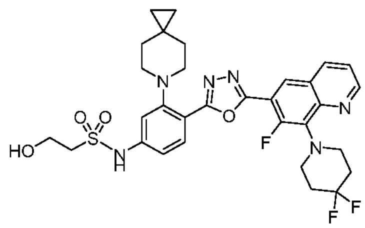 |

(continued)

| | | | |
|---|---|---|---|
| 31 | 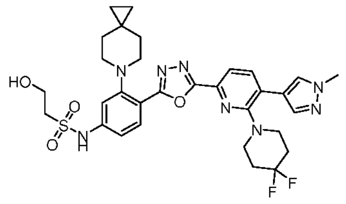 | 32 | 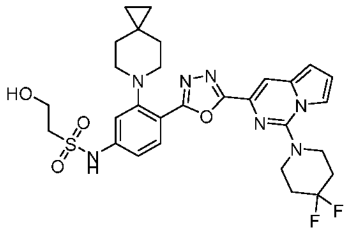 |
| 33 | 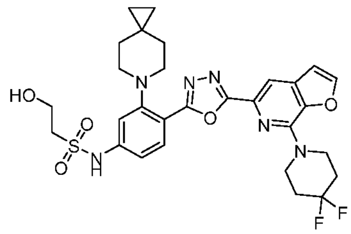 | 34 | 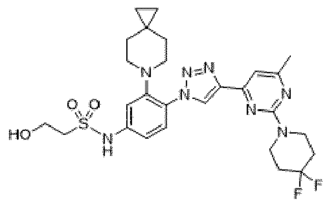 |
| 35 | 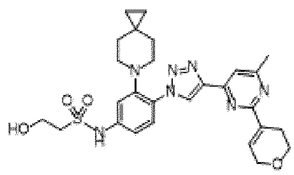 | 36 | 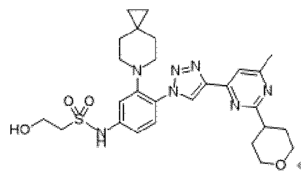 |
| 37 | 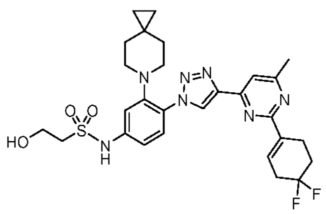 | 38 | 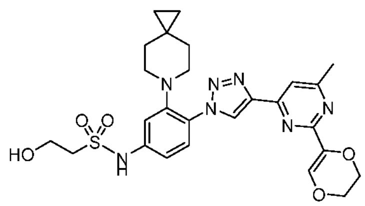 |
| 39 | 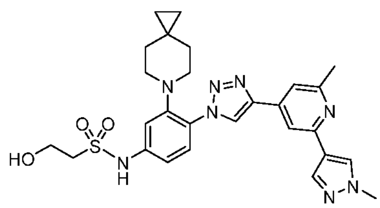 | 40 | 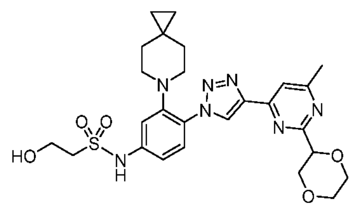 |
| 41 | 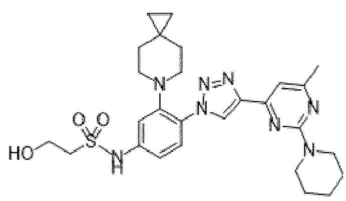 | 42 | 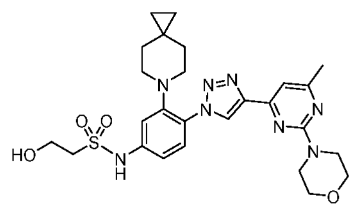 |
| 43 | 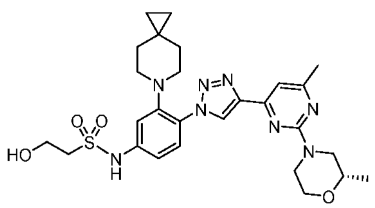 | 44 | 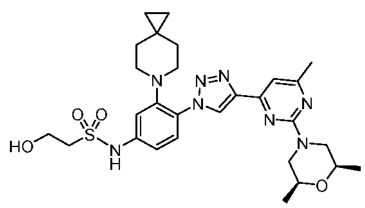 |

(continued)

| | | | |
|---|---|---|---|
| 45 | 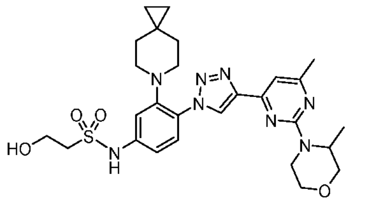 | 46 | 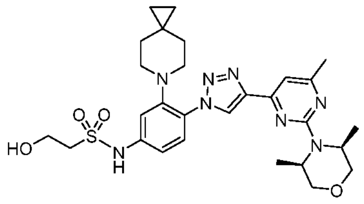 |
| 47 | 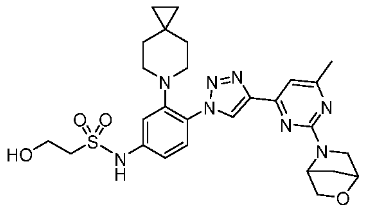 | 48 | 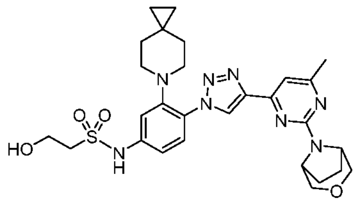 |
| 49 | 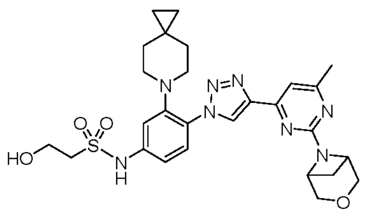 | 50 | 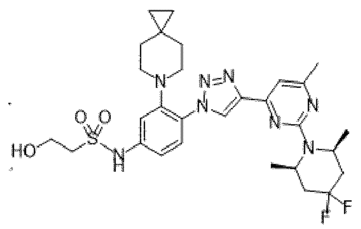 |
| 51 | 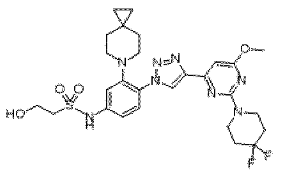 | 52 | 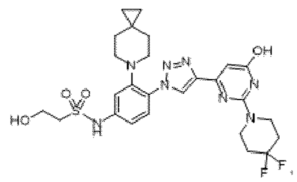 |
| 53 | 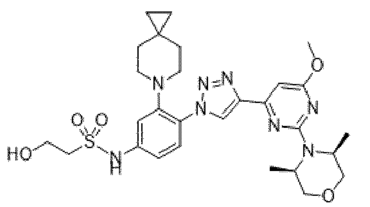 | 54 | 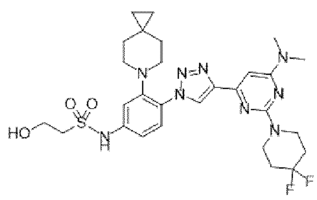 |
| 55 | 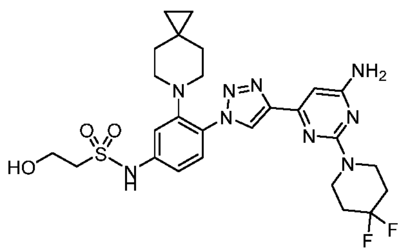 | 56 | 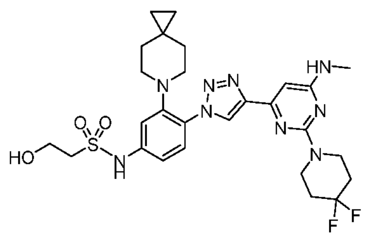 |
| 57 | 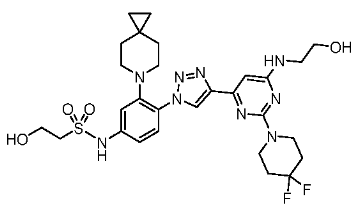 | 58 | 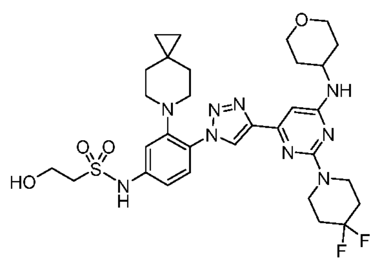 |

(continued)

| | | | |
|---|---|---|---|
| 59 | 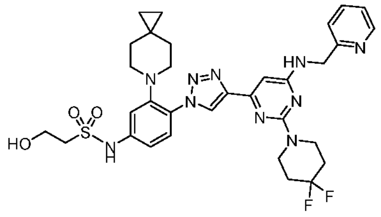 | 60 | 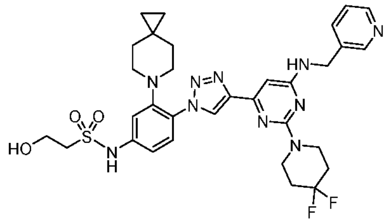 |
| 61 | 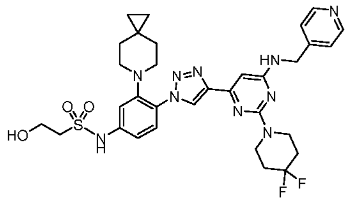 | 62 | 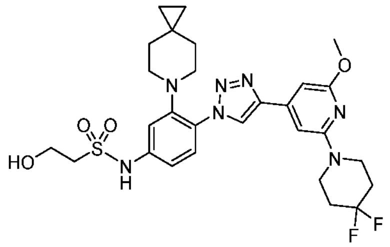 |
| 63 | 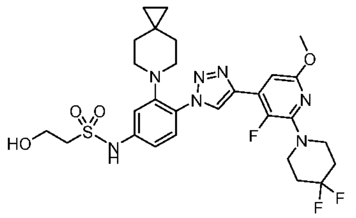 | 64 | 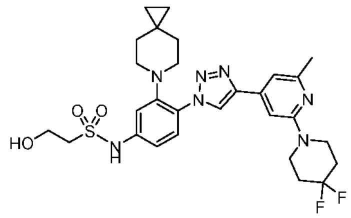 |
| 65 | 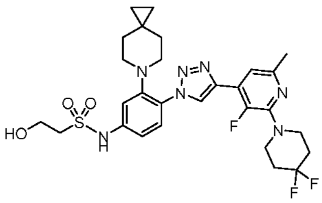 | 66 | 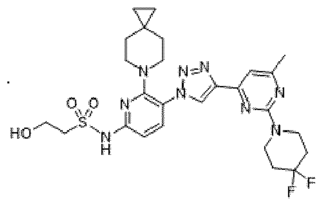 |
| 67 | 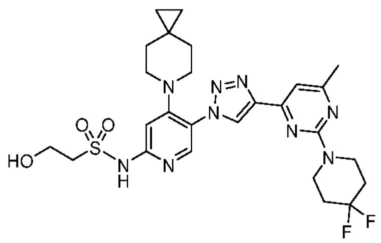 | 68 | 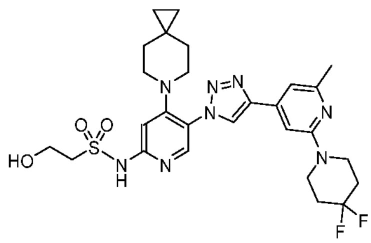 |
| 69 | 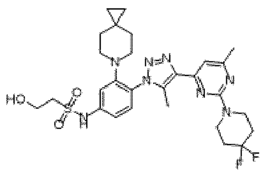 | 70 | 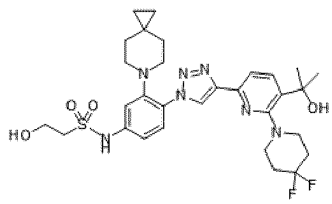 |
| 71 | 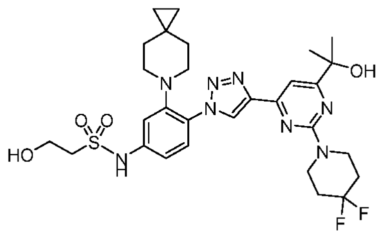 | 72 | 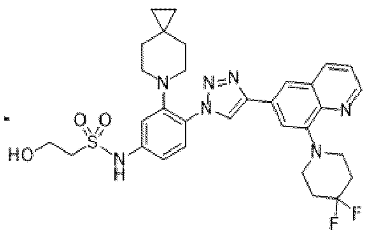 |

(continued)

| | | | |
|---|---|---|---|
| 73 | 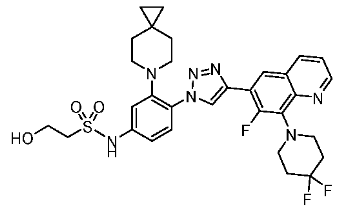 | 74 | 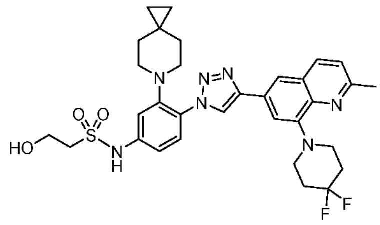 |
| 75 | 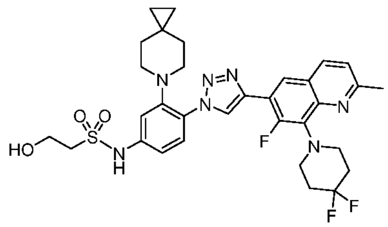 | 76 | 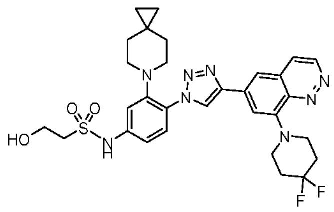 |
| 77 | 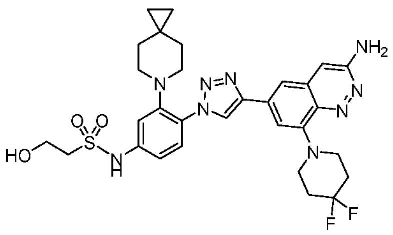 | 78 | 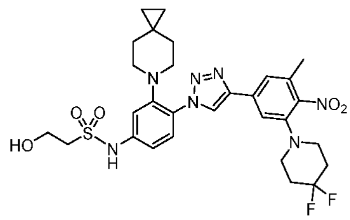 |
| 79 | 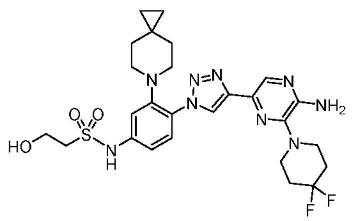 | 80 | 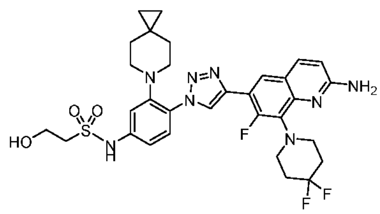 |
| 81 | 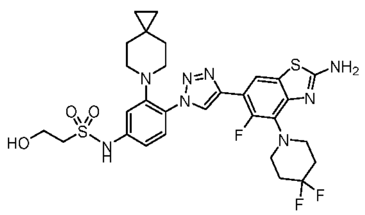 | 82 | 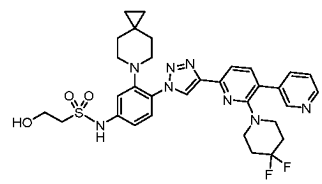 |
| 83 | 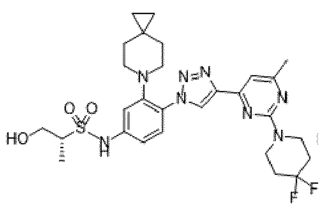 | 84 | 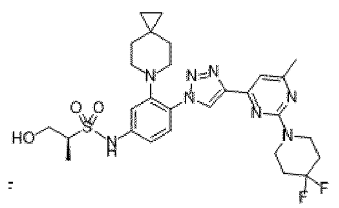 |
| 85 | 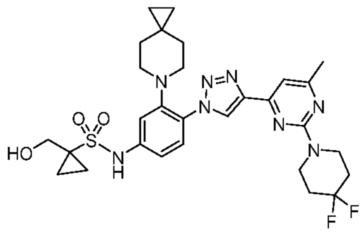 | 86 | 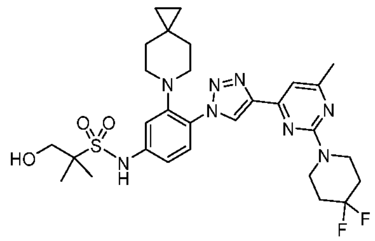 |

(continued)

| | | | |
|---|---|---|---|
| 87A | 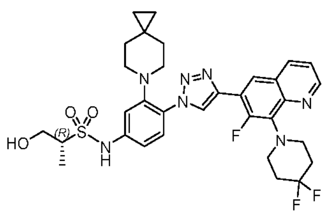 | 87B | 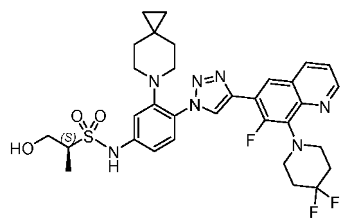 |
| 88 | 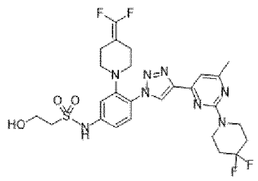 | 89 | 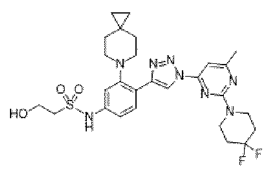 |
| 90 | 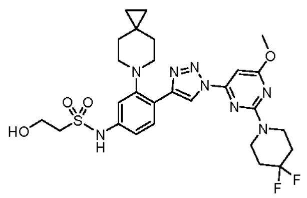 | 91 | 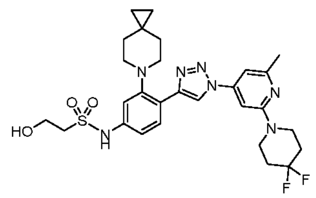 |
| 92 | 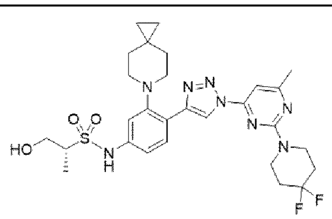 | 93 | 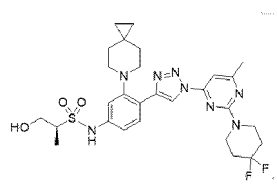 |
| 94 | 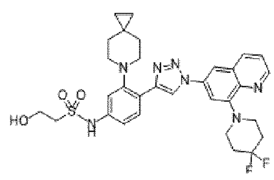 | 95 | 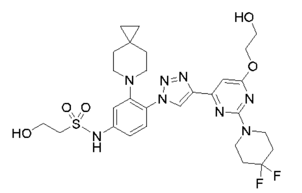 |
| 96 | 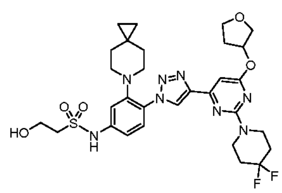 | 97 | 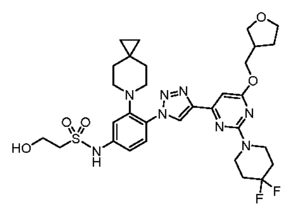 |
| 98 | 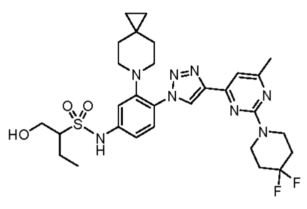 | 99 | 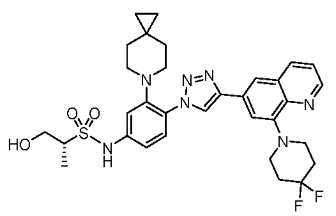 |
| 100 | 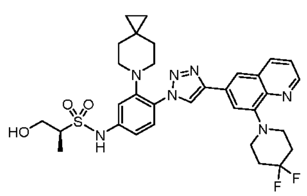 | 101 | 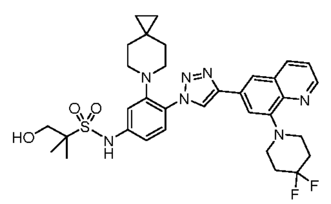 |

(continued)

| | | | |
|---|---|---|---|
| 102 | 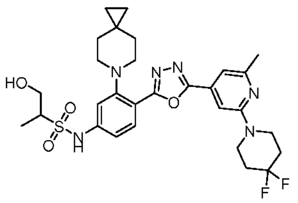 | 103 | 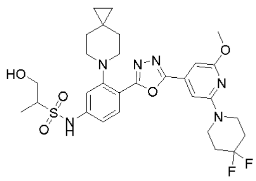 |
| 104 | 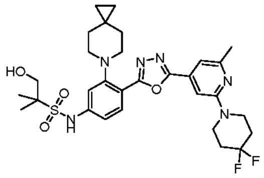 | 105 | 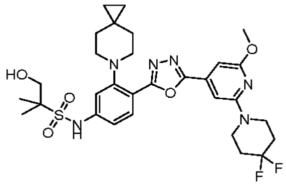 |
| 106 | 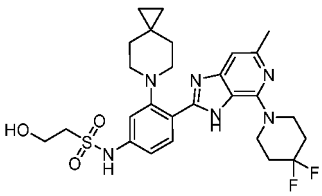 | | |

**INTERNATIONAL SEARCH REPORT**

International application No.
**PCT/CN2023/093538**

**A. CLASSIFICATION OF SUBJECT MATTER**

C07D401/14(2006.01)i； C07D405/14(2006.01)i； C07D413/14(2006.01)i； A61K31/497(2006.01)i； A61K31/506(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNKI; CNABS; CNTXT; VCN; VEN; WPABS; ENTXT; ENTXTC; ISI-WEB OF SCIENCE; STN: 湃隆生物科技, 肖贻崧, 谷晓辉, 赖焜民, KIF18A, 氮唑, 哌嗪, 哌啶, 噁 1w 唑, 吡唑, 吡啶, 嘧啶, 吡嗪, 磺酰胺, 驱动蛋白, 癌症, 肿瘤, triazol, tetrazol, pyrazol, pyridin, piperazin, piperidin, pyrimidin, pyrazin, sulfonamide, sulphonamide, cancer, tumour, tumor, 结构式(I)

**C. DOCUMENTS CONSIDERED TO BE RELEVANT**

| Category* | Citation of document, with indication, where appropriate, of the relevant passages | Relevant to claim No. |
|---|---|---|
| A | CN 113226473 A (AMGEN INC.) 06 August 2021 (2021-08-06)<br>claims 1-42 | 1-24 |
| A | CN 114401953 A (AMGEN INC.) 26 April 2022 (2022-04-26)<br>claims 1-29 | 1-24 |
| A | US 2022073504 A1 (AMGEN INC.) 10 March 2022 (2022-03-10)<br>entire document | 1-24 |
| A | US 2022056015 A1 (AMGEN INC.) 24 February 2022 (2022-02-24)<br>entire document | 1-24 |
| A | WO 2004058762 A1 (PHARMACIA CORP.) 15 July 2004 (2004-07-15)<br>description, embodiments 683, 684, 697, 713, 723, and 746, and claims 1-26 | 1-24 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 August 2023** | **19 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/093538**

| Patent document cited in search report | | | Publication date (day/month/year) | Patent family member(s) | | | Publication date (day/month/year) |
|---|---|---|---|---|---|---|---|
| CN | 113226473 | A | 06 August 2021 | CA | 3123871 | A1 | 25 June 2020 |
| | | | | US | 2022106293 | A1 | 07 April 2022 |
| | | | | US | 2020239441 | A1 | 30 July 2020 |
| | | | | US | 11236069 | B2 | 01 February 2022 |
| | | | | TW | 202034924 | A | 01 October 2020 |
| | | | | JP | 2022513972 | A | 09 February 2022 |
| | | | | WO | 2020132648 | A1 | 25 June 2020 |
| | | | | AU | 2019403486 | A1 | 24 June 2021 |
| | | | | BR | 112021011989 | A2 | 08 September 2021 |
| | | | | KR | 20210106474 | A | 30 August 2021 |
| | | | | EP | 3897852 | A1 | 27 October 2021 |
| | | | | SG | 11202106520 | VA | 29 July 2021 |
| | | | | AR | 117490 | A1 | 11 August 2021 |
| | | | | IN | 202117031747 | A | 10 December 2021 |
| | | | | VN | 82664 | A | 27 December 2021 |
| | | | | HK | 40062253 | A0 | 10 June 2022 |
| CN | 114401953 | A | 26 April 2022 | JP | 2022542319 | A | 30 September 2022 |
| | | | | AU | 2020324406 | A1 | 17 March 2022 |
| | | | | CA | 3147272 | A1 | 11 February 2021 |
| | | | | WO | 2021026099 | A1 | 11 February 2021 |
| | | | | EP | 4007753 | A1 | 08 June 2022 |
| US | 2022073504 | A1 | 10 March 2022 | CA | 3123042 | A1 | 25 June 2020 |
| | | | | WO | 2020132651 | A1 | 25 June 2020 |
| | | | | AU | 2019403488 | A1 | 24 June 2021 |
| | | | | EP | 3897855 | A1 | 27 October 2021 |
| | | | | JP | 2022514268 | A | 10 February 2022 |
| US | 2022056015 | A1 | 24 February 2022 | CA | 3123227 | A1 | 25 June 2020 |
| | | | | WO | 2020132649 | A1 | 25 June 2020 |
| | | | | AU | 2019404576 | A1 | 24 June 2021 |
| | | | | EP | 3898616 | A1 | 27 October 2021 |
| | | | | JP | 2022513967 | A | 09 February 2022 |
| WO | 2004058762 | A1 | 15 July 2004 | BR | 0317430 | A | 25 October 2005 |
| | | | | JP | 2006514043 | A | 27 April 2006 |
| | | | | EP | 1572693 | A1 | 14 September 2005 |
| | | | | US | 2004209897 | A1 | 21 October 2004 |
| | | | | MX | 2005006569 | A1 | 01 October 2005 |
| | | | | AU | 2003297431 | A1 | 22 July 2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210520744
- CN 202210878192
- CN 202211183092
- CN 202211537992
- CN 202310071265X
- CN 202310288871
- CN 202310475180


### Non-patent literature cited in the description


- **MI MAYR et al.** *Current Biology*, 2007, vol. 17, 488-98
- **ALLEN L. V. JR. et al.** Remington: The Science and Practice of Pharmacy. Pharmaceutical Press, 2012